(19) Europäisches Patentamt
European Patent Office
Office européen des brevets

(11) EP 4 434 984 A1

(12) **EUROPEAN PATENT APPLICATION**
published in accordance with Art. 153(4) EPC

(43) Date of publication:
**25.09.2024 Bulletin 2024/39**

(21) Application number: **22894778.4**

(22) Date of filing: **15.11.2022**

(51) International Patent Classification (IPC):
***C07D 471/04*** *(2006.01)* ***A61K 31/45*** *(2006.01)*
***A61P 35/00*** *(2006.01)* ***A61P 35/02*** *(2006.01)*

(52) Cooperative Patent Classification (CPC):
**A61K 31/45; A61P 35/00; A61P 35/02; C07D 471/04**

(86) International application number:
**PCT/CN2022/131968**

(87) International publication number:
**WO 2023/088245 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.11.2021 CN 202111375053**
**10.06.2022 CN 202210653756**

(71) Applicants:
- **Hitgen Inc.**
  **Chengdu, Sichuan 610200 (CN)**
- **Sichuan Kelun-Biotech Biopharmaceutical Co., Ltd.**
  **Chengdu, Sichuan 611138 (CN)**

(72) Inventors:
- **LIU, Chuan**
  **CHENGDU, Sichuan 610200 (CN)**
- **LIU, Jinming**
  **CHENGDU, Sichuan 611138 (CN)**
- **DOU, Dengfeng**
  **CHENGDU, Sichuan 610200 (CN)**
- **TAI, Zhengfu**
  **CHENGDU, Sichuan 611138 (CN)**
- **LI, Jin**
  **CHENGDU, Sichuan 610200 (CN)**
- **GE, Junyou**
  **CHENGDU, Sichuan 611138 (CN)**
- **ZHANG, Wei**
  **CHENGDU, Sichuan 610200 (CN)**
- **XIA, Shuai**
  **CHENGDU, Sichuan 610200 (CN)**
- **XIANG, Sichuan**
  **CHENGDU, Sichuan 610200 (CN)**
- **LUO, Linfu**
  **CHENGDU, Sichuan 610200 (CN)**
- **SHEN, Jianbo**
  **CHENGDU, Sichuan 610200 (CN)**
- **CAI, Longying**
  **CHENGDU, Sichuan 610200 (CN)**
- **CHEN, Qiuxia**
  **CHENGDU, Sichuan 610200 (CN)**
- **LIU, Qian**
  **CHENGDU, Sichuan 611138 (CN)**
- **HUANG, Hui**
  **CHENGDU, Sichuan 611138 (CN)**
- **YANG, Xiaojiao**
  **CHENGDU, Sichuan 611138 (CN)**
- **TIAN, Qiang**
  **CHENGDU, Sichuan 611138 (CN)**
- **SONG, Hongmei**
  **CHENGDU, Sichuan 611138 (CN)**

(74) Representative: **Meissner Bolte Partnerschaft mbB**
**Patentanwälte Rechtsanwälte**
**Postfach 86 06 24**
**81633 München (DE)**

(54) **DEGRADATION AGENT AND USE THEREOF**

(57) A compound having a BCL-XL protein degradation function as shown in Formula I and a use thereof in preparation of a drug for diseases associated with BCL-XL activity.

X-Y-Z Formula I

EP 4 434 984 A1

## Description

### Field of the Invention

The present invention relates to a compound with BCL-XI, protein degradation effect and use thereof in preparation of a medicament for a disease related to BCL-XI, activity.

### Background of the Invention

BCL-XI, is an anti-apoptotic protein belonging to the BCL-2 family. This protein family comprises anti-apoptotic proteins (e.g., Bcl-2, Bcl-xL and Mcl-1) and pro-apoptotic molecules (e.g., Bid, Bim, Bad, Bak and Bax). Although the anti-apoptotic BCL-XI, protein has a low expression level in normal cells, it is found to be highly overexpressed in many different types of human tumors and is considered to be potentially related to the occurrence, development and drug resistance of tumors. Targeting BCL-XI, has been pursued as a cancer treatment strategy.

Ubiquitin is a small molecule protein consisting of 76 amino acids with a highly conserved sequence that exists in eukaryotic cells. The main function of ubiquitin is to label target proteins. The labeled target proteins can be recognized and degraded by proteasome. This process is called the ubiquitin/proteasome system. Among them, E3 ubiquitin ligase (E3 ubiquitin-protein ligase) directly binds to proteins, determining the specificity of degradation. The degradation process of the ubiquitin/proteasome system mainly comprises the following four steps: 1) Ubiquitin activation: the carboxyl residue of ubiquitin combines with the sulfhydryl group of ubiquitin activating enzyme E1; 2) Ubiquitin cross-linking: E1 hands over the activated ubiquitin to a ubiquitin-conjugating enzyme E2 through a transesterification process; 3) Binding ubiquitin complex to target protein by E3: ubiquitin-protein ligase E3 links the ubiquitin bound to E2 to the target protein; if ubiquitin already exists on the protein, the ubiquitin bound to E2 can be directly linked to the target protein; 4) Proteasome degradation: proteasome recognizes the labeled target protein and hydrolyzes the target protein into peptide chains with a length of 7 to 8 amino acids, thereby completing degradation of the target protein.

Proteolysis targeting chimeric molecule is a bifunctional molecule that can bind to E3 ubiquitin ligase and target protein at the same time, ubiquitinating the target protein that cannot originally bind to E3, and performing selective degradation through ubiquitin/proteasome system, thereby controlling intracellular target protein level. Currently, E3 ubiquitin ligases mainly include CRBN, VHL, MDM2 and cIAP1.

### Summary of the Invention

The present invention provides a compound with BCL-XI, protein degradation effect and use thereof in preparation of a drug for a disease related to BCL-XI, activity.

The present invention provides a compound represented by Formula I, or a deuterated compound thereof, or a stereoisomer thereof, or a tautomer thereof, or a polymorph thereof, or a solvate thereof, or a N-oxide thereof, or an isotopically labeled compound thereof, or a metabolite thereof, or a prodrug thereof, or a pharmaceutically acceptable salt thereof:

X-Y-Z    Formula I

wherein,

X represents a group binding to a BCL-XI, protein;

Y represents a connecting group;

Z represents a group binding to an E3 ubiquitin ligase.

According to some embodiments of the present invention, X is selected from

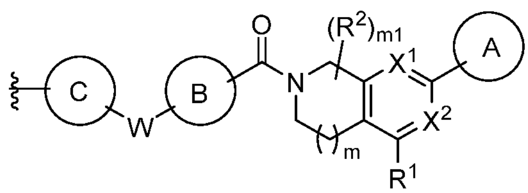

wherein,

Ring A is selected from $C_{6\sim10}$ aromatic ring or 6- to 10-membered aromatic heterocycle; in which the aromatic ring and aromatic heterocycle can be further substituted by one, two or three $R^{A1}$;

each $R^{A1}$ is independently selected from hydrogen, halogen, cyano, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{A2}$ or $-C_{0\sim4}$ alkylene-$NR^{A2}R^{A3}$;

$R^{A2}$ and $R^{A3}$ are each independently selected from hydrogen, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl or halogen-substituted $-C_{2\sim6}$ alkynyl;

$X^1$ and $X^2$ are each independently selected from Nor $CR^{X1}$;

each $R^{X1}$ is independently selected from hydrogen, halogen, cyano, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{X2}$ or $-C_{0\sim4}$ alkylene-$NR^{X2}R^{X3}$;

$R^{X2}$ and $R^{X3}$ are each independently selected from hydrogen, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl or halogen-substituted $-C_{2\sim6}$ alkynyl;

$R^1$ is selected from hydrogen, halogen, cyano, $-C_{1\sim6}$ alkyl, $-C_{2\sim6}$ alkenyl, $-C_{2\sim6}$ alkynyl, - halogen-substituted $C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{11}$ or $-C_{0\sim4}$ alkylene-$NR^{11}R^{12}$;

$R^{11}$ and $R^{12}$ are each independently selected from hydrogen, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl or halogen-substituted $-C_{2\sim6}$ alkynyl;

each $R^2$ is independently selected from hydrogen, halogen, cyano, =O, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{21}$ or $-C_{0\sim4}$ alkylene-$NR^{21}R^{22}$;

$R^{21}$ and $R^{22}$ are each independently selected from hydrogen, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl or halogen-substituted $-C_{2\sim6}$ alkynyl;

m1 is selected from 0, 1, 2 or 3;

m is selected from 0, 1 or 2;

Ring B is selected from $C_{3\sim10}$ cycloalkane, 3- to 10-membered heterocycloalkane, benzene ring, 5- to 6-membered aromatic heterocycle or 5- to 12-membered bridged ring; wherein, cycloalkane, heterocycloalkane, benzene ring, aromatic heterocycle and bridged ring can be further substituted by one, two or three $R^{B1}$; alternatively, Ring B is selected from $C_{3\sim10}$ cycloalkane, 3- to 10-membered heterocycloalkane, benzene ring or 5- to 6-membered aromatic heterocycle; wherein, the cycloalkane, heterocycloalkane, benzene ring, and aromatic heterocycle can be further substituted by one, two, or three $R^{B1}$;

each $R^{B1}$ is independently selected from hydrogen, halogen, cyano, =O, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{B2}$ or $-C_{0\sim4}$ alkylene-$NR^{B2}R^{B3}$;

$R^{B2}$ and $R^{B3}$ are each independently selected from hydrogen, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl or halogen-substituted $-C_{2\sim6}$ alkynyl;

W is selected from $-C_{1\sim6}$ alkylene-, $-C_{1\sim6}$ alkylene-O-, $-O-C_{1\sim6}$ alkylene-, $-C_{2\sim6}$ alkenylene-, - $C_{2\sim6}$ alkenylene-O-, $-O-C_{2\sim6}$ alkenylene-, $-C_{2\sim6}$ alkynylene-, $-C_{2\sim6}$ alkynylene-O- or $-O-C_{2\sim6}$ alkynylene-;

Ring C is selected from benzene ring or 5- to 6-membered aromatic heterocycle; wherein, the benzene ring and aromatic heterocycle can be further substituted by one, two or three $R^{C1}$;

each $R^{C1}$ is independently selected from hydrogen, halogen, cyano, -$C_{1\sim6}$ alkyl, -$C_{2\sim6}$ alkenyl, -$C_{2\sim6}$ alkynyl, halogen-substituted $C_{1\sim6}$ alkyl, halogen-substituted -$C_{2\sim6}$ alkenyl, halogen-substituted -$C_{2\sim6}$ alkynyl, -$C_{0\sim4}$ alkylene-O$R^{C2}$ or -$C_{0\sim4}$ alkylene-N$R^{C2}R^{C3}$;

$R^{C2}$ and $R^{C3}$ are each independently selected from hydrogen, -$C_{1\sim6}$ alkyl, -$C_{2\sim6}$ alkenyl, -$C_{2\sim6}$ alkynyl, halogen-substituted -$C_{1\sim6}$ alkyl, halogen-substituted -$C_{2\sim6}$ alkenyl or halogen-substituted -$C_{2\sim6}$ alkynyl.

According to some embodiments of the present invention, Ring A is selected from naphthalene ring or 8- to 10-membered aromatic heterocycle; wherein, the naphthalene ring and aromatic heterocycle can be further substituted by one, two or three $R^{A1}$, and each $R^{A1}$ is independently selected from hydrogen, halogen, cyano or -$C_{1\sim6}$ alkyl.

In some embodiments, Ring A is selected from naphthalene ring, benzopyrimidine ring, or quinoline ring.

In some embodiments, Ring A is selected from naphthalene ring or quinoline ring.

In some embodiments, Ring A is selected from quinoline ring.

According to some embodiments of the present invention, $X^1$ and $X^2$ are each independently selected from N or CH.

In some embodiments, $X^1$ and $X^2$ are each independently selected from N.

According to some embodiments of the present invention, $R^1$ is selected from hydrogen, halogen, cyano, -$C_{1\sim6}$ alkyl or -N$R^{11}R^{12}$, and $R^{11}$ and $R^{12}$ are each independently selected from hydrogen or -$C_{1\sim6}$ alkyl.

In some embodiments, $R^1$ is selected from hydrogen or -N$R^{11}R^{12}$, and $R^{11}$ and $R^{12}$ are each independently selected from hydrogen or -$C_{1\sim6}$ alkyl.

In some embodiments, $R^1$ is selected from hydrogen,

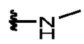 or 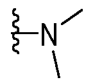.

According to some embodiments of the present invention, m is selected from 1.

According to some embodiments of the present invention, Ring B is selected from 6-membered nitrogen-containing heterocycloalkane, benzene ring or 6-membered nitrogen-containing aromatic heterocycle, 5- to 6-membered cycloalkane or 5- to 8-membered bridged ring; wherein, the benzene ring, heterocycloalkane, aromatic heterocycle, cycloalkane and bridged ring can be further substituted by one, two or three $R^{B1}$; each $R^{B1}$ is independently selected from hydrogen, halogen, cyano, =O or -$C_{1\sim6}$ alkyl.

According to some embodiments of the present invention, Ring B is selected from 6-membered nitrogen-containing heterocycloalkane, benzene ring or 6-membered nitrogen-containing aromatic heterocycle, or 5- to 6-membered cycloalkane; wherein, the benzene ring, heterocycloalkane, aromatic heterocycle, and cycloalkane can be further substituted by one, two or three $R^{B1}$; each $R^{B1}$ is independently selected from hydrogen, halogen, cyano, =O or -$C_{1\sim6}$ alkyl.

In some embodiments, Ring B is selected from benzene ring, pyridine ring or

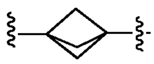.

In some embodiments, Ring B is selected from benzene ring or pyridine ring.

In some embodiments, Ring B is selected from benzene ring.

According to some embodiments of the present invention, W is selected from -$C_{1\sim4}$ alkylene-, -$C_{1\sim4}$ alkylene-O-, -O-$C_{1\sim4}$ alkylene-, -$C_{2\sim4}$ alkenylene-, -$C_{2\sim4}$ alkenylene-O-, -O-$C_{2\sim4}$ alkenylene-, -$C_{2\sim4}$ alkynylene-, -$C_{2\sim4}$ alkynylene-O- or -O-$C_{2\sim4}$ alkynylene.

In some embodiments, W is selected from -$C_{1\sim4}$ alkylene-, -$C_{1\sim4}$ alkylene-O-, -O-$C_{1\sim4}$ alkylene-, -$C_{2\sim4}$ alkenylene-, - $C_{2\sim4}$ alkenylene-O- or -O-$C_{2\sim4}$ alkenylene-.

In some embodiments, W is selected from -$C_{1\sim3}$ alkylene, -$C_{1\sim3}$ alkylene-O-, -O-$C_{1\sim3}$ alkylene or -$C_{2\sim3}$ alkenylene.

In some embodiments, W is selected from ethylene,

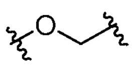, 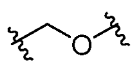, 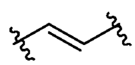

or

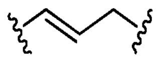.

In some embodiments, W is selected from ethylene,

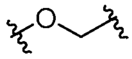, 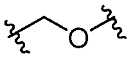 or 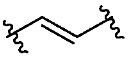.

According to some embodiments of the present invention, Ring C is selected from benzene ring or 6-membered nitrogen-containing aromatic heterocycle; wherein, the benzene ring and aromatic heterocycle can be further substituted by one, two or three $R^{C1}$, each $R^{C1}$ is independently selected from hydrogen, halogen, cyano or $-C_{1\sim6}$ alkyl. In some embodiments, Ring C is selected from benzene ring or pyridine ring.

According to some embodiments of the present invention, each $R^2$ is independently selected from halogen, cyano, =O, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{21}$ or $-C_{0\sim4}$ alkylene-$NR^{21}R^{22}$; preferably, each $R^2$ is independently selected from halogen, cyano, $-C_{1\sim6}$ alkyl, halogen-substituted $-C_{1\sim6}$ alkyl, halogen-substituted $-C_{2\sim6}$ alkenyl, $-C_{0\sim4}$ alkylene-$OR^{21}$ or $-C_{0\sim4}$ alkylene-$NR^{21}R^{22}$; further preferably, each $R^2$ is independently selected from $-C_{0\sim4}$ alkylene-$NR^{21}R^{22}$;

preferably, m1 is selected from 0 or 1; further preferably, m1 is selected from 0. When m1 is 0, it means that $R^2$ does not exist, that is, the carbon vacancies on 6-membered nitrogen-containing heterocycle of

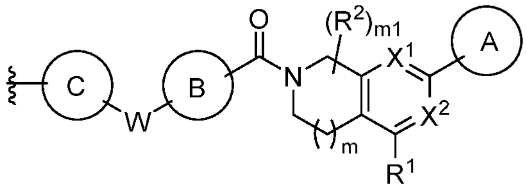

are all filled with H according to the principle of chemical bonding;

preferably, $R^{21}$ and $R^{22}$ are each independently selected from hydrogen, $-C_{1\sim6}$ alkyl, and halogen-substituted $-C_{1\sim6}$ alkyl; further preferably, $R^{21}$ and $R^{22}$ are each independently selected from hydrogen.

According to some embodiments of the present invention, Ring A is selected from naphthalene ring or 8- to 10-membered aromatic heterocycle; wherein, the naphthalene ring and aromatic heterocycle can be further substituted by one, two or three $R^{A1}$;

$X^1$ and $X^2$ are each independently selected from N or CH;

$R^1$ is selected from hydrogen or $-NR^{11}R^{12}$;

m is selected from 0 or 1;

Ring B is selected from 6-membered nitrogen-containing heterocycloalkane, benzene ring, 6-membered nitrogen-containing aromatic heterocycle, 5-membered bridged cycloalkane, or 5-membered cycloalkane; wherein, the benzene ring, aromatic heterocycle, cycloalkane, and bridged cycloalkane can be further substituted by one, two or three $R^{B1}$; or Ring B is selected from 6-membered nitrogen-containing heterocycloalkane, benzene ring, 6-membered nitrogen-containing aromatic heterocycle or 5-membered cycloalkane; wherein, the benzene ring, aromatic heterocycle, cycloalkane can be further substituted by one, two or three $R^{B1}$;

W is selected from $-C_{1\sim4}$ alkylene-, $-C_{1\sim4}$ alkylene-O-, $-O-C_{1\sim4}$ alkylene-, $-C_{2\sim4}$ alkenylene-, - $C_{2\sim4}$ alkenylene-O-, $-O-C_{2\sim4}$ alkenylene-, $-C_{2\sim4}$ alkynylene-, $-C_{2\sim4}$ alkynylene-O- or $-O-C_{2\sim4}$ alkynylene-;

Ring C is selected from benzene ring or 6-membered nitrogen-containing aromatic heterocycle; wherein, benzene ring and aromatic heterocycle can be further substituted by one, two or three $R^{C1}$.

According to some embodiments of the present invention,

Ring A is selected from

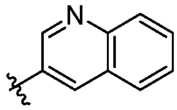,

Ring A can also be selected from

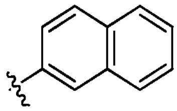,

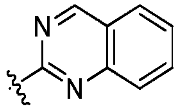;

$X^1$ and $X^2$ are selected from N or CH; or $X^1$ and $X^2$ are selected from N;

$R^1$ is selected from hydrogen,

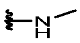 or 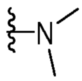;

m is selected from 1;

m1 is selected from 0;

Ring B is selected from

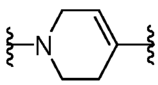, 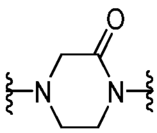, 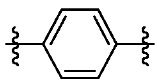, 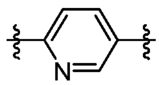

or

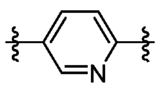,

Ring B can also be selected from

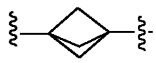;

preferably, Ring B is selected from

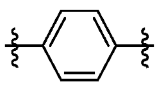, 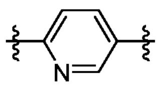 or 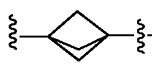;

preferably, Ring B is selected from

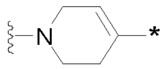,

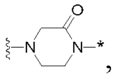, 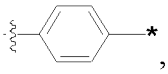, 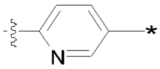, 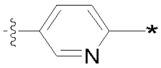 or 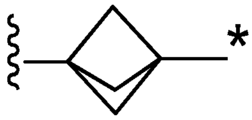,

wherein the * end is linked to W, and the

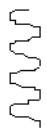

end is linked to the carbonyl on the main chain X;

W is selected from ethylene,

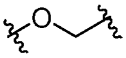, 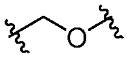 or 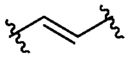,

W can also be selected from

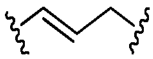;

preferably, W is selected from ethylene,

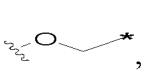, 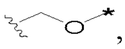, 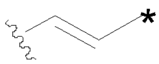

or

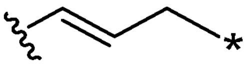,

wherein, the * end is linked to the ring B, and the

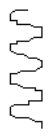

end is linked to the ring C;

Ring C is selected from

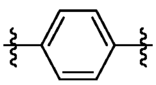, 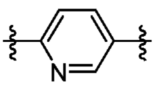 or 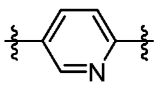,

preferably, Ring C is selected from

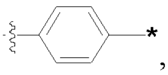,

wherein the * end is linked to W and the

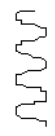

end is linked to Y.

According to some embodiments of the present invention, X is selected from

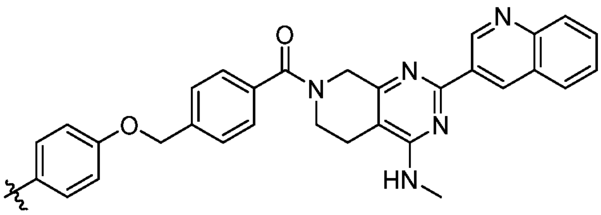, 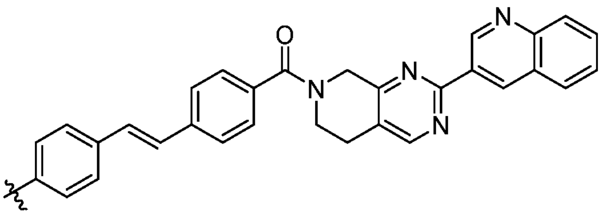,

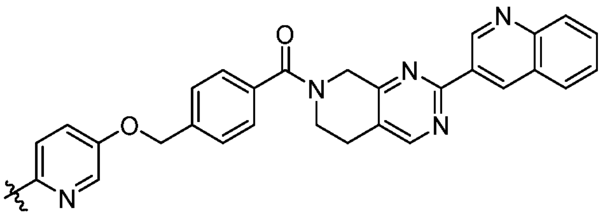, 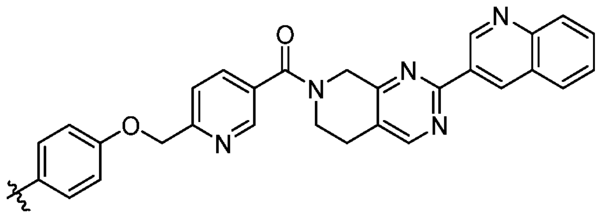,

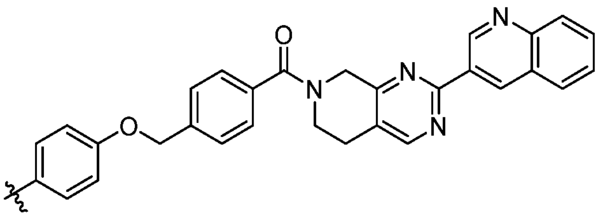, 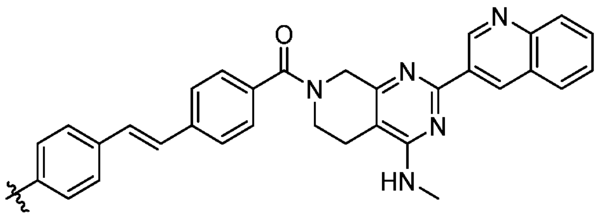,

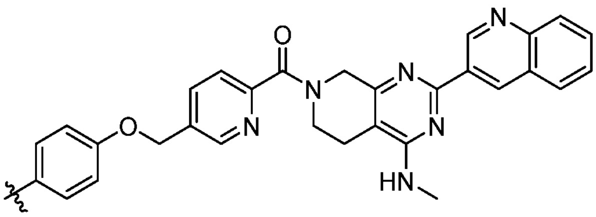, 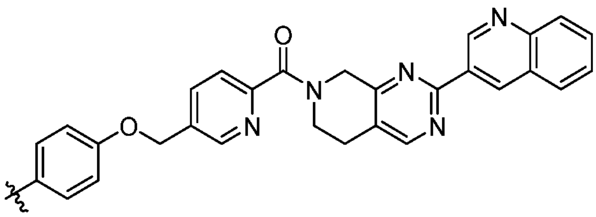,

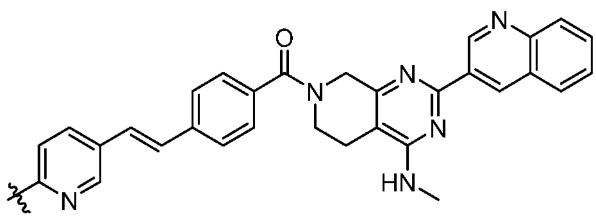, 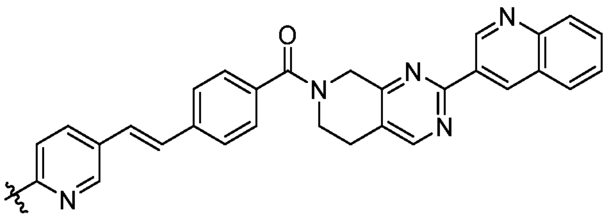.

X can also be selected from

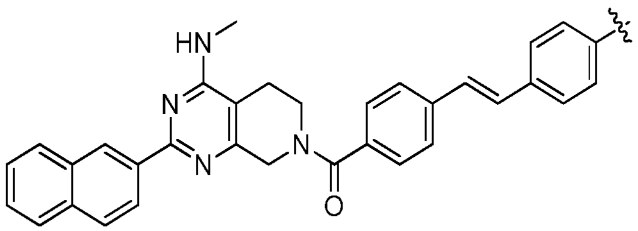

,

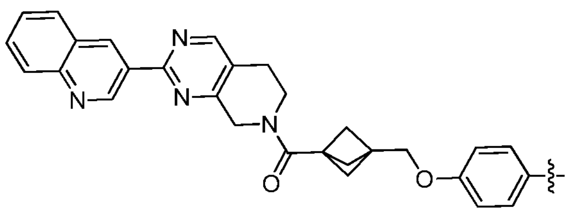

,

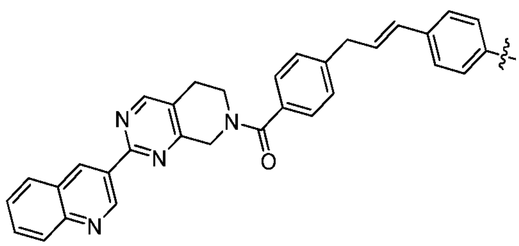

,

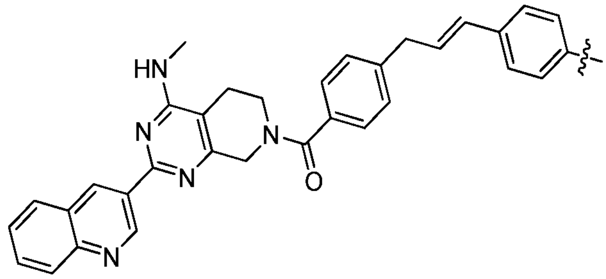

,

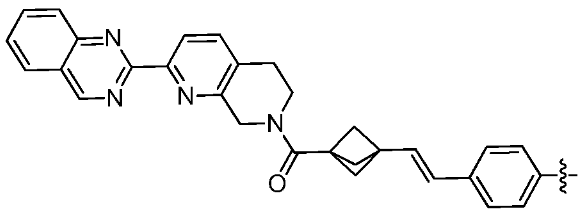

,

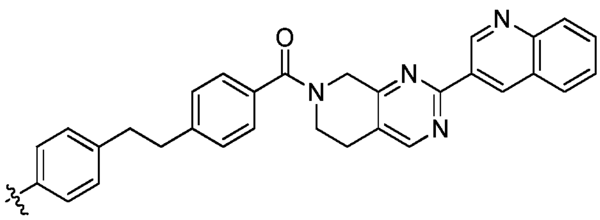

.

According to some embodiments of the present invention, X is selected from

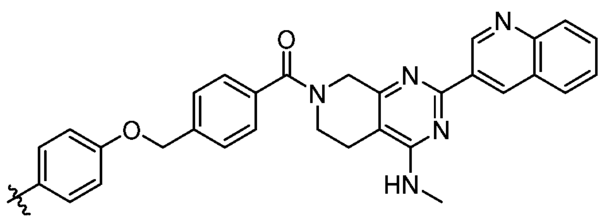

,

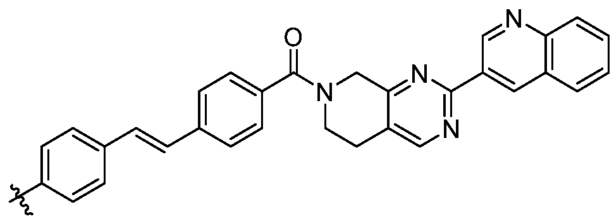

,

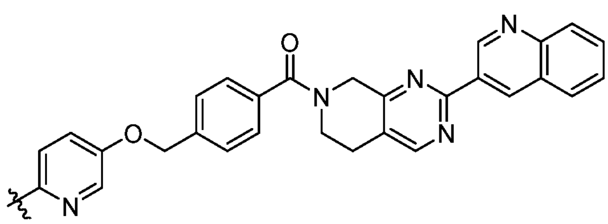

,

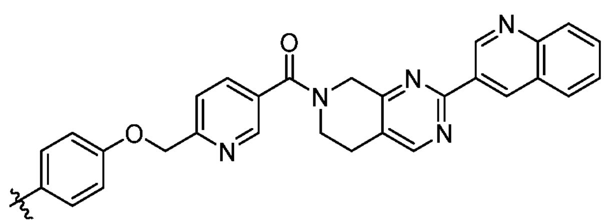

,

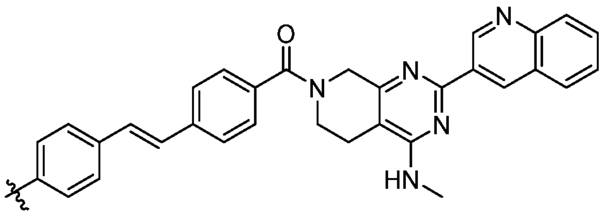,
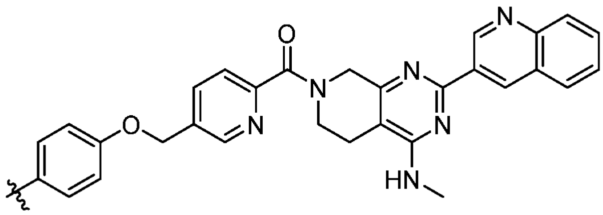,
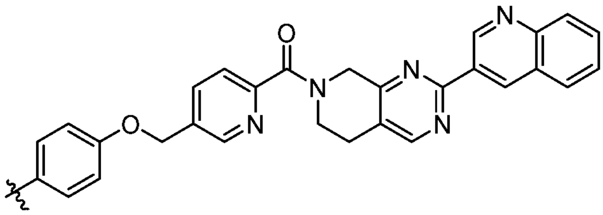,
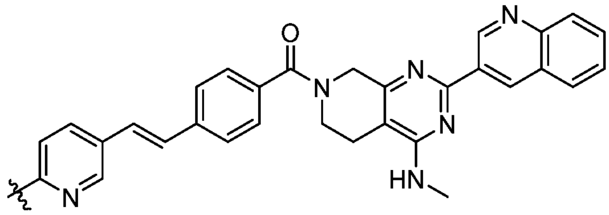,
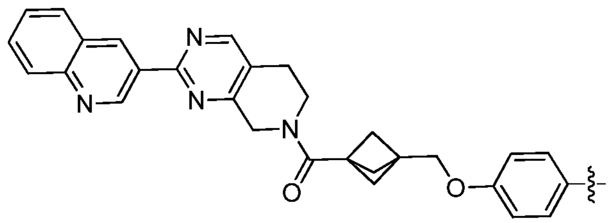,
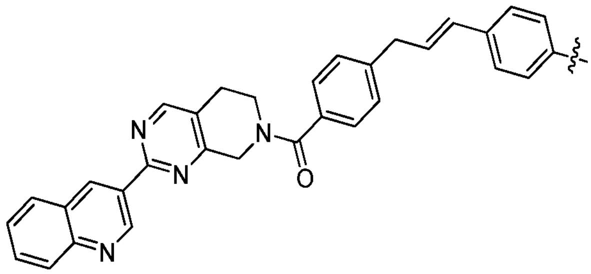.

According to some embodiments of the present invention, X can also optionally be independently substituted by one or more (e.g., 2, 3, 4, 5, 6, 7, 8, 9 or 10) $R^{A1'}$.

According to some embodiments of the present invention, each $R^{A1'}$ is independently selected from halogen, cyano, -$C_{1\sim6}$ alkyl, -$C_{2\sim6}$ alkenyl, -$C_{2\sim6}$ alkynyl, halogen-substituted -$C_{1\sim6}$ alkyl, halogen-substituted -$C_{2\sim6}$ alkenyl, halogen-substituted -$C_{2\sim6}$ alkynyl, -$C_{0\sim4}$ alkylene-$OR^{A2'}$ or -$C_{0\sim4}$ alkylene-$NR^{A2'}R^{A3'}$;
$R^{A2'}$ and $R^{A3'}$ are each independently selected from hydrogen, -$C_{1\sim3}$ alkyl, -$C_{2\sim6}$ alkenyl, -$C_{2\sim6}$ alkynyl, halogen-substituted -$C_{1\sim3}$ alkyl, halogen-substituted -$C_{2\sim6}$ alkenyl, or halogen-substituted -$C_{2\sim6}$ alkynyl.

According to some embodiments of the present invention, each $R^{A1'}$ is independently selected from halogen, cyano, -$C_{1\sim3}$ alkyl, -$C_{2\sim4}$ alkenyl, halogen-substituted -$C_{1\sim3}$ alkyl, halogen-substituted -$C_{2\sim4}$ alkenyl, -$C_{0\sim4}$ alkylene-$OR^{A2'}$ or -$C_{0\sim4}$ alkylene-$NR^{A2'}R^{A3'}$;
$R^{A2'}$ and $R^{A3'}$ are each independently selected from hydrogen, -$C_{1\sim3}$ alkyl, -$C_{2\sim4}$ alkenyl, halogen-substituted -$C_{1\sim3}$ alkyl, or halogen-substituted -$C_{2\sim4}$ alkenyl.

According to some embodiments of the present invention,

the Y is selected from -$(L^Y)_q$-;
q is an integer from 1 to 30;
each $L^Y$ is independently selected from structural fragments consisting of any one or more members selected from the group consisting of $C(R)_2$, C(O), O, S, S(O), $S(O)_2$, NR, -CR=CR-, - C=C-, $C_{3\sim10}$ cycloalkane, 3- to 10-membered heterocycloalkane, $C_{6\sim10}$ aromatic ring, 5- to 10-membered aromatic heterocycle, 5- to 12-membered spiro ring, 5- to 12-membered spiro heterocycle, 5- to 12-membered bridged ring, and 5- to 12-membered bridged heterocycle; wherein the cycloalkane, heterocycloalkane, aromatic ring, aromatic heterocycle, spiro ring, spiro heterocycle, bridged ring, and bridged heterocycle can be further substituted by one, two, or three kYL ;
each $R^{YL}$ is independently selected from the group consisting of hydrogen, halogen, cyano, nitro, $C_{1\sim6}$ alkyl, halogen-substituted $C_{1\sim6}$ alkyl, -OR, -$N(R)_2$;
each R is independently selected from the group consisting of hydrogen, halogen, -$C_{1\sim6}$ alkyl, halogen-substituted -$C_{1\sim6}$ alkyl, -$C_{0\sim2}$ alkylene-($C_{3\sim10}$ carbocyclic group), and -$C_{0\sim2}$ alkylene-(3-to 10-membered heterocycloalkyl).

According to some embodiments of the present invention, the q is 1 to 25; preferably, q is 1 to 20 (for example, 1 to 10); preferably, q is 2, 3, 4, 5, 6, 7, 8, 9, 10.

According to some embodiments of the present invention, Y is selected from the group consisting of:

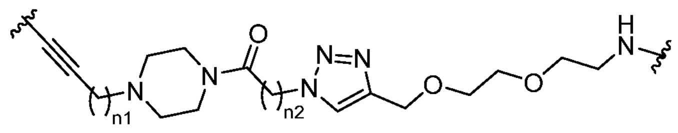 , 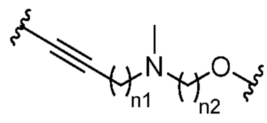 ,

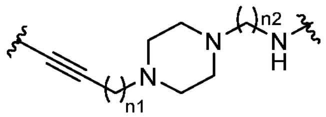 , 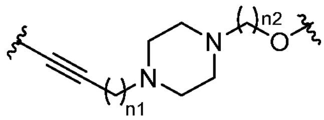 , 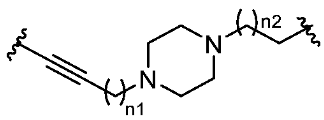 ,

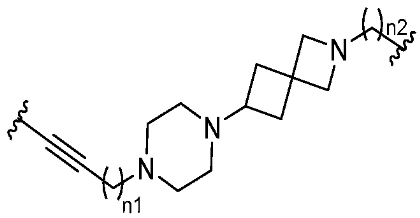 , 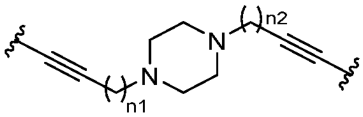 , 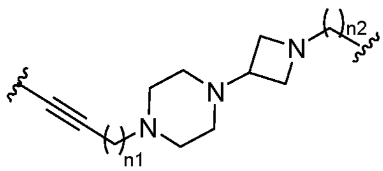 ,

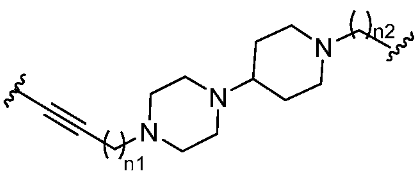 , 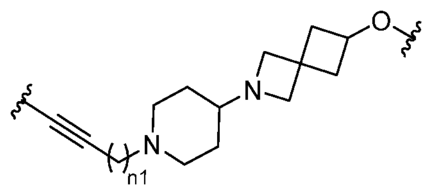

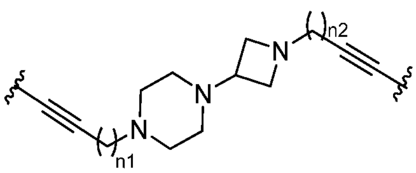 , 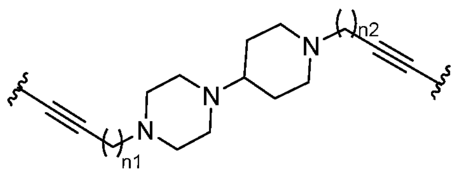 ,

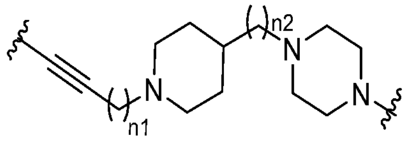 , 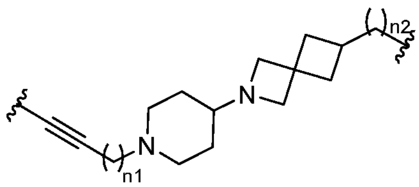 ,

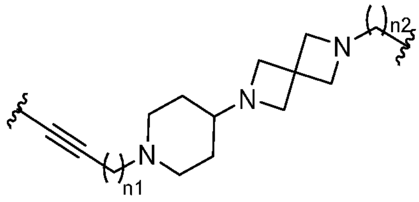 , 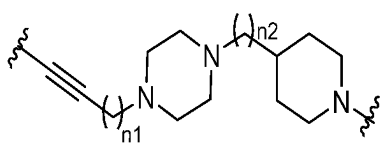 ,

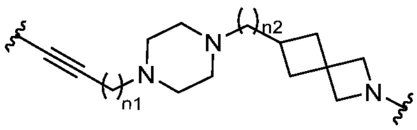, 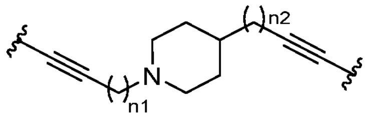, 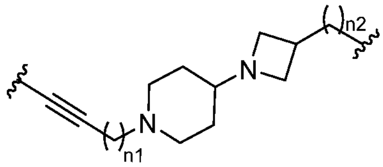,

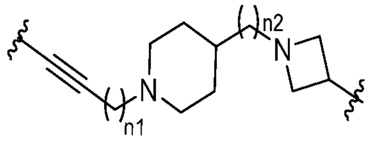, 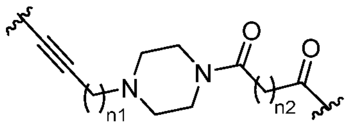, and 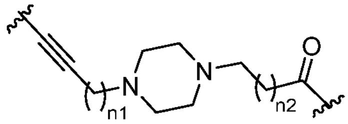.

The Y can also be selected from

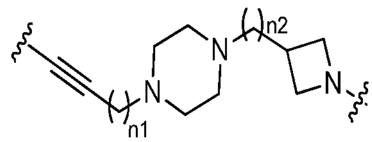,

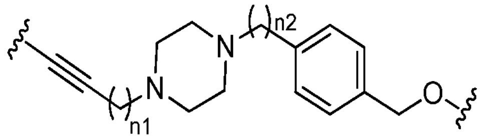,

wherein n1 in each structural fragment is independently an integer selected from 0 to 10, and n2 in each structural fragment is independently an integer selected from 0 to 10.

According to some embodiments of the present invention, n1 in each structural fragment is independently selected from 0, 1, 2, 3, 4, 5, 6, 7, 8, and n2 in each structural fragment is independently selected from 0, 1, 2, 3, 4, 5, 6, 7, 8.

In some embodiments, Y is selected from

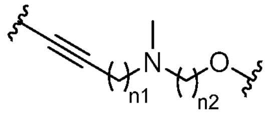,

wherein n1 is selected from 1, and n2 is selected from 3, 4 or 5; or

Y is selected from

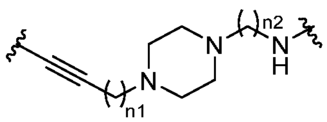,

wherein n1 is selected from 1 and n2 is selected from 3, 4, 5, 6, 7 or 8; or

Y is selected from

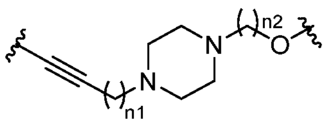
,

wherein n1 is selected from 1 and n2 is selected from 3, 4, 5, 6, 7 or 8; or

Y is selected from

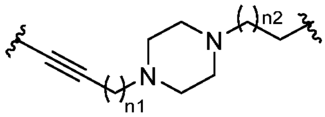
,

wherein n1 is selected from 1 and n2 is selected from 3, 4, 5 or 6; or

Y is selected from

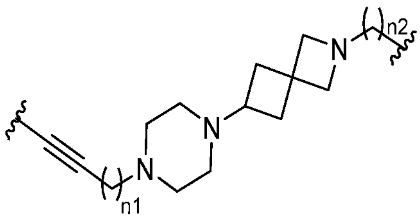
,

wherein n1 is selected from 1 and n2 is selected from 0 or 1; or

Y is selected from

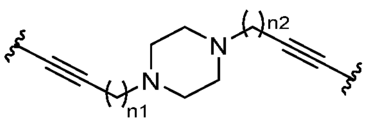
,

wherein n1 is selected from 1, 2, 3 or 4, and n2 is selected from 1; or

Y is selected from

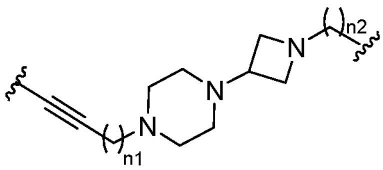
,

wherein n1 is selected from 1 and n2 is selected from 0, 1, 2 or 3; or

Y is selected from

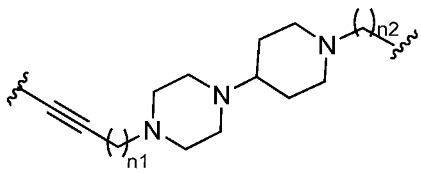,

wherein n1 is selected from 1 and n2 is selected from 0, 1, 2 or 3; or

Y is selected from

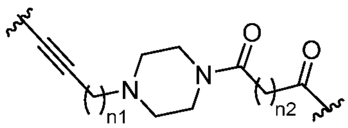,

wherein n1 is selected from 1 and n2 is selected from 1, 2, 3, 4, 5, 6, 7 or 8; or

Y is selected from

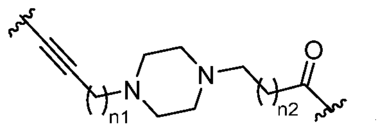,

wherein n1 is selected from 1 and n2 is selected from 2, 3 or 4.

According to some embodiments of the present invention, Y is selected from

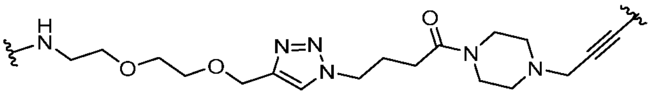, 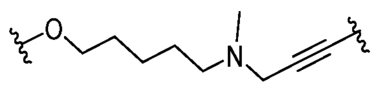,

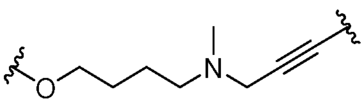, 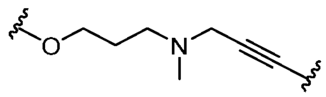, 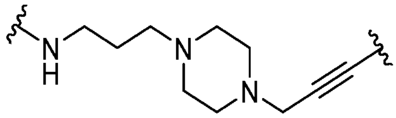,

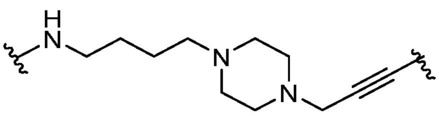, 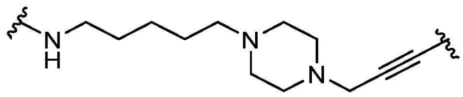,

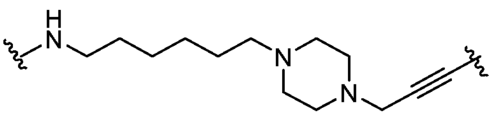, 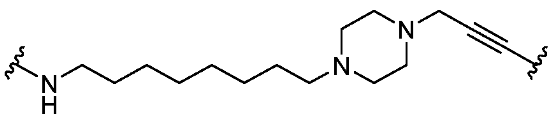,

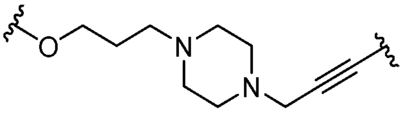, 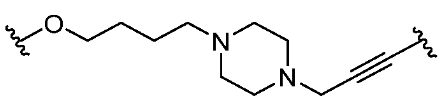,

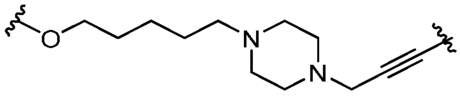 , 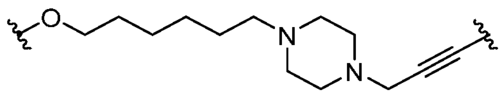 ,

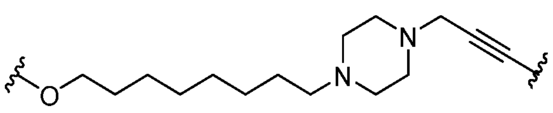 , 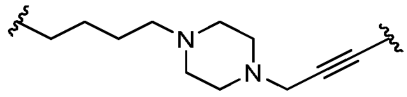 ,

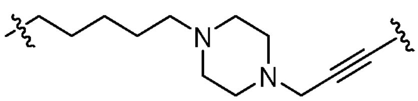 , 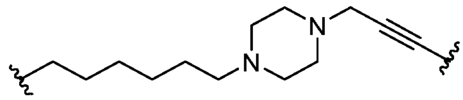 ,

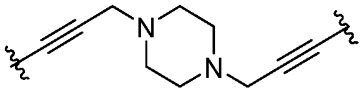 , 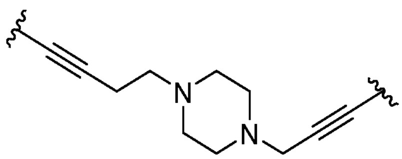 , 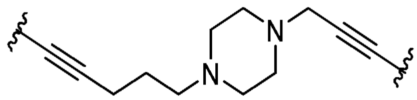 ,

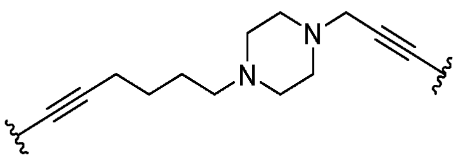 , 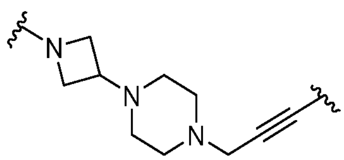 , 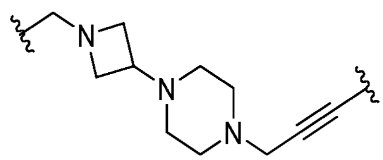 ,

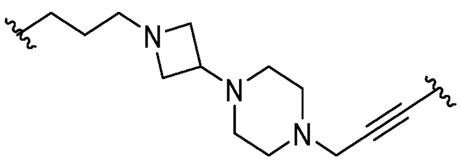 , 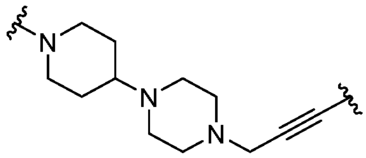 ,

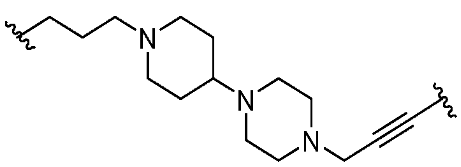 , 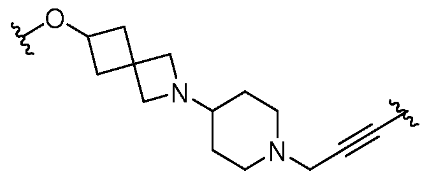 ,

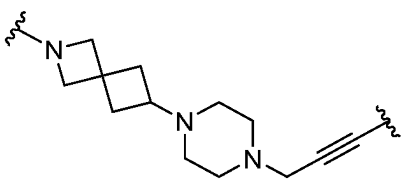 , 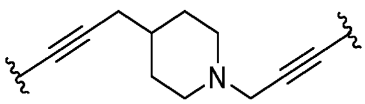 , 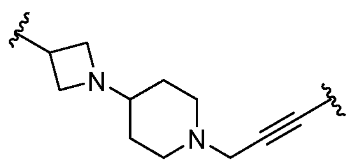 ,

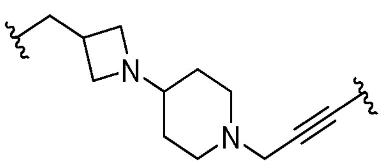 , 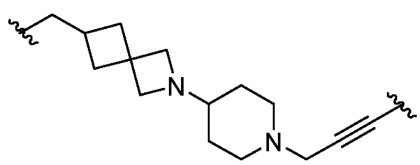 ,

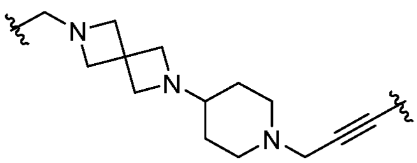,
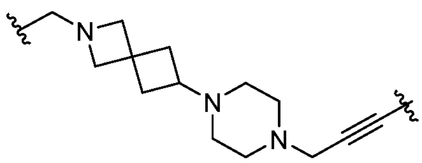,
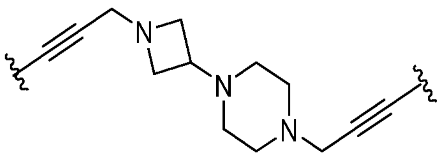,
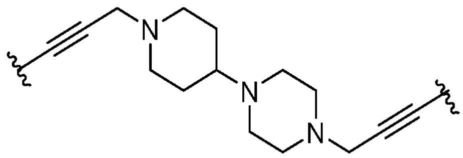,
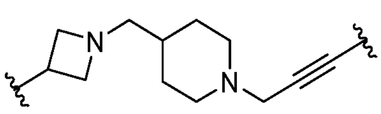,
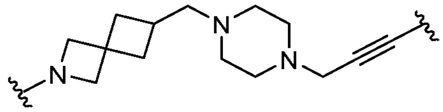,
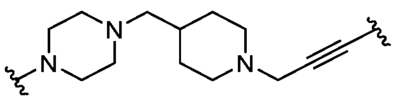,
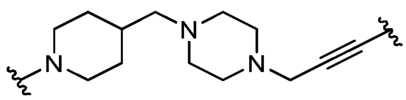,
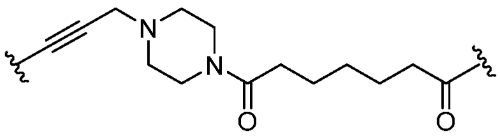,
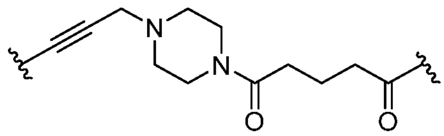,
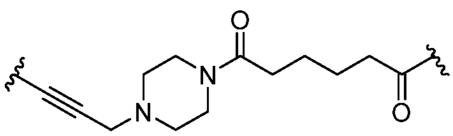,
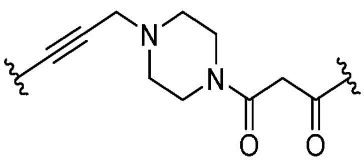,
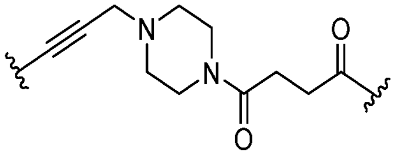,
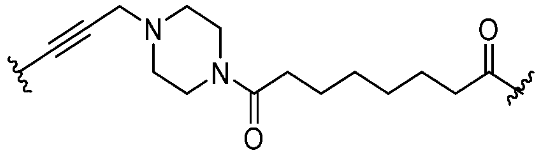,
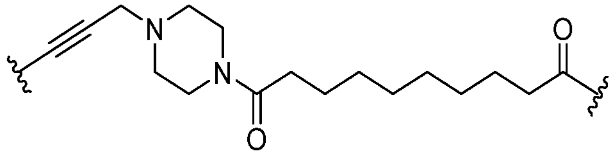,
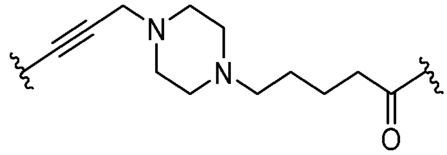,

or

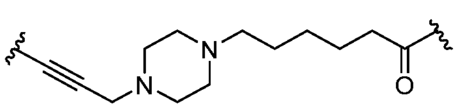.

The Y can also be selected from

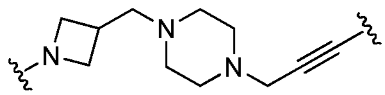

,

or

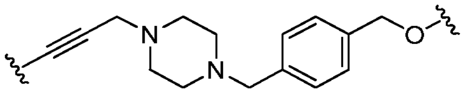

.

According to some embodiments of the present invention, the Y can also be optionally substituted independently by one or more (for example, 2, 3, 4, 5, 6, 7, 8, 9 or 10) $R^{YL'}$.

According to some embodiments of the present invention, $R^{YL'}$ is independently selected from halogen, cyano, nitro, $C_{1\sim6}$ alkyl, halogen-substituted $C_{1\sim6}$ alkyl, -OR', -N(R')$_2$; each R' is independently selected from hydrogen, halogen, -$C_{1\sim6}$ alkyl, halogen-substituted -$C_{1\sim6}$ alkyl, -$C_{0\sim2}$ alkylene-($C_{3\sim10}$ carbocyclic group), -$C_{0\sim2}$ alkylene-(3- to 10-membered heterocycloalkyl).

According to some embodiments of the present invention, $R^{YL'}$ is independently selected from halogen, cyano, nitro, $C_{1\sim6}$ alkyl, halogen-substituted $C_{1\sim6}$ alkyl, -OR', -N(R')$_2$; each R' is independently selected from hydrogen, halogen, -$C_{1\sim6}$ alkyl, halogen-substituted -$C_{1\sim6}$ alkyl, -$C_{0\sim2}$ alkylene-($C_{3\sim8}$ carbocyclic group), -$C_{0\sim2}$ alkylene-(3- to 8-membered heterocycloalkyl).

According to some embodiments of the present invention, $R^{YL'}$ is independently selected from halogen, cyano, nitro, $C_{1\sim3}$ alkyl, halogen-substituted $C_{1\sim3}$ alkyl, -OR' and -N(R')$_2$; each R' is independently selected from hydrogen, halogen, -$C_{1\sim3}$ alkyl, halogen-substituted -$C_{1\sim3}$ alkyl, -$C_{0\sim2}$ alkylene-($C_{3\sim6}$ carbocyclic group) and -$C_{0\sim2}$ alkylene-(3- to 6-membered heterocycloalkyl).

In some embodiments of the present invention, the E3 ubiquitin ligase is selected from CRBN, von Hippel-Lindau (VHL), XIAP, MDM2, cIAP-1.

According to some embodiments of the present invention, Z is selected from

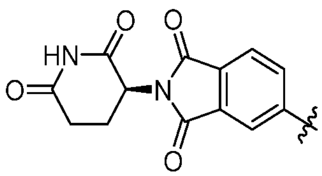 , 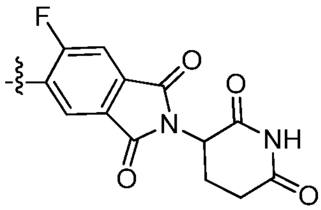 , 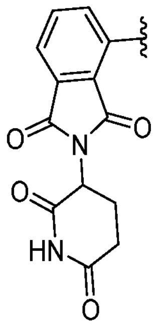 , 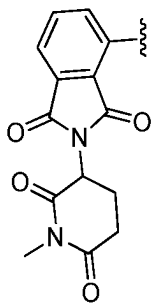 ,

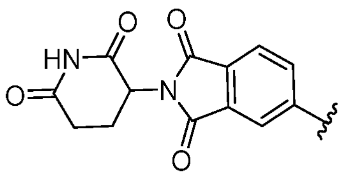 , 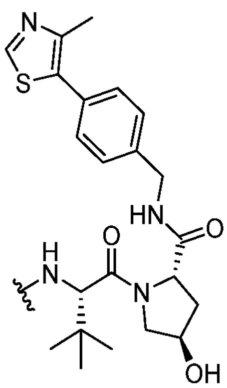 , 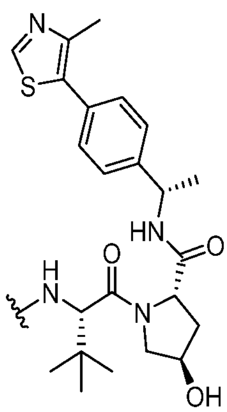 , 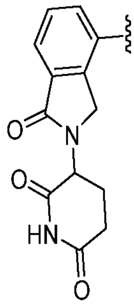 ,

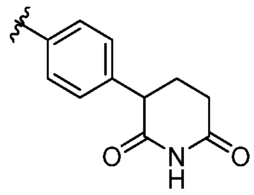, 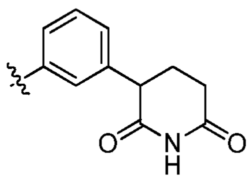, 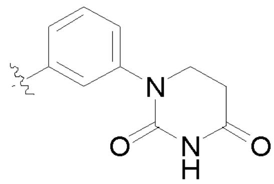, 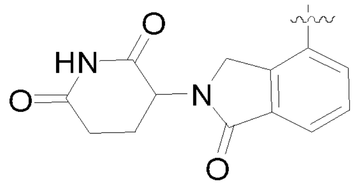,

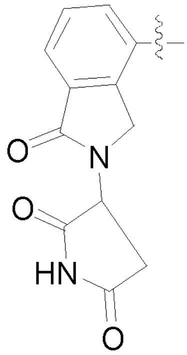, 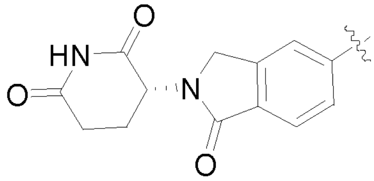, 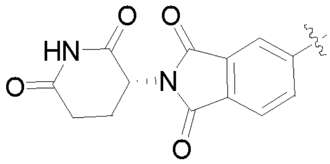

or

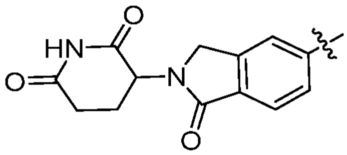.

According to some embodiments of the present invention, Z is selected from

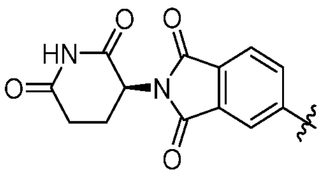, 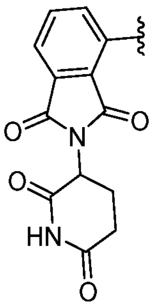, 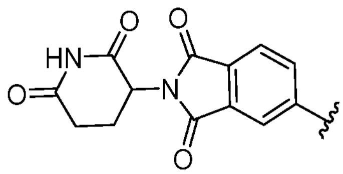, 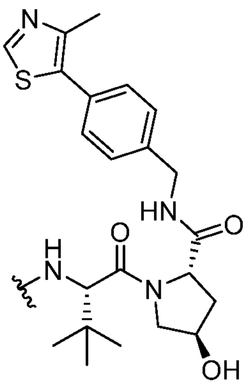,

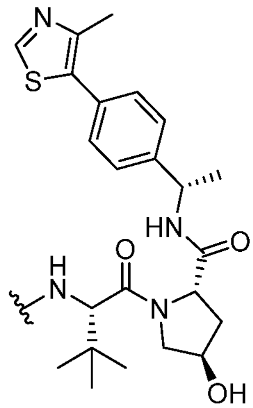, 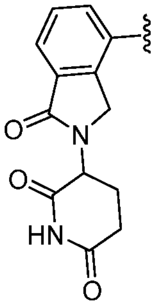, 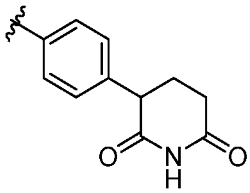, 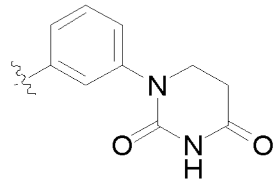

or

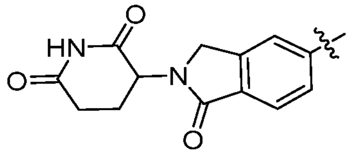.

According to some embodiments of the present invention, Z is selected from

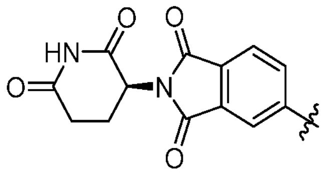, 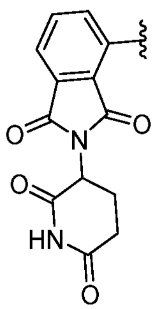, 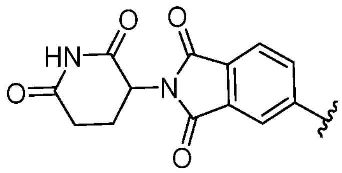, 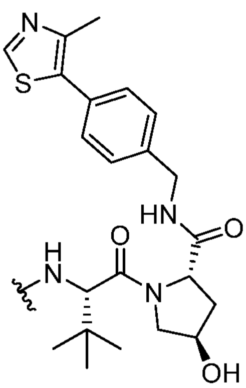,

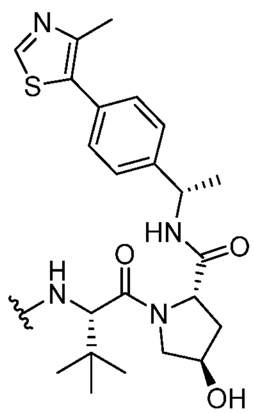, 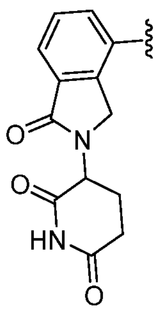, 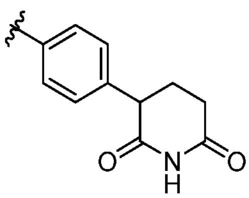 or 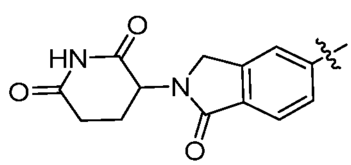.

Z can also be selected from

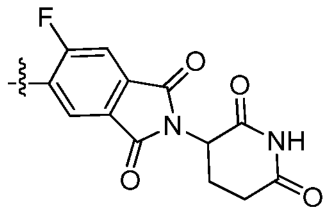, 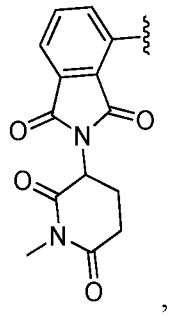, 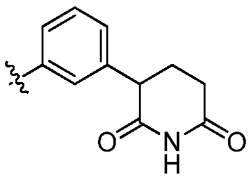,

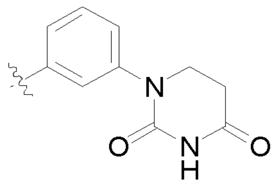, 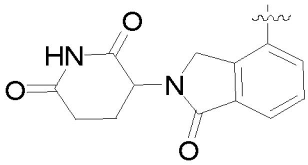, 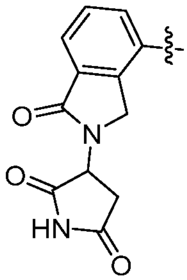, 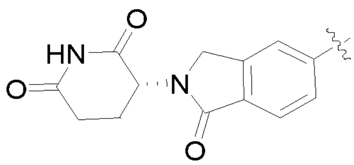

or

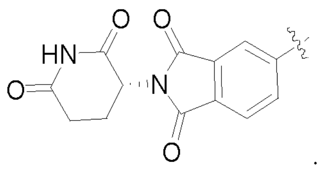.

Z can also be selected from

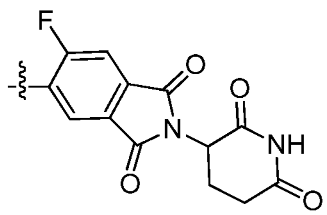, 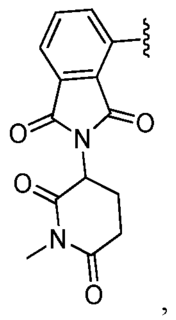, 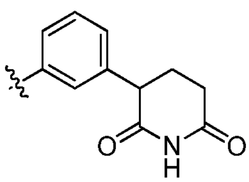,

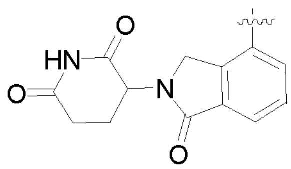, 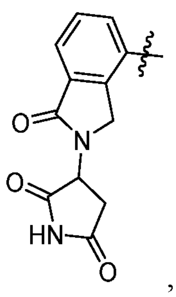, 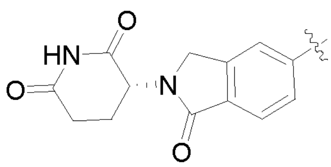 or 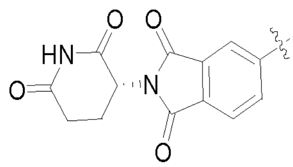.

According to some embodiments of the present invention, Z is selected from

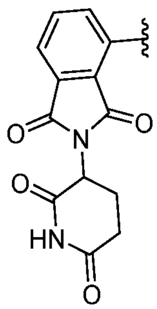,

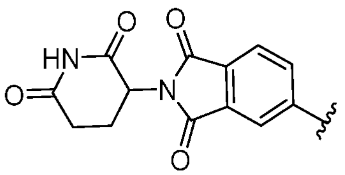, 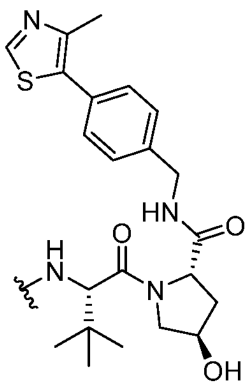, 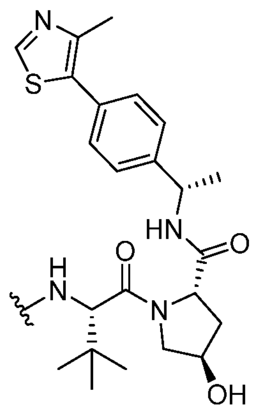, 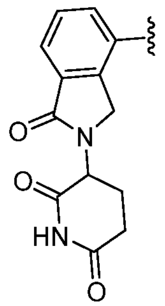,

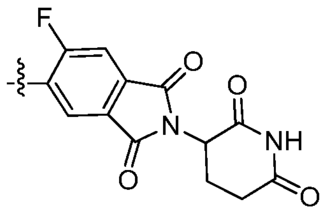, 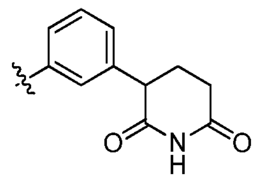, 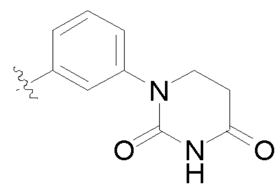,

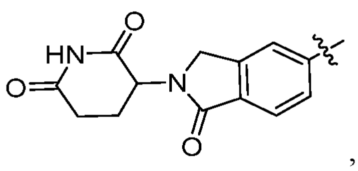, 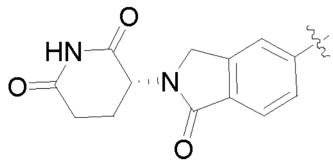, or 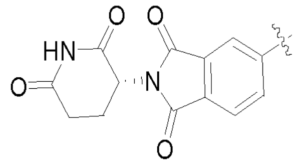.

In some specific embodiments of the present invention, the compound of Formula I is specifically:

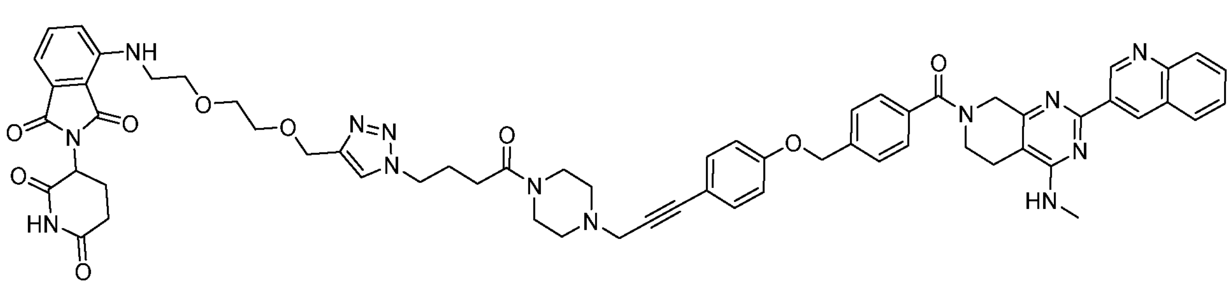,

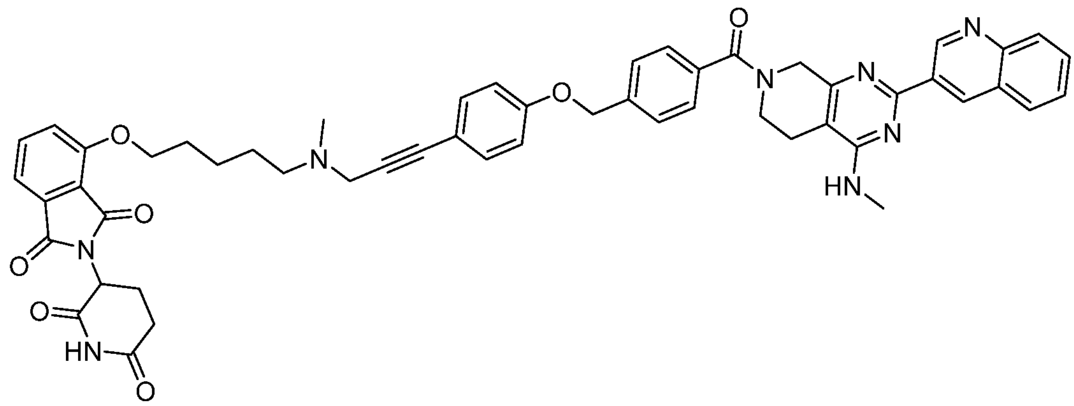

,

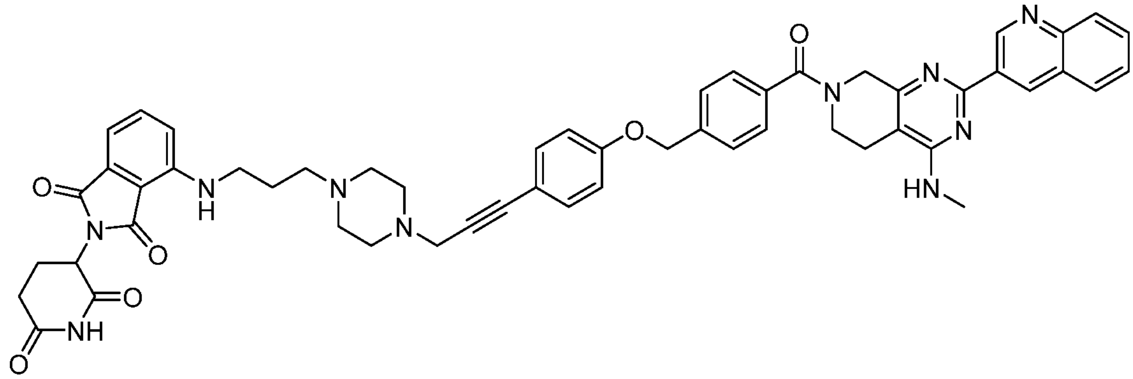

,

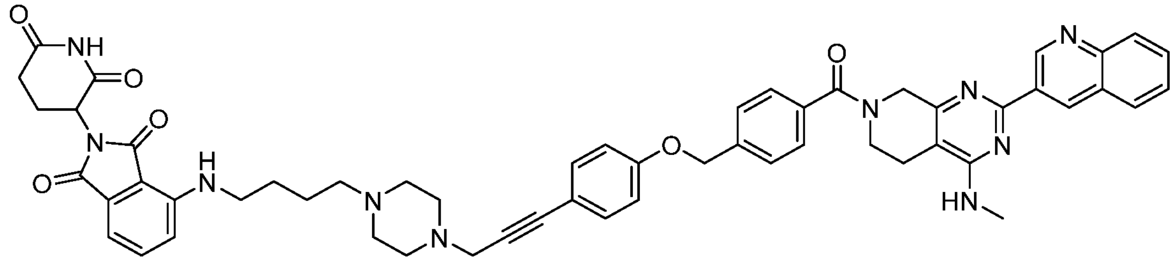

,

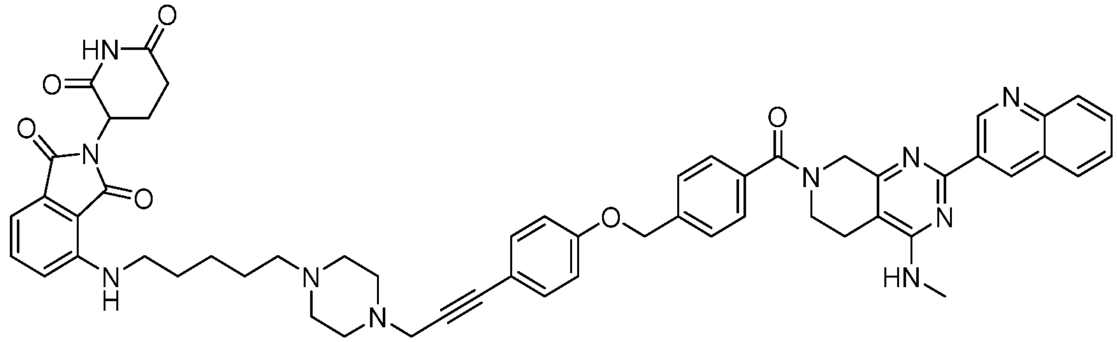

,

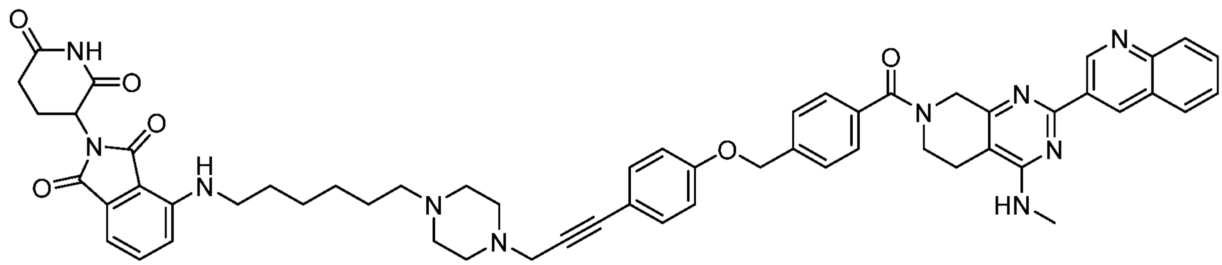

,

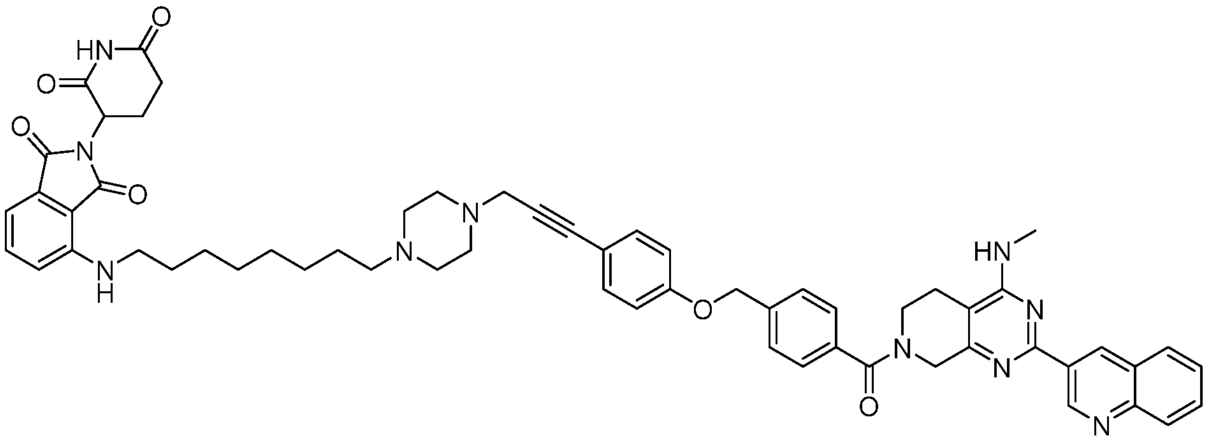

,

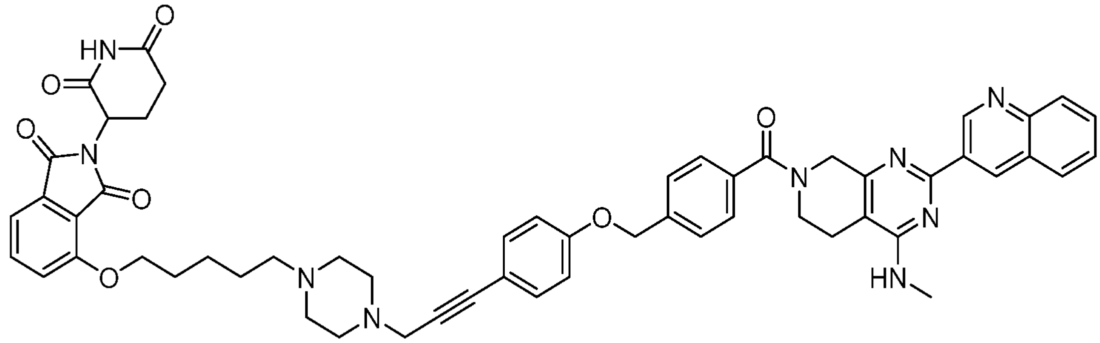

,

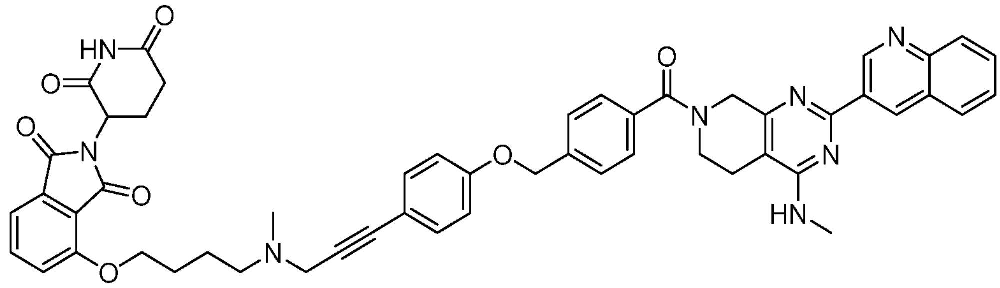

,

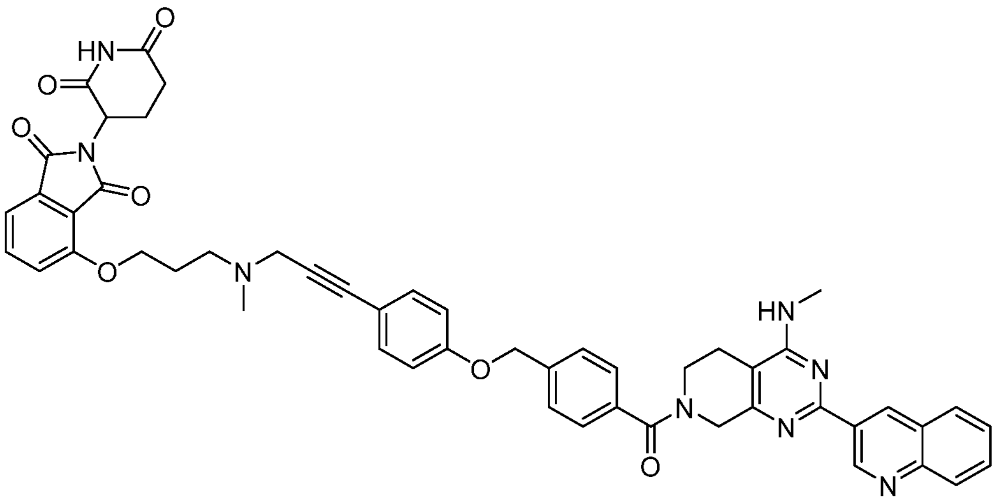

,

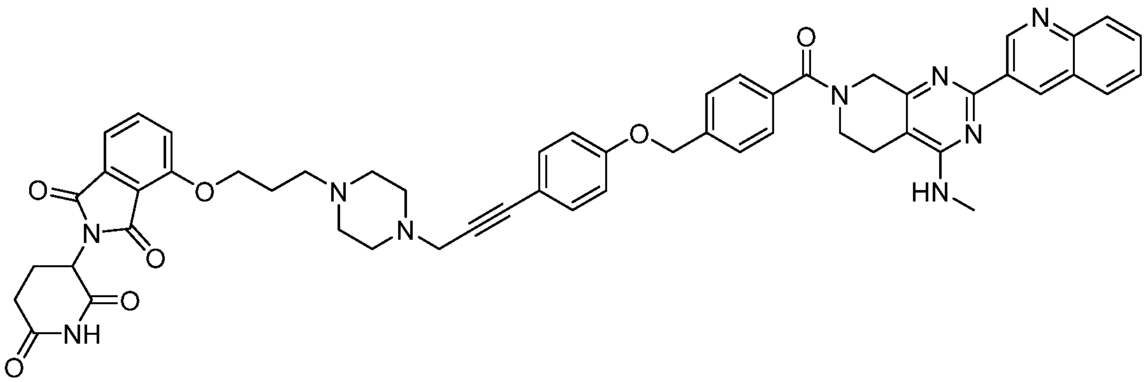

,

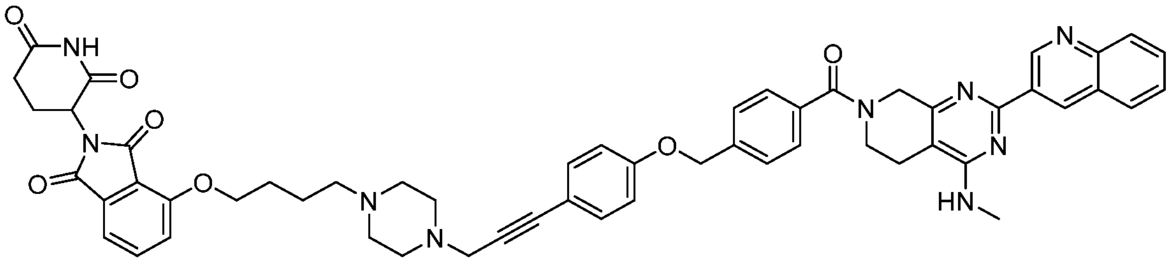

,

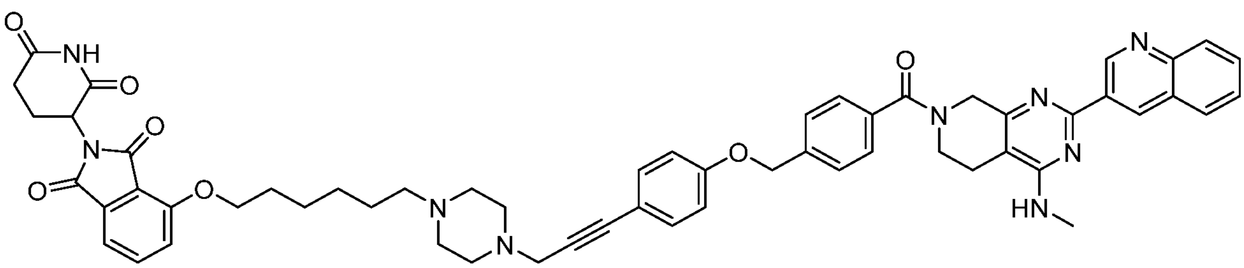

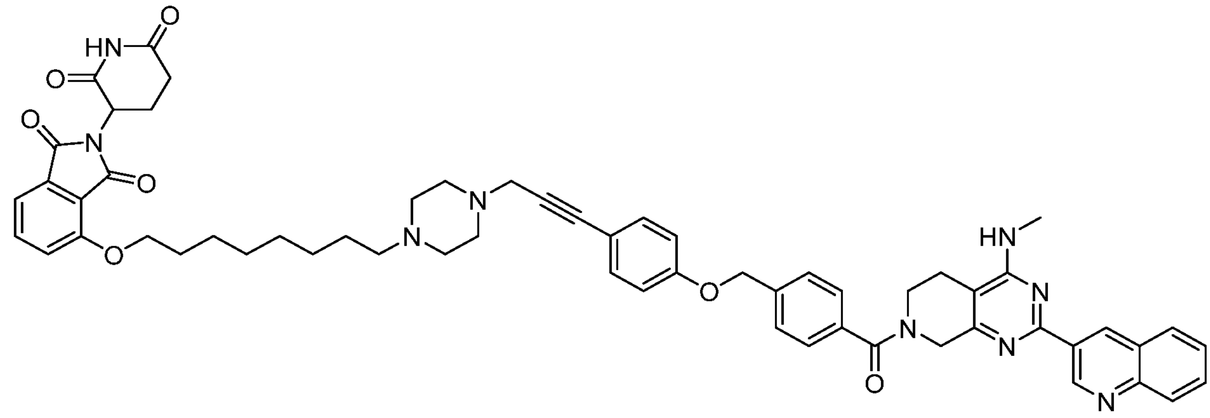

, ,

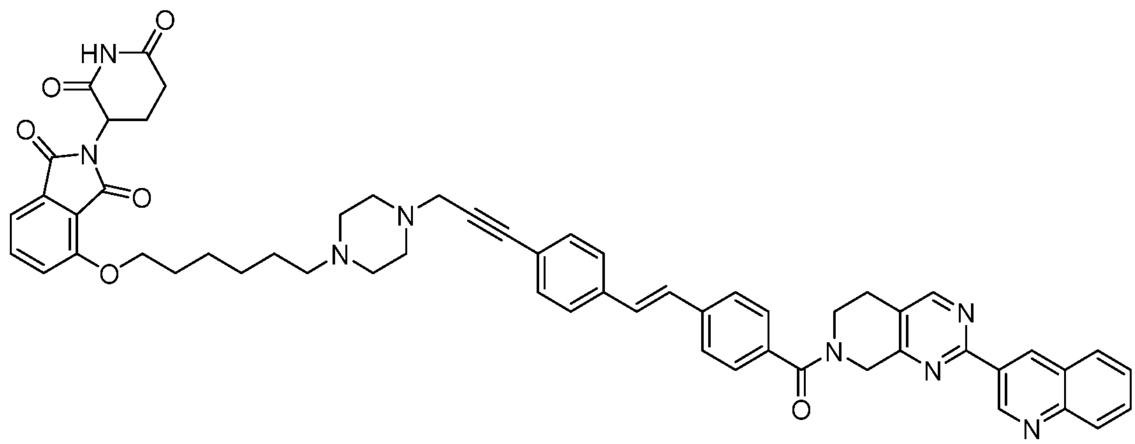

,

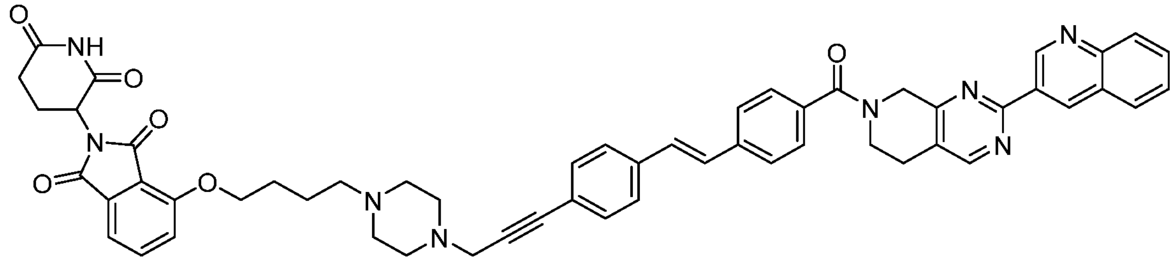

,

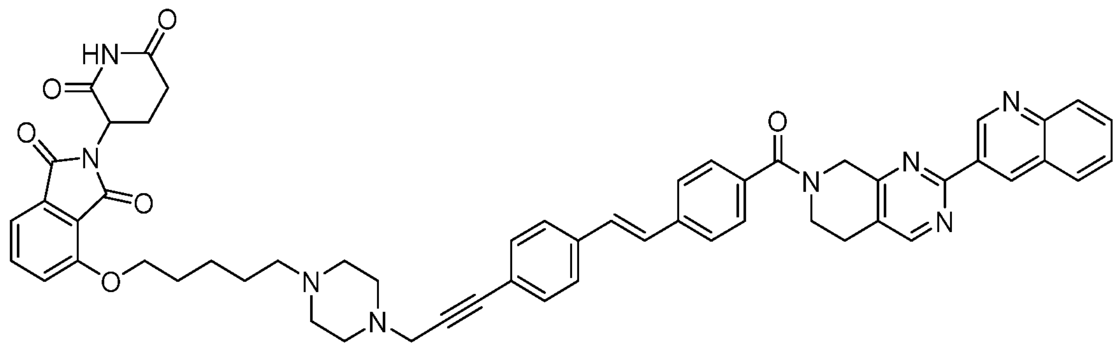

,

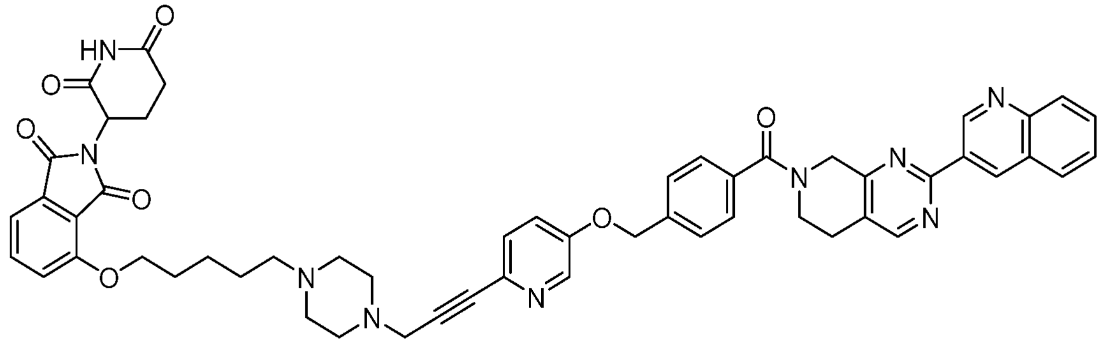

,

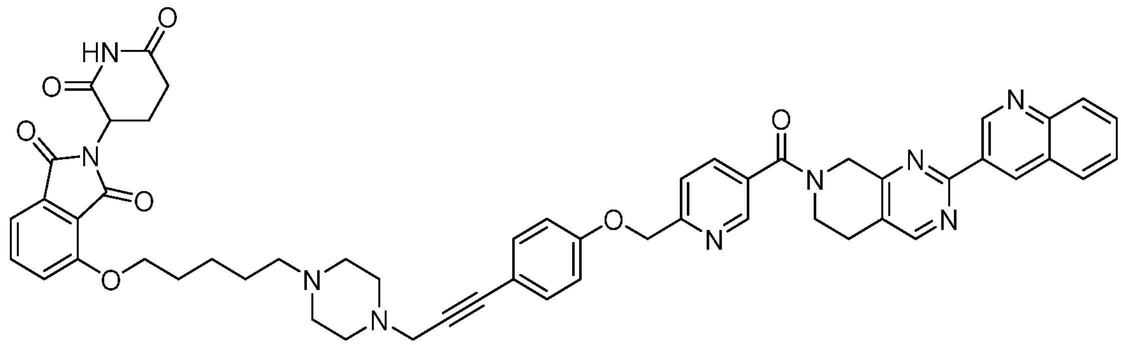

,

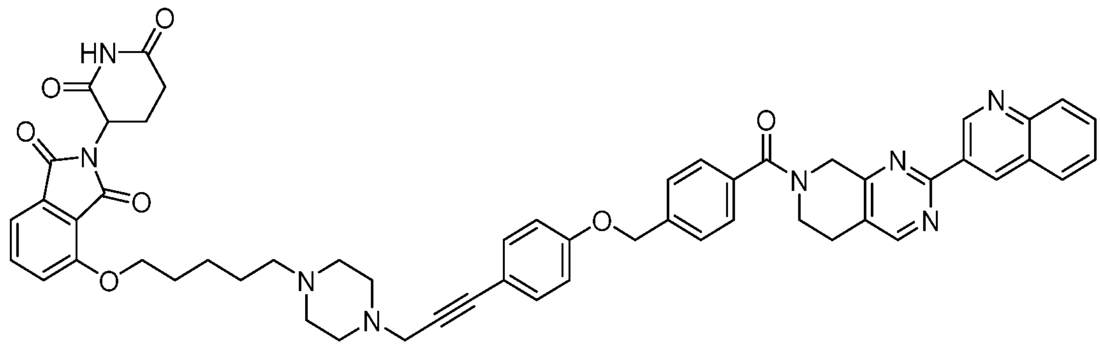

,

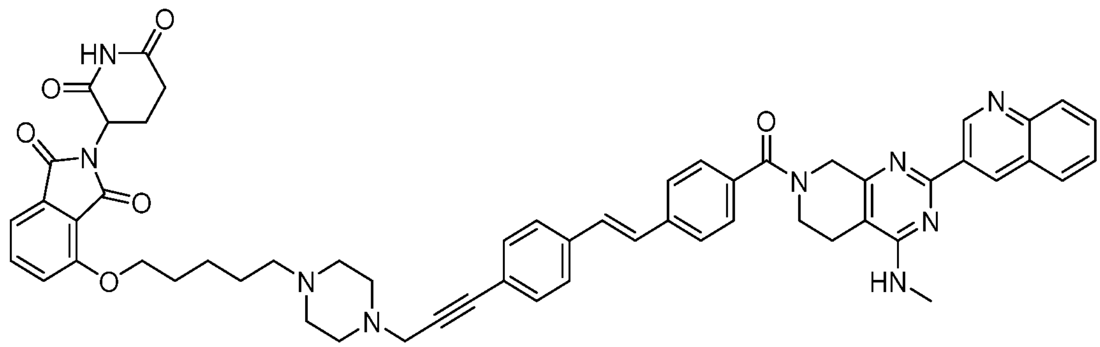

,

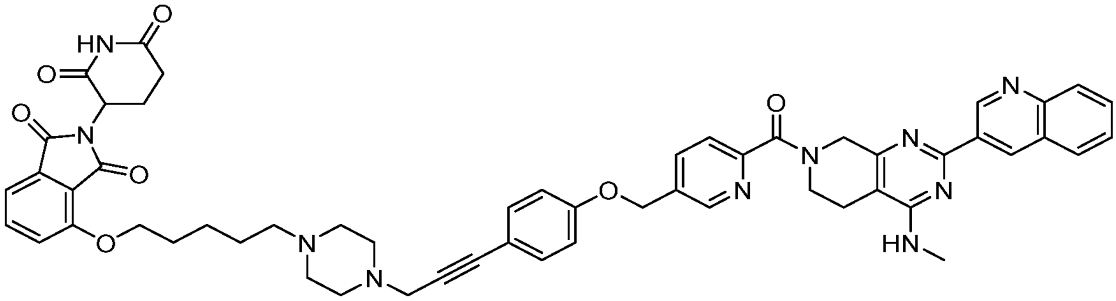

,

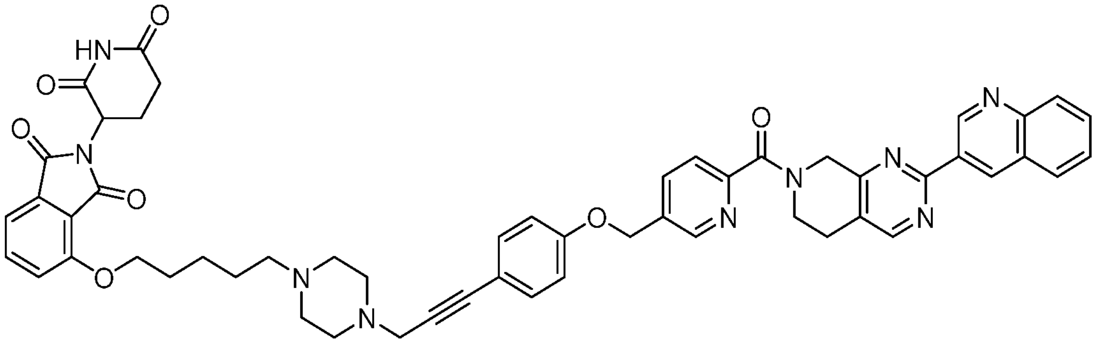

,

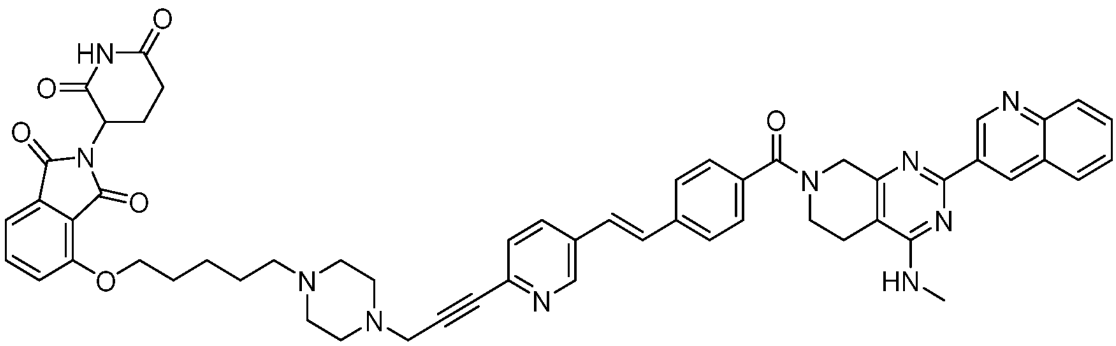

,

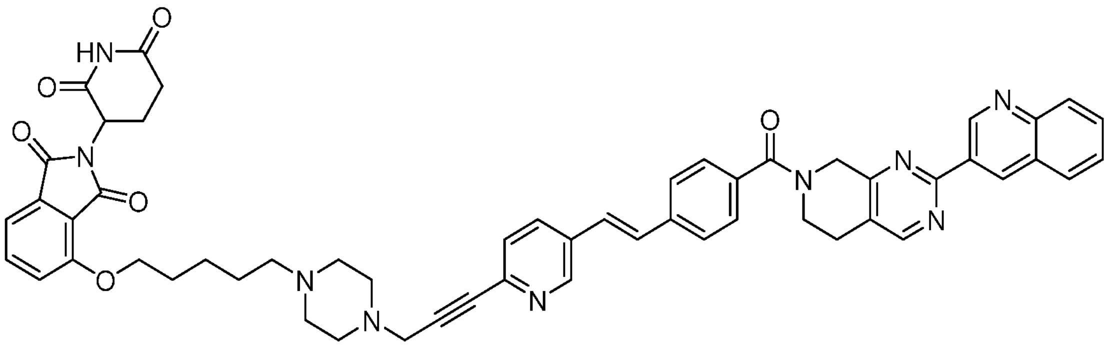

,

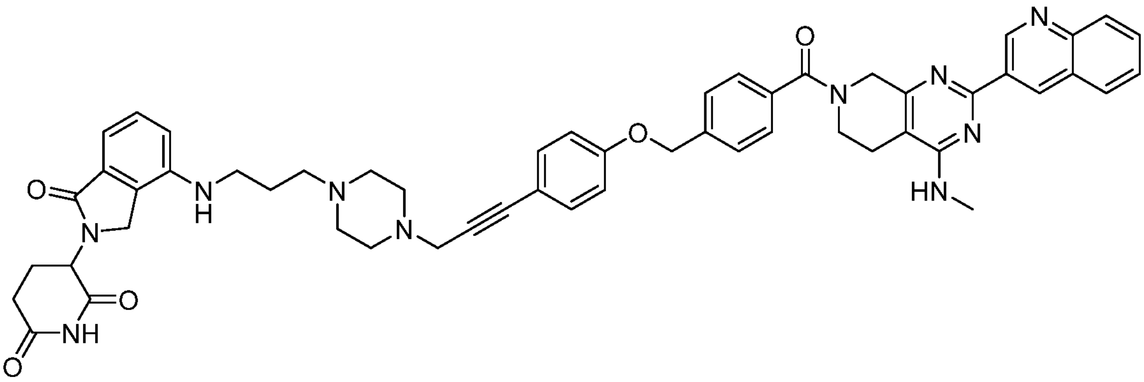

,

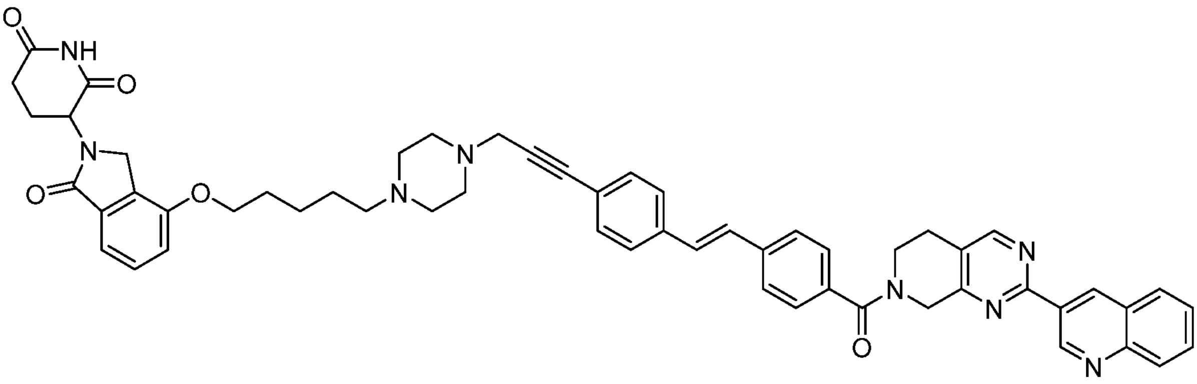

,

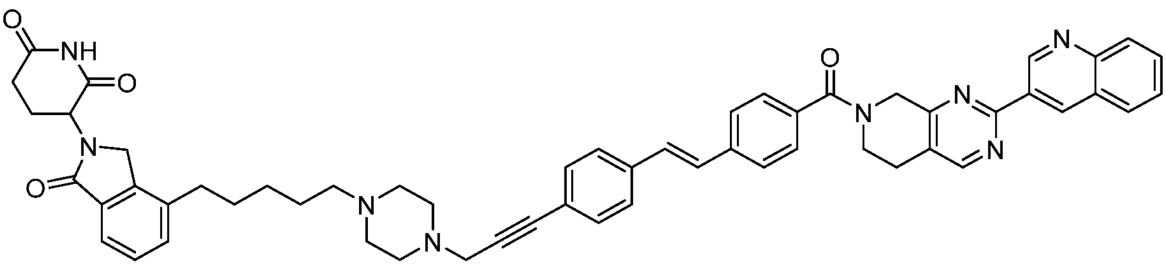

,

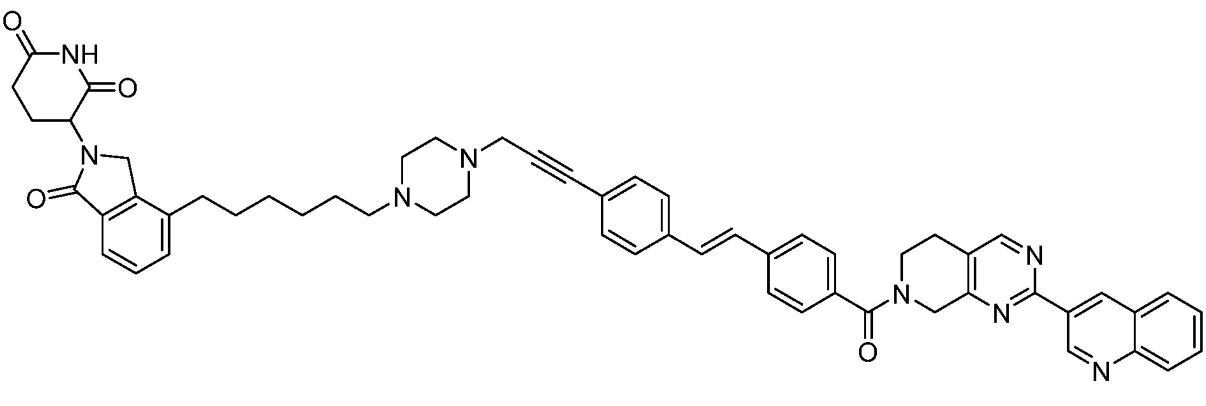

,

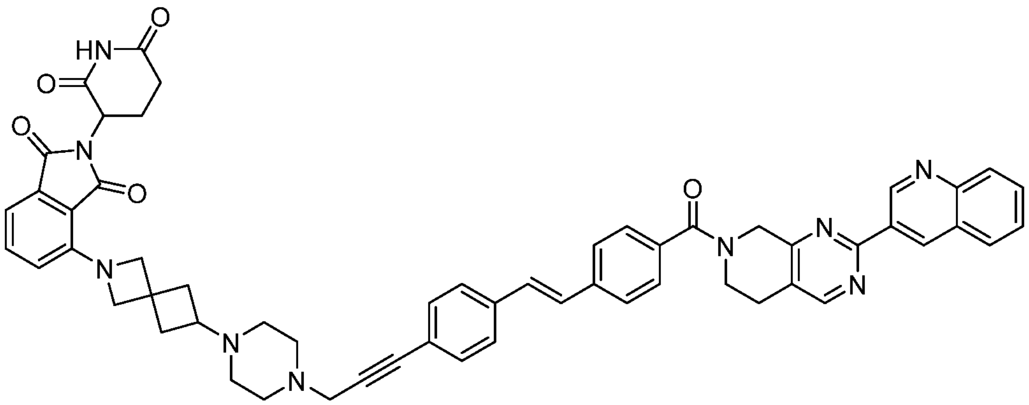

,

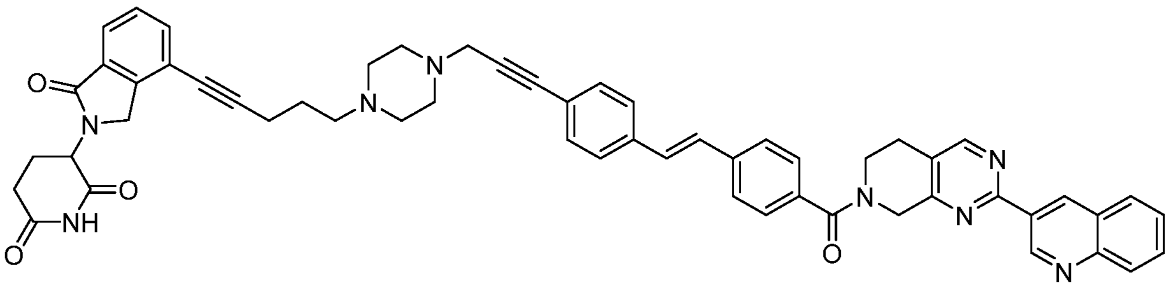

,

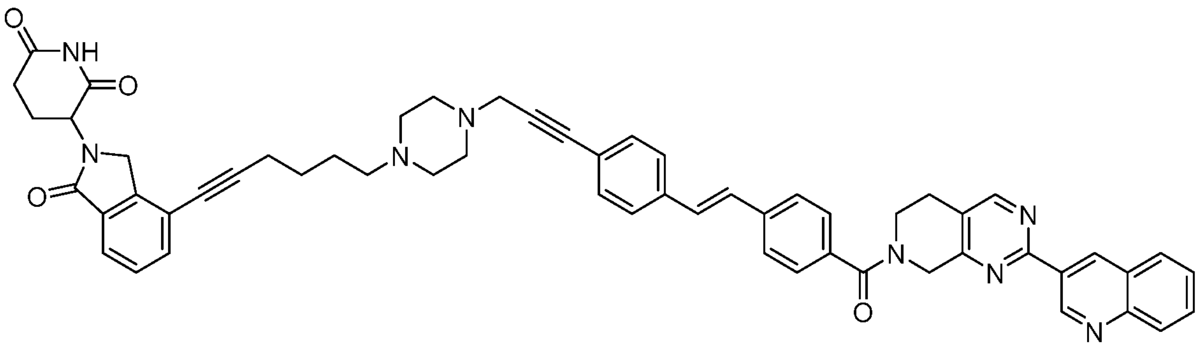

,

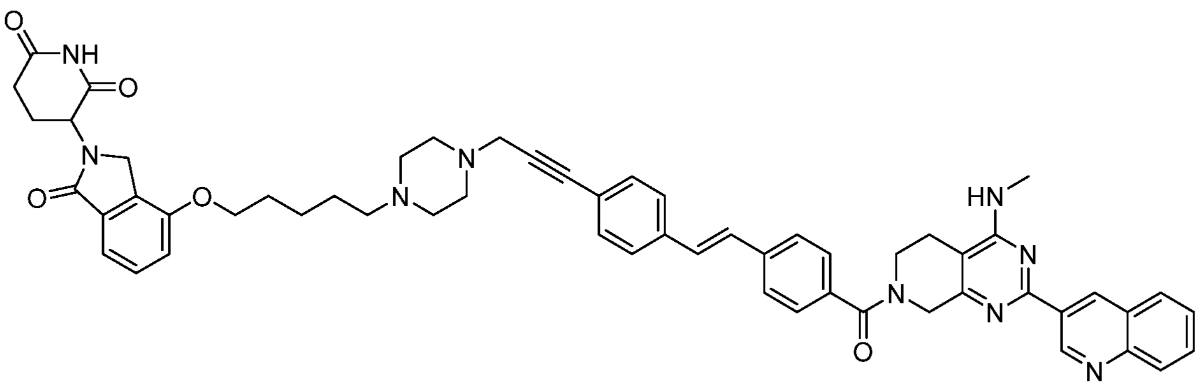

,

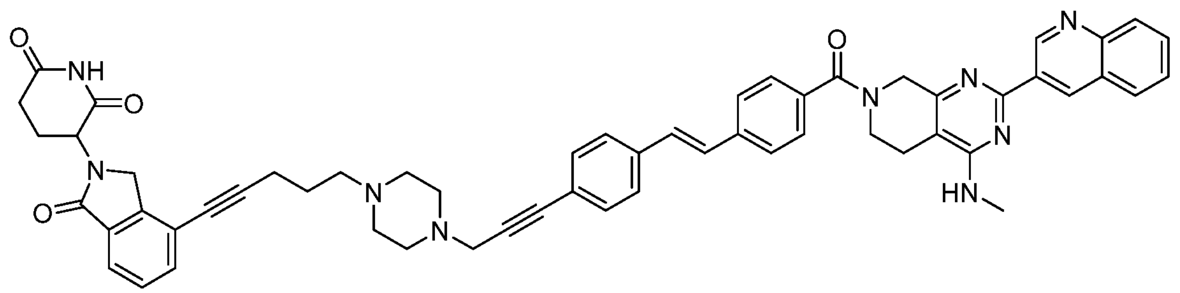

,

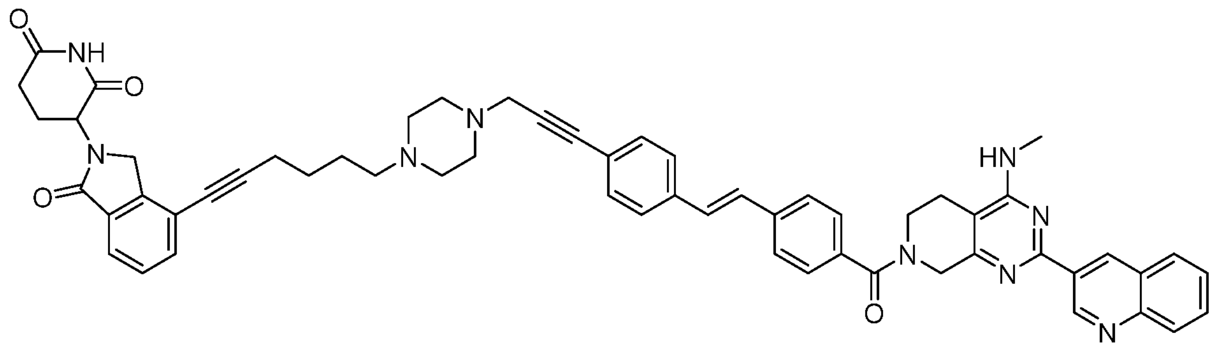

,

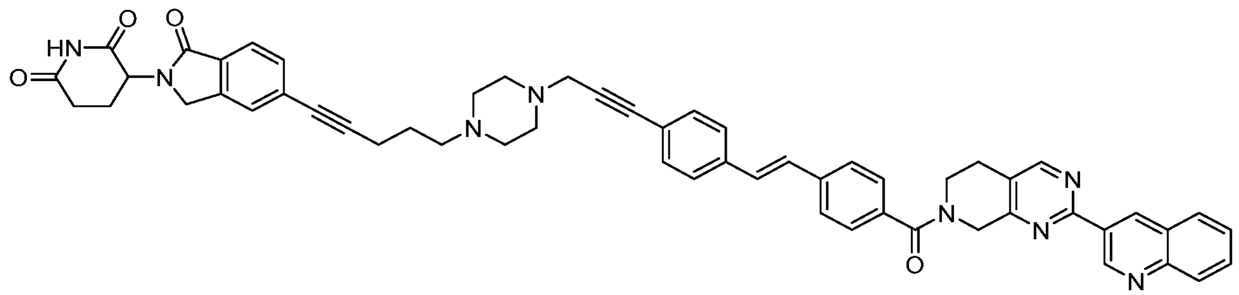

,

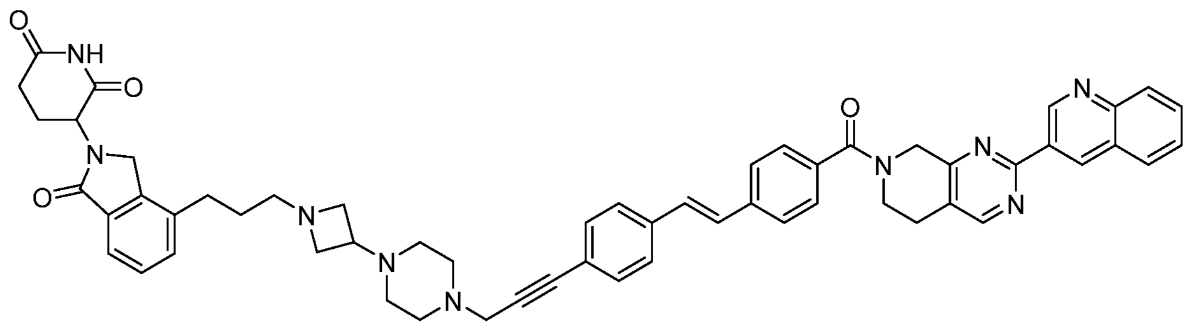

,

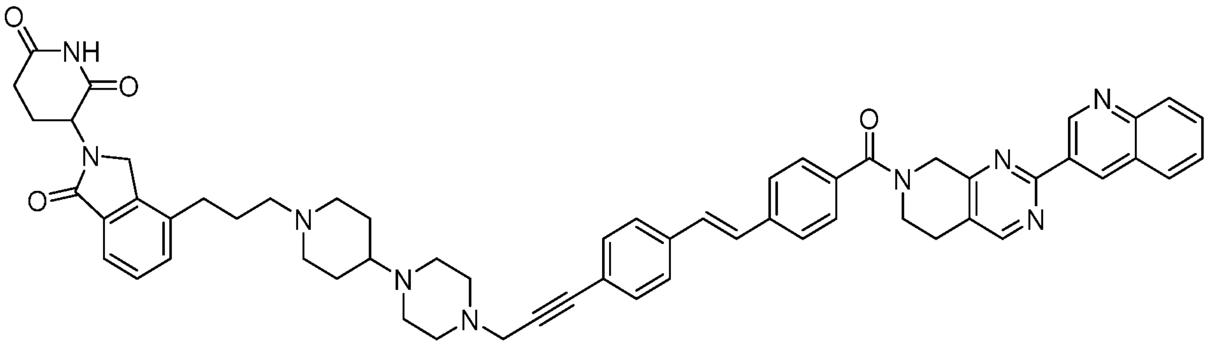

,

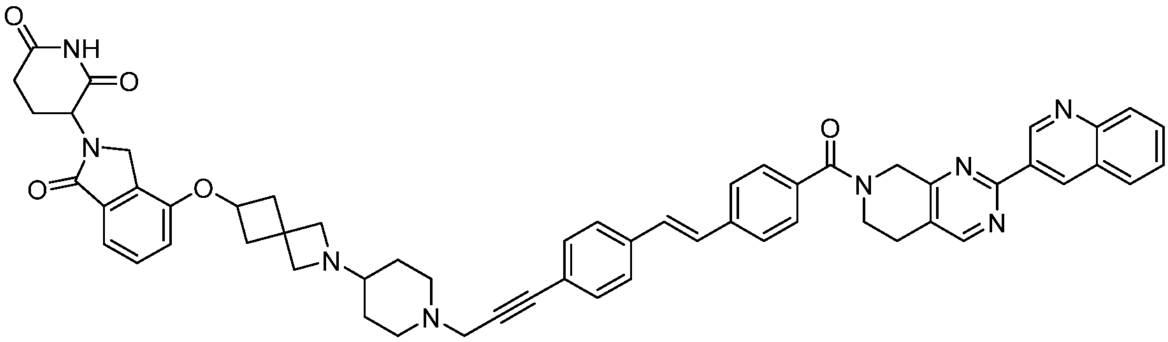

,

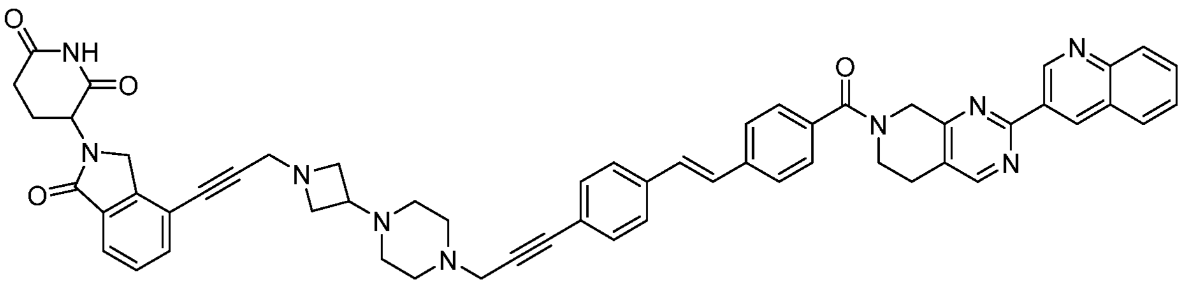

,

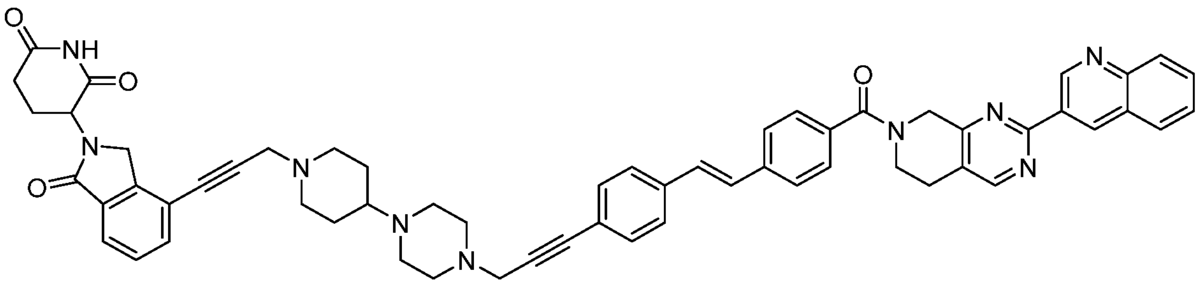

,

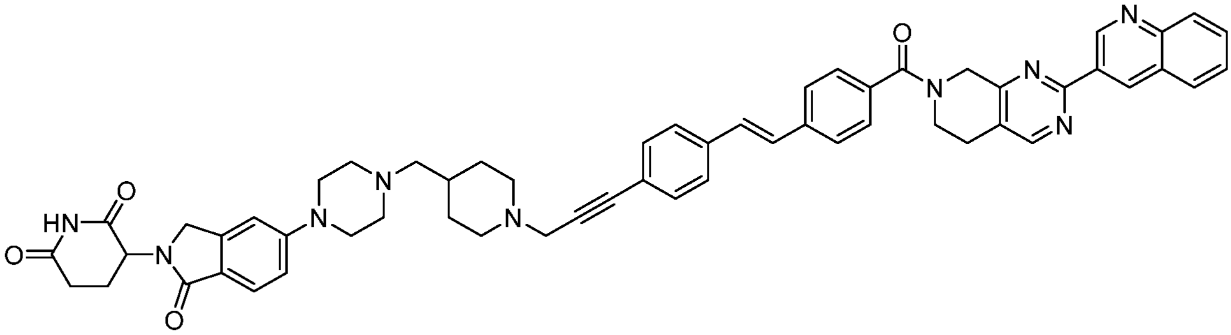

,

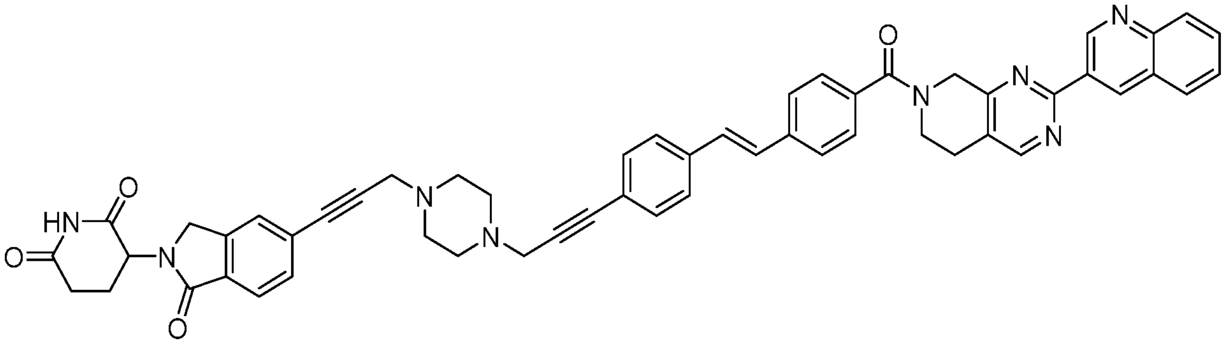

,

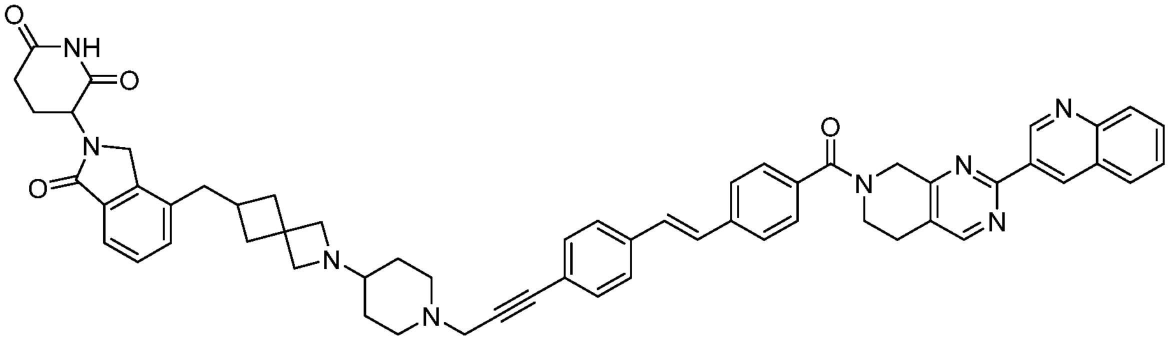

,

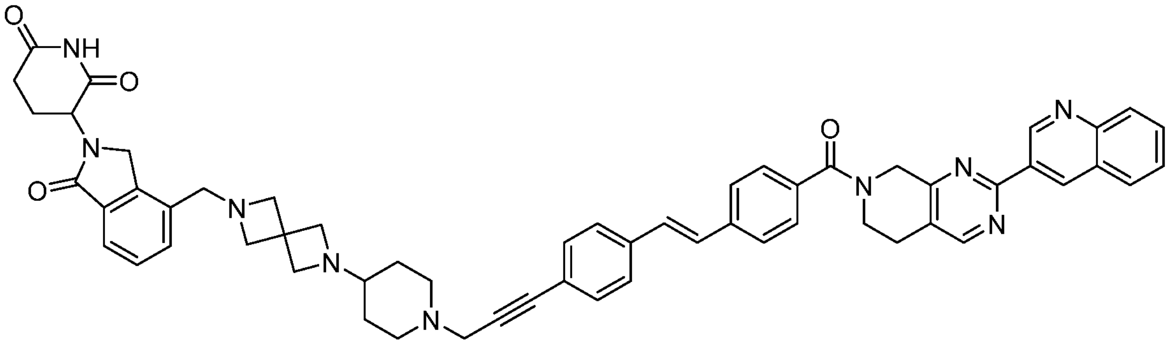

,

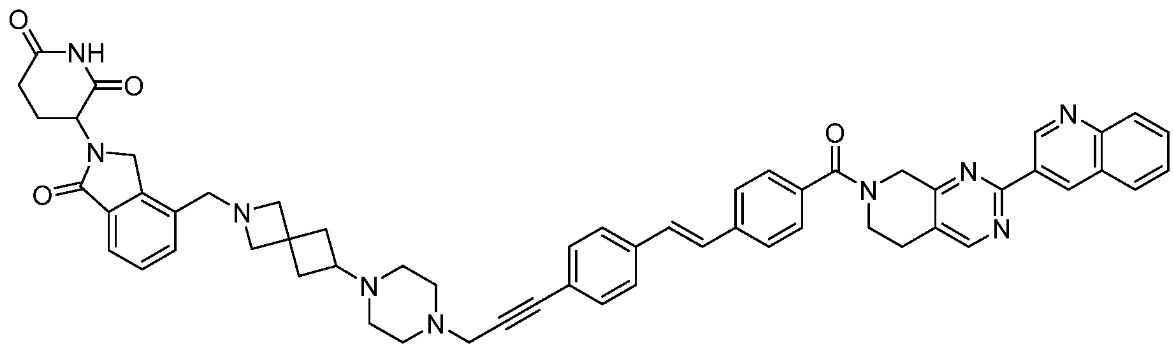

,

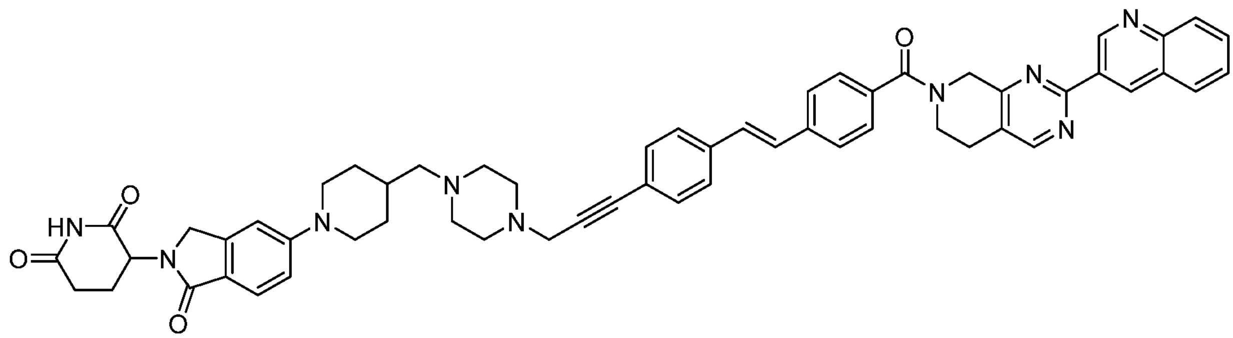

,

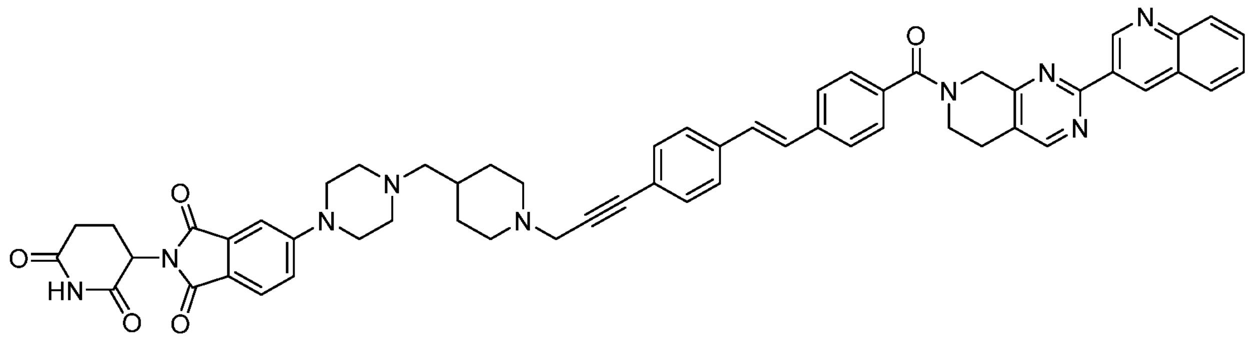

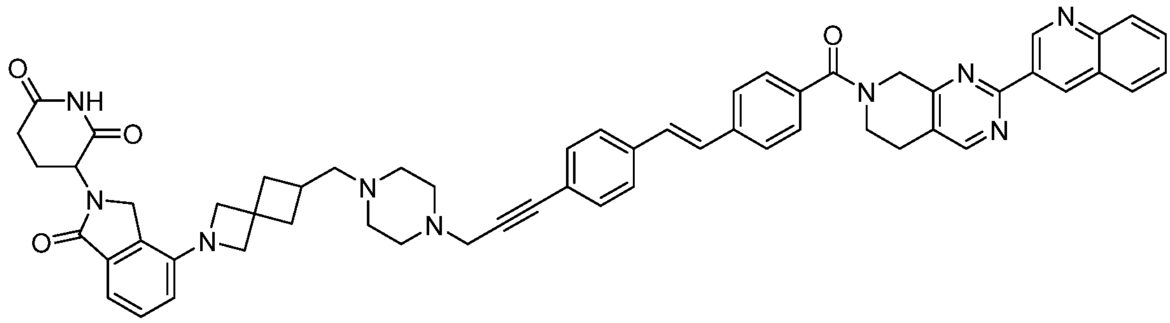

, ,

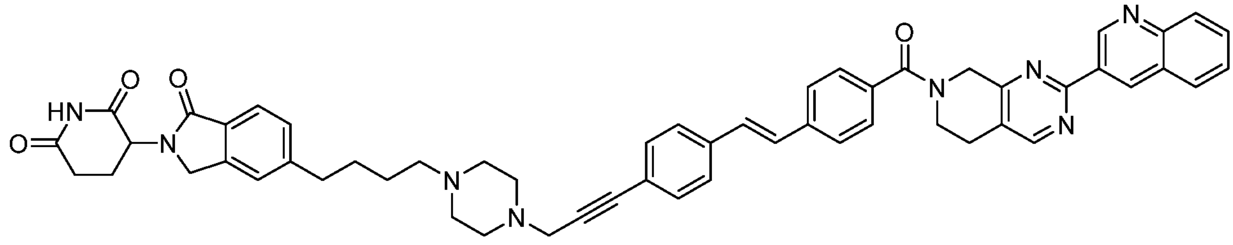

,

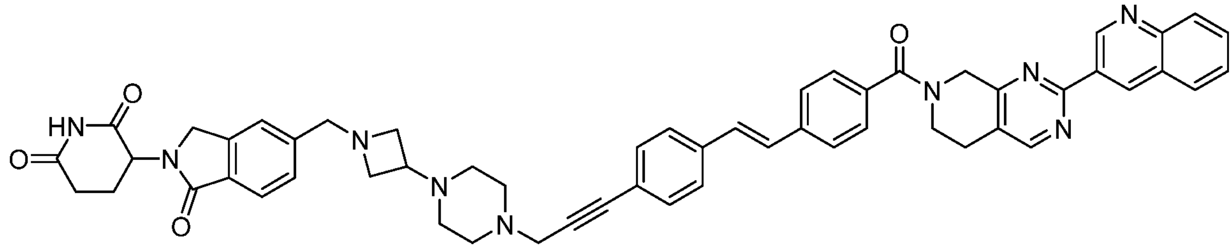

,

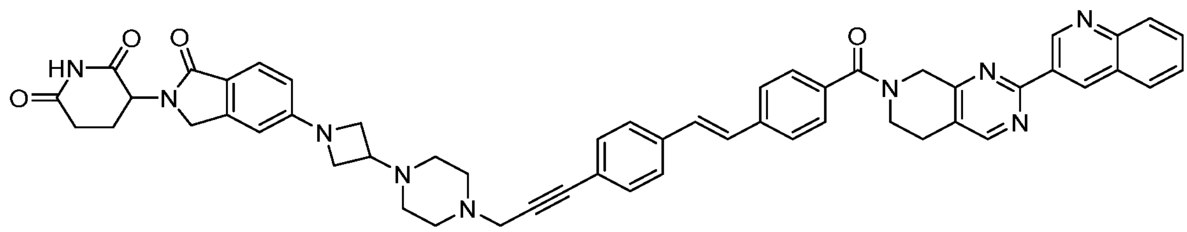

,

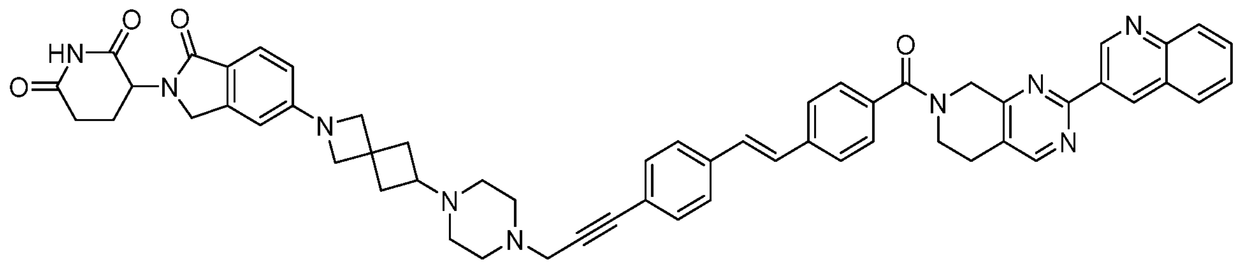

,

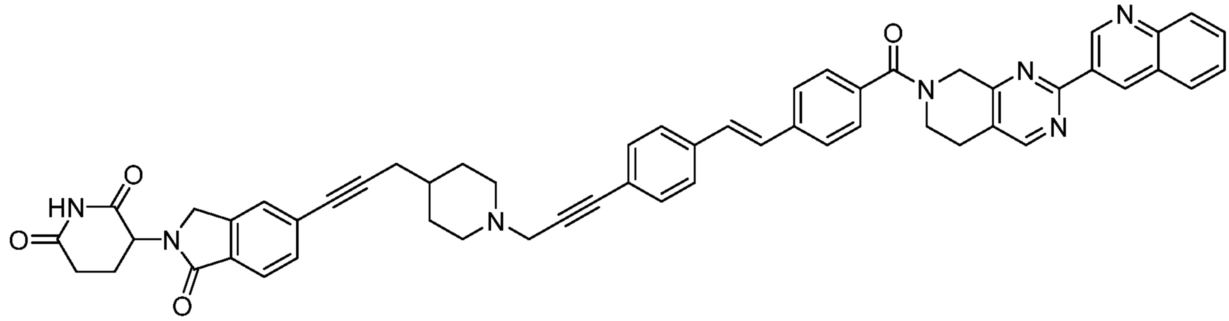

,

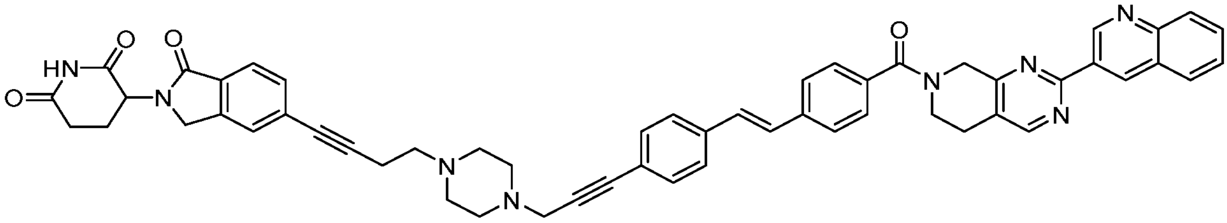

,

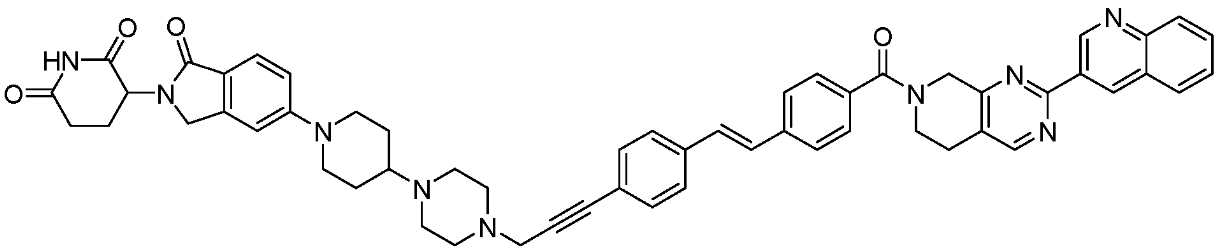

,

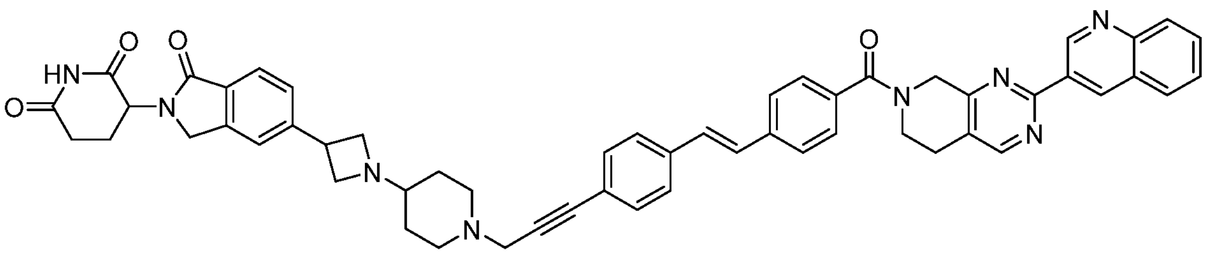

,

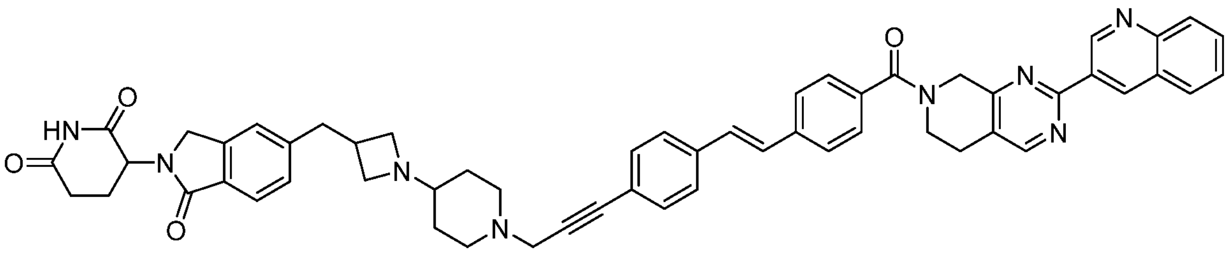

,

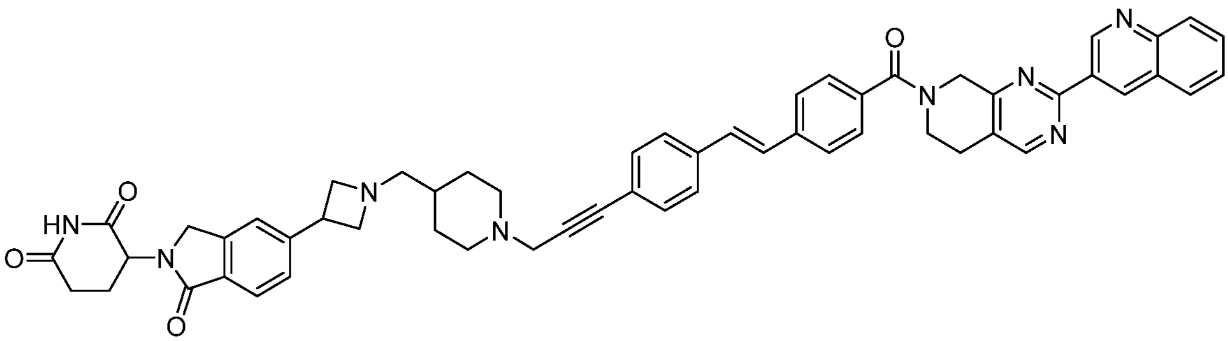

,

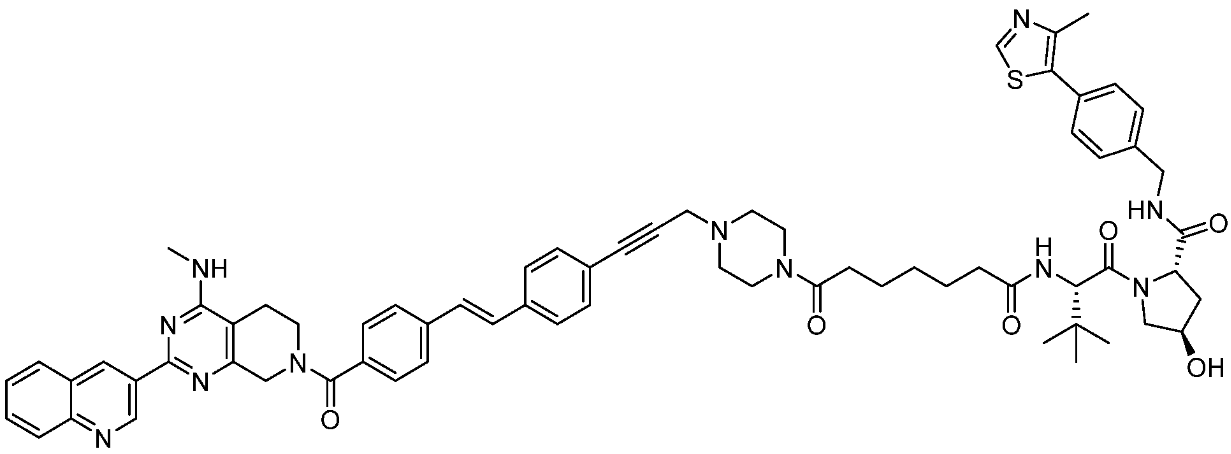

,

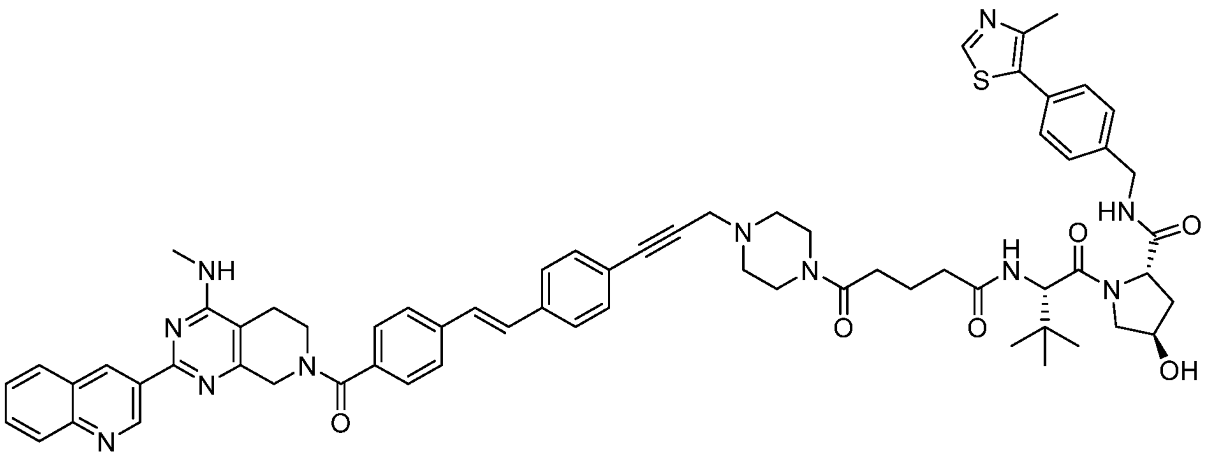

,

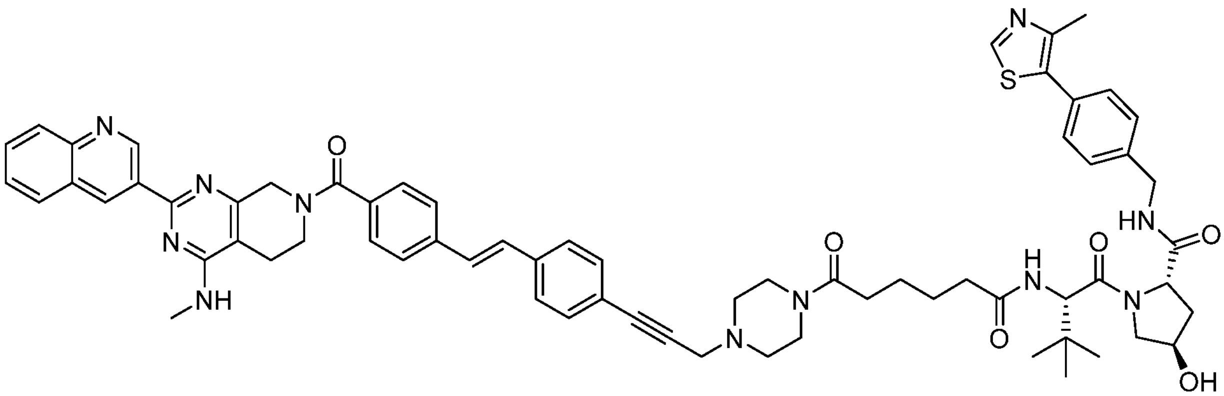

,

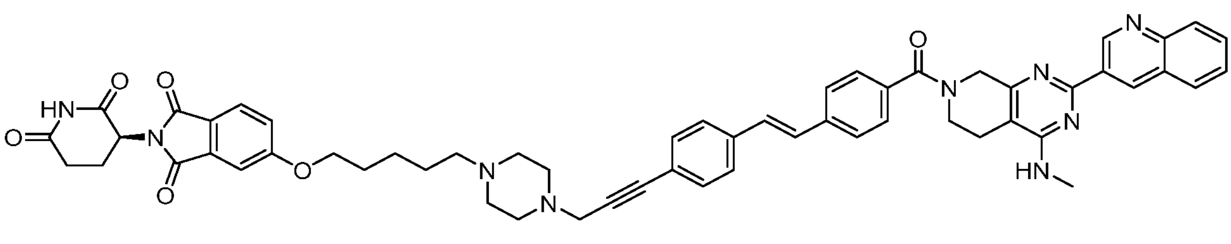

,

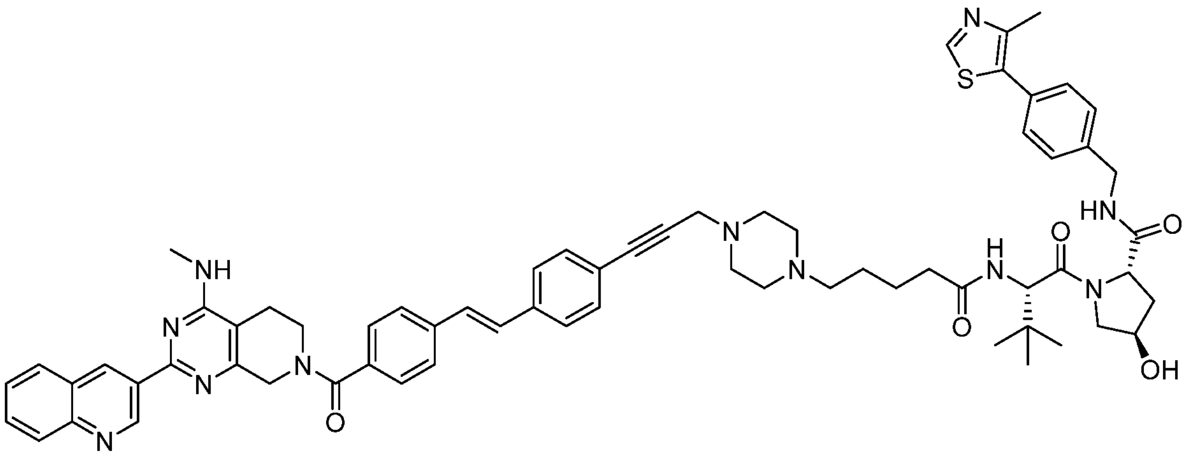

,

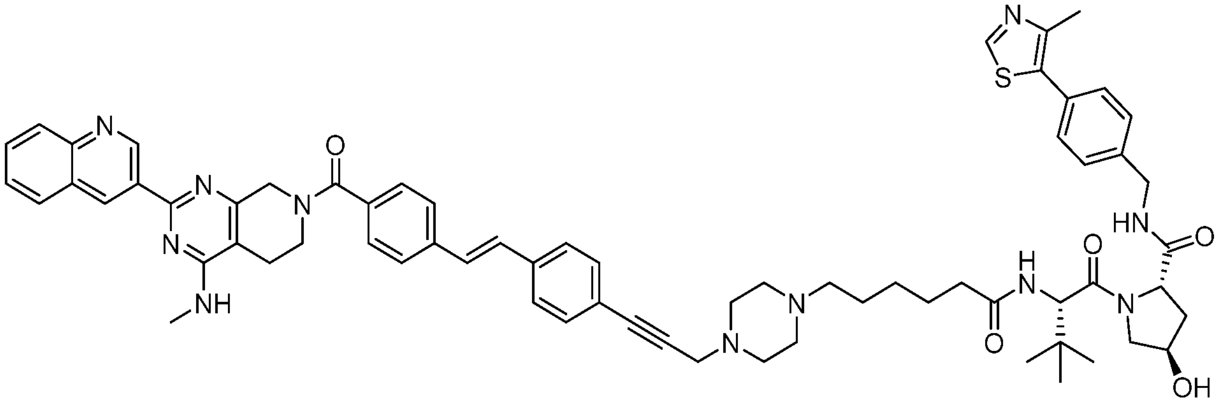

,

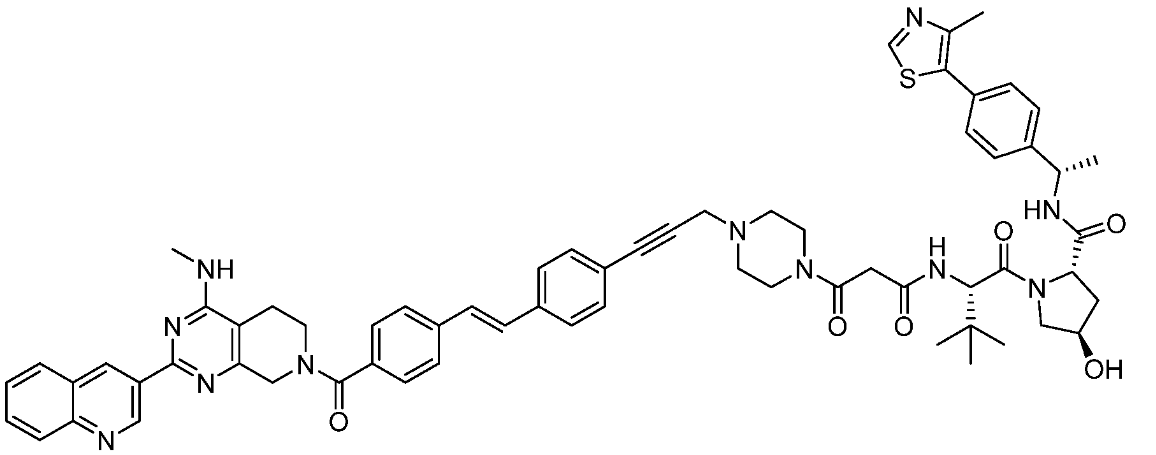

,

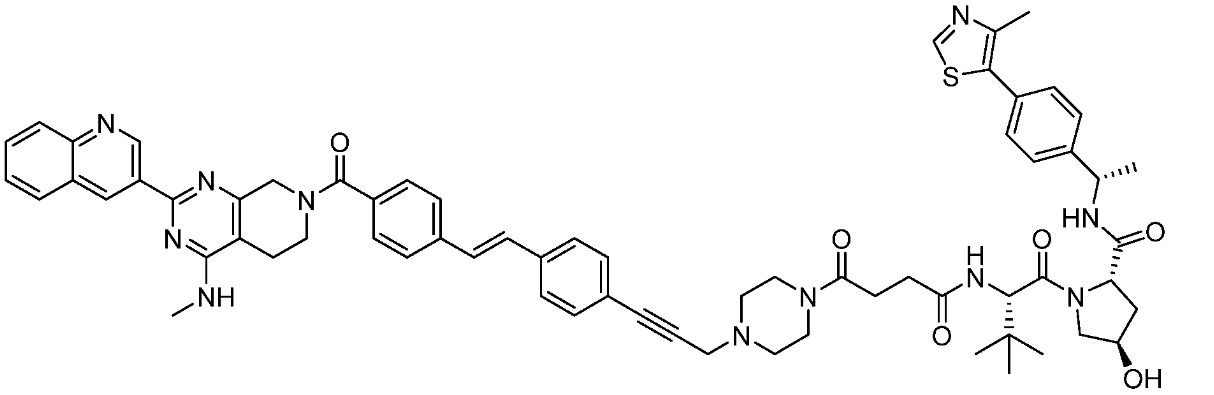

,

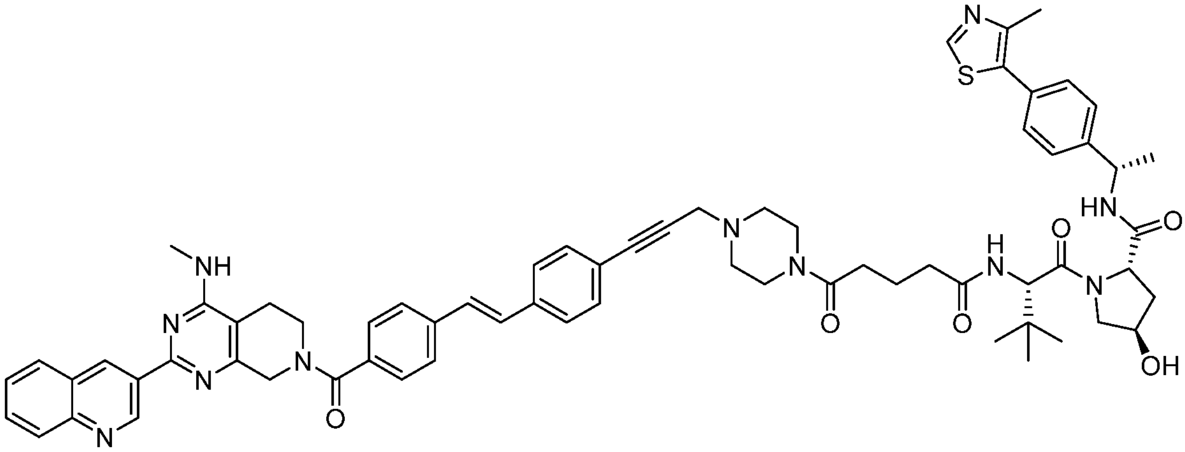

,

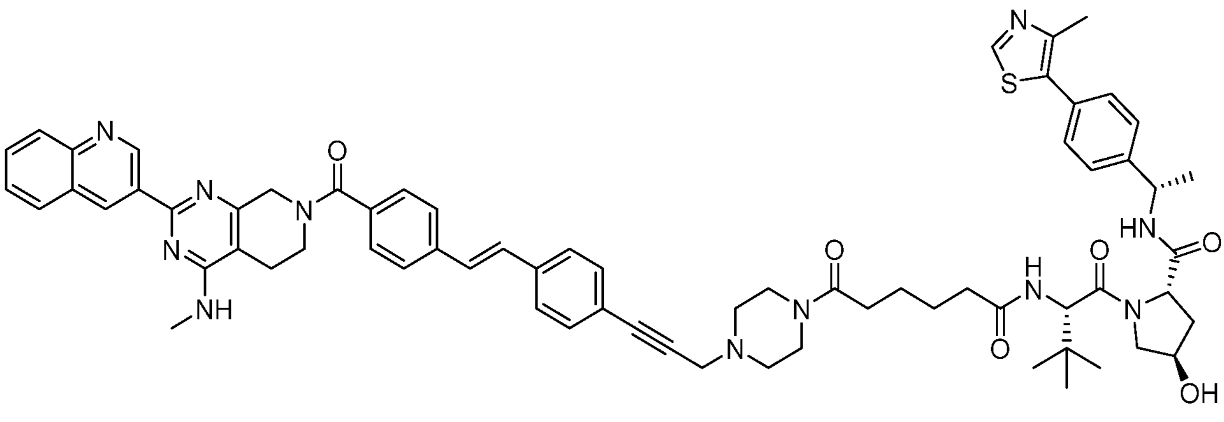

,

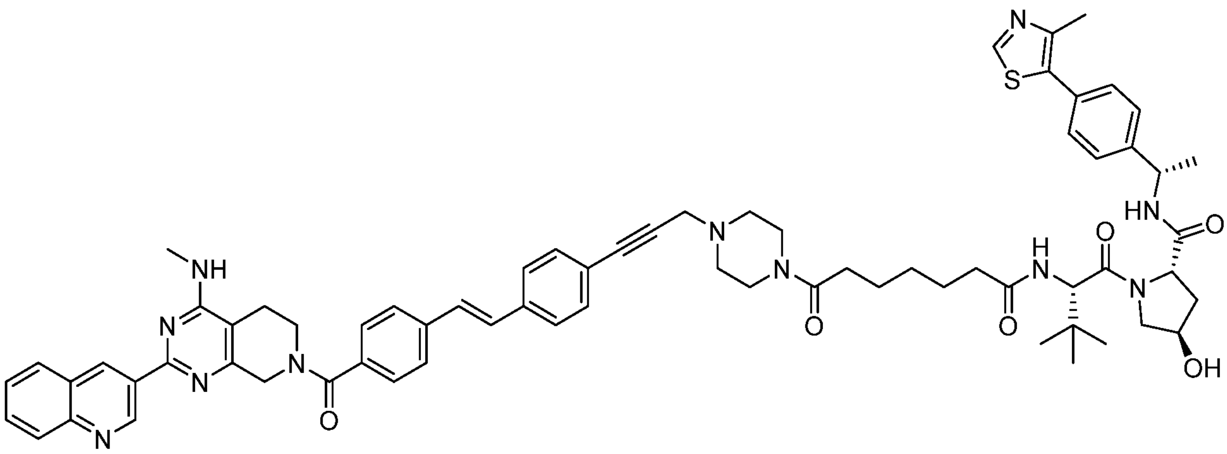

,

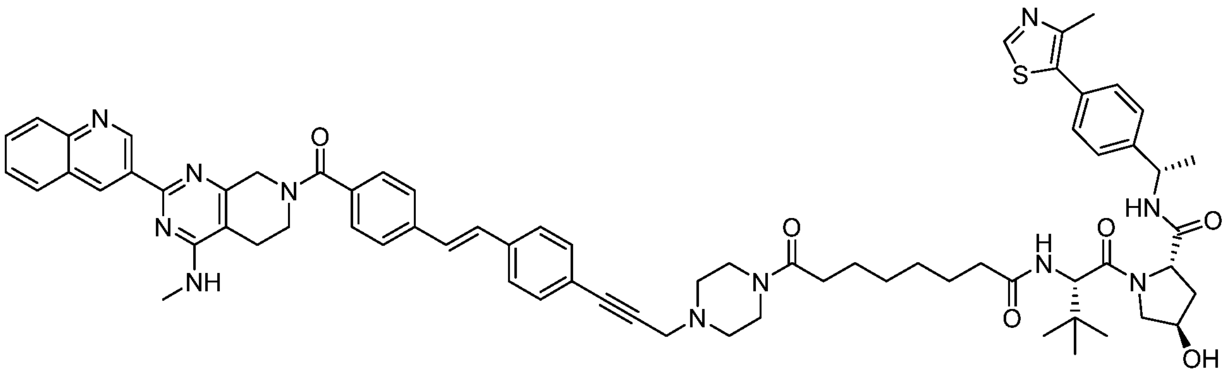

,

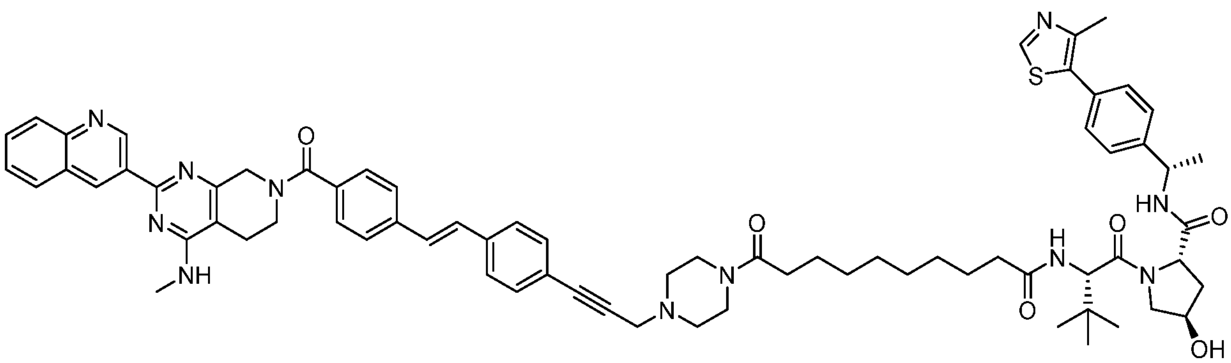

,

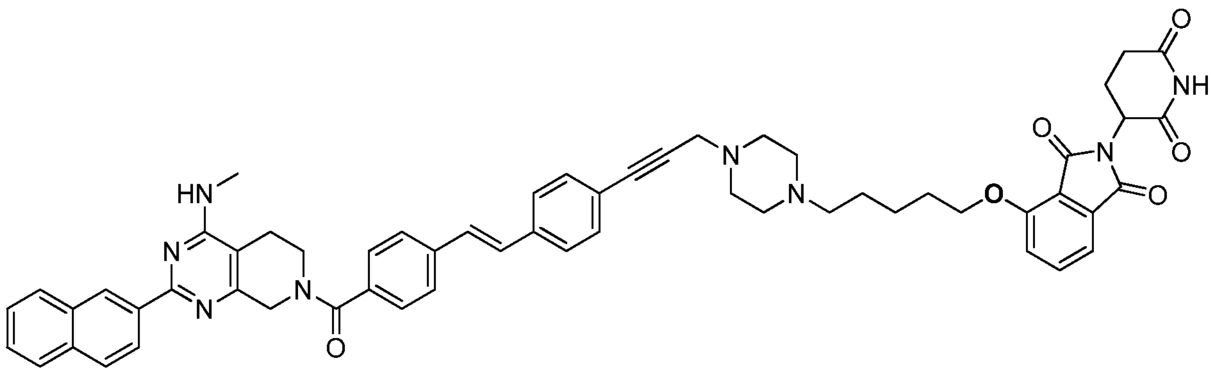

,

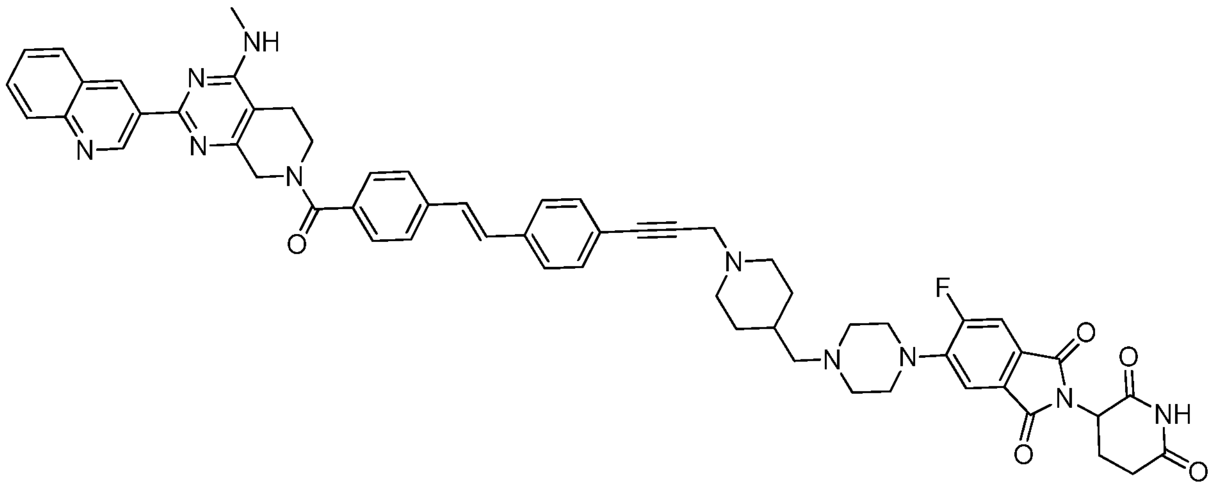,

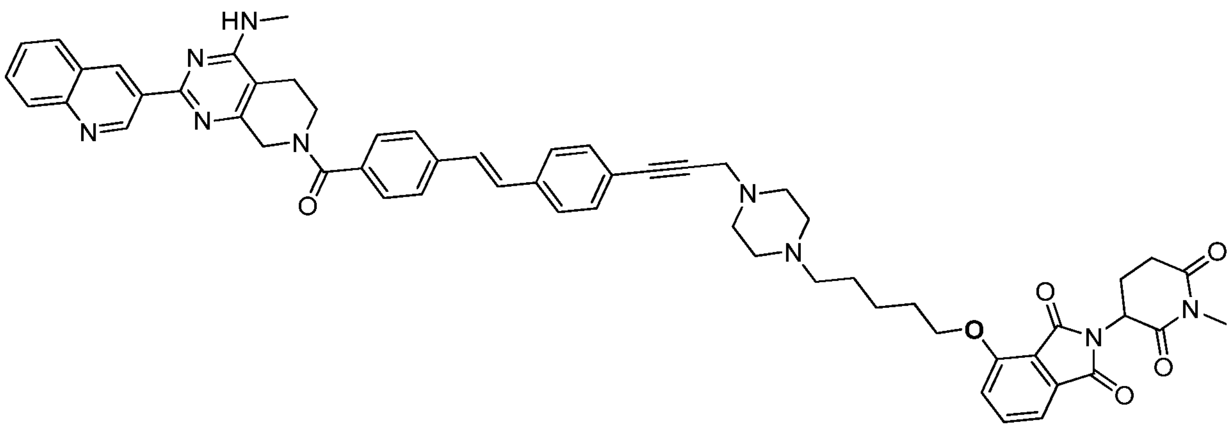,

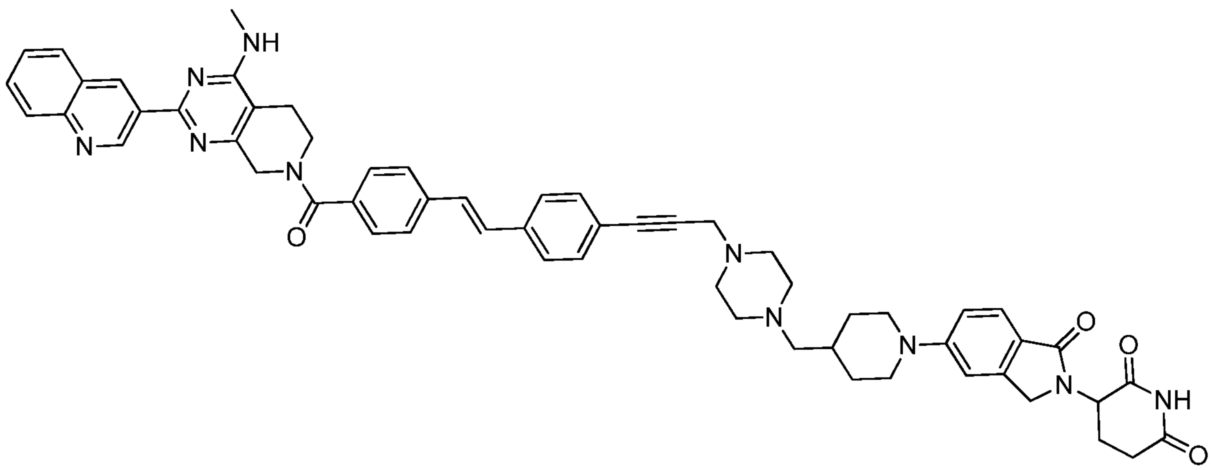,

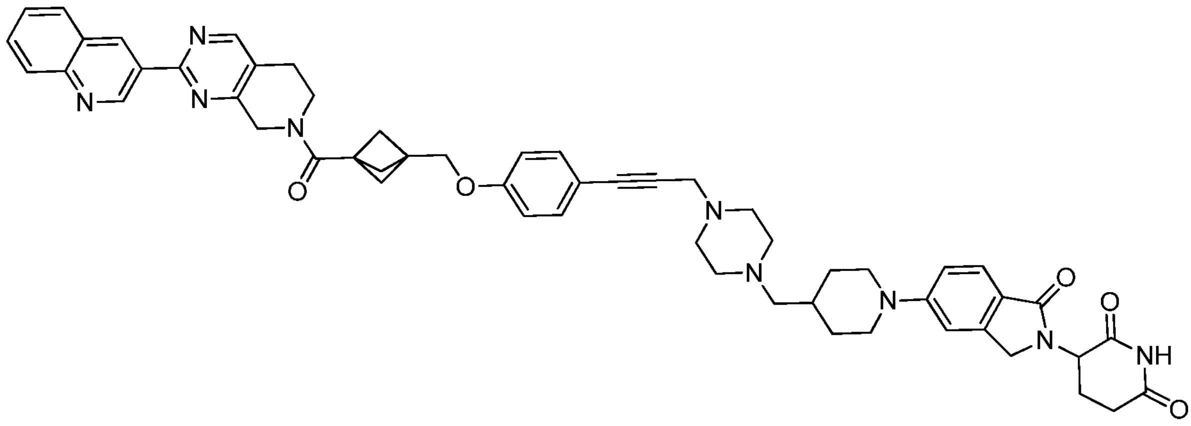
,

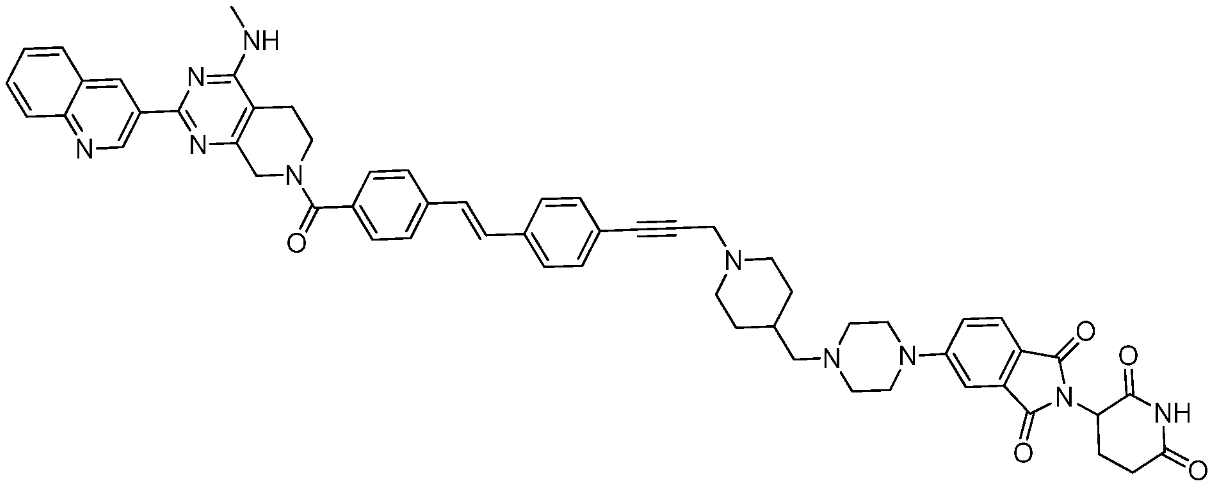
,

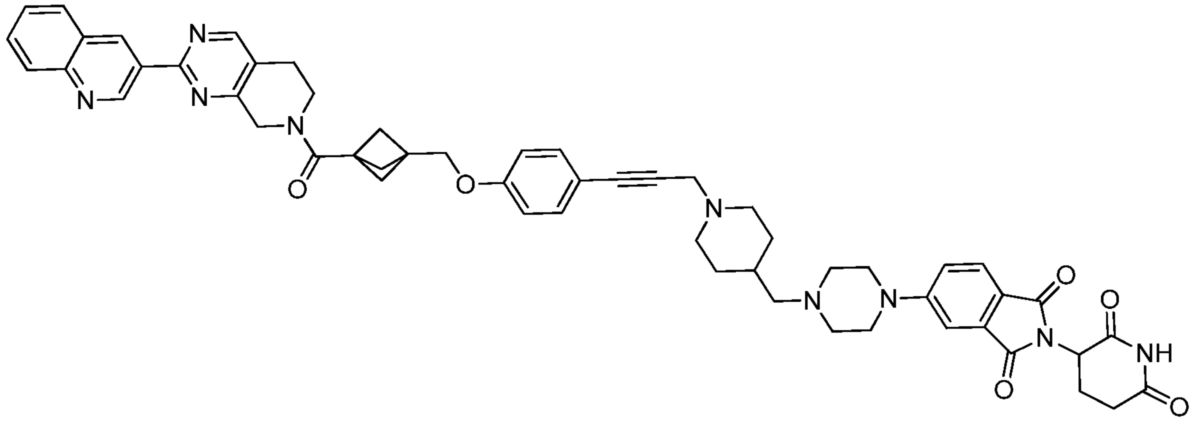
,

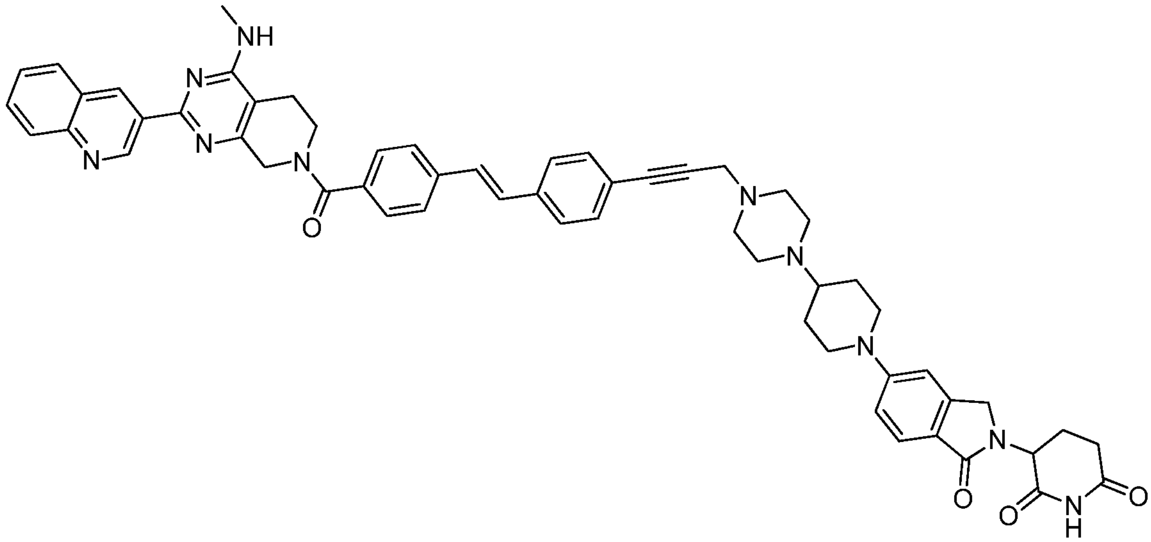

,

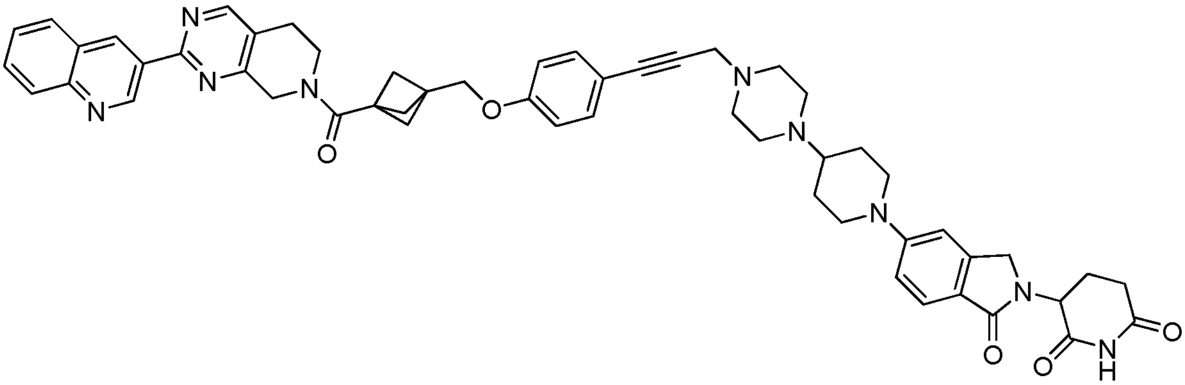

,

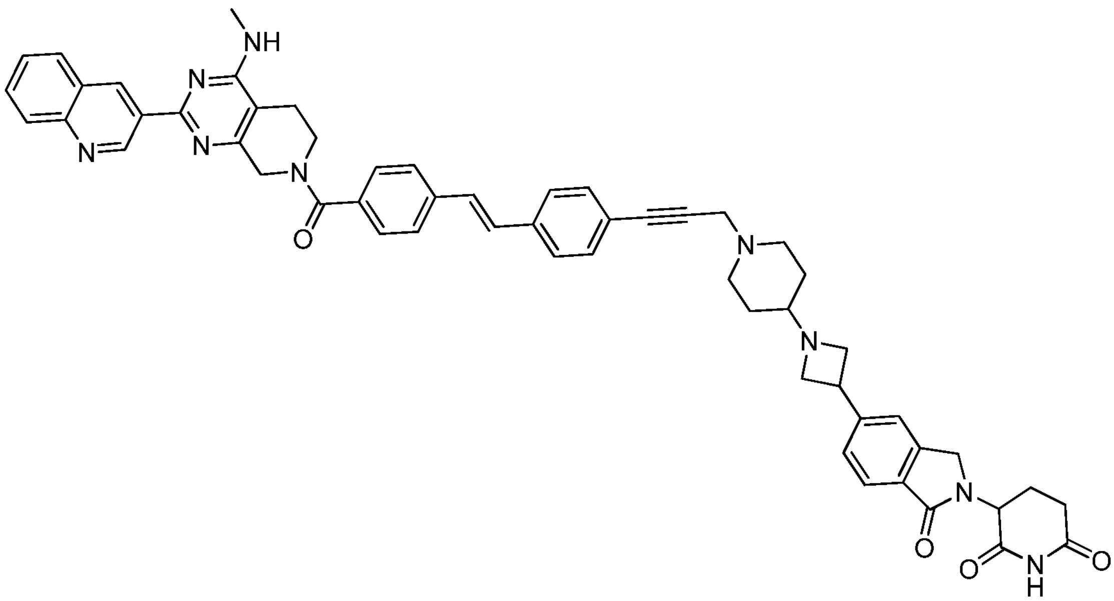

,

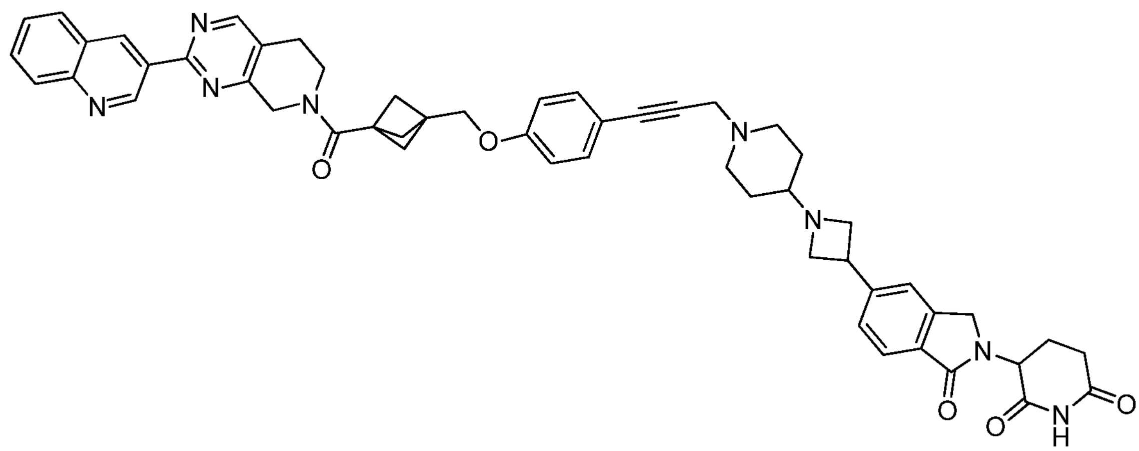

,

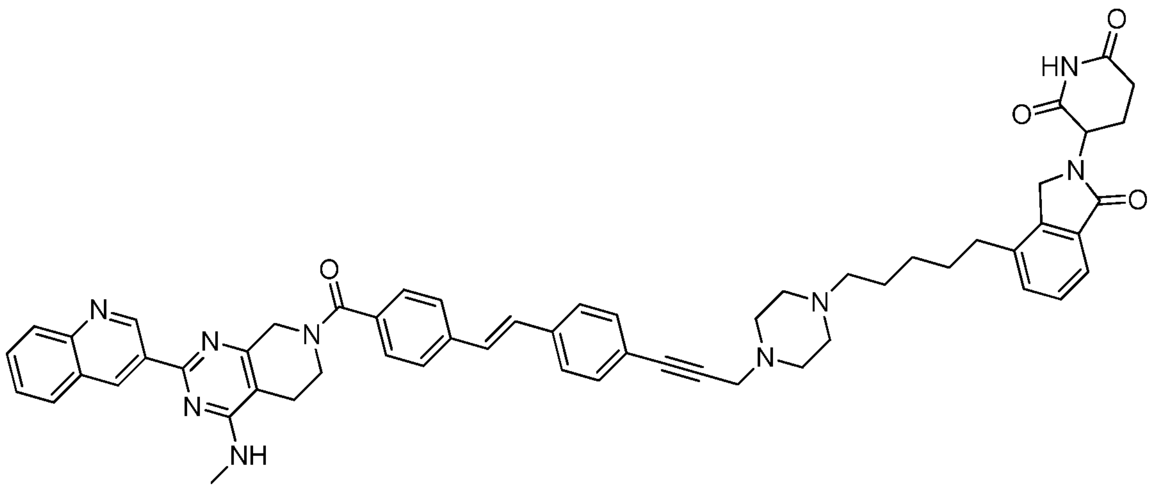

,

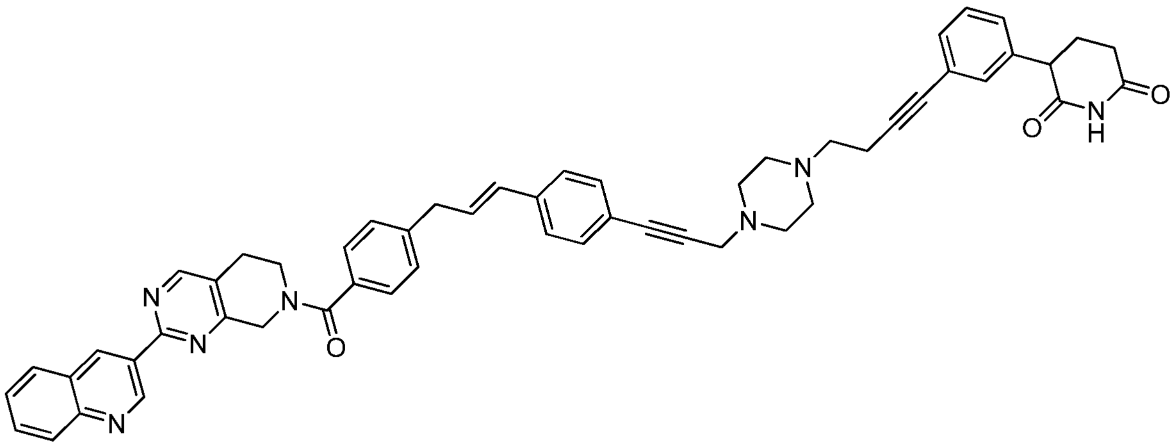

,

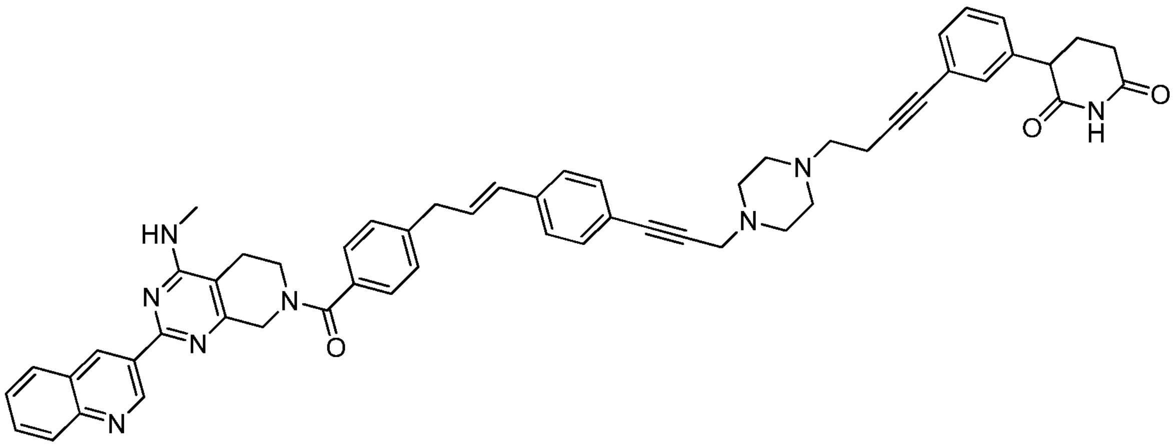

,

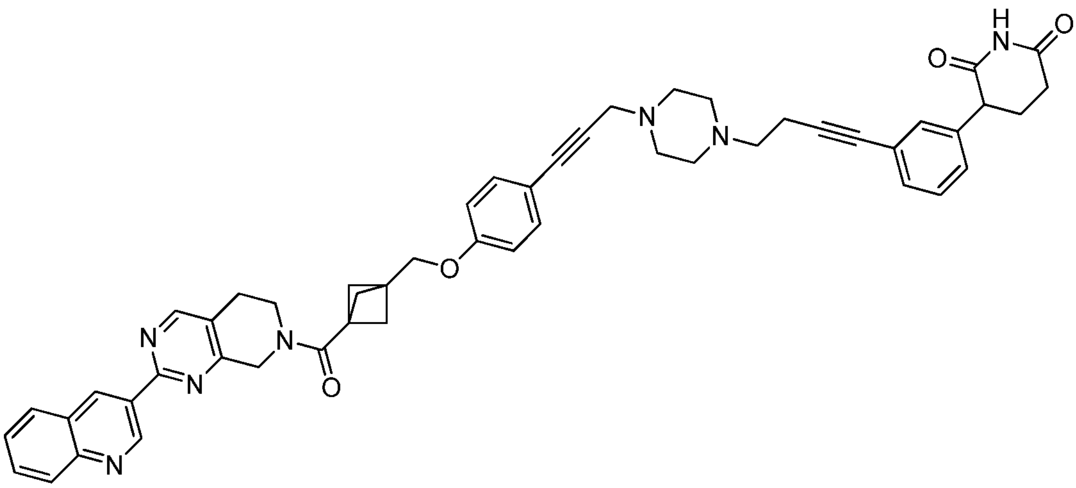

,

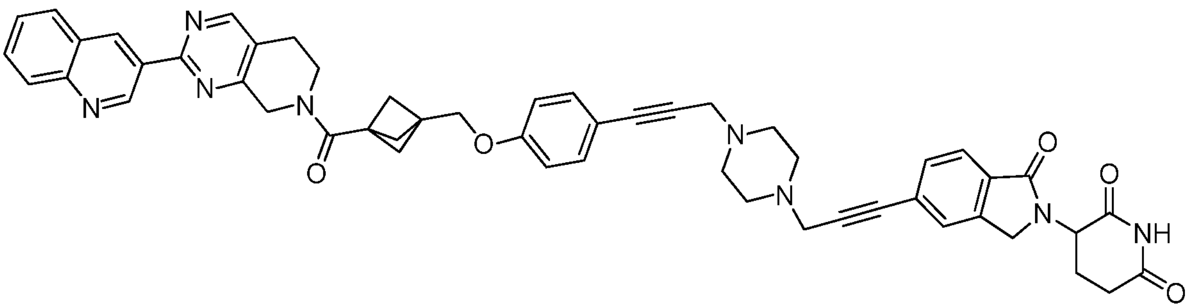

,

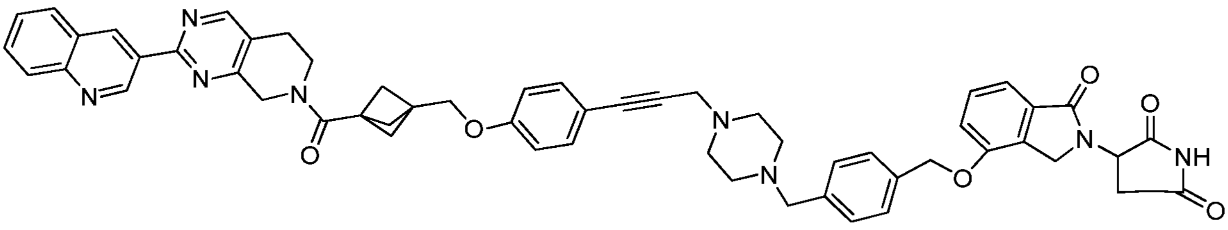

,

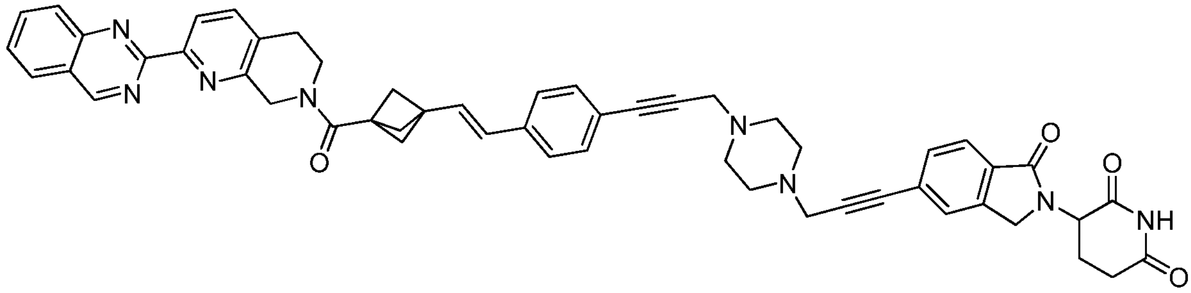,

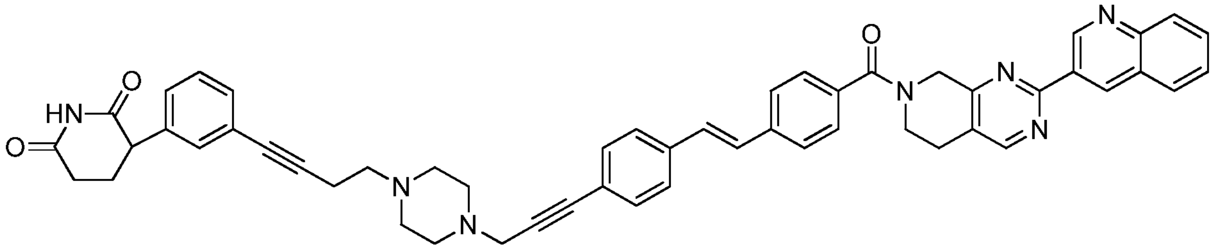,

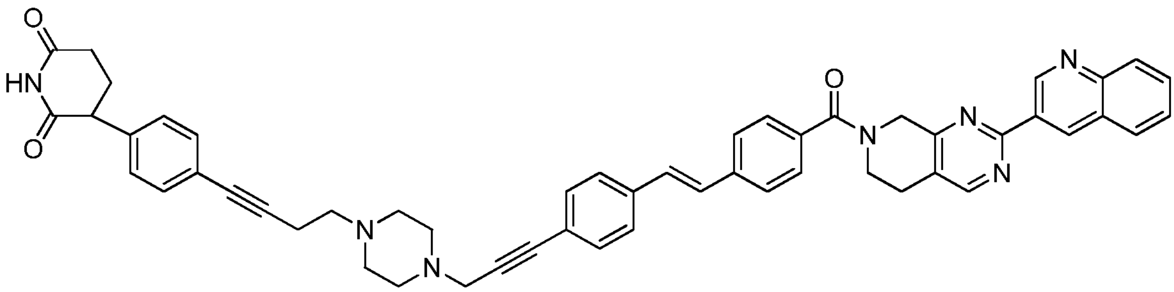,

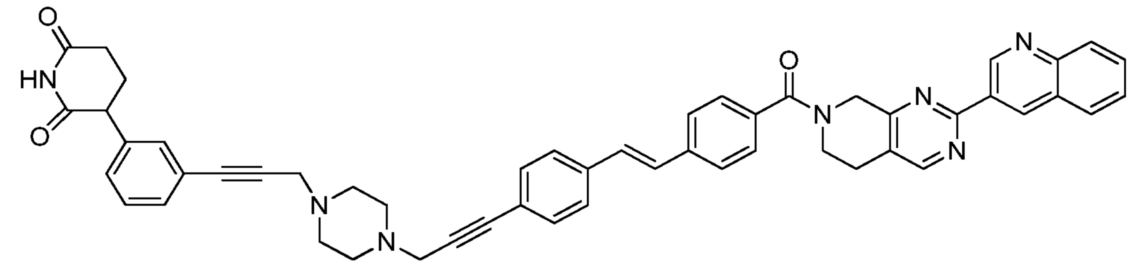,

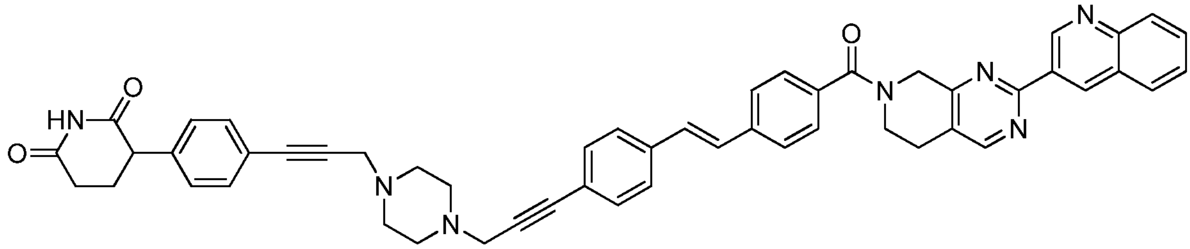,

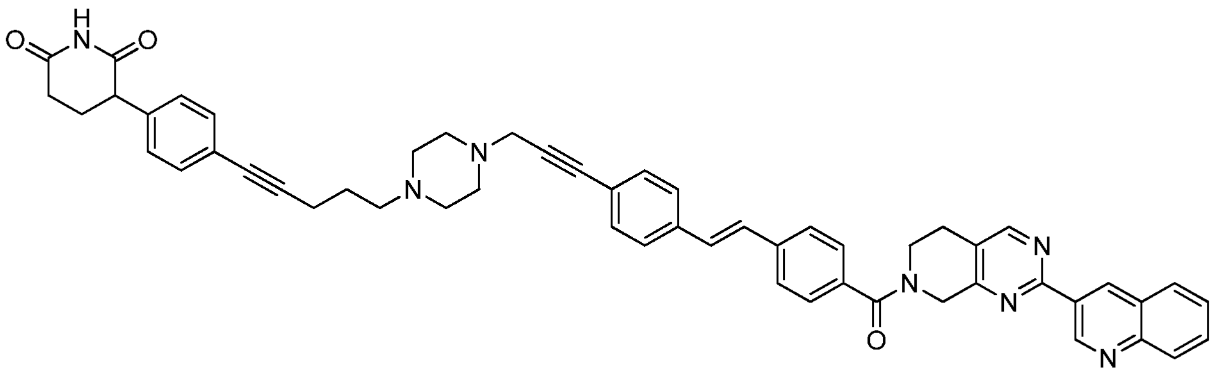

,

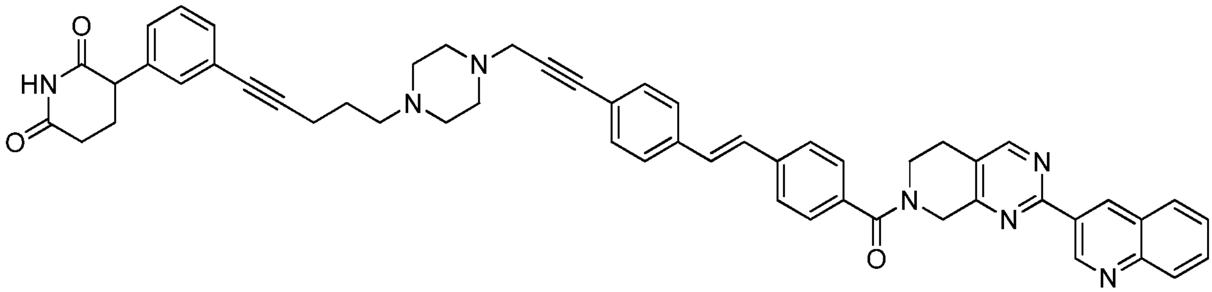

,

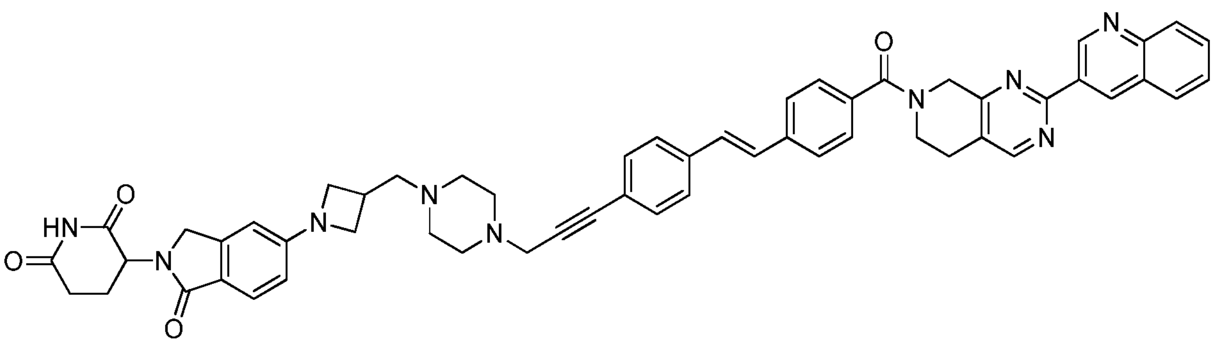

,

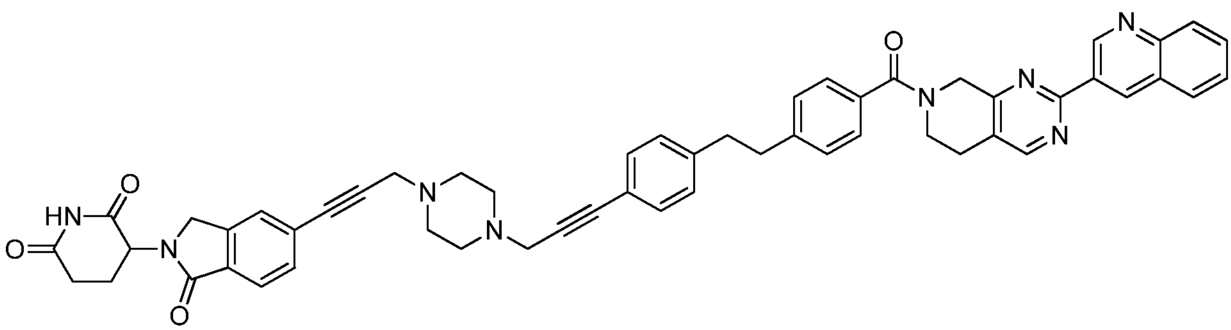

,

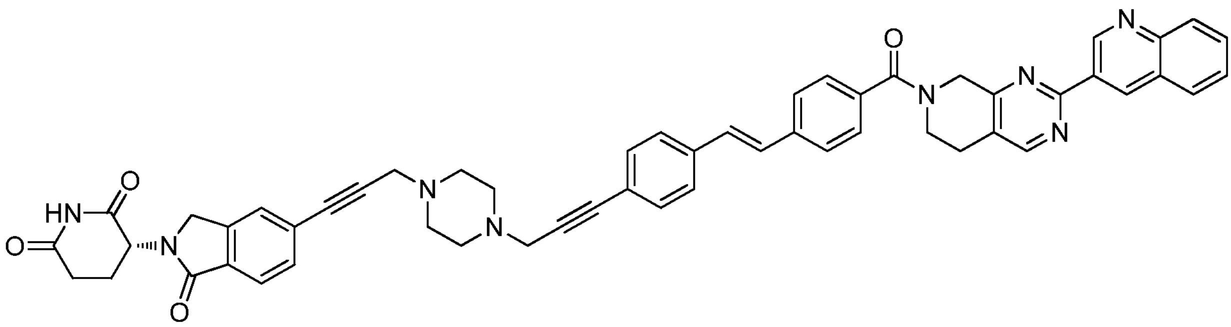

,

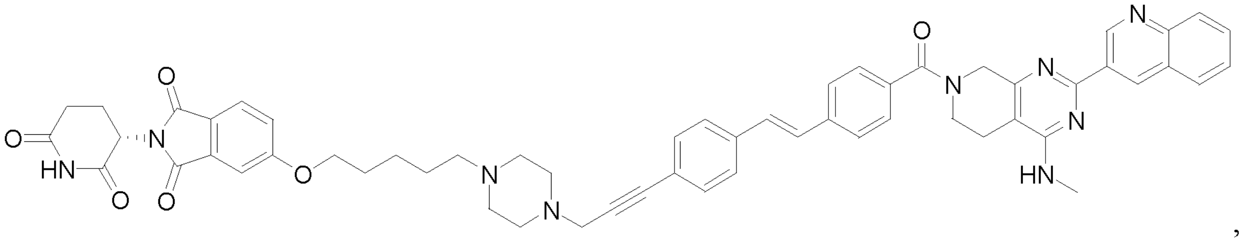

,

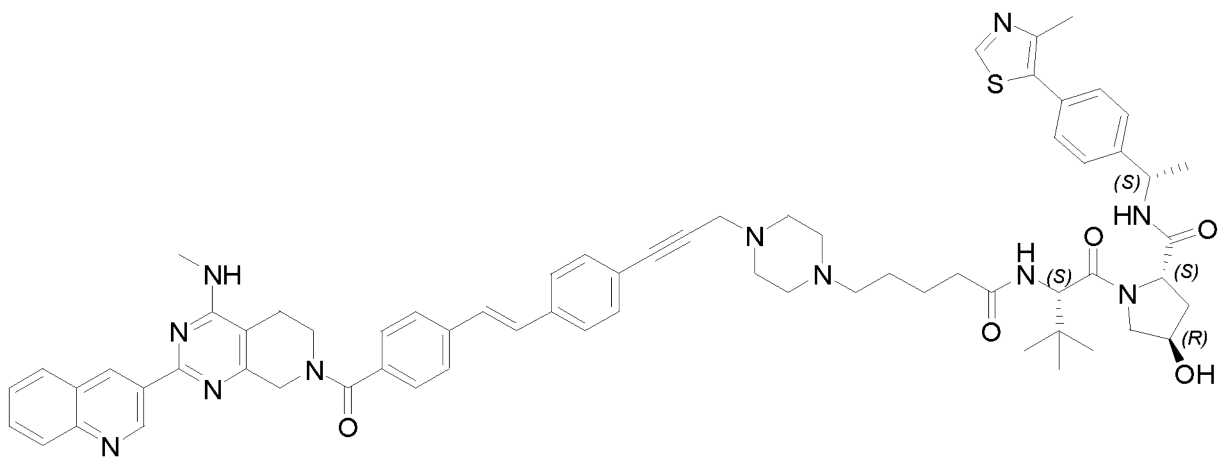

,

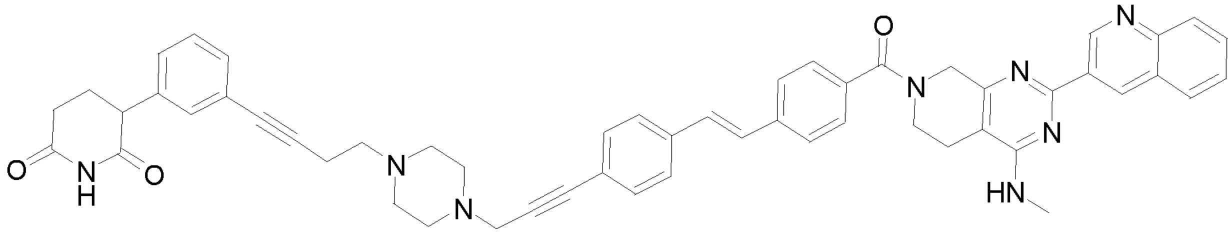

,

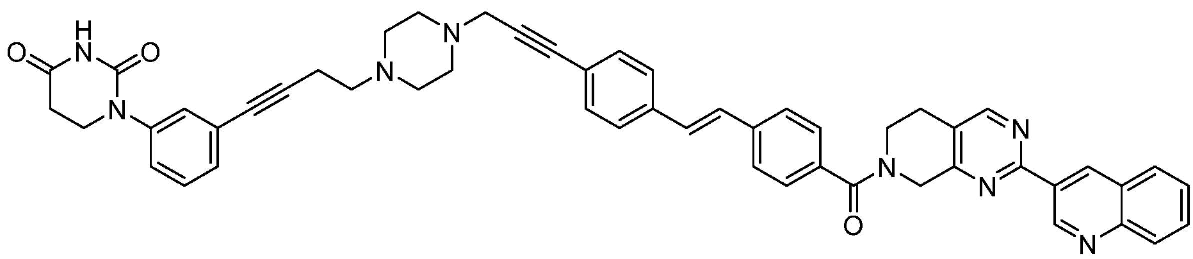

,

or

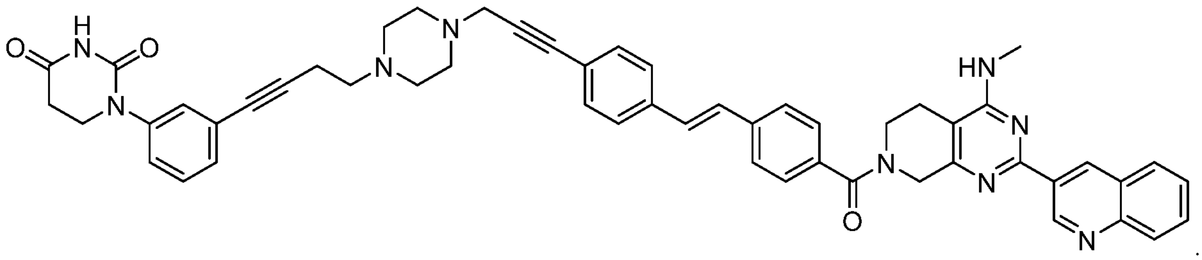

According to some embodiments of the present invention, the compound of Formula I may also be optionally and independently substituted by one or more (for example, 2, 3, 4, 5, 6, 7, 8, 9 or 10) $R^{YL'}$.

According to some embodiments of the present invention, each $R^{YL'}$ is independently selected from the group consisting of halogen, cyano, nitro, $C_{1\sim6}$ alkyl, halogen-substituted $C_{1\sim6}$ alkyl, - OR', and -N(R')$_2$; each R' is independently selected from the group consisting of hydrogen, halogen, -$C_{1\sim6}$ alkyl, halogen-substituted -$C_{1\sim6}$ alkyl, -$C_{0\sim2}$ alkylene-($C_{3\sim10}$ carbocyclic group), and -$C_{0\sim2}$ alkylene-(3- to 10-membered heterocycloalkyl).

According to some embodiments of the present invention, each $R^{YL'}$ is independently selected from the group consisting of halogen, cyano, nitro, $C_{1\sim6}$ alkyl, halogen-substituted $C_{1\sim6}$ alkyl, - OR', and -N(R')$_2$; each R' is independently selected from the group consisting of hydrogen, halogen, -$C_{1\sim6}$ alkyl, halogen-substituted -$C_{1\sim6}$ alkyl, -$C_{0\sim2}$ alkylene-($C_{3\sim8}$ carbocyclic group), and -$C_{0\sim2}$ alkylene-(3- to 8-membered heterocycloalkyl).

According to some embodiments of the present invention, each $R^{YL'}$ is independently selected from the group consisting of halogen, cyano, nitro, $C_{1\sim3}$ alkyl, halogen-substituted $C_{1\sim3}$ alkyl, -OR' and -N(R')$_2$; each R' is independently selected from the group consisting of hydrogen, halogen, - $C_{1\sim3}$ alkyl, halogen-substituted -$C_{1\sim3}$ alkyl, -$C_{0\sim2}$ alkylene-($C_{3\sim6}$ carbocyclic group) and -$C_{0\sim2}$ alkylene-(3- to 6-membered heterocycloalkyl).

The present invention also comprises technical solutions formed by any combination of various groups as described above in the present application, such as compounds of Formula I composed of X, Y and Z as described above, compounds of Formula I comprising X and Y as described above, compounds of Formula I comprising X and Z as described above, and compounds of Formula I comprising Y and Z as described above.

The present invention also provides a pharmaceutical composition, comprising a preparation made from any of the above-mentioned compounds, or deuterated compounds thereof, or stereoisomers thereof, or tautomers thereof, or polymorphs thereof, or solvates thereof, or N-oxides thereof, or isotopically labeled compounds thereof, or metabolites thereof, or prodrugs thereof, or pharmaceutically acceptable salts thereof.

The above pharmaceutical composition further comprises a pharmaceutically acceptable carrier, excipient, or vehicle.

The present invention also provides a use of any of the above-mentioned compounds, or deuterated compounds thereof, or stereoisomers thereof, or tautomers thereof, or polymorphs thereof, or solvates thereof, or N-oxides thereof, or isotopically labeled compounds thereof, or metabolites thereof, or prodrugs thereof, or pharmaceutically acceptable salts thereof, or the pharmaceutical composition in the preparation of a medicament for preventing and/or treating a disease related to BCL-XI, activity.

The present invention also provides a use of any of the above-mentioned compounds, or deuterated compounds thereof, or stereoisomers thereof, or tautomers thereof, or polymorphs thereof, or solvates thereof, or N-oxides thereof, or isotopically labeled compounds thereof, or metabolites thereof, or prodrugs thereof, or pharmaceutically acceptable salts thereof, or the pharmaceutical composition in the preparation of a medicament for preventing and/or treating a cancer.

The present invention also provides any of the above-mentioned compounds, or deuterated compounds thereof, or stereoisomers thereof, or tautomers thereof, or polymorphs thereof, or solvates thereof, or N-oxides thereof, or isotopically labeled compounds thereof, or metabolites thereof, or prodrugs thereof, or pharmaceutically acceptable salts thereof, or the pharmaceutical composition for use in preventing and/or treating a disease associated with BCL-XI, activity.

In some embodiments, the above-mentioned disease associated with BCL-XI, activity is autoimmune disease, bladder cancer, brain cancer, breast cancer, bone marrow cancer, cervical cancer, colorectal cancer, esophageal cancer, hepatocellular carcinoma, follicular lymphoma, lymphatic malignancy of T cell or B cell origin, melanoma, myeloma, oral cancer, ovarian cancer, non-small cell lung cancer, prostate cancer, leukemia, small cell lung cancer or spleen cancer; the leukemia is preferably chronic lymphocytic leukemia, lymphoblastic leukemia, or granulocytic leukemia.

The present invention also provides any of the above-mentioned compounds, or deuterated compounds thereof, or stereoisomers thereof, or tautomers thereof, or polymorphs thereof, or solvates thereof, or N-oxides thereof, or isotopically labeled compounds thereof, or metabolites thereof, or prodrugs thereof, or pharmaceutically acceptable salts thereof, or the pharmaceutical composition for use in preventing and/or treating a cancer.

A method for preventing and/or treating a disease associated with BCL-XI, activity, which comprises administering an effective amount of any of the above compounds, or deuterated compounds thereof, or stereoisomers thereof, or tautomers thereof, or polymorphs thereof, or solvates thereof, or N-oxides thereof, or isotopically labeled compounds thereof, or metabolites thereof, or prodrugs thereof, or pharmaceutically acceptable salts thereof, or the pharmaceutical composition to a subject in need thereof.

In some embodiments, the above-mentioned disease associated with BCL-XI, activity is autoimmune disease, bladder cancer, brain cancer, breast cancer, bone marrow cancer, cervical cancer, colorectal cancer, esophageal cancer, hepatocellular carcinoma, follicular lymphoma, lymphatic malignancy of T cell or B cell origin, melanoma, myeloma, oral cancer, ovarian cancer, non-small cell lung cancer, prostate cancer, small cell lung cancer or spleen cancer; the leukemia is preferably chronic lymphocytic leukemia, lymphoblastic leukemia, or granulocytic leukemia.

A method for preventing and/or treating a cancer, which comprises administering an effective amount of any of the above compounds, or deuterated compounds thereof, or stereoisomers thereof, or tautomers thereof, or polymorphs thereof, or solvates thereof, or N-oxides thereof, or isotopically labeled compounds thereof, or metabolites thereof, or prodrugs thereof, or pharmaceutically acceptable salts thereof, or the pharmaceutical composition to a subject in need thereof.

The compounds and derivatives provided in the present invention may be named according to the nomenclature systems of IUPAC (International Union of Pure and Applied Chemistry) or CAS (Chemical Abstracts Service, Columbus, OH).

Definitions of terms used in the present invention: Unless otherwise stated, the initial definition provided for a group or term herein applies to the group or term in the entire description; for terms that are not specifically defined herein, their meanings should be that the skilled in the art can give them based on the invention content and context.

"Substitution" means that a hydrogen atom in a molecule is replaced by other different atom or group; or a lone pair of electrons of an atom in a molecule is replaced by other atom or group. For example, the lone pair of electrons on S atom can be replaced by O atom to form

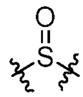

or

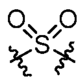.

"Can be further substituted" means that "substitution" may but does not have to occur, and this description includes whether it occurs or not.

The minimum and maximum content of carbon atoms in a hydrocarbon group is indicated by a prefix, for example, the prefix $C_{a\sim b}$ alkyl refers to any alkyl containing "a" to "b" carbon atoms. Thus, for example, $C_{1\sim 6}$ alkyl refers to alkyl containing 1 to 6 carbon atoms.

"Alkyl" refers to a saturated hydrocarbon chain having the specified number of member atoms. Alkyl groups can be straight or branched. Representative branched alkyl groups have one, two or three branches. Alkyl groups may be optionally substituted with one or more substituents as defined herein. Alkyl groups include methyl, ethyl, propyl (n-propyl and isopropyl), butyl (n-butyl, isobutyl and tert-butyl), pentyl (n-pentyl, isopentyl and neopentyl) and hexyl. Alkyl group can also be a part of other groups, such as -O($C_{1\text{-}6}$ alkyl).

"Alkylene" refers to a divalent saturated aliphatic hydrocarbon group having the specified number of member atoms. $C_{a\sim b}$ alkylene refers to an alkylene group having a to b carbon atoms. Alkylene groups include branched and straight chain hydrocarbyl groups. For example, the term "propylene" can be exemplified by the following structure: 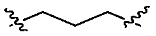. Likewise, the term "dimethylbutylene" may be exemplified, for example, by any of the following structures:

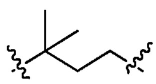 or 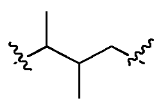.

The $C_{0\sim 4}$ alkylene group of the present invention can be $C_0$ alkylene group, $C_1$ alkylene group (e.g., $-CH_2-$), $C_2$ alkylene group (e.g., $-CH_2CH_2-$, etc.), $C_3$ alkylene group or $C_4$ alkylene group; $C_0$ alkylene means that the group

here does not exist and is connected in the form of a chemical bond. For example, A-$C_0$ alkylene-B means A-B, that is, the group A and the group B are directly connected through a chemical bond.

The term "carbocyclic group" in the present invention refers to a saturated or non-aromatic partially saturated cyclic group having a single ring or multiple rings (fused, bridged, spiro) and multiple carbon atoms, but having no ring heteroatoms. The term "carbocyclic group" includes cycloalkenyl groups such as cyclohexenyl. Examples of monocarbocyclic groups include, for example, cyclopropyl, cyclobutyl, cyclohexyl, cyclopentyl, cyclooctyl, cyclopentenyl and cyclohexenyl. Examples of carbocyclic groups of the fused carbocyclic system include bicyclohexyl, bicyclopentyl, bicyclooctyl, etc. Two such bicycloalkyl polycyclic structures are exemplified and named below:

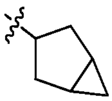

bicyclohexyl and

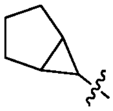

bicyclohexyl. Examples of carbocyclic groups of the bridged carbocyclic system include

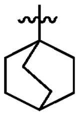, 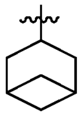,

adamantyl, and the like. Examples of carbocyclic groups of the spirocarbocyclic system include

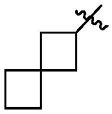, 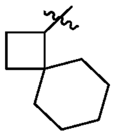,

and the like. The term "carbocyclic group" also comprises the case of partially saturated cyclic group formed by the fusion of an aromatic ring and a non-aromatic ring, and the connection site can be located at a non-aromatic carbon atom or an aromatic carbon atom. Examples include 1,2,3,4-tetrahydronaphthalen-5-yl, and 5,6,7,8-tetrahydronaphthalen-5-yl.

"Cycloalkane" as used in the present invention refers to a saturated or non-aromatic partially saturated divalent cyclic alkane having a single ring or multiple rings (fused) and multiple carbon atoms, but having no ring heteroatoms. The term "cycloalkane" includes cyclic olefins such as cyclohexene. Examples of monocarbocyclic groups include, for example, cyclopropane, cyclobutane, cyclohexane, cyclopentane, cyclooctane, cyclopentene, cyclohexene, and the like. Examples of the fused cycloalkane system include bicyclohexane, bicyclopentane, bicyclooctane, and the like. The "cycloalkane" mentioned in the present invention includes, but is not limited to,

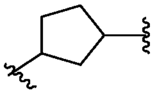, or 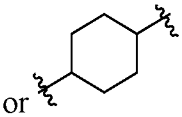 or 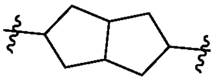.

The "heterocycloalkane" mentioned in the present invention refers to a saturated ring or a non-aromatic partially saturated divalent ring with a single ring or multiple rings (fused) containing at least one heteroatom; wherein the heteroatom refers to nitrogen atom, oxygen atom, sulfur atom, etc. Examples of heterocycloalkanes of monoheterocycloalkane systems are oxetane, azetidine, pyrrolidine, 2-oxo-pyrrolidine, tetrahydrofuran, tetrahydro-thiophene, pyrazolidine, imidazolidine, thiazolidine, piperidine, tetrahydropyran, tetrahydrothiopyran, piperazine, morpholine, thiomorpholine,

1,1-dioxo-thiomorpholine, azepane, diazepane, etc. Examples of fused heterocycloalkane systems include 8-aza-bicyclo[3.2.1]octane, quinuclidine, 8-oxa-3-aza-bicyclo[3.2.1]octane, 9-aza-bicyclo[3.3.1]nonane, etc. The term "heterocycloalkane" also includes the case where an aromatic ring is fused with a non-aromatic ring to form a partially saturated ring containing at least one heteroatom, in which the attachment point may be located at a non-aromatic carbon atom, an aromatic carbon atom or a heteroatom. The "heterocycloalkanes" mentioned in the present invention include, but are not limited to,

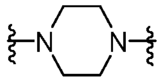, 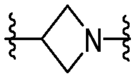, 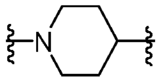,

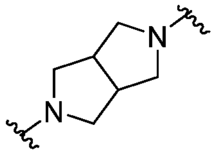,

etc.

The "spiro ring" mentioned in the present invention refers to a saturated or non-aromatic partially saturated bivalent ring formed by linking two or more rings having multiple carbon atoms and no ring heteroatom in the manner of spiro compound. Examples of spiro ring systems include

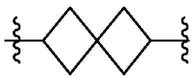, 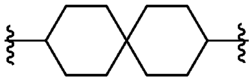,

etc.

The "spiro heterocycle" mentioned in the present invention refers to a saturated ring or a non-aromatic partially saturated bivalent ring formed by linking two or more rings containing at least one heteroatom in the manner of spiro compound; and examples of spiro heterocyclic systems include

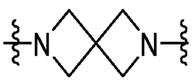, 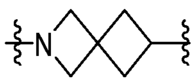, 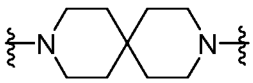,

etc. The "bridged ring" mentioned in the present invention refers to a saturated or non-aromatic partially saturated bivalent ring formed by bridging multiple rings having multiple carbon atoms and no ring heteroatom. Examples of bridge-ring systems include

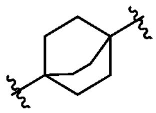, 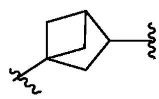,

etc.

The "bridged heterocycle" mentioned in the present invention refers to a saturated ring or a non-aromatic partially saturated divalent ring formed by bridging multiple rings containing at least one heteroatom; examples of bridged heterocyclic systems include

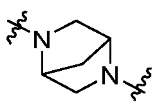, 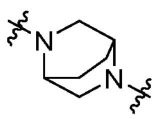,

etc.

The term "unsaturated" in the present invention refers to that a group or molecule contains a carbon-carbon

double bond, carbon-carbon triple bond, carbon-oxygen double bond, carbon-sulfur double bond, carbon-nitrogen triple bond, etc.

The "alkenyl" mentioned in the present invention refers to a straight-chain or branched-chain hydrocarbon group with at least one vinyl unsaturated site (>C=C<). For example, $C_{a-b}$ alkenyl refers to an alkenyl group having a to b carbon atoms and is intended to include, for example, vinyl, propenyl, isopropenyl, 1,3-butadienyl, and the like.

The "alkenylene" mentioned in the present invention refers to a straight-chain or branched divalent carbon chain having at least one carbon-carbon double bond. $C_{a-b}$ alkenylene refers to an alkenylene group having a to b carbon atoms.

The "alkynyl" mentioned in the present invention refers to a straight-chain monovalent hydrocarbon group or a branched chain monovalent hydrocarbon group containing at least one triple bond. The term "alkynyl" is also intended to include those hydrocarbyl groups having one triple bond and one double bond. For example, $C_{2-6}$ alkynyl is intended to include ethynyl, propynyl, and the like.

The "alkynylene" mentioned in the present invention refers to a straight-chain divalent hydrocarbon group or a branched chain divalent hydrocarbon group containing at least one triple bond. $C_{a-b}$ alkynylene refers to an alkynylene group having a to b carbon atoms.

The "heterocycloalkyl" mentioned in the present invention refers to a saturated ring or a non-aromatic partially saturated ring with a single ring or multiple rings (fused, bridged, spiro) containing at least one heteroatom; wherein heteroatom refers to nitrogen atom, oxygen atom, sulfur atom, etc. It usually represents a monovalent saturated or partially unsaturated monocyclic or polycyclic ring system with multiple ring atoms, which contains 1, 2 or 3 ring heteroatoms selected from N, O and S, and the remaining ring atoms are carbon atoms. Examples of heterocycloalkyl of monoheterocycloalkyl system are oxetanyl, azetidinyl, pyrrolidinyl, 2-oxopyrrolidin-3-yl, tetrahydrofuranyl, tetrahydrothienyl, pyrazolidinyl, imidazolidinyl, thiazolidinyl, piperidinyl, tetrahydropyranyl, tetrahydrothiopyranyl, piperazineheptyl, etc. Examples of heterocycloalkyl of fused heterocycloalkyl system include 8-aza-bicyclo[3.2.1]octyl, quinuclidinyl, 8-oxa-3-aza-bicyclo[3.2.1]octyl, 9-aza-bicyclo[3.3.1]nonyl, etc. Examples of heterocycloalkyl of bridged heterocycloalkyl system include

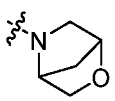 , 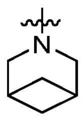 , 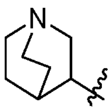 ,

and the like. Examples of heterocycloalkyl of spiroheterocycloalkyl system include

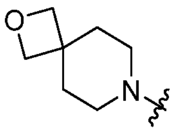 ,

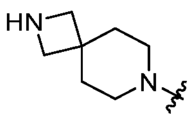 ,

etc. Examples of partially saturated heterocycloalkyl are dihydrofuryl, imidazolinyl, tetrahydropyridyl or dihydropyranyl, and the like. The term "heterocycloalkyl" also includes the case where an aromatic ring is fused with a non-aromatic ring to form a partially saturated cyclic group containing at least one heteroatom, in which the attachment point can be located at a non-aromatic carbon atom, an aromatic carbon atom or a heteroatom; and examples include

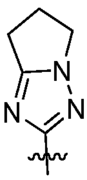 , 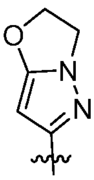 , 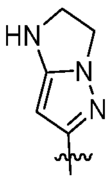 .

The "aromatic ring" mentioned in the present invention refers to an aromatic hydrocarbon group having multiple carbon atoms. Aryl usually refers to a monocyclic, bicyclic or tricyclic aryl group having multiple carbon atoms. Furthermore, the term "aryl" as used herein refers to an aromatic substituent that may be a single aromatic ring, or multiple aromatic rings fused together. Non-limiting examples include phenyl, naphthyl or tetrahydronaphthyl.

The "aromatic heterocycle" mentioned in the present invention refers to an aromatic unsaturated ring containing at least one heteroatom; wherein the heteroatom refers to nitrogen atom, oxygen atom, sulfur atom, etc. Aromatic heterocycles generally include aromatic monocyclic or bicyclic hydrocarbons that contain multiple ring atoms, one or more of which are heteroatoms selected from O, N, and S. Preferably, there are one to three heteroatoms. Heterocyclic aryl represents, for example: pyridyl, indolyl, quinoxalinyl, quinolyl, isoquinolinyl, benzothienyl, benzofuranyl, benzothienyl, benzothienyl, benzopyranyl, benzothiopyranyl, furyl, pyrrolyl, thiazolyl, oxazolyl, isoxazolyl, triazolyl, tetrazolyl, pyrazolyl, imidazolyl, thienyl, oxadiazolyl, benzimidazolyl, benzothiazolyl, benzoxazolyl.

The "halogen" mentioned in the present invention refers to fluorine, chlorine, bromine or iodine.

The "halogen-substituted alkyl" mentioned in the present invention means that one or more hydrogen atoms in alkyl are substituted by halogen; for example, "halogen-substituted $C_{1-4}$ alkyl", which is an alkyl containing 1 to 4 carbon atoms in which the hydrogen atoms are substituted by one or more halogen atoms; for further example, monofluoromethyl, bisfluoromethyl, and trifluoromethyl.

The "halogen-substituted alkenyl" mentioned in the present invention means that one or more hydrogen atoms in alkenyl are substituted by halogen; for example, "halogen-substituted $C_{2-6}$ alkenyl", which is an alkenyl containing 2 to 6 carbon atoms in which the hydrogen atoms are substituted by one or more halogen atoms; for further example, monofluorovinyl, bifluorovinyl, and trifluoropropenyl.

The "halogen-substituted alkynyl" mentioned in the present invention means that one or more hydrogen atoms in alkynyl are substituted by halogen; for example, "halogen-substituted $C_{2-6}$ alkynyl", which is an alkynyl containing 2 to 6 carbon atoms in which the hydrogen atoms are substituted by one or more halogen atoms; for further example, monofluoroethynyl, bisfluoroethynyl, and trifluoropropynyl.

In the present invention, "-OR", "-N(R)$_2$" and the like mean that the R group is connected to the oxygen atom or the nitrogen atom via a single bond.

In the present invention, "=O" means that the oxygen atom replaces two hydrogen atoms in a molecule through a double bond.

In the present invention, "-C(O)R", "-S(O)$_2$R" and the like mean that the oxygen atom is connected with the carbon atom or the sulfur atom via a double bond, and the R group is connected with the oxygen atom or the sulfur atom via a single bond. For another example, "-S(O)(NH)R" means that the oxygen atom and the nitrogen atom are connected to the sulfur atom via double bonds, and the R group is connected to the sulfur atom via a single bond.

In the present invention, "- - -" and

" 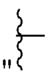 "

in a group are used to describe a position substituted by the group.

The "deuterated compound" of the present invention means that one or more hydrogen atoms in a molecule or group are replaced by deuterium atoms, in which the proportion of deuterium atoms is greater than the abundance of deuterium in nature.

The term "pharmaceutically acceptable" means that carrier, vehicle, diluent, excipient, and/or formed salt is generally chemically or physically compatible with the other ingredients constituting a pharmaceutical dosage form, and is physiologically compatible with a receptor.

The terms "salt" and "pharmaceutically acceptable salt" refer to an acidic and/or basic salt formed between the above-mentioned compound or stereoisomer thereof and an inorganic and/or organic acid and base, and also include zwitterionic salt (inner salt), also include quaternary ammonium salt, such as alkylammonium salt. These salts can be obtained directly from the final isolation and purification of the compound. They can also be obtained by appropriately mixing the above compound or stereoisomer thereof with a certain amount of acid or base (e.g., with equivalent amount). These salts may form a precipitate in a solution and be collected by filtration, or may be recovered after evaporation of solvent, or may be obtained by reacting in an aqueous medium and then freeze-drying.

The term "prevention" comprises inhibiting and delaying the onset of a disease, and comprises not only prevention before the disease develops, but also prevention of its recurrence after treatment.

The term "treatment" means reversing, alleviating or eliminating a disorder or condition to which such term applies or the progression of one or more symptoms of such disorder or condition.

In certain embodiments, one or more compounds of the present invention may be used in combination with each other. The compounds of the present invention can also be optionally used in combination with any other active

agent to prepare drugs or pharmaceutical compositions for regulating cell function or treating diseases. If a group of compounds is used, the compounds can be administered to a subject simultaneously, separately, or sequentially.

Obviously, according to the above contents of the present invention, according to the common technical knowledge and common means in the field, without departing from the above basic technical idea of the present invention, various other forms of modifications, replacements or changes can also be made.

The above contents of the present invention will be further described in detail below through examples. However, this should not be understood to mean that the scope of the above subject matter of the present invention is limited to the following examples. All technologies implemented based on the above contents of the present invention belong to the scope of the present invention.

## Detailed Description of the Invention

The known starting materials of the present invention could be synthesized by methods known in the art, or could be purchased from companies such as Energy Chemical, Chengdu Kelong Chemical, Accela ChemBio Co., Ltd, J&K Scientific, etc.

When there was no special instructions in the examples, the reaction was carried out in nitrogen atmosphere. When there was no special explanation in the examples, the solution was an aqueous solution. When there was no special instructions in the examples, the reaction temperature was room temperature. The room temperature was the most suitable reaction temperature, which was 20°C to 30°C. When there was no special explanation in the examples, M referred to mol per liter.

The structure of compound was determined by nuclear magnetic resonance (NMR) and mass spectrometry (MS). NMR shifts (δ) were given in unit of $10^{-6}$ (ppm). NMR was measured using (Bruker AvanceIII 400 and Bruker Avance 600) nuclear magnetic instruments. The measurement solvents were deuterated dimethyl sulfoxide (DMSO-*d6*), deuterated chloroform ($CDCl_3$), and deuterated methanol (Methanol-*d4*), and the internal standard was tetramethylsilane (TMS). LC-MS measurement was performed using a Shimadzu liquid-chromatography-mass spectrometry instrument (Shimadzu LC-MS 2020 (ESI)). HPLC measurement was performed using a Shimadzu high-pressure liquid chromatograph (Shimadzu LC-20A). MPLC (medium-pressure preparative chromatography) was performed using a Gilson GX-281 reversed phase preparative chromatograph. Thin layer chromatography silica gel plates were Yantai Huanghai HSGF254 or Qingdao GF254 silica gel plates, and the specifications of thin layer chromatography for the separation and purification of products were 0.4 mm to 0.5 mm. Column chromatography generally used Yantai Huanghai Silica Gel 200~300 mesh silica gel as carrier.

$Pd(dppf)Cl_2$: [1,1'-bis(diphenylphosphino) ferrocene] palladium dichloride; TMSCl: trimethylchlorosilane; Xphos: 2-bicyclohexylphosphine-2',4',6'-triisopropylbiphenyl; $Pd_2(dba)_3$: tris(dibenzylideneacetone)dipalladium; EDCI: 1-ethyl-3-(3-dimethylaminopropyl)carbodiimide; HOBt: hydroxybenzotriazole; Found: MS value of product when measured by LC-MS.

**Synthesis of intermediates:** Synthesis of intermediate compounds $\mathbf{R_{1A\text{-}1}}$, $\mathbf{R_{1A\text{-}2}}$, $\mathbf{R_{1A\text{-}3}}$, $\mathbf{R_{1A\text{-}4}}$, and $\mathbf{R_{1A\text{-}5}}$

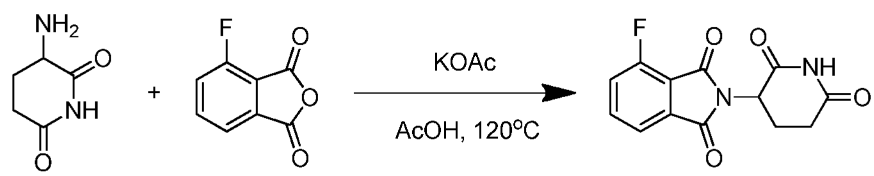


**1** **2a** $\mathbf{R_{1A\text{-}1}}$

Compound **1** (1.00 mmol), Compound **2a** (1.05 mmol), potassium acetate (1.20 mmol), and acetic acid (5 mL) were added to a 100 mL reaction bottle in sequence, heated to 120°C, stirred and reacted for 8 hours, and then water was added to quench the reaction (LC-MS monitoring). After extraction with saturated sodium chloride solution (25 mL) and ethyl acetate (3× 25 mL), the organic phases were combined, the combined organic phase was dried over anhydrous sodium sulfate, and concentrated under reduced pressure to remove the solvent to obtain Compound $\mathbf{R_{1A\text{-}1}}$. ($C_{13}H_{10}FN_2O_4$, $[M+H]^+$ 277.1; found 277.2, yield 89.8%).

Referring to the synthesis method of Compound $\mathbf{R_{1A\text{-}1}}$, the raw materials in the following table were used to replace Compound **2a,** and other raw materials and operating methods were kept unchanged to obtain intermediate compounds $\mathbf{R_{1A\text{-}2}}$, $\mathbf{R_{1A\text{-}3}}$, $\mathbf{R_{1A\text{-}4}}$, $\mathbf{R_{1A\text{-}5}}$, $\mathbf{R_{1A\text{-}6}}$.

| Compound No. | Structure and characterization data of compound | Raw material | Yield |
|---|---|---|---|
| $R_{1A-2}$ | 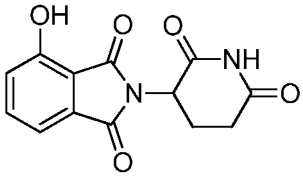<br>$R_{1A-2}$ | 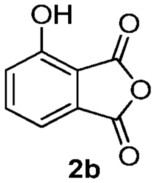<br>2b | 89.8% |
| | LC-MS: $C_{13}H_{11}N_2O_5$ [M+H]+ 275.1; found 275.2 | | |
| $R_{1A-3}$ | 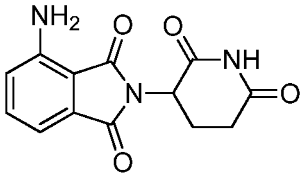<br>$R_{1A-3}$ | 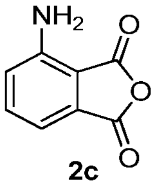<br>2c | 65.3% |
| | LC-MS: $C_{13}H_{12}N_3O_4$, [M+H]+ 274.1; found 274.3 | | |
| $R_{1A-4}$ | 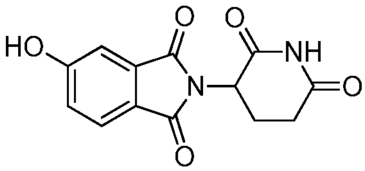<br>$R_{1A-4}$ | 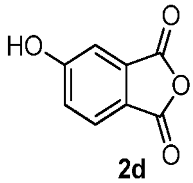<br>2d | 68.9% |
| | LC-MS: $C_{13}H_{11}N_2O_5$, [M+H]+ 275.1; found 275.2 | | |
| $R_{1A-5}$ | 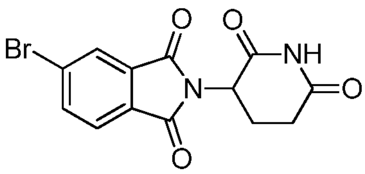<br>$R_{1A-5}$ | 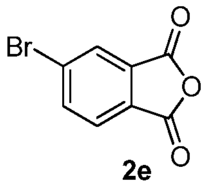<br>2e | 83.6% |
| | LC-MS: $C_{13}H_{10}BrN_2O_4$, $[M+H]^+$ 337.0; found 337.1 | | |
| $R_{1A-6}$ | 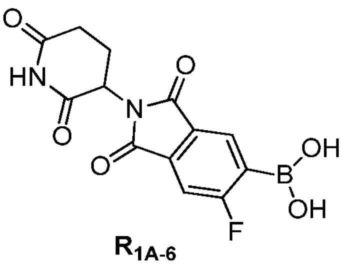<br>$R_{1A-6}$ | 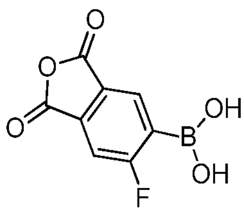<br>2f | 78.9% |
| | LC-MS: $C_{13}H_{11}BFN_2O_6$, $[M+H]^+$ 321.2; found 321.1 | | |

**Synthesis of intermediate:** Synthesis of intermediate $\mathbf{R_{1A-7}}$

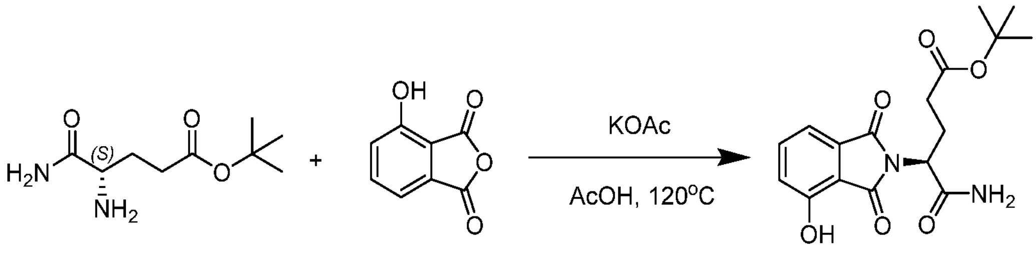


1S 2b $R_{1A-7}$

Compound **1S** (1.00 mmol), Compound **2b** (1.05 mmol), potassium acetate (1.20 mmol), and acetic acid (5 mL) were added to a 100 mL reaction bottle in sequence, heated to 120°C, stirred and reacted for 1 hour, and then water was added to quench the reaction (LC-MS monitoring). After extraction with saturated sodium chloride solution (25 mL) and ethyl acetate (3× 25 mL), the organic phases were combined, the combined organic phase was dried over anhydrous sodium sulfate, and concentrated under reduced pressure to remove the solvent to obtain Compound $\mathbf{R_{1A-7}}$. ($C_{17}H_{21}N_2O_6$, $[M+H]^+$ 349.4; found 349.4, yield 49.6%).

**Synthesis of intermediates:** Synthesis of intermediates $\mathbf{R_{1B-1}}$, $\mathbf{R_{1B-2}}$, $\mathbf{R_{1B-3}}$, $\mathbf{R_{1B-4}}$, $\mathbf{R_{1B-5}}$, $\mathbf{R_{1B-6}}$, $\mathbf{R_{1B-7}}$

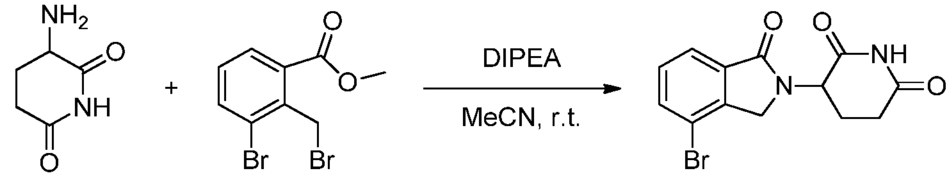


1 3a $R_{1B-1}$

Compound **1** (1.00 mmol), Compound **3a** (1.05 mmol), *N*,*N*-diisopropylethylamine (2.10 mmol), and acetonitrile (10 mL) were added in sequence to a 100 mL reaction bottle, reacted at room temperature for 8 hours, and then water was added to quench the reaction (LC-MS monitoring). After extraction with saturated sodium chloride solution (25 mL) and ethyl acetate (3× 25 mL), the organic phases were combined, the combined organic phase was dried over anhydrous sodium sulfate, and concentrated under reduced pressure to remove the solvent to obtain Compound $\mathbf{R_{1B-1}}$ ($C_{13}H_{12}BrN_2O_3$, $[M+H]^+$ 323.0; found 323.2, yield 90.3%).

Referring to the synthesis method of Compound $\mathbf{R_{1B-1}}$, the raw materials in the following table were used to replace Compound **3a,** and the other raw materials and operating methods remained unchanged, to obtain intermediate compounds $\mathbf{R_{1B-2}}$, $\mathbf{R_{1B-3}}$, $\mathbf{R_{1B-4}}$, $\mathbf{R_{1B-5}}$, $\mathbf{R_{1B-6}}$, $\mathbf{R_{1B-7}}$.

| Compound No. | Structure and characterization data of compound | Raw material | Yield |
|---|---|---|---|
| $R_{1B-2}$ | 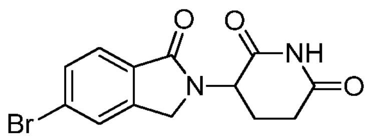 $R_{1B-2}$ | 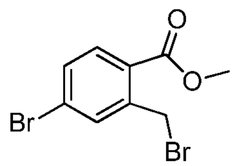 3b | 91.6% |
| | LC-MS: $C_{13}H_{12}BrN_2O_3$, $[M+H]^+$ 323.0; found 323.1 | | |

(continued)

| Compound No. | Structure and characterization data of compound | Raw material | Yield |
|---|---|---|---|
| $R_{1B-3}$ | 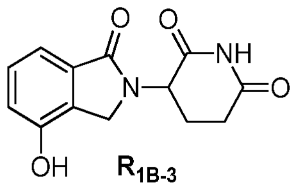 $R_{1B-3}$<br>LC-MS: $C_{13}H_{13}N_2O_4$, $[M+H]^+$ 261.1; found 261.3 | 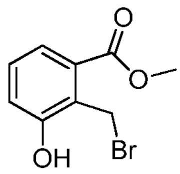<br>**3c** | 89.9% |
| $R_{1B-4}$ | 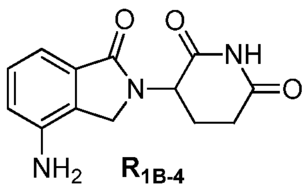 $R_{1B-4}$<br>LC-MS: $C_{13}H_{14}N_3O_3$, $[M+H]^+$ 260.1; found 260.2 | 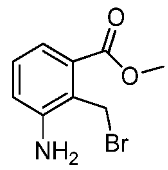<br>**3d** | 78.9% |
| $R_{1B-5}$ | 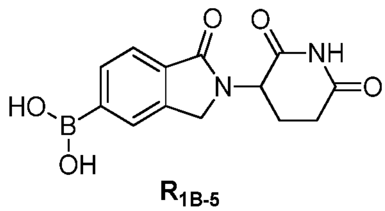<br>$R_{1B-5}$<br>LC-MS: $C_{13}H_{14}BN_2O_5$, $[M+H]^+$ 289.1; found 289.2 | 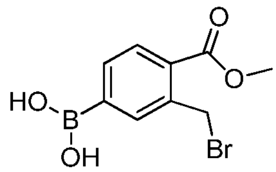<br>**3e** | 63.1% |
| $R_{1B-6}$ | 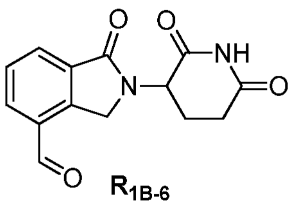 $R_{1B-6}$<br>LC-MS: $C_{14}H_{13}N_2O_4$, $[M+H]^+$ 273.1; found 273.3 | 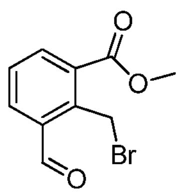<br>**3f** | 47.6% |
| $R_{1B-7}$ | 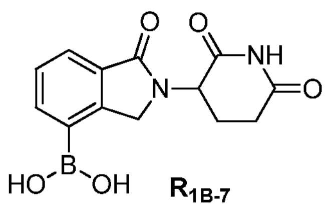 $R_{1B-7}$<br>LC-MS: $C_{13}H_{14}BN_2O_5$, $[M+H]^+$ 289.1; found 289.2 | 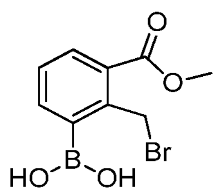<br>**3g** | 60.9% |

**Synthesis of intermediates**: Synthesis of intermediate compounds $R_{2-A1}$, $R_{2-A2}$, $R_{2-B1}$, $R_{2-B1}$

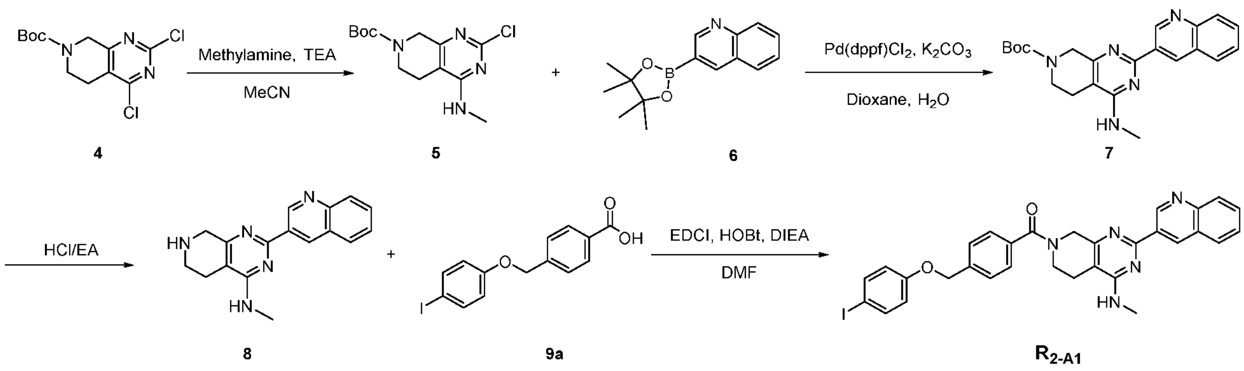


4 5 6 7

8 9a $R_{2\text{-}A1}$

**Step 1. Synthesis of Compound 5**

Compound **4** (1.00 mmol), methylamine (1.15 mmol), triethylamine (2 mL) and acetonitrile (10 mL) were added to a 50 mL reaction bottle, reacted at room temperature for 8 hours, then water was added to quench the reaction (LC-MS monitoring). After extraction with saturated sodium chloride solution (25 mL) and ethyl acetate (3× 25 mL), the organic phases were combined, the combined organic phase was dried over anhydrous sodium sulfate, and concentrated under reduced pressure to remove the solvent to obtain Compound 5 (LC-MS: $C_{13}H_{20}ClN_4O_2$, $[M+H]^+$ 299.1; found 299.3, crude product).

**Step 2. Synthesis of Compound 7**

Under nitrogen protection, Compound **5** (1.00 mmol), Compound **6** (1.05 mmol), potassium carbonate (2.00 mmol), 1,4-dioxane and water (4 mL/1 mL) and Pd(dppf)$Cl_2$ (0.25 mol%) were added in sequence to a 50 mL reaction bottle. The reaction system was sealed, the reaction was carried out at 80°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1: 100 to 1:5), and the solvent was removed by concentration under reduced pressure to obtain Compound 7 (LC-MS: $C_{22}H_{26}N_5O_2$, $[M+H]^+$ 392.2; found 392.4, yield 53.6%).

**Step 3. Synthesis of Compound 8**

Compound 7 was dissolved in a mixed solution of 6N hydrochloric acid and ethyl acetate (5 mL) in a 50 mL reaction bottle, stirred and reacted at room temperature for 0.5 hour, and concentrated under reduced pressure to remove the solvent to obtain Compound **8** (LC-MS: $C_{17}H_{18}N_5$, $[M+H]^+$ 292.2; found 292.1, crude product).

**Step 4. Synthesis of Compound $R_{2\text{-}A1}$**

Compound **9a** (1.00 mmol), EDCI (1.05 mmol), HOBt (1.05 mmol) and *N*,*N*-dimethylformamide (2 mL) were added in sequence to a 50 mL reaction bottle. After the temperature of the reaction system dropped to 0°C, Compound **8** (1.05 mmol) was added. The reaction was carried out under ice bath conditions and stirring for 1 hour, and then quenched (LC-MS monitoring). The extraction was carried out with saturated sodium chloride solution (10 mL) and ethyl acetate (3× 25 mL). The organic phases were combined, and the combined organic phase was dried over anhydrous sodium sulfate, evaporated to remove the solvent, purified by medium-pressure preparative chromatography, and concentrated under reduced pressure to remove the solvent to obtain Compound **$R_{2\text{-}A1}$** (LC-MS: $C_{31}H_{27}IN_5O_2$, $[M+H]^+$ 628.1; found 628.3, yield 86.5%).

Referring to the synthesis method of Compound **$R_{2\text{-}A1}$**, the raw materials in the following table were used to replace Compound **9a,** and the other raw materials and operating methods remained unchanged, to obtain intermediate Compound **$R_{2\text{-}A2}$**.

| Compound No. | Structure and characterization data of compound | Raw material | Yield |
|---|---|---|---|
| $R_{2\text{-}A2}$ | 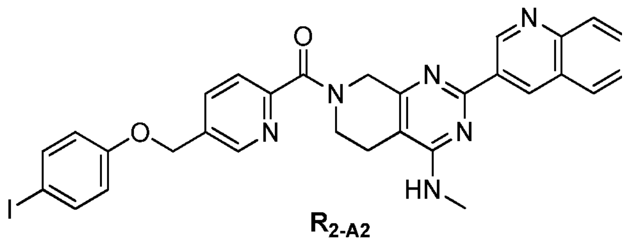 $R_{2\text{-}A2}$ | 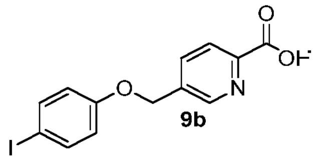 9b | 84.3 % |
| | LC-MS: $C_{30}H_{26}IN_6O_2$, $[M+H]^+$ 629.1; found 629.3 | | |

Referring to the synthesis method of Compound $R_{2\text{-}A1}$, the raw materials in the following table were used to replace Compound **9a,** and the other raw materials and operating methods remained unchanged, to obtain intermediate compounds $R_{2\text{-}B1}$ and $R_{2\text{-}B2}$.

| Compound No. | Structure and characterization data of compound | Raw material | Yield |
|---|---|---|---|
| $R_{2\text{-}B1}$ | 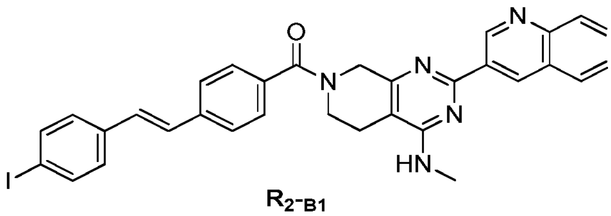 $R_{2\text{-}B1}$ | 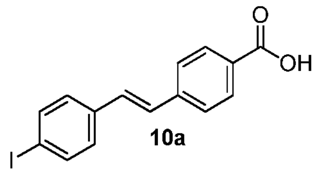 10a | 78.6 % |
| | LC-MS: $C_{32}H_{27}IN_5O$, $[M+H]^+$ 624.1; found 624.2 | | |
| $R_{2\text{-}B2}$ | 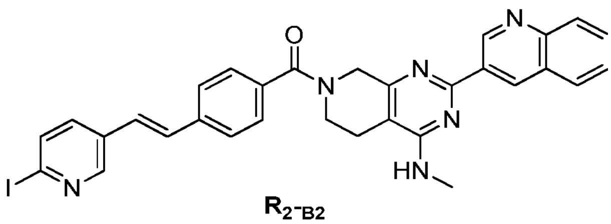 $R_{2\text{-}B2}$ | 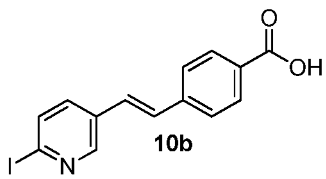 10b | 59.8 % |
| | LC-MS: $C_{31}H_{26}IN_6O$, $[M+H]^+$ 625.1; found 625.2 | | |

**Synthesis of intermediates:** Synthesis of intermediate compounds $R_{2\text{-}C1}$, $R_{2\text{-}C2}$, $R_{2\text{-}C3}$, $R_{2\text{-}C4}$, $R_{2\text{-}C5}$, $R_{2\text{-}D1}$, $R_{2\text{-}D2}$, $R_{2\text{-}D3}$, $R_{2\text{-}D4}$

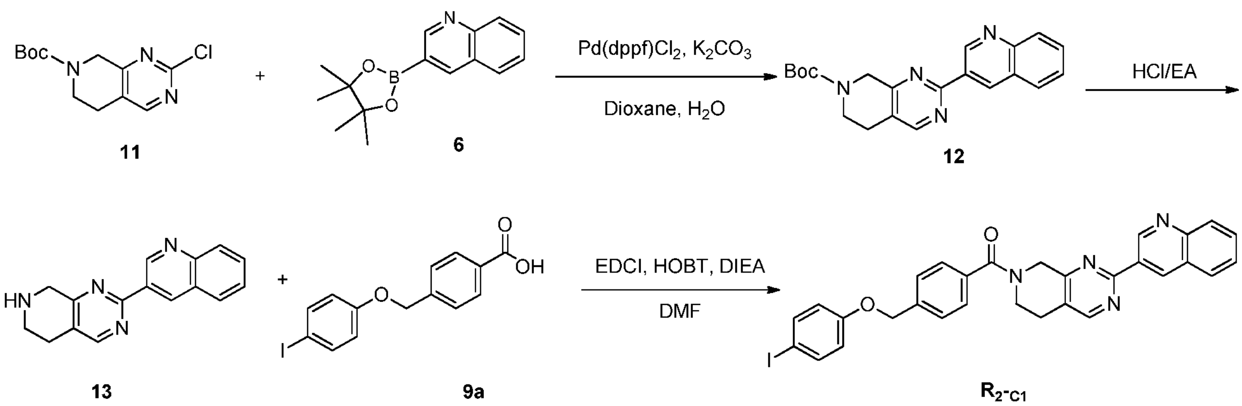


11 6 12

13 9a $R_{2\text{-}C1}$

**Step 1. Synthesis of Compound 12**

Under nitrogen protection, Compound **11** (1.00 mmol), Compound **6** (1.05 mmol), potassium carbonate (2.00 mmol), 1,4-dioxane and water (4 mL/1 mL) and Pd(dppf)$Cl_2$ (0.25 mol%) were added in sequence to a 50 mL reaction bottle. The reaction system was sealed, the reaction was carried out under stirring at 80°C for 2 hours, and then the reaction was quenched (LC-MS monitoring). The extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1: 100 to 1:5), and the solvent was removed after concentration under reduced pressure to obtain Compound **12** (LC-MS: $C_{21}H_{23}N_4O_2$, $[M+H]^+$ 363.2; found 363.3, yield 61.8%).

**Step 2. Synthesis of Compound 13**

Compound **12** was dissolved in a mixed solution of 6N hydrochloric acid and ethyl acetate (5 mL) in a 50 mL reaction bottle, stirred and reacted at room temperature for 0.5 hour, and concentrated under reduced pressure to remove the solvent to obtain Compound **13** (LC-MS: $C_{16}H_{15}N_4$, $[M+H]^+$ 263.1; found 263.2, crude product).

**Step 3. Synthesis of Compound $R_{2\text{-}C1}$**

Compound **9a** (1.00 mmol), EDCI (1.05 mmol), HOBT (1.05 mmol) and *N*,*N*-dimethylformamide (2 mL) were added in sequence to a 50 mL reaction bottle. After the temperature of the reaction system dropped to 0°C, Compound **13** (1.00 mmol) was added, the reaction was carried out under ice bath conditions and stirring for 1 hour, and then the reaction was quenched (LC-MS monitoring). The extraction was carried out with saturated sodium chloride solution (10 mL) and ethyl acetate (3× 25 mL), the organic phases were combined, the combined organic phase was dried over anhydrous sodium sulfate, evaporated to remove the solvent, purified by medium-pressure preparative chromatography, and concentrated under reduced pressure to remove the solvent to obtain Compound **$R_{2\text{-}C1}$** (LC-MS: $C_{30}H_{24}IN_4O_2$, $[M+H]^+$ 599.1; found 599.3, yield 73.2%).

Referring to the synthesis method of Compound **$R_{2\text{-}C1}$**, the raw materials in the following table were used to replace Compound **9a,** and the other raw materials and operating methods remained unchanged to obtain intermediate compounds **$R_{2\text{-}C2}$**, **$R_{2\text{-}C3}$**, **$R_{2\text{-}C4}$** and **$R_{2\text{-}C5}$**.

| Compound No. | Structure and characterization data of compound | Raw material | Yield |
|---|---|---|---|
| **$R_{2\text{-}C2}$** | 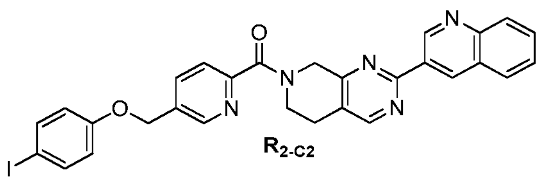 **$R_{2\text{-}C2}$** | 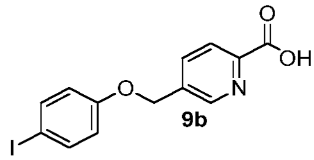 **9b** | 58.2 % |
| | LC-MS: $C_{29}H_{23}IN_5O_2$, $[M+H]^+$ 600.1; found 600.2 | | |
| **$R_{2\text{-}C3}$** | 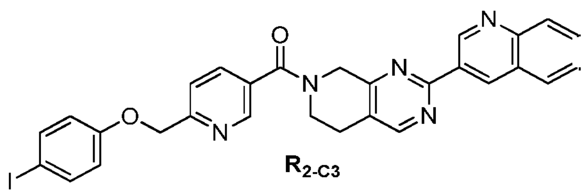 **$R_{2\text{-}C3}$** | 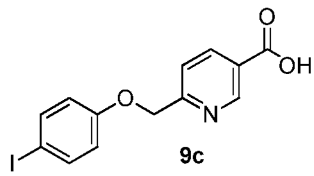 **9c** | 59.6 % |
| | LC-MS: $C_{29}H_{23}IN_5O_2$, $[M+H]^+$ 600.1; found 600.3 | | |

(continued)

| Compound No. | Structure and characterization data of compound | Raw material | Yield |
|---|---|---|---|
| **$R_{2\text{-}C4}$** | 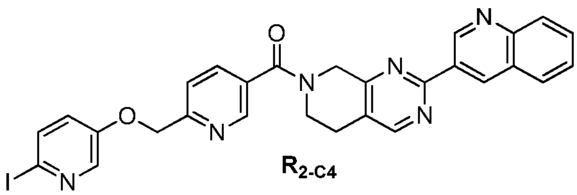 **$R_{2\text{-}C4}$** | 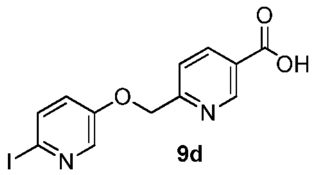 **9d** | 43.8 % |
| | LC-MS: $C_{28}H_{22}IN_6O_2$, $[M+H]^+$ 601.1; found 601.3 | | |
| **$R_{2\text{-}C5}$** | 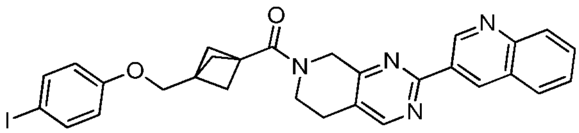 **$R_{2\text{-}C5}$** | 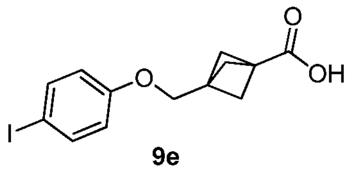 **9e** | 51.2 % |
| | LC-MS: $C_{29}H_{26}IN_4O_2$, $[M+H]^+$ 589.1; found 589.2 | | |

Referring to the synthesis method of Compound **$R_{2\text{-}C1}$**, the raw materials in the following table were used to replace Compound **9a,** and the other raw materials and operating methods remained unchanged to obtain intermediate compounds **$R_{2\text{-}D1}$**, **$R_{2\text{-}D2}$**, **$R_{2\text{-}D3}$** and **$R_{2\text{-}D4}$**.

| Compound No. | Structure and characterization data of compound | Raw material | Yield |
|---|---|---|---|
| **$R_{2\text{-}D1}$** | 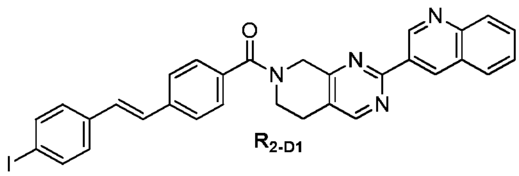 **$R_{2\text{-}D1}$** | 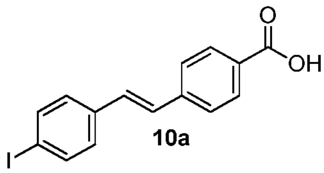 **10a** | 63.8 % |
| | LC-MS: $C_{31}H_{24}IN_4O$, $[M+H]^+$ 595.1; found 595.2 | | |
| **$R_{2\text{-}D2}$** | 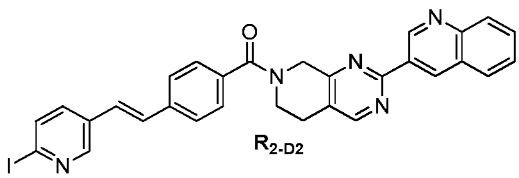 **$R_{2\text{-}D2}$** | 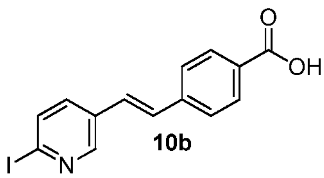 **10b** | 51.9 % |
| | LC-MS: $C_{30}H_{23}IN_5O$, $[M+H]^+$ 596.1; found 596.3 | | |
| **$R_{2\text{-}D3}$** | 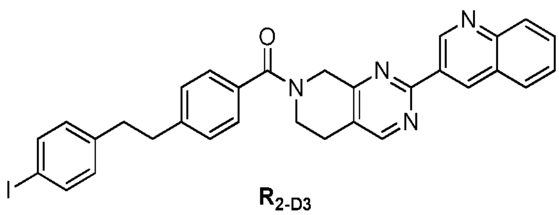 **$R_{2\text{-}D3}$** | 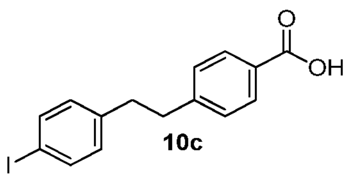 **10c** | 67.8 % |
| | LC-MS: $C_{31}H_{26}IN_4O$, $[M+H]^+$ 597.1; found 597.2 | | |

(continued)

| Compound No. | Structure and characterization data of compound | Raw material | Yield |
|---|---|---|---|
| $R_{2\text{-}D4}$ | 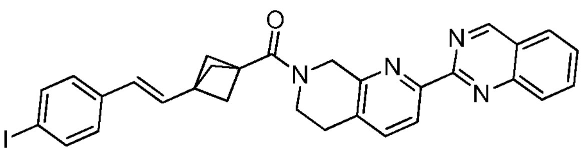<br>$R_{2\text{-}D4}$<br>LC-MS: $C_{30}H_{26}IN_4O$, $[M+H]^+$ 585.1; found 585.2 | 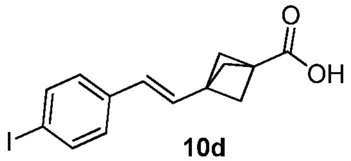<br>10d | 59.2 % |

**Example 1.** Synthesis of Compound A of the present invention

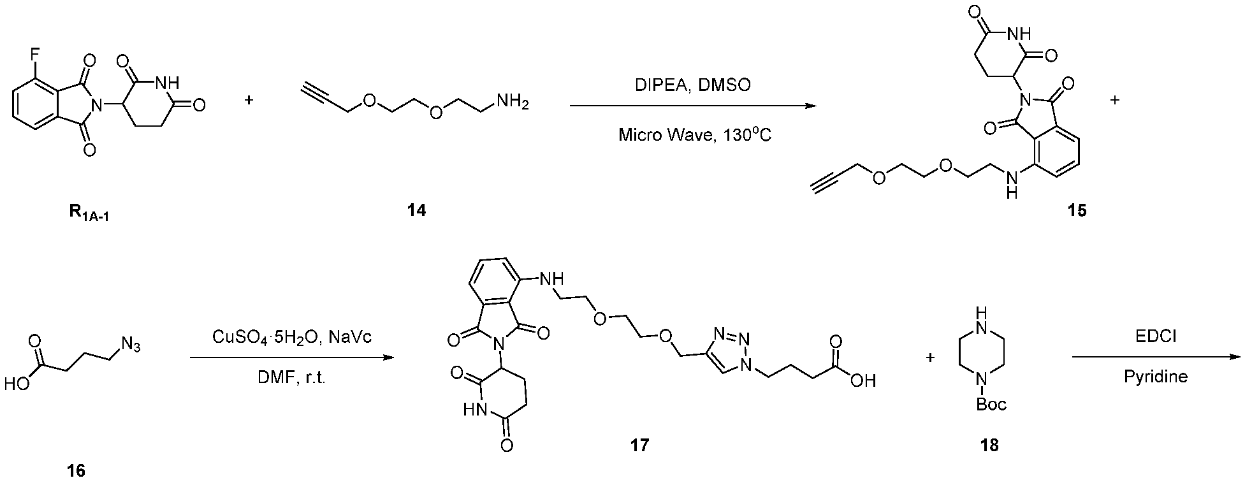

$R_{1A\text{-}1}$ 14 15 16 17 18

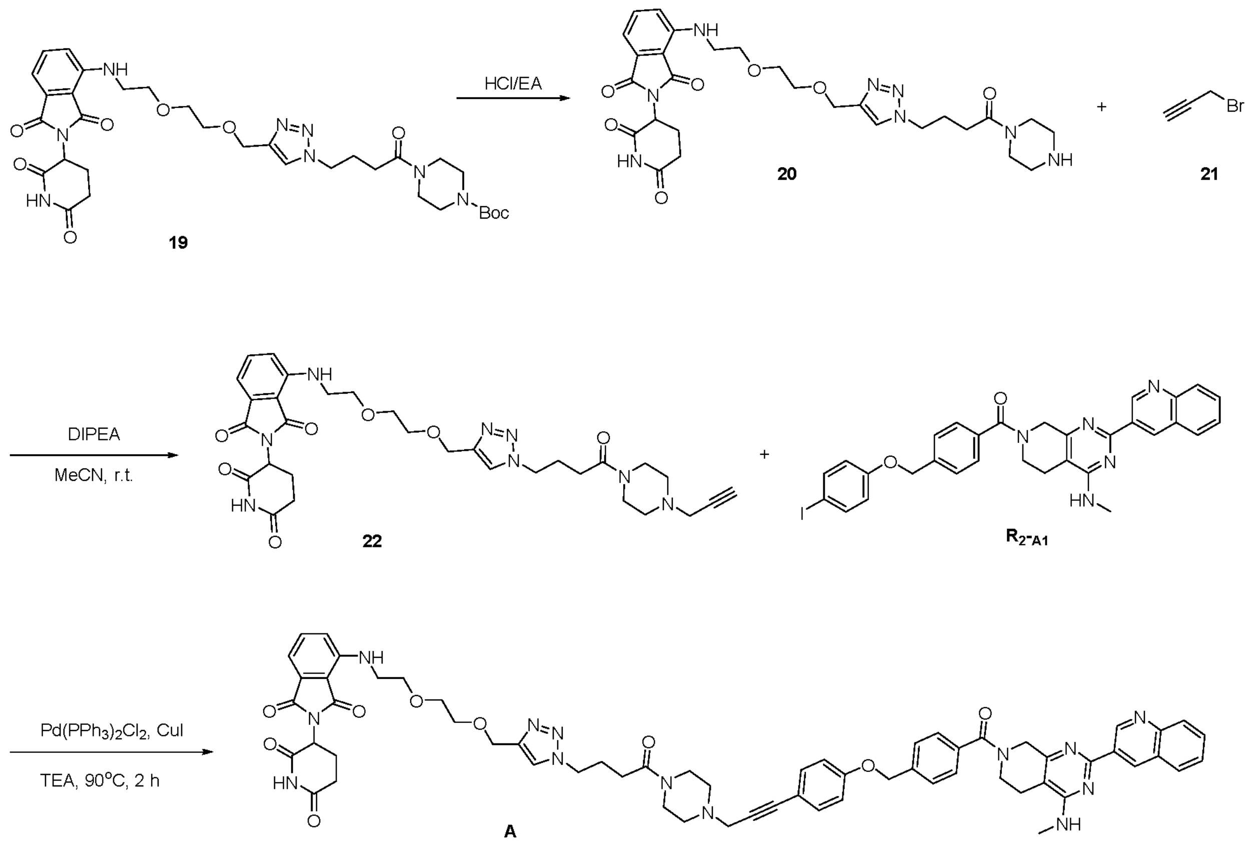


19 20 21 22 R2-A1 A

**Step 1. Synthesis of Compound 15**

Compound $\mathbf{R_{1A\text{-}1}}$ (1.15 mmol), Compound **14** (1.15 mmol), *N,N*-diisopropylethylamine (2.80 mmol) and DMSO (5 mL) were added into a 50 mL reaction bottle, heated by a microwave reactor to 130°C and reacted for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **15** ($C_{20}H_{22}N_3O_6$, $[M+H]^+$ 400.2; found 400.3, crude product).

**Step 2. Synthesis of Compound 17**

Compound **15** (0.85 mmol), Compound **16** (0.90 mmol) and *N,N*-dimethylformamide (2 mL) were added into a 50 mL reaction bottle. After the solution was clear, copper sulfate pentahydrate (1.75 mmol) and NaVc (0.90 mmol) were added in sequence, reacted at room temperature until the end of the reaction (LC-MS monitoring), the solid was filtered out, and the reaction solution was concentrated under reduced pressure to obtain Compound **17** ($C_{24}H_{29}N_6O_8$, $[M+H]^+$ 529.2; found 529.1, yield 91.8%).

**Step 3. Synthesis of Compound 19**

Compound **17** (0.75 mmol), EDCI (0.80 mmol) and pyridine (2 mL) were added in sequence to a 50 mL reaction bottle. After the temperature of the reaction system dropped to 0°C, Compound **18** (0.75 mmol) was added. The reaction was carried out under ice bath conditions and stirring for 0.5 hours, and then the reaction was quenched. Extraction was carried out with saturated sodium chloride solution (20 mL) and methylene chloride (3× 15 mL). The organic phases were combined, and the combined organic phase was dried over anhydrous sodium sulfate and evaporated to dryness to remove the solvent, and purified by medium-pressure preparative chromatography, and the solvent was removed by concentration under reduced pressure to obtain Compound **19** ($C_{33}H_{45}N_8O_9$, $[M+H]^+$ 697.3; found 697.4, yield 83.2%).

**Step 4. Synthesis of Compound 20**

Compound **19** was dissolved in a mixed solution of 6N hydrochloric acid and ethyl acetate (5 mL) in a 50 mL reaction bottle, stirred and reacted at room temperature for 0.5 hours, and concentrated under reduced pressure to remove the solvent, to obtain Compound **20** ($C_{28}H_{37}N_8O_7$, [M+H]+ 597.3; found 597.2, crude product).

**Step 5. Synthesis of Compound 22**

Compound **20** (0.55 mmol), Compound **21** (0.60 mmol), *N,N*-diisopropylethylamine (1.40 mmol) and acetonitrile (5 mL) were added into a 50 mL reaction bottle, stirred and reacted at room temperature for 2 hours, then added with water to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, and purified by medium-pressure preparative chromatography, and the solvent was removed by concentration under reduced pressure to obtain Compound **22** ($C_{31}H_{39}N_8O_7$, $[M+H]^+$ 635.3; found 635.2, yield 68.2%).

**Step 6. Synthesis of Compound A**

Under nitrogen protection, Compound **22** (0.25 mmol), Compound $\mathbf{R_{2\text{-}A1}}$ (0.25 mmol), copper iodide (0.55 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and the solvent was removed by concentration under reduced pressure to obtain Compound A (42.8 mg, 37.76 μmol, purity 99.9%). LC-MS: $C_{62}H_{64}N_{13}O_9$, $[M/2+H]^+$ 568.1; found 568.1. $^1$H NMR (400 MHz, DMSO-*d6*) δ 11.10 (s, 1H), 9.82-9.74 (m, 1H), 9.31-9.21 (m, 1H), 8.22 (s, 1H), 8.12-8.05 (m, 2H), 7.88 (s, 1H), 7.72-7.70 (m, 1H), 7.67-7.58 (m, 5H), 7.53-7.49 (m, 3H), 7.15-7.11 (m, 1H), 7.09-7.04 (m, 2H), 7.01 (d, *J* = 8.0 Hz, 1H), 6.60 (s, 1H), 5.23 (s, 2H), 5.08 -5.03 (m, 1H), 4.76 (s, 2H), 4.52 (s, 2H), 4.45-4.34 (m, 4H), 3.98 (s, 2H), 3.72-3.58 (m, 8H), 3.47 (s, 3H), 3.32 (s, 3H), 3.10 (d, *J* = 4.0 Hz, 3H), 2.93-2.85 (m, 1H), 2.67-2.57 (m, 3H), 2.53-2.50 (m, 4H), 2.39 (s, 2H), 2.06-2.03 (m, 2H). Purity > 99%.

**Example 2.** Synthesis of Compounds **B1, B2** and **B3** of the present invention

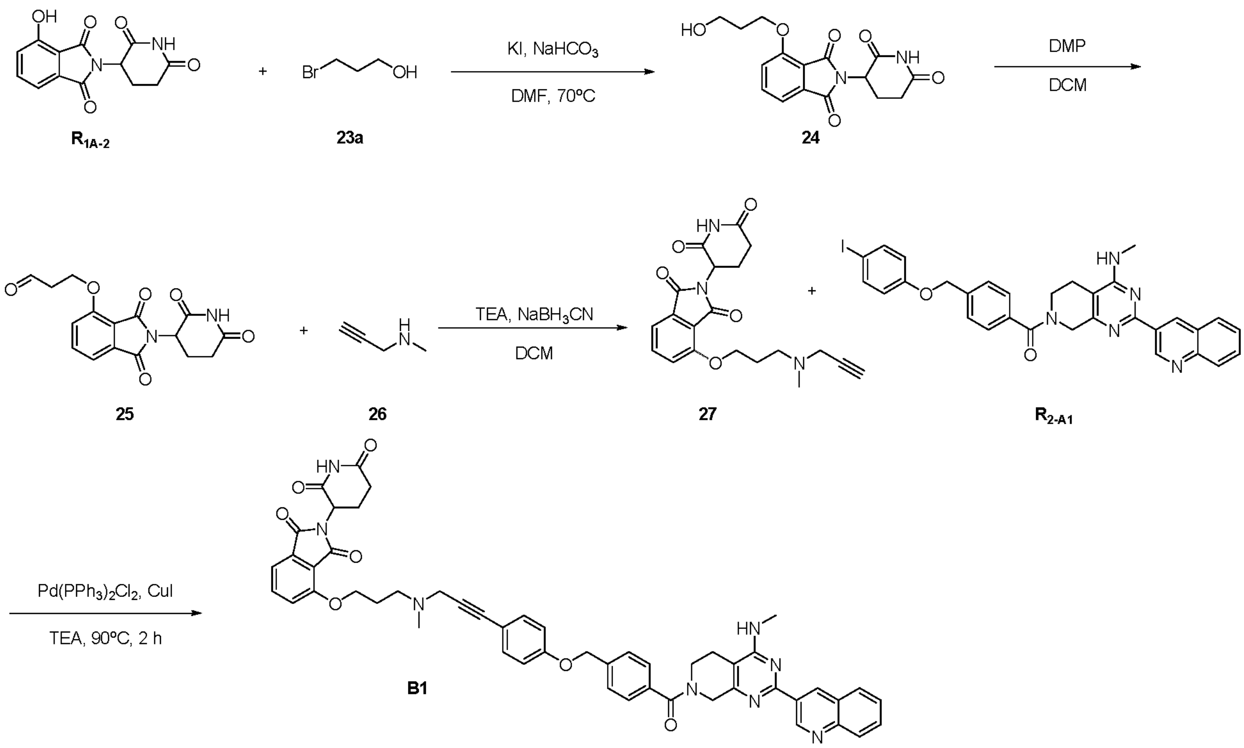


$R_{1A-2}$ 23a 24 25 26 27 $R_{2-A1}$ B1

### Step 1. Synthesis of Compound 24

Compound $\mathbf{R_{1A-2}}$ (1.00 mmol), Compound **23a** (2.25 mmol), potassium iodide (1.05 mmol), sodium bicarbonate (2.80 mmol) and *N*,*N*-dimethylformamide (5 mL) were added into a 50 mL reaction bottle, heated to 70°C, stirred and reacted for 8 hours, and then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **24** ($C_{16}H_{17}N_2O_6$, $[M+H]^+$ 333.1; found 333.3, yield 59.8%).

### Step 2. Synthesis of Compound 25

Compound **24** (0.55 mmol), Dess-Martin Periodinane (1.20 mmol) and dichloroethane (5 mL) were added to a 50 mL reaction bottle, stirred and reacted at room temperature for 1 hour, and then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **25** ($C_{16}H_{15}N_2O_6$, $[M+H]^+$ 331.1; found 331.2, yield 81.5%).

### Step 3. Synthesis of Compound 27

Compound **25** (0.50 mmol), Compound **26** (0.95 mmol), triethylamine (2 mL), sodium cyanoborohydride (1.02 mmol) and dichloroethane (5 mL) were added in sequence to a 50 mL reaction bottle, stirred and reacted at room temperature for 2 hours, and then the reaction was quenched (LC-MS monitoring). The solvent was evaporated to dryness, purification was carried out by medium-pressure preparative chromatography, and the solvent was removed by concentration under reduced pressure to obtain Compound **27** ($C_{20}H_{22}N_3O_5$, $[M+H]^+$ 384.2; found 384.3, yield 68.9%).

### Step 4. Synthesis of Compound B1

Under nitrogen protection, Compound 27 (0.25 mmol), Compound $\mathbf{R_{2-A1}}$ (0.30 mmol), copper iodide (0.50 mmol), triethylamine (5 mL), and $Pd(PPh_3)_2Cl_2$ (0.25 mol%) were added in sequence to a 50 mL reaction bottle. The reaction system was sealed, the reaction was carried out at 90°C under stirring for 2 hours, and then the reaction was

quenched (LC-MS monitoring). Extraction was carried out with saturated sodium chloride solution (20 mL) and ethyl acetate (3 × 25mL), the organic phases were combined, the combined organic phase was dried over anhydrous sodium sulfate, evaporated to remove the solvent, separated by column chromatography (the volume ratio of the eluents used was petroleum ether/ethyl acetate = 1:100 to 1 :5), and the solvent was removed by concentration under reduced pressure to obtain Compound **B1** (3.0 mg, 3.39 μmol, purity 89.7%). LC-MS: $C_{51}H_{47}N_8O_7$, $[M/2+H]^+$ 442.4; found 422.6. $^1H$ NMR (400 MHz, Methanol-*d4*) δ 9.80-9.68 (m, 1H),9.25-9.22 (m, 1H), 8.09- 8.07 (m, 2H), 7.82-7.53 (m, 8H), 7.40-7.29 (m, 3H), 7.02-6.92 (m, 2H), 5.18 (s, 2H), 4.31 (s, 3H), 4.11 (s, 2H), 3.80-3.70 (m, 3H), 3.17 (s, 3H), 2.94 (s, 2H), 2.82-2.53 (m, 9H), 2.11-2.03 (m, 3H). Purity > 85%.

According to the step 1 to step 4 of the synthesis method of Compound **B1**, the raw materials in the following table were used to replace Compound **23a,** and the other raw materials and operating methods remained unchanged, to obtain Compounds **B2** and **B3.**

| Compound No. | Structure and characterization data of compound | Raw material | Yield |
|---|---|---|---|
| **B2** | 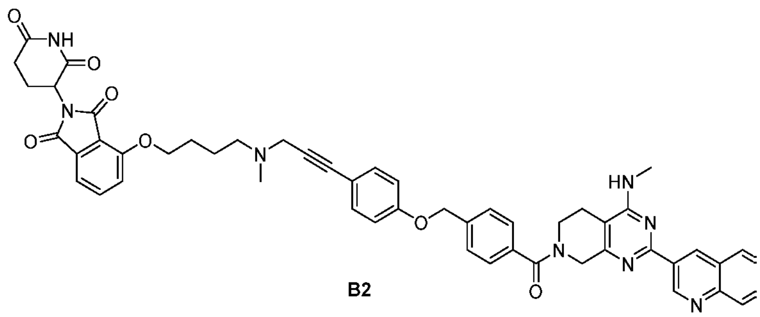 B2<br>LC-MS: $C_{52}H_{49}N_8O_7$, $[M/2+H]^+$ 449.2; found 449.5. $^1H$ NMR (400 MHz, Methanol-*d4*) δ 9.78 (s, 1H), 9.54 (s, 1H), 8.29 (s, 1H), 8.23-8.21 (m, 1H), 8.07 (s, 1H), 7.88 (s, 1H), 7.74 (t, *J* = 8.0 Hz, 1H), 7.60 (q, *J* = 8.0 Hz, 4H), 7.43-7.39 (m, 2H), 7.37-7.32 (m, 2H), 6.91 (d, *J* = 8.3 Hz, 2H), 5.18 (s, 2H), 5.08-5.03 (m, 1H), 4.92 (s, 1H), 4.73 (s, 1H), 4.32-4.26 (m, 4H), 4.12 (s, 1H), 3.83 (s, 1H), 3.73-3.67 (m, 1H), 3.47 (s, 1H), 3.25 (s, 3H), 3.03 (s, 3H), 2.83-2.78 (m, 1H), 2.72-2.62 (m, 4H), 2.04 (s, 5H). | 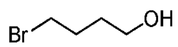 **23b** | 12.8 % |
| **B3** | 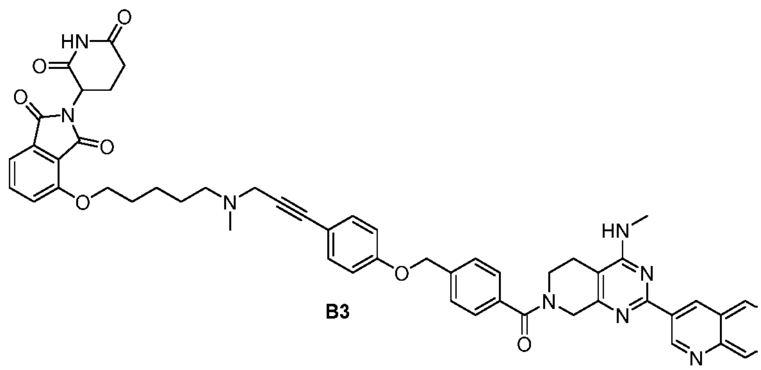 B3<br>LC-MS: $C_{53}H_{51}N_8O_7$, $[M/2+H]^+$ 456.2; found 456.6. $^1H$ NMR (400 MHz, DMSO-$d_6$) δ 11.11 (s, 1H), 10.75-10.68 (m, 1H), 9.82-9.74 (m, 1H), 9.34-9.24 (m, 1H), 8.23 (s, 1H), 8.13 (s, 1H), 7.89 (s, 1H), 7.83-7.44 (m, 8H), 7.08 (d, *J* = 8.5 Hz, 2H), 5.23 (s, 2H), 5.09-5.06 (m, 1H), 4.76 (s, 1H), 4.56 (s, 1H), 4.32 (d, *J* = 4.8 Hz, 2H), 4.23 (t, *J* = 6.2 Hz, 2H), 3.84 (s, 1H), 3.21-3.16 (m, 2H), 3.10 (d, *J* = 3.8 Hz, 3H), 2.92-2.86 (m, 4H), 2.68-2.56 (m, 4H), 2.08-2.01 (m, 1H), 1.82-1.80 (m, 4H), 1.55-1.52 (m, 2H). | 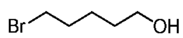 **23c** | 10.9 % |

**Example 3.** Synthesis of compounds **C1-1**, **C1-2** and **C1-3** of the present invention

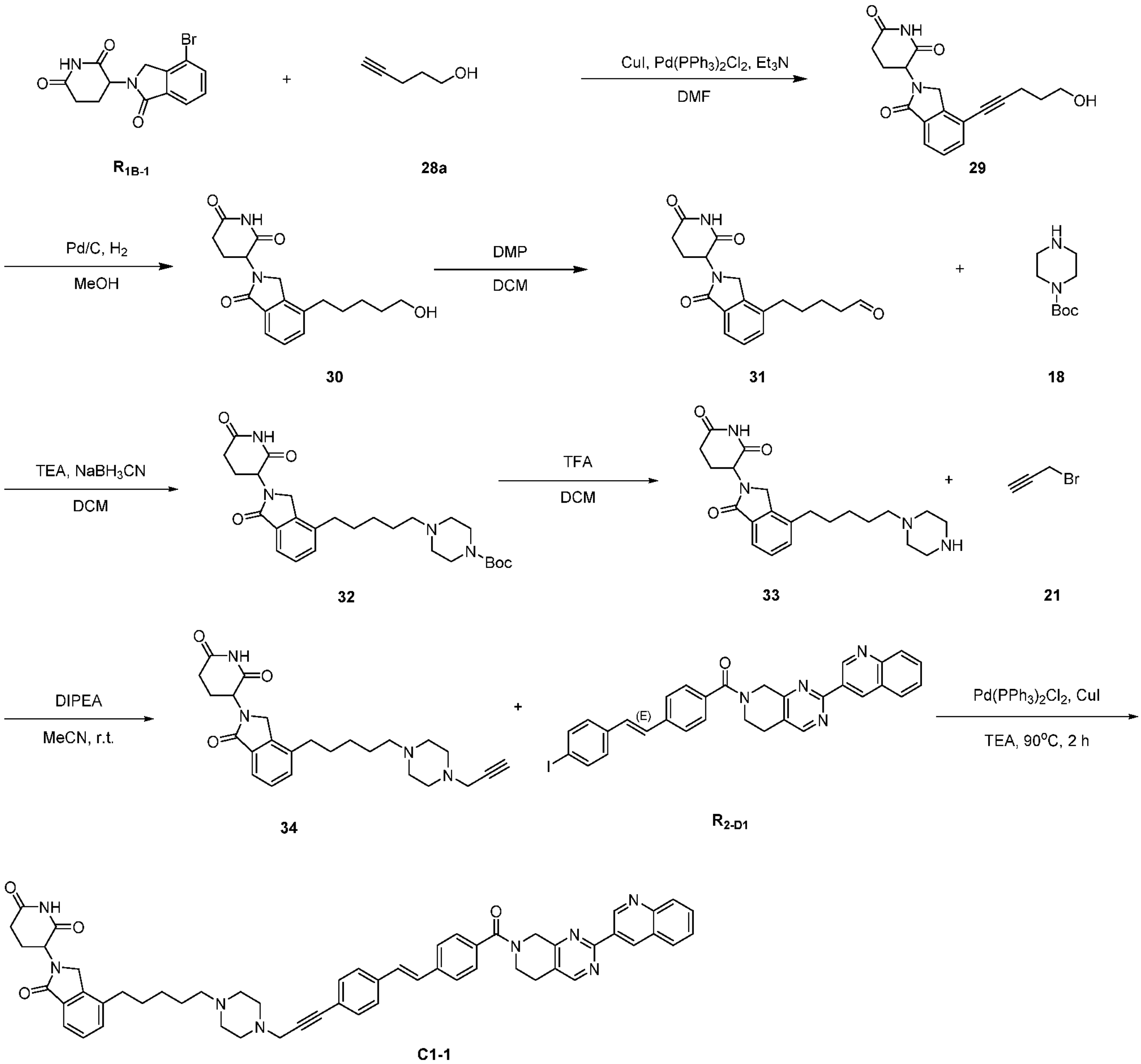


$R_{1B-1}$ + 28a → 29

→ 30 → 31 + 18

→ 32 → 33 + 21

→ 34 + $R_{2-D1}$ →

C1-1

### Step 1. Synthesis of Compound 29

Under nitrogen protection, Compound $\mathbf{R_{1B-1}}$ (1.00 mmol), Compound **28a** (1.05 mmol), copper iodide (2.00 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of eluents used was petroleum ether/ethyl acetate = 1:100 to 1:5), and the solvent was removed by concentration under reduced pressure to obtain Compound **29** ($C_{18}H_{19}N_2O_4$, $[M+H]^+$ 327.1; found 327.2, yield 68.5%).

### Step 2. Synthesis of Compound 30

Compound **29** (0.65 mmol), methanol (5 mL), and palladium-on-carbon (30.00 mg) were added in sequence to a 50 mL reaction bottle. The reaction system was replaced with hydrogen, maintained at an atmospheric pressure hydrogen atmosphere, and the reaction was carried out under stirring at room temperature for 3 hours (LC-MS monitoring). Filtration was carried to remove the palladium-on-carbon, washing was carried out with methanol (2×20 mL), the filtrate was collected, and concentrated under reduced pressure to remove the solvent to obtain Compound **30** ($C_{18}H_{23}N_2O_4$, $[M+H]^+$ 331.2; found 331.1, crude product).

**Step 3. Synthesis of Compound 31**

Compound **30** (0.55 mmol), Dess-Martin Periodinane (1.50 mmol) and dichloroethane (5 mL) were added to a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **31** ($C_{18}H_{21}N_2O_4$, $[M+H]^+$ 329.2; found 329.1, yield 82.1%).

**Step 4. Synthesis of Compound 32**

Compound **31** (0.45 mmol), Compound **18** (0.50 mmol), triethylamine (2 mL), sodium cyanoborohydride (0.55 mmol) and dichloroethane (5 mL) were added in sequence to a 50 mL reaction bottle. The reaction was carried out under stirring at room temperature for 2 hours, and then the reaction was quenched (LC-MS monitoring). The solvent was removed by evaporation to dryness, and purification was carried by medium-pressure preparative chromatography. The solvent was removed by concentration under reduced pressure to obtain Compound **32** ($C_{27}H_{39}N_4O_5$, $[M+H]^+$ 499.3; found 499.2, yield 85.9%).

**Step 5. Synthesis of Compound 33**

Compound **32** (0.35 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **33** ($C_{22}H_{31}N_4O_3$, $[M+H]^+$ 399.2; found 399.1, crude product).

**Step 6. Synthesis of Compound 34**

Compound **33** (0.35 mmol), Compound **21** (0.45 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **34** ($C_{25}H_{33}N_4O_3$, $[M+H]^+$ 437.3; found 437.2, yield 71.4%).

**Step 7. Synthesis of Compound C1-1**

Under nitrogen protection, Compound $\mathbf{R_{2\text{-}D1}}$ (0.25 mmol), Compound **34** (0.25 mmol), copper iodide (0.55 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and the solvent was removed by concentration under reduced pressure to obtain Compound **C1-1** (16.4 mg, 18.14 μmol). LC-MS: $C_{56}H_{55}N_8O_4$, $[M/2+H]^+$ 452.2; found 452.6. $^1$H NMR (400 MHz, DMSO-*d6*) δ 9.85-9.73 (m, 1H), 9.41-9.31 (m, 1H), 8.85 (s, 1H), 8.27-8.25 (m, 2H), 8.15-7.94 (m, 1H), 7.78-7.73 (m, 3H), 7.71-7.69 (m, 2H), 7.68 (d, *J* = 8.0 Hz, 1H), 7.56 (d, *J* = 4.0 Hz, 2H), 7.50-7.40 (m, 4H), 7.38-7.28 (m, 2H), 5.10-5.07 (m, 1H), 4.96-4.80 (m, 2H), 4.36-4.25 (m, 1H), 4.20-3.92 (m, 1H), 3.74 (s, 3H), 3.66-3.45 (m, 3H), 3.09-2.95 (m, 6H), 2.94-2.83 (m, 3H), 2.72-2.68 (m, 4H), 2.54-2.42 (m, 2H), 2.38-2.06 (m, 1H), 1.69-1.63 (m, 4H), 1.40-1.34 (m, 2H).

According to the step 1 to step 7 of the method for Compound **C1-1,** the raw material **1** in the following table was used to replace Compound $\mathbf{R_{1B\text{-}1}}$, and the raw material **2** in the following table was used to replace Compound **28a,** and the other raw materials and operating methods remained unchanged to obtain Compounds **C1-2** and **C1-3.**

| Compound No. | Structure and characterization data of compound | Raw material 1 | Raw material 2 | Yield |
|---|---|---|---|---|
| C1-2 | 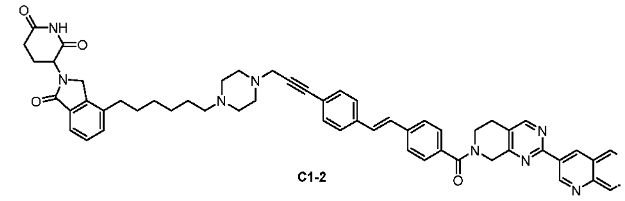<br>C1-2<br>LC-MS: $C_{57}H_{57}N_8O_4$, $[M/2+H]^+$ 459.2; found 459.6. $^1H$ NMR (400 MHz, DMSO-$d_6$) $\delta$ 9.86-9.76 (m, 1H), 9.48-9.36 (m, 1H), 8.86 (s, 1H), 8.29-8.25 (m, 2H), 8.18-8.17 (m, 1H), 7.97-7.77 (m, 3H), 7.75-7.72 (m, 2H), 7.68 (d, *J* = 8.0 Hz, 1H), 7.63 (d, *J* = 4.0 Hz, 2H), 7.60-7.50 (m, 4H), 7.49-7.38 (m, 2H), 5.10-5.07 (m, 1H), 4.97-4.81 (m, 2H), 4.51-4.48 (m, 1H), 4.36-3.93 (m, 1H), 3.79 (s, 3H), 3.12-3.01 (m, 7H), 2.94-2.86 (m, 3H), 2.72-2.65 (m, 5H), 2.54-2.37 (m, 3H), 2.36-2.05 (m, 1H), 1.63 (s, 4H). 1.35 (s, 4H). | 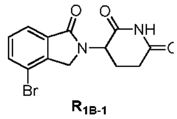<br>$R_{1B-1}$ | 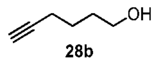<br>28b | 15.7 % |
| C1-3 | 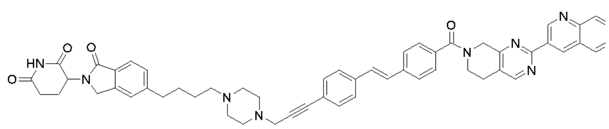<br>C1-3<br>LC-MS: $C_{55}H_{53}N_8O_4$, $[M/2+H]^+$ 445.6; found 445.4. $^1H$ NMR (400 MHz, DMSO-$d_6$) $\delta$ 11.01 (s, 1H), 9.82-9.76 (m, 1H), 9.25-9.21 (m, 1H), 8.85 (s, 1H), 8.22 (s, 1H), 8.10 (s, 1H), 7.87-7.85 (m, 1H), 7.75-7.73 (m, 2H), 7.68-7.66 (m, 4H), 7.57-7.55 (m, 2H), 7.48-7.46 (m, 3H), 7.40-7.37 (m, 3H), 5.05-5.03 (m, 1H), 4.92-4.78 (m, 2H), 4.45-4.40 (m, 1H), 4.32-4.27 (m, 1H), 3.71-3.69 (m, 4H), 3.32-3.17 (m, 2H), 3.12-2.99 (m, 6H), 2.95-2.91 (m, 1H), 2.86-2.76 (m, 2H), 2.67-2.62 (m, 4H), 2.44-2.41 (m, 1H), 2.38-2.32 (m, 1H), 2.01-1.99 (m, 1H), 1.98-1.65 (m, 4H). | 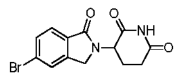<br>$R_{1B-2}$ | 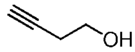<br>28c | 16.8 % |

Compound **C1-4** could be obtained by following the step 1 to step 7 of the method for Compound **C1-1**, in which the raw material **1** in the following table was used to replace Compound $\mathbf{R_{2D-1}}$, and the other raw materials and operating methods remained unchanged.

| Compound No. | Structure and characterization data of compound | Raw material 1 | Yield |
|---|---|---|---|
| C1-4 | 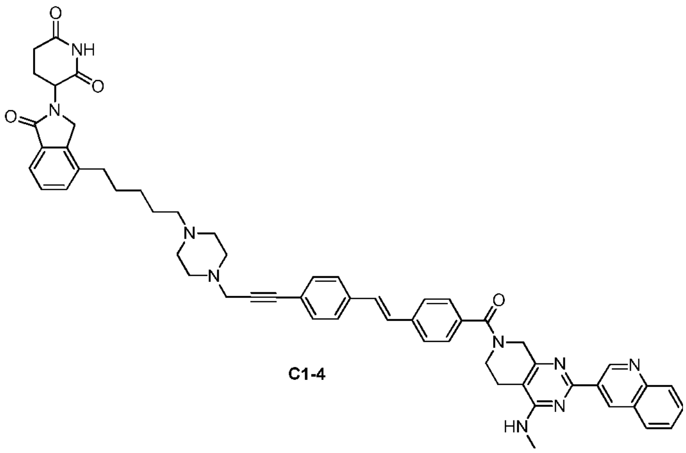 C1-4 | 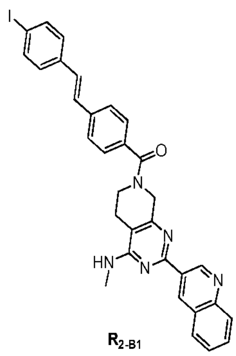 $R_{2\text{-}B1}$ | 15.7 % |
| | Compound **C1-4** (25.7 mg, 37.76 μ mol, 98.5% Purity). LC-MS: $C_{57}H_{58}N_9O_4$, $[M/2+H]^+$ 467.1; found 467.2. $^1H$ NMR (600 MHz, DMSO-$d_6$) δ 9.78 - 9.72 (m, 1H), 9.41 - 9.33 (m, 1H), 8.28 - 8.18 (m, 2H), 7.99 (s, 1H), 7.76 - 7.39 (m, 14H), 5.13 - 5.12 (m, 1H), 4.82 - 4.64 (m, 2H), 4.50 - 4.31 (m, 2H), 3.99 (s, 1H), 3.84 (s, 1H), 3.14 - 3.11 (m, 7H), 2.92 (s, 2H), 2.67 - 2.43 (m, 13H), 2.06 (s, 1H), 1.69 - 1.67 (m, 4H), 1.36 (s, 2H). Purity> 95%. | | |

**Example 4.** Synthesis of compounds **C2-1, C2-2, C2-3, C2-4, C2-5** and **C2-6** of the present invention

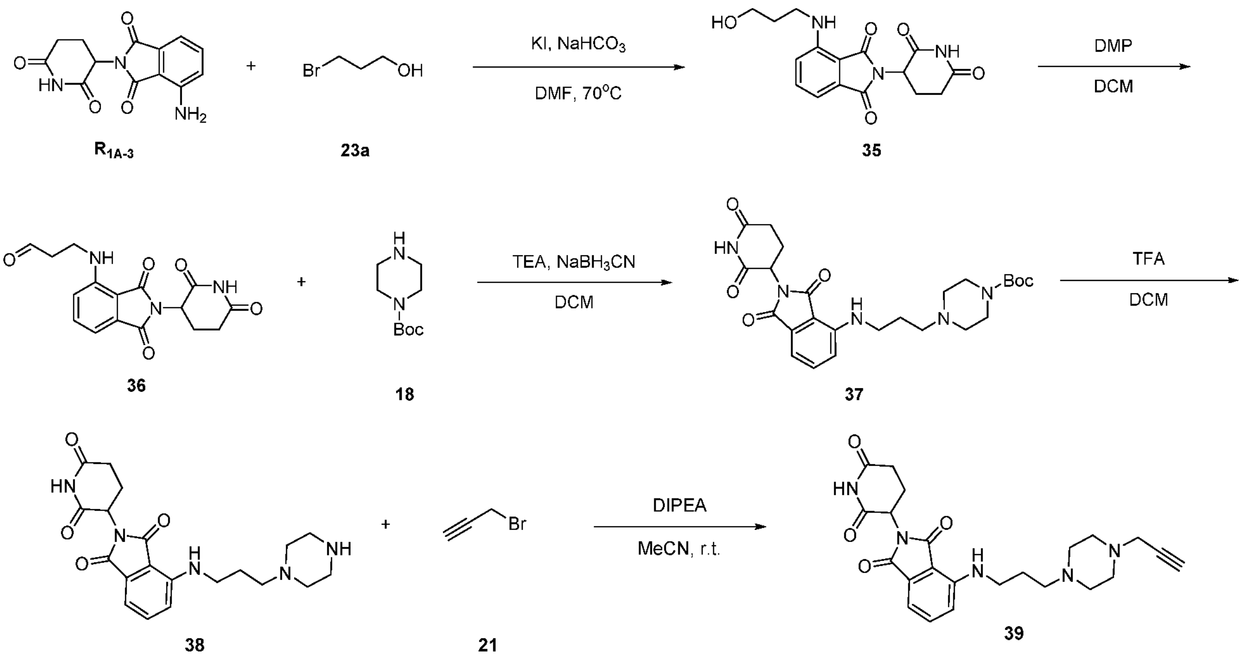


$R_{1A\text{-}3}$ 23a 35 36 18 37 38 21 39

+

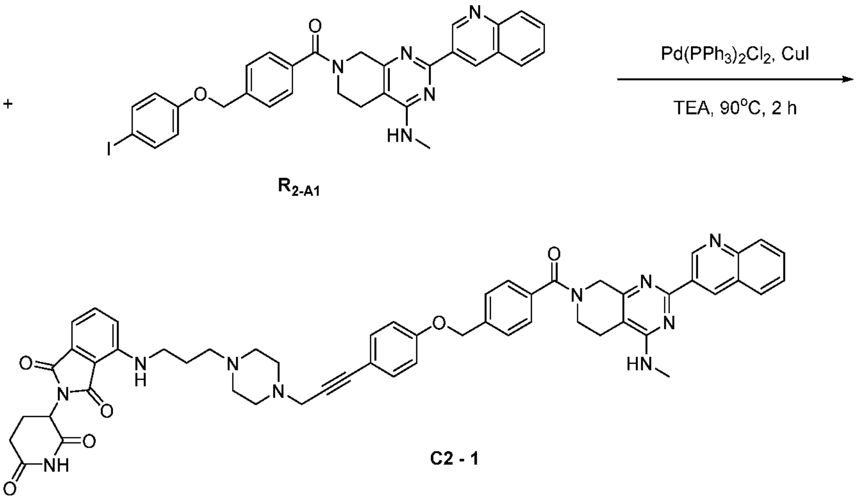


$R_{2\text{-}A1}$

C2 - 1

**Step 1. Synthesis of Compound 35**

Compound $\mathbf{R_{1A\text{-}3}}$ (1.00 mmol), Compound **23a** (1.80 mmol), potassium iodide (1.05 mmol), sodium bicarbonate (2.20 mmol) and *N*,*N*-dimethylformamide (5 mL) were added into a 50 mL reaction bottle, heated to 70°C, reacted under stirring for 8 hours, and then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **35** ($C_{16}H_{18}N_3O_5$, $[M+H]^+$ 332.1; found 332.3, yield 61.2%).

**Step 2. Synthesis of Compound 36**

Compound **35** (0.60 mmol), Dess-Martin Periodinane (1.80 mmol) and dichloroethane (5 mL) were added to a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **36** ($C_{16}H_{15}N_2O_6$, $[M+H]^+$ 331.1; found 331.3, yield 91.3%).

**Step 3. Synthesis of Compound 37**

Compound **36** (0.55 mmol), Compound **18** (0.75 mmol), triethylamine (2 mL), sodium cyanoborohydride (0.80 mmol) and dichloroethane (5 mL) were added in sequence to a 50 mL reaction bottle. The reaction was carried out under stirring at room temperature for 2 hours, and then the reaction was quenched (LC-MS monitoring), the solvent was removed by evaporation to dryness, purification was carried out by medium-pressure preparative chromatography, and the solvent was removed by concentration under reduced pressure to obtain Compound **37** ($C_{25}H_{34}N_5O_6$, $[M+H]^+$ 500.2; found 500.4, yield 34.8%).

**Step 4. Synthesis of Compound 38**

Compound 37 (0.30 mmol) was dissolved in dichloromethane (5 mL) in a 50 mL reaction bottle, then trifluoroacetic acid (1.5 mL) was added dropwise, the reaction was carried out under stirring at room temperature for 0.5 hour, the solvent was removed by concentration under reduced pressure to obtain Compound **38** ($C_{20}H_{26}N_5O_4$, $[M+H]^+$ 400.2; found 400.1, crude product).

**Step 5. Synthesis of Compound 39**

Compound **38** (0.30 mmol), Compound **21** (0.65 mmol), *N*,*N*-diisopropylethylamine (1.20 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution

(25 mL) and ethyl acetate (3× 25 mL), the organic phases were combined, the organic combined phase was dried over anhydrous sodium sulfate, evaporated to dryness to remove the solvent, purified by medium-pressure preparative chromatography, and concentrated under reduced pressure to remove the solvent to obtain Compound **39** ($C_{23}H_{28}N_5O_4$, $[M+H]^+$ 438.2; found 438.3, yield 87.6%).

**Step 6. Synthesis of Compound C2-1**

Under nitrogen protection, Compound **39** (0.25 mmol), Compound $\mathbf{R_{2\text{-}A1}}$ (0.25 mmol), copper iodide (0.55 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate =1:100 to 1:5), and concentrated under reduced pressure to remove the solvent to obtain Compound **C2-1** (5.9 mg, 6.38 μmol, 99.9% purity). LC-MS: $C_{54}H_{53}N_{10}O_6$, $[M/2+H]^+$ 469.6; found 469.4. $^1H$ NMR (400 MHz, Methanol-*d4*) δ 9.79-9.69 (m, 1H), 9.24-9.13 (m, 1H), 8.22- 8.07 (m, 2H), 7.82 (s, 1H), 7.73-7.52 (m, 6H), 7.36 (d, *J* = 8.0 Hz, 2H), 7.07-6.96 (m, 4H), 5.19 (s, 2H), 5.05-5.00 (m, 1H), 4.58 (s, 4H), 4.28 (t, *J* = 4.0 Hz, 1H), 4.27 (s, 1H), 3.76-3.74 (m, 2H), 3.51-3.48 (m, 2H), 3.38 (s, 2H), 3.16 (s, 3H), 2.79-2.51 (m, 12H), 1.87-1.85 (m, 2H).

Compounds **C2-2** to **C2-5** could be obtained by following the step 1 to step 6 of the method for Compound **C2-1,** in which Compound **23a** was replaced with the raw materials in the below table, and the other raw materials and operating methods remained unchanged.

| Compound No. | Structure and characterization data of compound | Raw material | Yield |
|---|---|---|---|
| **C2-2** | 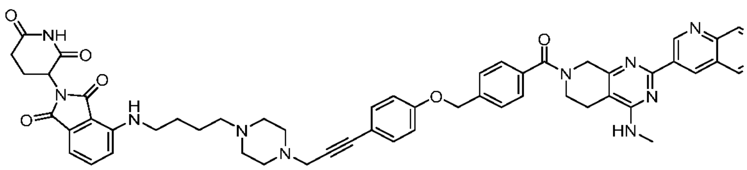<br>C2 - 2<br>LC-MS: $C_{55}H_{55}N_{10}O_6$, $[M/2+H]^+$ 476.6; found 476.4. $^1H$ NMR (400 MHz, Methanol-$d_4$) δ 9.80-9.69 (m, 1H), 9.25-9.19 (m, 1H), 8.12 (s, 2H), 7.82 (s, 1H), 7.73-7.53 (m, 6H), 7.36 (d, *J* = 8.0 Hz, 2H), 7.07-6.93 (m, 4H), 5.19 (s, 2H), 5.05-5.00 (m, 1H), 4.58 (s, 5H), 4.28 (t, *J* = 4.0 Hz, 1H), 4.25 (s, 2H), 3.79-3.72 (m, 2H), 3.59-3.48 (m, 2H), 3.16 (s, 3H), 2.83-2.68 (m, 12H), 2.48-2.46 (m, 2H), 2.09-2.08 (m, 2H). | 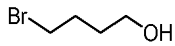<br>**23b** | 15.3 % |
| **C2-3** | 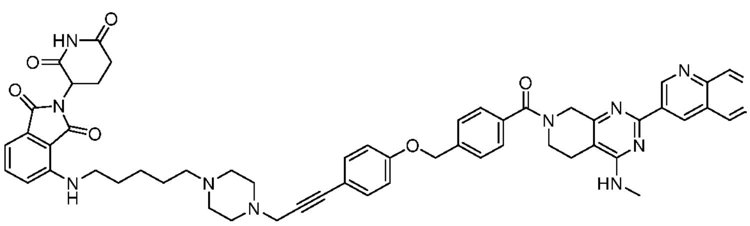<br>C2 - 3<br>LC-MS: $C_{56}H_{57}N_{10}O_6$, $[M/2+H]^+$ 483.2; found 483.6. $^1H$ NMR (400 MHz, Methanol-$d_4$) δ 9.78-9.69 (m, 1H), 9.24-9.14 (m, 1H), 8.12 (s, 2H), 7.82 (s, 1H), 7.73-7.48 (m, 6H), 7.35 (d, *J* = 8.0 Hz, 2H), 7.06-6.96 (m, 4H), 5.19 (s, 2H), 5.07-5.01 (m, 1H), 4.58 (s, 4H), 4.28 (t, *J* = 4.0 Hz, 1H), 4.25 (s, 1H), 3.77-3.71 (m, 2H), 3.52-3.47 (m, 3H), 3.16 (s, 4H), 2.84-2.65 (m, 12H), 2.58-2.46 (m, 2H), 1.73-1.69 (m, 2H), 1.67-1.58 (m, 2H). | 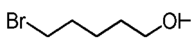<br>**23c** | 11.6 % |

(continued)

| Compound No. | Structure and characterization data of compound | Raw material | Yield |
|---|---|---|---|
| C2-4 | 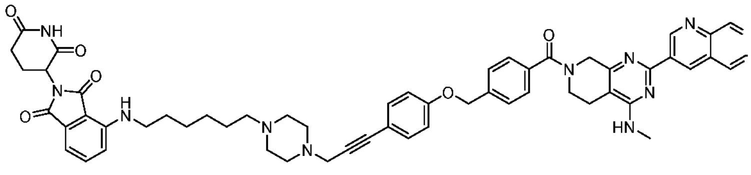<br>C2 - 4<br>LC-MS: $C_{57}H_{59}N_{10}O_6$, $[M/2+H]^+$ 490.6; found 490.5. $^1H$ NMR (400 MHz, Methanol-$d_4$) δ 9.78-9.69 (m, 1H), 9.24-9.14 (m, 1H), 8.12 (s, 2H), 7.82 (s, 1H), 7.73-7.48 (m, 6H), 7.35 (d, *J* = 8.0 Hz, 2H), 7.06-6.96 (m, 4H), 5.19 (s, 3H), 4.58 (s, 4H), 4.30 (t, *J* = 4.0 Hz, 1H), 4.10 (s, 2H), 3.76-3.71 (m, 2H), 3.53 (s, 3H), 3.16 (s, 3H), 2.84-2.65 (m, 12H), 2.48 (s, 2H), 2.10-2.08 (m, 2H), 1.70-1.56 (m, 4H). | 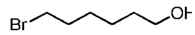<br>23d | 13.6 % |
| C2-5 | 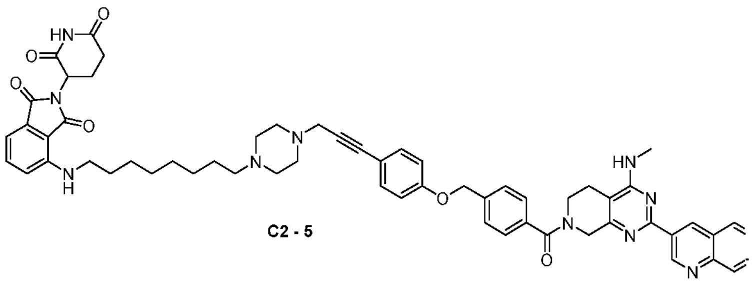<br>C2 - 5<br>LC-MS: $C_{59}H_{63}N_{10}O_6$, $[M/2+H]^+$ 504.6; found 504.7. $^1H$ NMR (400 MHz, Methanol-$d_4$) δ 9.78-9.69 (m, 1H), 9.24-9.14 (m, 1H), 8.12 (s, 2H), 7.82 (s, 1H), 7.73-7.48 (m, 6H), 7.35 (d, *J* = 8.0 Hz, 2H), 7.06-6.96 (m, 4H), 5.19 (s, 2H), 5.07-5.01 (m, 1H), 4.58 (s, 4H), 4.30 (t, *J* = 4.0 Hz, 1H), 4.10 (s, 2H), 3.76-3.71 (m, 2H), 3.53 (s, 3H), 3.16 (s, 3H), 2.84-2.65 (m, 12H), 2.49-2.47 (m, 2H), 2.10-2.08 (m, 2H), 2.01-1.85 (m, 6H), 1.67-1.53 (m, 2H). | 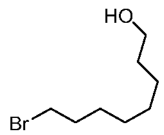<br>23e | 10.2 % |

Compound **C2-6** could be obtained by following the step 1 to step 6 of the method for Compound **C2-1**, in which Compound $\mathbf{R_{1A-3}}$ was replaced with the raw materials in the following table, and the other raw materials and operating methods remained unchanged.

| Compound No. | Structure and characterization data of compound | Raw material | Yield |
|---|---|---|---|
| C2-6 | 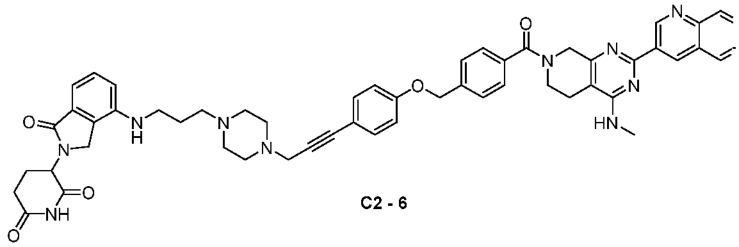 C2 - 6 | 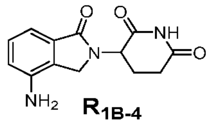 $R_{1B-4}$ | 20.1 % |
| | LC-MS: $C_{54}H_{55}N_{10}O_5$, $[M/2+H]^+$ 462.2; found 462.6. $^1H$ NMR (400 MHz, Methanol-$d_4$) δ 9.78 (s, 1H), 9.51 (s, 1H), 8.26-8.20 (m, 2H), 8.05 (s, 1H), 7.86 (s, 1H), 7.61-7.54 (m, 5H), 7.37-7.33 (m, 2H), 7.11 (d, *J* = 8.0 Hz, 1H), 7.05-6.97 (m, 2H), 6.87 (d, *J* = 8.0 Hz, 1H), 5.22-5.13 (m, 5H), 4.73 (s, 2H), 4.29-4.23 (m, 2H), 4.15 (s, 1H), 3.81-3.77 (m, 2H), 3.71 (s, 2H), 3.57-3.36 (m, 6H), 3.26 (s, 5H), 3.19-2.91 (m, 6H), 2.88-2.76 (m, 1H), 2.66 (s, 1H), 2.19-2.17 (m, 1H), 2.15-2.03 (m, 2H). | | |

**Example 5. Synthesis of Compounds C3-1 to C3-18 of the present invention**

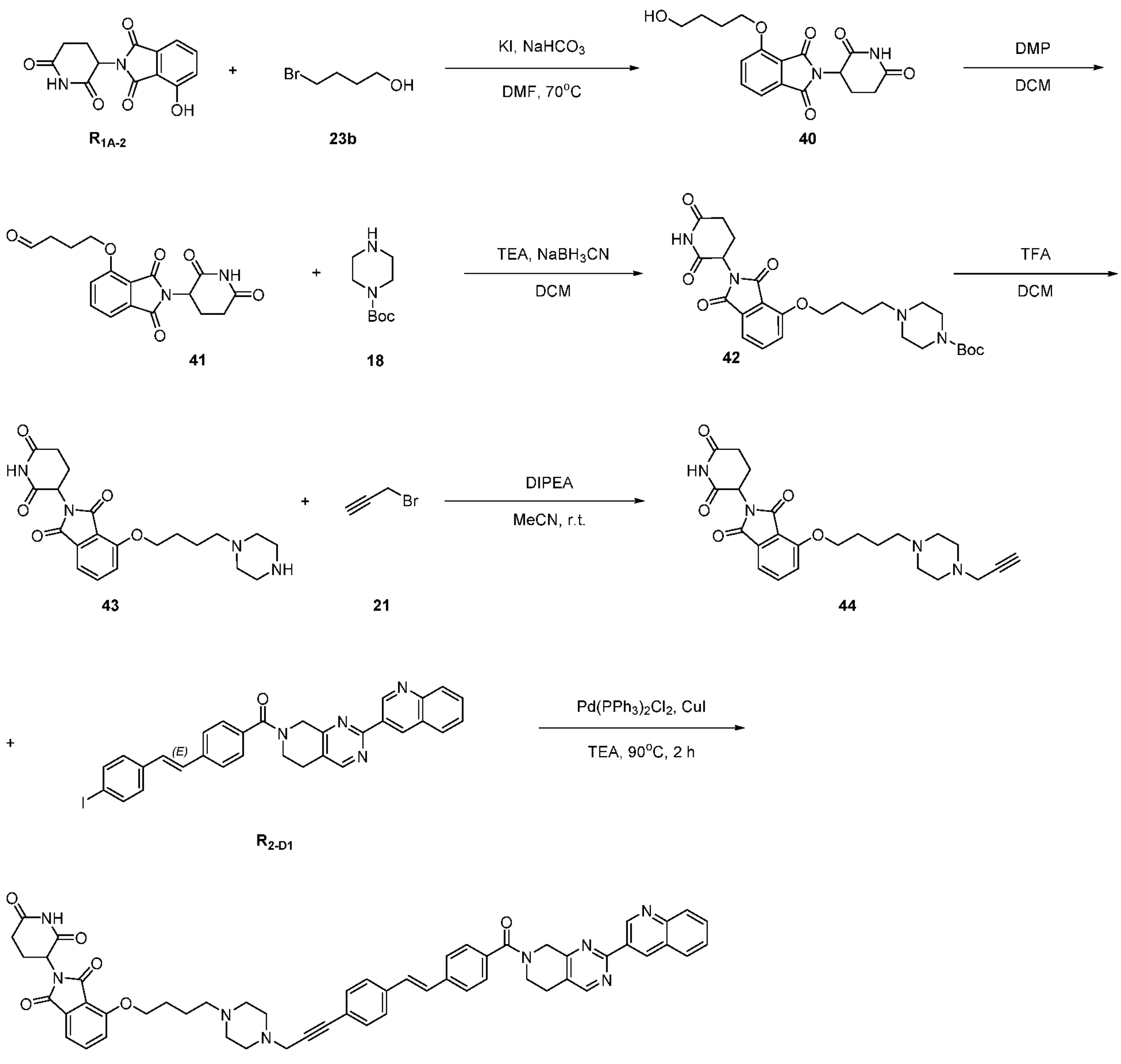


$R_{1A-2}$ 23b 40

41 18 42

43 21 44

$R_{2-D1}$

C3 - 1

### Step 1. Synthesis of Compound 40

Compound $\mathbf{R_{1A-2}}$ (1.00 mmol), Compound **23b** (1.12 mmol), potassium iodide (1.05 mmol), sodium bicarbonate (2.20 mmol) and *N,N*-dimethylformamide (5 mL) were added into a 50 mL reaction bottle, heated to 70°C, reacted under stirring for 8 hours, and then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **40** ($C_{17}H_{19}N_2O_6$, $[M+H]^+$ 347.1; found 347.3, yield 63.8%).

### Step 2. Synthesis of Compound 41

Compound **40** (0.60 mmol), Dess-Martin Periodinane (1.80 mmol) and dichloroethane (5 mL) were added to a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **41** ($C_{17}H_{17}N_2O_6$, $[M+H]^+$ 345.1; found 345.1,

yield 91.6%).

**Step 3. Synthesis of Compound 42**

Compound **41** (0.55 mmol), Compound **18** (0.65 mmol), triethylamine (2 mL), sodium cyanoborohydride (0.95 mmol) and dichloroethane (5 mL) were added in sequence to a 50 mL reaction bottle, reacted under stirring at room temperature for 2 hours, and then the reaction was quenched (LC-MS monitoring), the solvent was removed by evaporation to dryness, purification was carried out by medium-pressure preparative chromatography, and the solvent was removed by concentration under reduced pressure to obtain Compound **42** ($C_{26}H_{35}N_4O_7$, $[M+H]^+$ 515.2; found 515.1, yield 63.6%).

**Step 4. Synthesis of Compound 43**

Compound **42** (0.35 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **43** ($C_{21}H_{27}N_4O_5$, $[M+H]^+$ 415.2; found 415.1, crude product).

**Step 5. Synthesis of Compound 44**

Compound **43** (0.30 mmol), Compound **21** (0.65 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **44** ($C_{24}H_{29}N_4O_5$, $[M+H]^+$ 453.2; found 453.3, yield 83.3%).

**Step 6. Synthesis of Compound C3-1**

Under nitrogen protection, Compound $\mathbf{R_{2\text{-}D1}}$ (0.25 mmol), Compound **44** (0.25 mmol), copper iodide (0.55 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and the solvent was removed by concentration under reduced pressure to obtain Compound **C3-1** (9.0 mg, 9.78 μmol). LC-MS: $C_{55}H_{51}N_8O_6$, $[M/2+H]^+$ 460.2; found 460.5. $^1H$ NMR (400 MHz, Methanol-*d4*) δ 10.10-9.80 (m, 2H), 8.83 (s, 1H), 8.39 (s, 1H), 8.24 (s, 1H), 8.13 (s, 1H), 7.94 (s, 1H), 7.80 (td, *J* = 8.0, 4.0 Hz, 1H), 7.71 (d, *J* = 4.0 Hz, 2H), 7.62-7.54 (m, 4H), 7.48-7.43 (m, 4H), 7.30 (s, 2H), 5.13-5.06 (m, 2H), 4.87 (s, 1H), 4.32-4.27 (m, 2H), 4.26-4.05 (m, 1H), 3.98 (s, 1H), 3.78 (s, 2H), 3.51-3.47 (m, 5H), 3.45-3.42 (m, 1H), 3.19-3.07 (m, 6H), 2.86-2.81 (m, 1H), 2.76-2.75 (m, 1H), 2.71-2.66 (m, 1H), 2.15-2.11 (m, 1H), 2.04-1.99 (m, 4H).

According to the step 1 to step 6 of the method for Compound **C3-1,** the raw materials in the following table were used to replace Compound **23b,** and other raw materials and operating methods remained unchanged to obtain Compounds **C3-2** and **C3-3.**

| Compound No. | Structure and characterization data of compound | Raw material | Yield |
|---|---|---|---|
| **C3-2** | 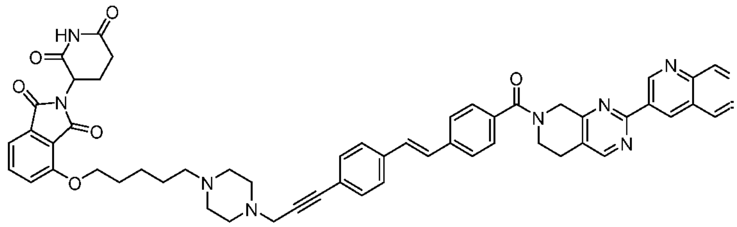<br>**C3 - 2** | 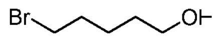<br>**23c** | 13.2 % |
| | LC-MS: $C_{56}H_{53}N_8O_6$, $[M+H]^+$ 933.4; found 933.2. $^1H$ NMR (400 MHz, Methanol-$d_4$) δ 9.93-9.62 (m, 2H), 8.80 (s, 1H), 8.23-8.17 (m, 2H), 8.16 (s, 1H), 7.99-7.70 (m, 4H), 7.62-7.54 (m, 4H), 7.47-7.43 (m, 4H), 7.30 (s, 2H), 5.12-5.07 (m, 2H), 4.30-4.26 (m, 3H), 4.23-3.81 (m, 3H), 3.73 (s, 2H), 3.67-3.42 (m, 3H), 3.31-3.21 (m, 3H), 3.19-3.05 (m, 4H), 2.85-2.70 (m, 5H), 2.00-1.92 (m, 4H), 1.72-1.67 (m, 2H). | | |
| **C3-3** | 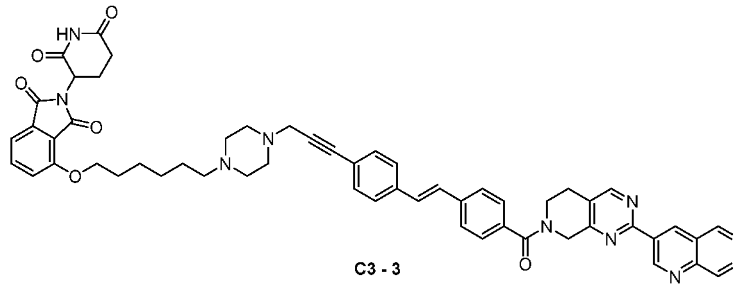<br>**C3 - 3** | 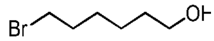<br>**23d** | 9.8% |
| | LC-MS: $C_{57}H_{55}N_8O_6$, $[M/2+H]^+$ 474.2; found 474.5. $^1H$ NMR (400 MHz, DMSO-$d_6$) δ 9.78-9.69 (m, 1H), 9.37-9.25 (s, 1H), 8.78 (s, 1H), 8.21-8.17 (m, 2H), 8.10 (s, 1H), 7.91-7.77 (m, 4H), 7.72-7.70 (m, 2H), 7.62 (d, *J* = 8.0 Hz, 2H), 7.52-7.41 (m, 4H), 7.32 (s, 2H), 5.40-5.00 (m, 1H), 4.91-4.76 (m, 2H), 4.17 (s, 2H), 3.72 (s, 4H), 3.42-3.38 (m, 2H), 3.22-3.05 (m, 8H), 2.95-2.78 (m, 3H), 2.68-2.59 (m, 2H), 2.04-2.01 (m, 1H), 1.75 (s, 2H), 1.65 (s, 2H), 1.47 (s, 2H), 1.36 (d, *J* = 4.0 Hz, 2H). | | |

According to the step 1 to step 6 of the method for Compound **C3-1,** Compound **23c** was used to replace Compound **23b,** the raw material **1** in the following table was used to replace Compound $\mathbf{R_{1A-2}}$, the raw material **2** was used to replace Compound $\mathbf{R_{2-D1}}$, and the other raw materials and operating methods remained unchanged to obtain Compounds **C3-4** to **C3-13.**

| Compound No. | Structure and characterization data of compound | Raw material 1 | Raw material 2 | Yiel d |
|---|---|---|---|---|
| C3-4 | 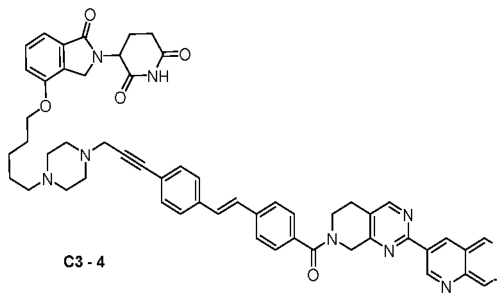 C3 - 4<br>LC-MS: $C_{56}H_{55}N_8O_5$, $[M/2+H]^+$ 460.2; found 460.6. $^1H$ NMR (400 MHz, DMSO-$d_6$) δ 9.83-9.74 (m, 1H), 9.37-9.26 (m, 1H), 8.85 (s, 1H), 8.24-8.09 (m, 2H), 8.01-7.93 (m, 1H), 7.78-7.72 (m, 3H), 7.72-7.70 (m, 2H), 7.68 (d, *J* = 4.0 Hz, 2H), 7.56 (d, *J* = 4.0 Hz, 2H), 7.50-7.44 (m, 3H), 7.38-7.24 (m, 1H), 7.06 (d, *J* = 8.0 Hz, 1H), 5.06 (s, 1H), 4.96-4.80 (m, 2H), 4.36-4.25 (m, 1H), 4.20-3.92 (m, 1H), 3.72 (s, 2H), 3.66 (d, *J* = 6.2 Hz, 2H), 3.50-3.45 (m, 2H), 3.05-2.92 (m, 9H), 2.82 (s, 2H), 2.78-2.71 (m, 2H), 2.54-2.40 (m, 2H), 2.08 (t, *J* = 8.0 Hz, 1H), 1.66 (s, 2H), 1.48 (d, *J* = 6.8 Hz, 2H), 1.28-1.17 (m, 2H). | 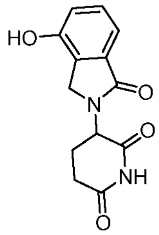 $R_{1B-3}$ | 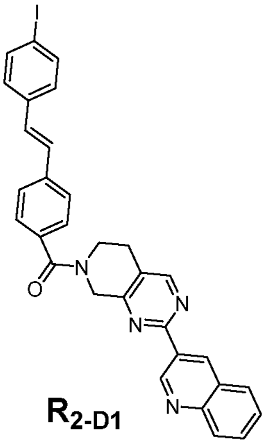 $R_{2-D1}$ | 10.6 % |
| C3-5 | 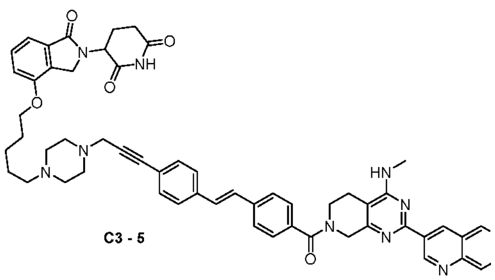 C3 - 5<br>LC-MS: $C_{57}H_{58}N_9O_5$, $[M/2+H]^+$ 474.7; found 475.1. $^1H$ NMR (400 MHz, DMSO-$d_6$) δ 9.77-9.68 (m, 1H), 9.39-9.25 (m, 1H), 8.24-8.15 (m, 2H), 7.99 (s, 1H), 7.97-7.93 (m, 3H), 7.77-7.75 (m, 2H), 7.71-7.48 (m, 5H), 7.40-7.28 (m, 3H), 7.26-7.24 (m, 1H), 5.12-5.09 (m, 1H), 4.97-4.81 (m, 1H), 4.69-4.52 (m, 1H), 4.26-4.22 (m, 1H), 4.16-4.13 (m, 1H), 3.75-3.54 (m, 6H), 3.48-3.40 (m, 4H), 3.12-2.92 (m, 12H), 2.89-2.68 (m, 4H), 2.44-2.41 (m, 2H), 2.09-2.06 (m, 1H), 1.81-1.69 (m, 2H), 1.67-1.48 (m, 2H), 1.29-1.23 (m, 2H). | 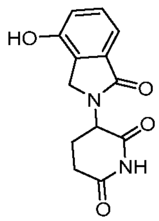 $R_{1B-3}$ | 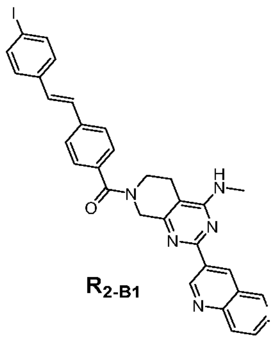 $R_{2-B1}$ | 12.3 % |

(continued)

| Compound No. | Structure and characterization data of compound | Raw material 1 | Raw material 2 | Yiel d |
|---|---|---|---|---|
| C3-6 | 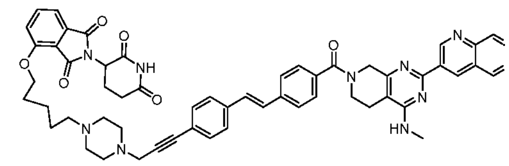 C3 - 6<br>LC-MS: $C_{57}H_{56}N_9O_6$, $[M/2+H]^+$ 481.7; found 482.0. $^1$H NMR (400 MHz, DMSO-$d_6$) δ 11.16 (s, 1H), 9.86-9.73 (m, 1H), 9.41-9.25 (m, 2H), 8.25-8.14 (m, 2H), 7.89-7.87 (m, 2H), 7.85-7.69 (m, 5H), 7.58-7.43 (m, 6H), 7.39 (s, 2H), 5.14-5.10 (m, 2H), 4.78 (s, 2H), 4.64 (s, 1H), 4.23-4.21 (m, 2H), 4.01 (s, 1H), 3.89 (s, 3H), 3.56 (s, 2H), 3.19-3.07 (m, 8H), 2.97-2.94 (m, 1H), 2.93-2.88 (m, 2H), 2.72-2.71 (m, 3H), 2.09-2.06 (m, 1H), 1.87-1.83 (m, 4H), 1.57-1.54 (m, 2H). | 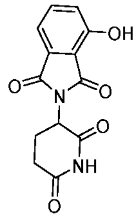 $R_{1A-2}$ | 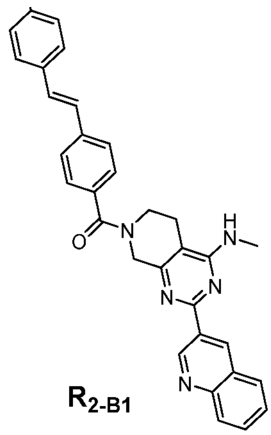 $R_{2-B1}$ | 15.6 % |
| C3-7 | 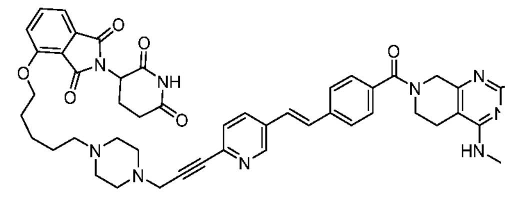 C3 - 7<br>LC-MS: $C_{56}H_{55}N_{10}O_6$, $[M/2+H]^+$ 482.2; found 482.4. $^1$H NMR (400 MHz, Methanol-$d_4$) δ 9.78 (s, 1H), 9.51 (s, 1H), 8.71 (s, 1H), 8.28-8.20 (m, 2H), 8.12 (dd, $J$ = 4.0 Hz, 1H), 8.07-8.04 (m, 1H), 7.86 (s, 1H), 7.80-7.76 (m, 3H), 7.59-7.53 (m, 3H), 7.48-7.38 (m, 3H), 7.10 (s, 1H), 5.13-5.08 (m, 1H), 4.86 (s, 1H), 4.78 (s, 1H), 4.28 (t, $J$ = 8.8 Hz, 2H), 4.18 (s, 1H), 3.95 (s, 1H), 3.78 (s, 1H), 3.59 (s, 2H), 3.53 (s, 2H), 3.33-3.29 (m, 6H), 3.23-3.13 (m, 4H), 2.86-2.82 (m, 2H), 2.76-2.74 (m, 2H), 2.72-2.67 (m, 2H), 2.15-2.10 (m, 1H), 1.99-1.93 (m, 4H), 1.72-1.67 (m, 2H). | 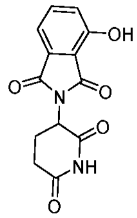 $R_{1A-2}$ | 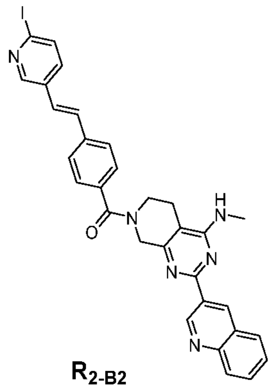 $R_{2-B2}$ | 11.8 % |

(continued)

| Compound No. | Structure and characterization data of compound | Raw material 1 | Raw material 2 | Yiel d |
|---|---|---|---|---|
| C3-8 | 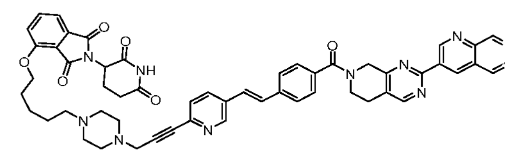<br>C3 - 8<br>LC-MS: $C_{55}H_{52}N_9O_6$, $[M/2+H]^+$ 468.0; found 468.0. $^1H$ NMR (400 MHz, Methanol-$d_4$) δ 9.98-9.94 (m, 1H), 9.73-9.49 (m, 1H), 8.81 (s, 1H), 8.72 (d, *J* = 0.8 Hz, 1H), 8.27-8.18 (m, 2H), 8.12 (dd, *J* = 1.2 Hz, 1H), 8.01 (s, 1H), 7.81-7.77 (m, 4H), 7.56 (dd, *J* = 5.6 Hz, 3H), 7.48-7.38 (m, 3H), 7.36-7.33 (m, 1H), 5.13-5.09 (m, 1H), 4.86 (s, 1H), 4.78 (s, 1H), 4.28 (t, *J* = 5.2 Hz, 2H), 4.18 (s, 1H), 3.95 (s, 1H), 3.78 (s, 2H), 3.53-3.51 (m, 2H), 3.30-3.23 (m, 5H), 3.16-3.06 (m, 3H), 2.91-2.66 (m, 5H), 2.16-2.11 (m, 1H), 2.03-1.95 (m, 4H), 1.73-1.88 (m, 2H). | 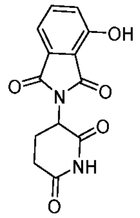<br>$R_{1A-2}$ | 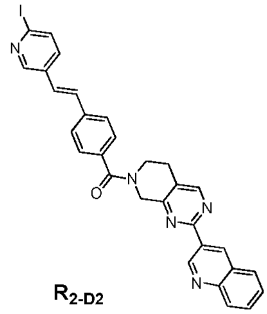<br>$R_{2-D2}$ | 11.3 % |
| C3-9 | 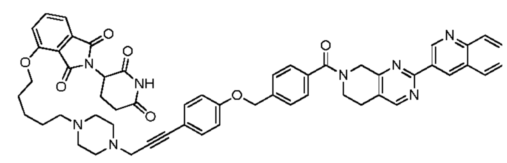<br>C3-9<br>LC-MS: $C_{55}H_{53}N_8O_7$, $[M/2+H]^+$ 469.5; found 469.5. $^1H$ NMR (400 MHz, DMSO-$d_6$) δ 9.82-9.72 (m, 1H), 9.36-9.25 (m, 1H), 8.85 (s, 1H), 8.23-8.13 (m, 2H), 7.93-7.73 (m, 3H), 7.64-7.41 (m, 8H), 7.13-7.06 (m, 2H), 5.22 (s, 2H), 5.09-5.04 (m, 1H), 4.97 (s, 1H), 4.76 (s, 1H), 4.22 (s, 2H), 3.67 (s, 2H), 3.09-2.86 (m, 9H), 2.71 (t, *J* = 2.0 Hz, 4H), 2.36 (t, *J* = 2.0 Hz, 4H), 2.05 (t, *J* = 4.4 Hz, 2H), 1.82 (s, 2H), 1.74 (s, 2H), 1.51 (s, 2H). | 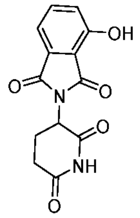<br>$R_{1A-2}$ | 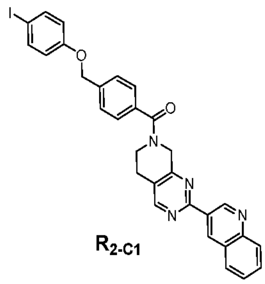<br>$R_{2-C1}$ | 13.8 % |

(continued)

| Compound No. | Structure and characterization data of compound | Raw material 1 | Raw material 2 | Yiel d |
|---|---|---|---|---|
| C3-10 | 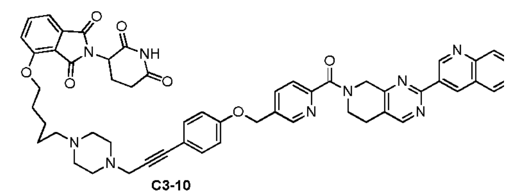 C3-10<br>LC-MS: $C_{54}H_{52}N_9O_7$, $[M/2+H]^+$ 469.7; found 469.6. $^1H$ NMR (400 MHz, Methanol-$d_4$) δ 9.98-9.84 (m, 1H), 9.73-9.55 (m, 1H), 8.82 (s, 1H), 8.75 (s, 1H), 8.32-8.14 (m, 2H), 8.10-7.96 (m, 2H), 7.89-7.78 (m, 3H), 7.46-7.39 (m, 4H), 7.03 (d, *J* = 8.0 Hz, 2H), 5.27 (s, 2H), 5.09 (s, 2H), 4.95 (s, 1H), 4.29-4.23 (m, 2H), 4.16 (s, 1H), 3.88 (t, *J* = 4.0 Hz, 1H), 3.69 (d, *J* = 4.0 Hz, 2H), 3.54-3.47 (m, 2H), 3.24-3.08 (m, 7H), 2.90-2.63 (m, 5H), 2.12-2.09 (m, 1H), 1.95-1.92 (m, 4H), 1.68 (s, 2H), 1.37-1.28 (m, 1H). | 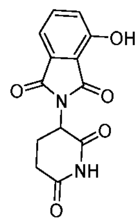 $R_{1A-2}$ | 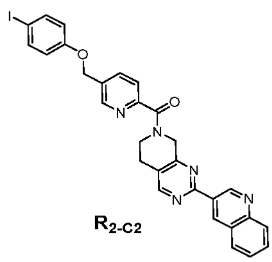 $R_{2-C2}$ | 11.6 % |
| C3-11 | 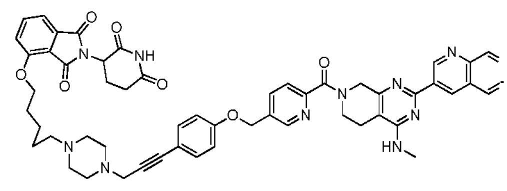 C3-11<br>LC-MS: $C_{55}H_{55}N_{10}O_7$, $[M/2+H]^+$ 484.2; found 484.5. $^1H$ NMR (400 MHz, Methanol-$d_4$) δ 9.80-9.72 (m, 1H), 9.52-9.42 (m, 1H), 8.74 (s, 1H), 8.29-8.18 (m, 2H), 8.15-8.01 (m, 2H), 7.99-7.76 (m, 3H), 7.45-7.38 (m, 4H), 7.04-7.00 (m, 2H), 5.25 (s, 2H), 5.09-5.06 (m, 1H), 4.95 (s, 2H), 4.28-4.25 (m, 2H), 4.26 (s, 1H), 3.90 (t, *J* = 5.6 Hz, 1H), 3.68 (d, *J* = 5.2 Hz, 2H), 3.48-3.47 (m, 2H), 3.25-3.17 (m, 6H), 3.14-2.87 (m, 4H), 2.86-2.61 (m, 5H), 2.12-2.09 (m, 1H), 1.94-1.93 (m, 4H), 1.69-1.65 (m, 2H), 1.44-1.42 (m, 1H). | 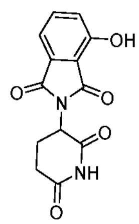 $R_{1A-2}$ | 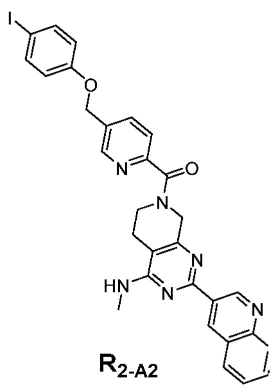 $R_{2-A2}$ | 9.8% |

(continued)

| Compound No. | Structure and characterization data of compound | Raw material 1 | Raw material 2 | Yiel d |
|---|---|---|---|---|
| C3-12 | 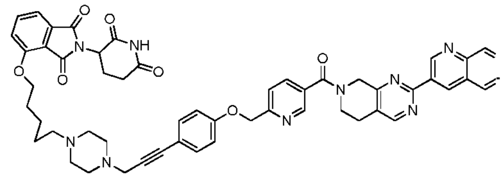<br>C3-12<br>LC-MS: $C_{54}H_{52}N_9O_7$, $[M/2+H]^+$ 469.7; found 469.0. $^1H$ NMR (400 MHz, DMSO-$d_6$) δ 9.82-9.72 (m, 1H), 9.36-9.25 (m, 1H), 8.85 (s, 2H), 8.23-8.13 (m, 2H), 7.93-7.73 (m, 3H), 7.64-7.41 (m, 8H), 7.13-7.06 (m, 2H), 5.22 (s, 2H), 5.09-5.04 (m, 1H), 4.97 (s, 3H), 4.76 (s, 2H), 4.22 (s, 4H), 3.67 (s, 2H), 3.09-2.86 (m, 10H), 2.71 (t, $J$ = 2.0 Hz, 4H), 2.36 (t, $J$ = 2.0 Hz, 4H), 2.05 (t, $J$ = 4.4 Hz, 2H), 1.82 (s, 2H), 1.74 (s, 2H), 1.51 (s, 2H). | 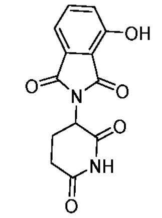<br>$R_{1A-2}$ | 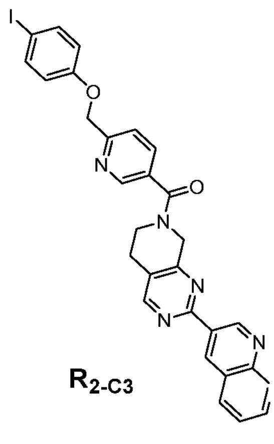<br>$R_{2-C3}$ | 8.6% |
| C3-13 | 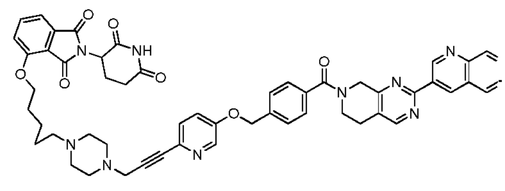<br>C3-13<br>LC-MS: $C_{54}H_{52}N_9O_7$, $[M/2+H]^+$ 469.7; found 470.2. $^1H$ NMR (400 MHz, DMSO-$d_6$) δ 9.91-9.87 (m, 1H), 9.62-9.50 (m, 1H), 8.85 (s, 1H), 8.38 (s, 2H), 8.22 (s, 1H), 8.06 (s, 1H), 7.84 (t, $J$ = 8.0 Hz, 2H), 7.64-7.46 (m, 8H), 5.31 (s, 2H), 5.10-5.05 (m, 1H), 4.98 (s, 1H), 4.86-4.77 (m, 1H), 4.23-4.21 (m, 2H), 3.91 (s, 2H), 3.72 (s, 2H), 3.44-3.38 (m, 4H), 3.23-3.09 (m, 7H), 2.99-2.90 (m, 1H), 2.87-2.63 (m, 2H), 2.56 (s, 1H), 2.09-2.06 (m, 1H), 1.84-1.77 (m, 4H), 1.60-1.50 (m, 2H). | 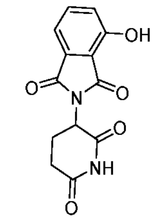<br>$R_{1A-2}$ | 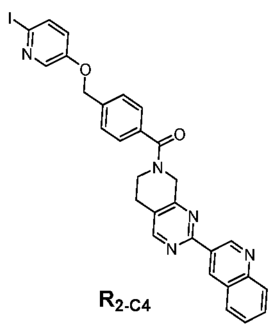<br>$R_{2-C4}$ | 10.2 % |

According to the step 1 to step 6 of the method for Compound **C3-1,** Compound **$R_{2-A1}$** was used to replace Compound **$R_{2-D1}$**, the raw materials in the following table were used to replace Compound **23b,** and the other raw materials and operating methods remained unchanged to obtain Compounds **C3-14** to **C3 -18.**

| Compound No. | Structure and characterization data of compound | Raw material | Yield |
|---|---|---|---|
| C3-14 | 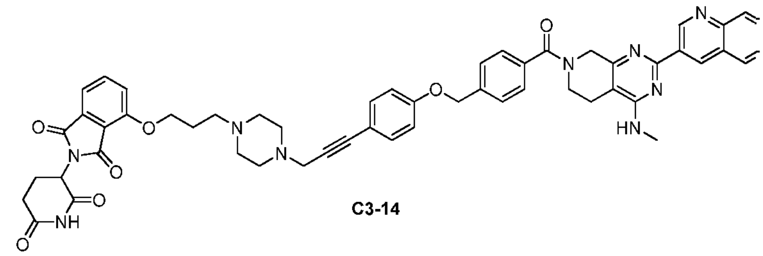<br>C3-14<br>LC-MS: $C_{54}H_{52}N_9O_7$, $[M/2+H]^+$ 470.0; found 469.9. $^1H$ NMR (400 MHz, Methanol-$d_4$) δ 9.78 (s, 1H), 9.54 (s, 1H), 8.29-8.21 (m, 2H), 8.07 (t $J$ = 8.0 Hz, 1H), 7.89-7.79 (m, 2H), 7.69-7.37 (m, 8H), 6.96 (d, $J$ = 8.0 Hz, 2H), 5.19 (s, 2H), 5.10-5.06 (m, 1H), 4.36-4.28 (m, 2H), 4.18-3.84 (m, 4H), 3.65-3.42 (m, 6H), 3.25-3.20 (m, 5H), 2.99-2.61 (m, 6H), 2.36 (t, $J$ = 8.0 Hz, 2H), 2.05-2.03 (m, 2H), 1.86-1.81 (m, 2H). | 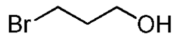<br>23a | 25.9 % |
| C3-15 | 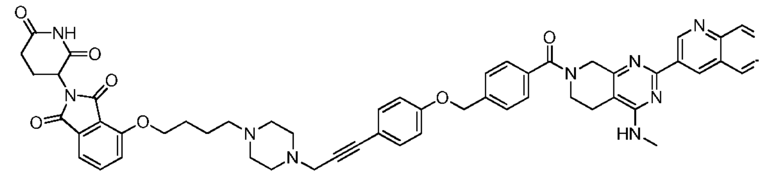<br>C3-15<br>LC-MS: $C_{55}H_{54}N_9O_7$, $[M/2+H]^+$ 477.0; found 477.0. $^1H$ NMR (400 MHz, Methanol-$d_4$) δ 9.78 (s, 1H), 9.51 (s, 1H), 8.27-8.20 (m, 2H), 8.03-7.97 (m, 1H), 7.85-7.70 (m, 2H), 7.69-7.36 (m, 8H), 6.97 (d, $J$ = 8.0 Hz, 2H), 5.19 (s, 2H), 5.12-5.07 (m, 1H), 4.38-4.29 (m, 2H), 4.12 (s, 1H), 3.90 (s, 1H), 3.88 (s, 2H), 3.70-3.44 (m, 4H), 3.24 (s, 3H), 2.99-2.65 (m, 10H), 2.13-1.98 (m, 5H), 1.46-1.26 (m, 2H), 0.99-0.87 (m, 1H). | 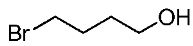<br>23b | 20.1 % |
| C3-16 | 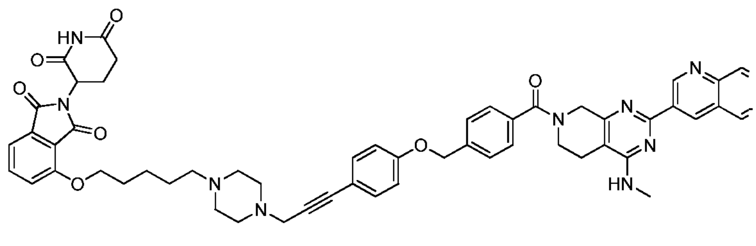<br>C3-16<br>LC-MS: $C_{56}H_{56}N_9O_7$, $[M/2+H]^+$ 483.7; found 484.0. $^1H$ NMR (400 MHz, Methanol-$d_4$) δ 9.78 (s, 1H), 9.52 (s, 1H), 8.28-8.22 (m, 2H), 8.20 (s, 1H), 8.06 (s, 1H), 7.87-7.76 (m, 1H), 7.62-7.55 (m, 4H), 7.45 (t, $J$ = 8.0 Hz, 2H), 7.38 (d, $J$ = 8.0 Hz, 2H), 6.98 (d, $J$ = 8.0 Hz, 2H), 5.19 (s, 2H), 5.10-5.06 (m, 1H), 4.86 (s, 1H), 4.56 (s, 1H), 4.26 (t, $J$ = 5.6 Hz, 2H), 4.18-4.12 (m, 1H), 3.82 (s, 1H), 3.69 (s, 2H), 3.58-3.45 (m, 3H), 3.31-3.25 (m, 6H), 3.22-2.93 (m, 4H), 2.86-2.66 (m, 5H), 2.13-2.08 (m, 1H), 1.97-1.91 (m, 4H), 1.71-1.66 (m, 2H). | 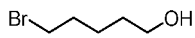<br>23c | 19.8 % |

(continued)

| Compound No. | Structure and characterization data of compound | Raw material | Yield |
|---|---|---|---|
| C3-17 | 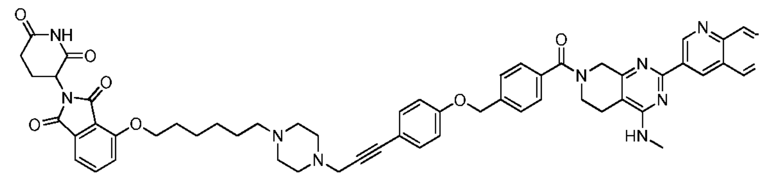 C3-17 | 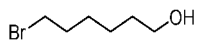 23d | 10.6 % |
| | LC-MS: $C_{57}H_{58}N_9O_7$, $[M/2+H]^+$ 491.0; found 491.0. $^1$H NMR (400 MHz, Methanol-$d_4$) $\delta$ 9.82-9.79 (m, 1H), 9.28-9.14 (m, 1H), 8.21-8.06 (m, 2H), 7.81-7.52 (m, 7H),7.43-7.05 (m, 4H), 6.97 (d, *J* = 8.0 Hz, 2H), 5.19 (s, 2H), 5.12-5.07 (m, 1H), 4.58 (s, 4H), 4.38-4.21 (m, 4H), 3.88 (s, 2H), 3.64 (s, 2H), 3.19 (s, 3H), 3.16-2.58 (m, 12H), 2.13-1.98 (m, 2H), 1.85 (t, *J* = 8.0 Hz, 2H), 1.60-1.58 (m, 2H), 1.44-1.42 (m, 2H), 1.31-1.28 (m, 2H). | | |
| C3-18 | 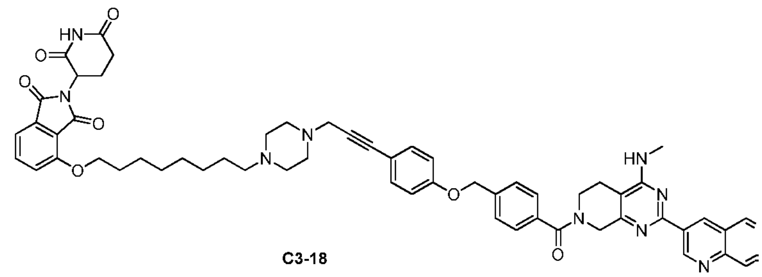 C3-18 | 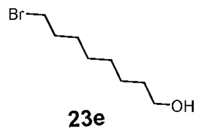 23e | 9.8% |
| | LC-MS: $C_{59}H_{62}N_9O_7$, $[M/2+H]^+$ 505.1; found 505.0. $^1$H NMR (400 MHz, Methanol-$d_4$) $\delta$ 9.80-9.76 (m, 1H), 9.25-9.14 (m, 1H), 8.06 (s, 2H), 7.97-7.52 (m, 7H),7.46-7.21 (m, 4H), 6.97 (d, *J* = 8.0 Hz, 2H), 5.19 (s, 2H), 5.12-5.07 (m, 1H), 4.58 (s, 4H), 4.28 (t, *J* = 8.0 Hz, 2H), 4.20 (t, *J* = 8.0 Hz, 2H), 3.54 (s, 2H), 3.16 (s, 3H), 2.99-2.65 (m, 12H), 2.51-2.49 (m, 4H), 2.20-2.09 (m, 2H), 1.84 (t, *J* = 8.0 Hz, 2H), 1.75-1.68 (m, 2H). | | |

Example 5B, Synthesis of Compound C3-19 of the present invention

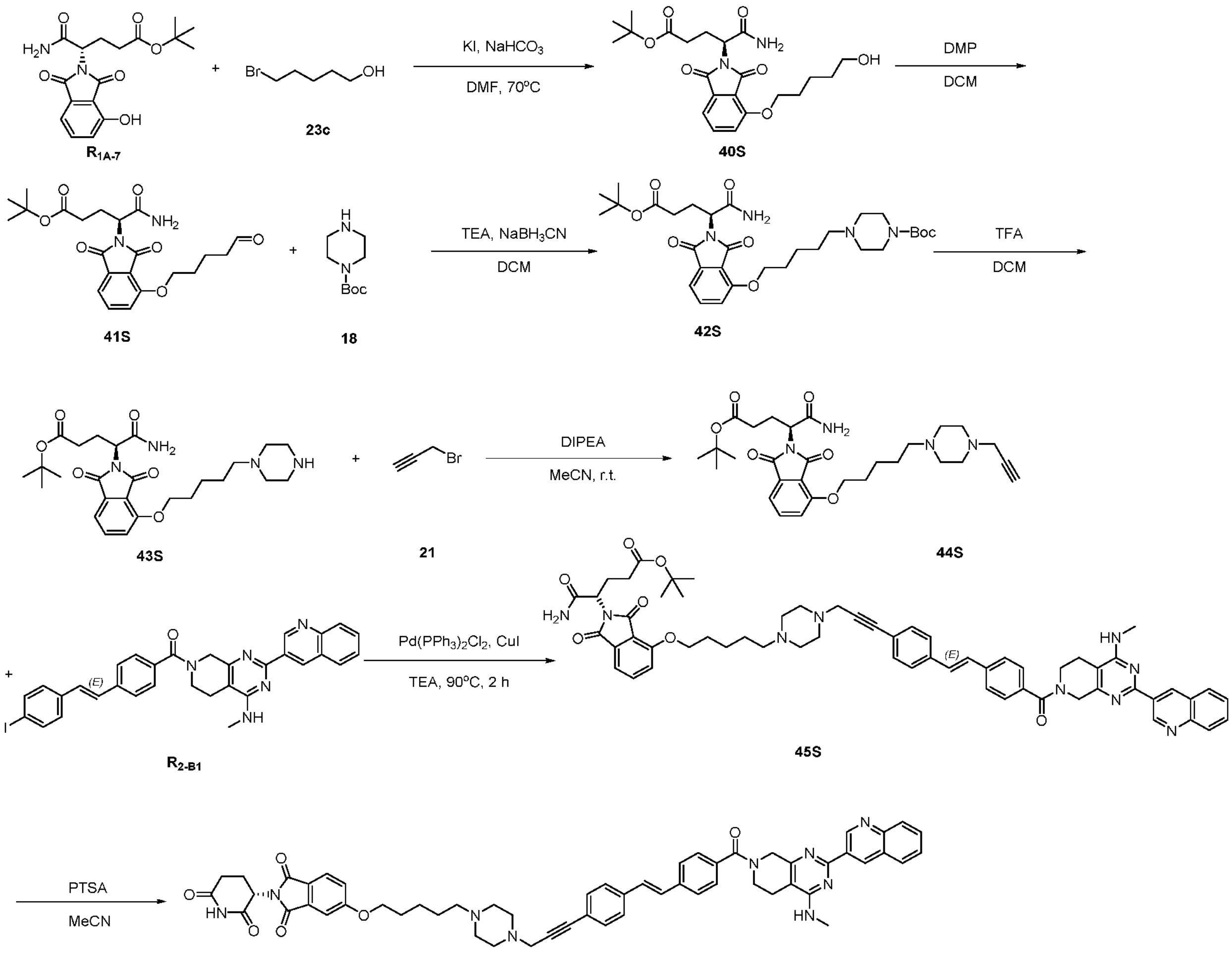


R1A-7 23c 40S 41S 18 42S 43S 21 44S R2-B1 45S

C3-19 H

**Step 1. Synthesis of Compound** 40S

Compound $\mathbf{R_{1A-7}}$ (1.00 mmol), Compound **23c** (1.12 mmol), potassium iodide (1.05 mmol), sodium bicarbonate (2.20 mmol) and *N,N*-dimethylformamide (5 mL) were added into a 50 mL reaction bottle, heated to 70°C, reacted under stirring for 8 hours, and then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **40S** ($C_{22}H_{31}N_2O_7$, $[M+H]^+$ 435.5; found 435.5, yield 45.1%).

**Step 2. Synthesis of Compound 41S**

Compound **40S** (0.45 mmol), Dess-Martin Periodinane (1.20 mmol) and dichloromethane (5 mL) were added to a 50 mL reaction bottle, reacted under stirring at room temperature for 1 hour, and then added with water to quench the reaction (LC-MS monitoring). Extraction was carried out with saturated sodium chloride solution (25 mL) and ethyl acetate (3× 25 mL), the organic phases were combined, the organic phase was dried over anhydrous sodium sulfate, evaporated to dryness to remove the solvent, purified by medium-pressure preparative chromatography, and concentrated under reduced pressure to remove the solvent to obtain Compound **41S** ($C_{22}H_{29}N_2O_7$, $[M+H]^+$ 433.5; found 433.2, yield 64.2%).

**Step 3. Synthesis of Compound 42S**

Compound **41S** (0.21 mmol), Compound **18** (0.42 mmol), triethylamine (2 mL), sodium cyanoborohydride (0.35 mmol) and dichloromethane (5 mL) were added in sequence to a 50 mL reaction bottle, and reacted under stirring at room temperature for 2 hours, then the reaction was quenched (LC-MS monitoring), the solvent was removed by evaporation to dryness, purification was carried out by medium-pressure preparative chromatography, and the solvent was removed by concentration under reduced pressure to obtain Compound **42S** ($C_{31}H_{47}N_4O_8$, $[M+H]^+$ 603.7; found 603.8, yield 68.9%).

**Step 4. Synthesis of Compound 43S**

Compound 42S (0.10 mmol) was dissolved in dichloromethane (5 mL) in a 50 mL reaction bottle, then trifluoroacetic acid (1.5 mL) was added dropwise, the reaction was carried out under stirring at room temperature for 0.5 hour, and the solvent was removed by concentration under reduced pressure to obtain Compound **43S** ($C_{21}H_{27}N_4O_5$, $[M+H]^+$ 415.2; found 415.1, crude product).

**Step 5. Synthesis of Compound 44S**

Compound **43S** (crude product), Compound **21** (0.35 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **44S** ($C_{29}H_{41}N_4O_6$, $[M+H]^+$ 541.6; found 541.3, yield 83.3%).

**Step 6. Synthesis of Compound 45S**

Under nitrogen protection, Compound $\mathbf{R_{2\text{-}B1}}$ (0.10 mmol), Compound **44S** (0.09 mmol), copper iodide (0.25 mmol), triethylamine (5 mL), and $Pd(PPh_3)_2Cl_2$ (0.25 mol%) were added in sequence into a 50mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and the solvent was removed by concentration under reduced pressure to obtain Compound **45S** ($C_{61}H_{66}N_9O_7$, $[M/2+H]^+$ 518.6; found 518.3, yield 34.3%).

**Step 7. Synthesis of Compound C3-19**

Compound **45S** (27.00 μmol), *p*-toluenesulfonic acid (0.10 mmol) and acetonitrile (5 mL) were added to a 50 mL reaction bottle, reacted under stirring at room temperature for 1 hour, and then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **C3-19** (5.00 mg, 5.19μmol). LC-MS: $C_{57}H_{56}N_9O_6$, $[M/2+H]^+$ 482.0; found 482.0. $^1$H NMR (600 MHz, DMSO-*d6*) δ 11.11 (s, 1H), 9.82 - 9.73 (m, 1H), 9.24-9.13 (m, 2H), 8.15-8.07 (m, 2H), 7.84-7.82 (m, 2H), 7.74-7.65 (m, 5H), 7.53 (dd, *J* = 8.5, 2.4 Hz, 3H), 7.47 (dd, *J* = 7.6, 3.8 Hz, 3H), 7.32 (s, 2H), 5.12-5.05 (m, 2H), 4.73 (s, 1H), 4.56 (s, 1H), 4.24 (t, *J* = 6.2 Hz, 2H), 3.97 (s, 1H), 3.69 (s, 3H), 3.12-2.91 (m, 7H), 2.88-2.85 (m, 2H), 2.65-2.62 (m, 6H), 2.39 (s, 2H), 2.07-2.02 (m, 2H), 1.83-1.81 (m, 2H), 1.75 (s, 2H), 1.52-1.51 (m, 2H).

**Example 6.** Synthesis of Compound **D1-1** of the present invention

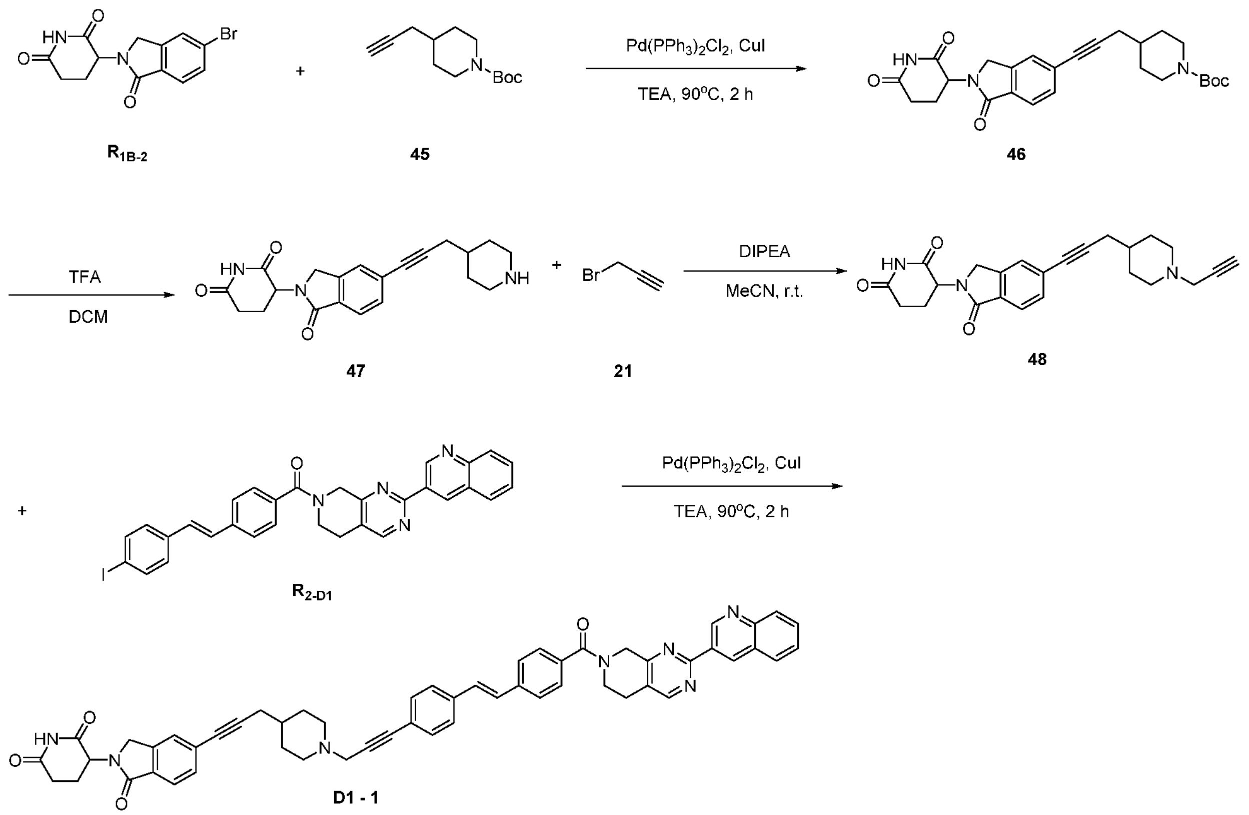


R1B-2 45 46 47 21 48 R2-D1 D1 - 1

**Step 1. Synthesis of Compound 46**

Under nitrogen protection, Compound **$R_{1B-2}$** (1.00 mmol), Compound **45** (1.05 mmol), copper iodide (2.05 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and the solvent was removed by concentration under reduced pressure to obtain Compound **46** ($C_{26}H_{32}N_3O_5$, $[M+H]^+$ 466.2; found 466.4, yield 46.8%).

**Step 2. Synthesis of Compound 47**

Compound **46** (0.45 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **47** ($C_{21}H_{24}N_3O_3$, $[M+H]^+$ 366.2; found 366.3, crude product).

**Step 3. Synthesis of Compound 48**

Compound **47** (0.35 mmol), Compound **21** (0.65 mmol), *N,N*-diisopropylethylamine (1.25 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **48** ($C_{24}H_{26}N_3O_3$, $[M+H]^+$ 366.2; found 366.3, crude product).

**Step 4. Synthesis of Compound D1-1**

Under nitrogen protection, Compound $\mathbf{R_{2\text{-}D1}}$ (0.25 mmol), Compound **48** (0.25 mmol), copper iodide (0.55 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the organic combined phase was dried over anhydrous sodium sulfate, evaporated to dryness to remove the solvent, and separated by column chromatography (the volume ratio of the eluents used was petroleum ether/ethyl acetate = 1:100 to 1:5), and the solvent was removed by concentration under reduced pressure to obtain Compound **D1-1** (1.1 mg, 1.26 μmol). LC-MS: $C_{55}H_{48}N_7O_4$, $[M+H]^+$ 435.7; found 436.2. $^1H$ NMR (400 MHz, DMSO-*d6*) δ 9.87-9.83 (m, 1H), 9.31-9.29 (m, 1H), 8.85 (s, 1H), 8.21 (s, 1H), 8.09 (s, 1H), 7.85 (s, 1H), 7.75-7.65 (m, 7H), 7.57-7.46 (m, 5H), 7.38 (s, 2H), 5.12 -5.10 (m, 1H), 4.94-4.80 (m, 2H), 4.46-4.43 (m, 1H), 4.34-4.31 (m, 1H), 3.99 (s, 1H), 3.73 (s, 1H), 3.53 (s, 2H), 2.99 (s, 2H), 2.91 (s, 3H), 2.62-2.59 (m, 1H), 2.45-2.39 (m, 2H), 2.22 (s, 2H), 2.01 (s, 1H), 1.83-1.81 (m, 2H), 1.54 (s, 1H), 1.39-1.37 (m, 2H), 1.26-1.23 (m, 2H).

**Example 7.** Synthesis of Compounds **D2-1, D2-1(R), D2-2, D2-3, D2-4, D2-4-A, D2-5, D2-6,** and **D2-7** of the present invention

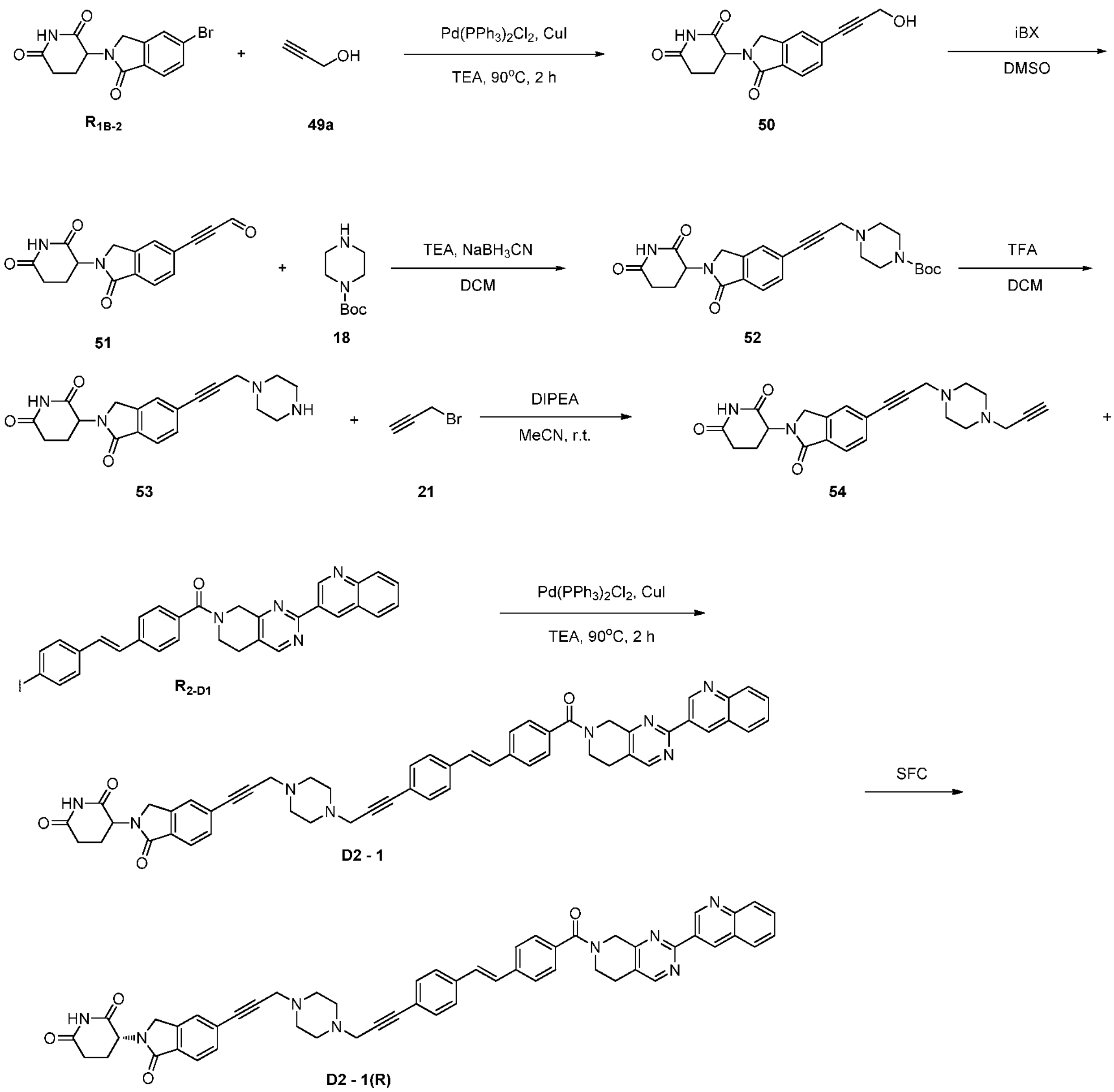


$R_{1B-2}$ 49a 50 51 18 52 53 21 54 $R_{2-D1}$ D2 - 1 D2 - 1(R)

**Step 1. Synthesis of Compound 50**

Under nitrogen protection, Compound $\mathbf{R_{1B-2}}$ (1.00 mmol), Compound **49a** (1.02 mmol), copper iodide (2.00 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1: 100 to 1:5), and the solvent was removed by concentration under reduced pressure to obtain Compound **50** ($C_{16}H_{15}N_2O_4$, $[M+H]^+$ 299.1; found 299.3, yield 48.9%).

**Step 2. Synthesis of Compound 51**

Compound 50 (0.45 mmol), 2-iodoxybenzoic acid (1.02 mmol) and DMSO (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated

under reduced pressure to remove the solvent to obtain Compound **51** ($C_{16}H_{13}N_2O_4$, $[M+H]^+$ 297.1; found 297.2, 66.7%).

**Step 3. Synthesis of Compound 52**

Compound **51** (0.30 mmol), Compound **18** (0.45 mmol), triethylamine (2 mL), sodium cyanoborohydride (0.55 mmol) and dichloroethane (5 mL) were added in sequence to a 50 mL reaction bottle, and reacted under stirring at room temperature for 2 hours, then the reaction was quenched (LC-MS monitoring), the solvent was removed by evaporation to dryness, purification was carried out by medium-pressure preparative chromatography, and the solvent was removed by concentration under reduced pressure to obtain Compound **52** ($C_{25}H_{31}N_4O_5$, $[M+H]^+$ 467.2; found 467.1, yield 71.2%).

**Step 4. Synthesis of Compound 53**

Compound **52** (0.21 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **53** ($C_{20}H_{23}N_4O_3$, $[M+H]^+$ 367.2; found 367.1, crude product).

**Step 5. Synthesis of Compound 54**

Compound **53** (0.20 mmol), Compound **21** (0.55 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **54** ($C_{23}H_{25}N_4O_3$, $[M+H]^+$ 405.2; found 405.1, yield 76.9%).

**Step 6. Synthesis of Compound D2-1**

Under nitrogen protection, Compound $\mathbf{R_{2\text{-}D1}}$ (0.15 mmol), Compound **54** (0.15 mmol), copper iodide (0.45 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, purified by column chromatography (the volume ratio of the eluents used was petroleum ether/ethyl acetate = 1:100 to 1:5), and the solvent was removed by concentration under reduced pressure to obtain Compound **D2-1** (19.0 mg, 21.8 μmol). LC-MS: $C_{54}H_{47}N_8O_4$, $[M+H]^+$ 872.01; found 871.3. $^1H$ NMR (400 MHz, DMSO-*d6*) δ 11.01 (s, 1H), 9.87-9.83 (m, 1H), 9.31-9.29 (m, 1H), 8.86 (s, 1H), 8.21 (s, 1H), 8.07 (s, 1H), 7.95 (s, 1H), 7.77-7.73 (m, 2H), 7.72-7.70 (m, 3H), 7.66 -7.63 (m, 2H), 7.58-7.54 (m, 3H), 7.48-7.46 (m, 2H), 7.40 (s, 2H), 5.14-5.09 (m, 1H), 4.95-4.81 (m, 1H), 4.48-4.43 (m, 1H), 4.35-4.31 (m, 1H), 4.06 (s, 1H), 3.59-3.56 (m, 3H), 3.17-3.16 (m, 1H), 3.00-2.97 (m, 2H), 2.92-2.86 (m, 2H), 2.68-2.67 (m, 3H), 2.66 (s, 4H), 2.23-2.02 (m, 3H), 2.00-1.97 (m, 2H).

**Step 7. Synthesis of Compound D2-1(R)**

Compound **D2-1** was separated by SFC (A:0.1%DEA/EtOH, B:0.1%DEA/DCM) to obtain Compound **D2-1(R)** (58.5 mg, found 871.4. $^1H$ NMR (600 MHz, DMSO-*d6*) δ 11.01 (s, 1H), 9.83 - 9.74 (m, 1H), 9.31 - 9.24 (m, 1H), 8.86 (s, 1H), 8.22 (d, *J* = 9.0 Hz, 1H), 8.11 (s, 1H), 7.86 (s, 1H), 7.81 - 7.77 (m, 4H), 7.76 - 7.74 (m, 3H), 7.68 (t, *J* = 8.4 Hz, 1H), 7.60 - 7.55 (m, 4H), 7.44 - 7.42 (m, 2H), 5.14 - 5.11 (m, 1H), 4.95 - 4.80 (m, 2H), 4.49 - 4.46 (m, 1H), 4.37 - 4.34 (m, 1H), 4.17 - 4.12 (m, 6H), 4.04 - 3.92 (m, 6H), 3.07 - 3.06 (m, 3H), 2.94 - 2.88 (m, 2H), 2.62 - 2.60 (m, 1H), 2.44 - 2.37 (m, 1H), 2.03 - 2.01 (m, 1H). Purity > 90%.

According to the step 1 to step 6 of the method for Compound **D2-1,** the raw material 1 in the following table was used to replace Compound $\mathbf{R_{1B\text{-}2}}$, and the raw material **2** in the following table was used to replace Compound **49a,** and the other raw materials and operating methods remained unchanged to obtain Compounds **D2-2** to **D2-5.**

| Compound No. | Structure and characterization data of compound | Raw material 1 | Raw material 2 | Yield |
|---|---|---|---|---|
| **D2-2** | 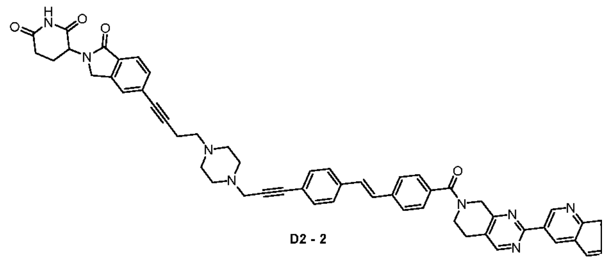<br>D2 - 2<br>LC-MS: $C_{55}H_{49}N_8O_4$, $[M/2+H]^+$ 443.5; found 443.7. $^1H$ NMR (400 MHz, DMSO-$d_6$) δ 11.01 (s, 1H), 9.84-9.78 (m, 1H), 9.34-9.28 (m, 1H), 8.86 (s, 1H), 8.23 (s, 1H), 8.11 (s, 1H), 7.87 (s, 1H), 7.74-7.63 (m, 7H), 7.56-7.49 (m, 5H), 7.40 (s, 2H), 5.12-5.10 (m, 1H), 4.95-4.80 (m, 2H), 4.47-4.47 (m, 1H), 4.35-4.32 (m, 1H), 4.04 (s, 2H), 3.36 (s, 5H), 2.98-2.89 (m, 11H), 2.61-2.50 (m, 1H), 2.39 (s, 2H), 2.01 (s, 1H). | 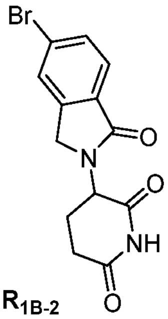<br>**$R_{1B-2}$** | 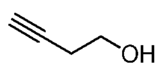<br>**49b** | 26.8 % |
| **D2-3** | 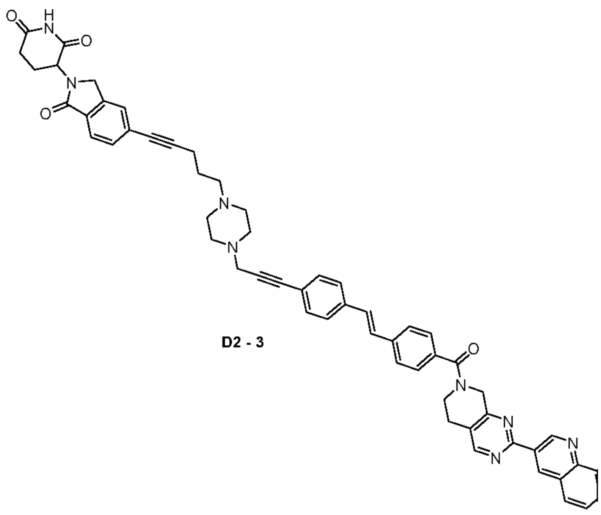<br>D2 - 3<br>LC-MS: $C_{56}H_{51}N_8O_4$, $[M/2+H]^+$ 450.5; found 450.5. $^1H$ NMR (400 MHz, Methanol-$d_4$) δ 9.95-9.80 (m, 1H), 9.72-9.60 (m, 1H), 8.81 (s, 1H), 8.30-8.25 (m, 2H), 8.18 (s, 1H), 8.04 (s, 1H), 7.75-7.72 (m, 3H), 7.61-7.54 (m, 6H), 7.43 (d, *J* = 8.0 Hz, 2H), 7.32-7.30 (m, 2H), 5.16-5.12 (m, 2H), 4.46 (d, *J* = 8.0 Hz, 3H), 3.97 (s, 2H), 3.77 (s, 2H), 3.72 (t, *J* = 4.0 Hz, 1H), 3.48-3.39 (m, 3H), 3.13-2.85 (m, 6H), 2.80-2.75 (m, 2H), 2.66-2.63 (m, 1H), 2.48 (t, *J* = 8.0 Hz, 2H), 2.46-2.45 (m, 1H), 2.18-2.15 (m, 1H), 2.14-2.07 (m, 2H), 1.88-1.85 (m, 1H). | 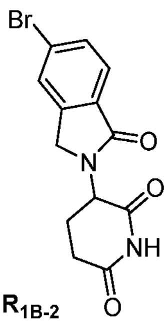<br>**$R_{1B-2}$** | 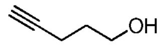<br>**49c** | 24.5 % |

(continued)

| Compound No. | Structure and characterization data of compound | Raw material 1 | Raw material 2 | Yield |
|---|---|---|---|---|
| D2-4 | 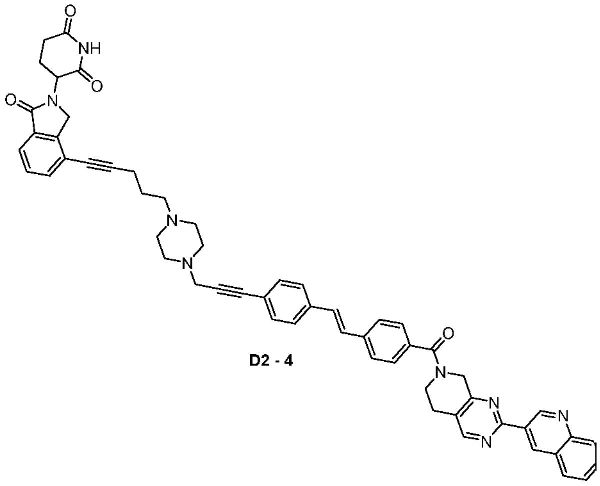<br>D2 - 4<br>LC-MS: $C_{56}H_{51}N_8O_4$, $[M/2+H]^+$ 450.6; found 450.7. $^1H$ NMR (400 MHz, DMSO-$d_6$) δ 9.86-9.78 (m, 1H), 9.46-9.34 (m, 1H), 8.86 (s, 1H), 8.29-8.21 (m, 2H), 8.16 (s, 1H), 7.97-7.78 (m, 4H), 7.76-7.67 (m, 3H), 7.59-7.55 (m, 3H), 7.50 (d, *J* = 8.0 Hz, 2H), 7.41-7.38 (m, 2H), 5.13-5.10 (m, 1H), 4.97-4.81 (m, 2H), 4.54-4.50 (m, 1H), 4.39-4.34 (m, 1H), 3.79-3.61 (m, 4H), 3.45-3.22 (m, 6H), 3.10-2.88 (m, 7H), 2.68-2.54 (m, 2H), 2.46-2.41 (m, 2H), 2.09-1.95 (m, 3H). | 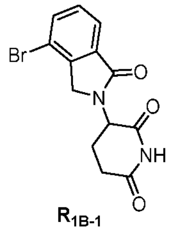<br>$R_{1B-1}$ | 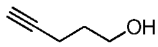<br>49c | 10.8 % |

(continued)

| Compound No. | Structure and characterization data of compound | Raw material 1 | Raw material 2 | Yield |
|---|---|---|---|---|
| D2-4-A | 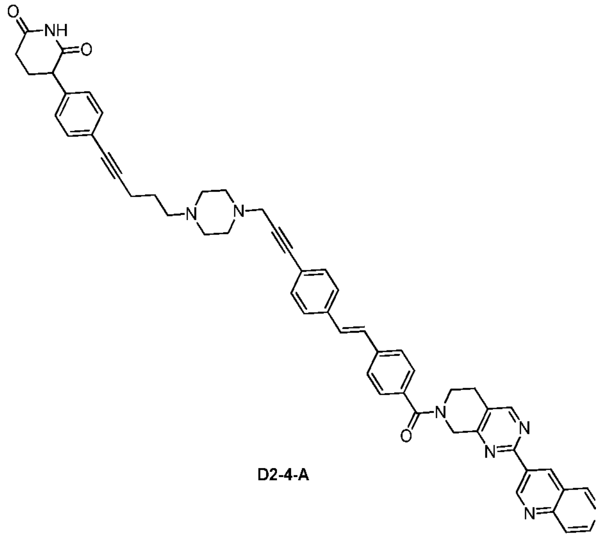<br>D2-4-A<br>LC-MS: $C_{54}H_{50}N_7O_3$, $[M+H]^+$ 844.4; found 844.3. $^1H$ NMR (600 MHz, Methanol-$d_4$) δ 10.86 (s, 1H), 9.83-9.76 (m, 1H), 9.31-9.26 (m, 1H), 8.86 (s, 1H), 8.23 (br, 1H), 8.11 (br, 1H), 7.86 (s, 1H), 7.74 (d, *J* = 8.4 Hz, 2H), 7.71 (d, *J* = 3.6 Hz, 2H), 7.67 (d, *J* = 8.4 Hz, 2H), 7.56 (d, *J* = 8.4 Hz, 2H), 7.49-7.39 (m, 5H), 7.22 (d, *J* = 8.4 Hz, 2H), 4.95-4.80 (m, 2H), 4.02 (s, 1H), 3.98-3.81 (m, 2H), 3.39 (s, 2H), 3.21 (s, 2H), 3.09-3.04 (m, 3H), 2.99-2.98 (m, 3H), 2.70-2.64 (m, 4H), 2.56-2.54 (m, 2H), 2.21-2.18 (m, 2H), 2.03-1.93 (m, 4H). Purity > 99%. | 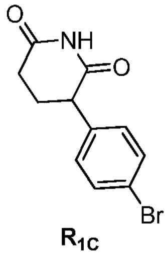<br>$R_{1C}$ | 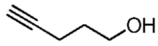<br>49c | 19.6 % |

(continued)

| Compound No. | Structure and characterization data of compound | Raw material 1 | Raw material 2 | Yield |
|---|---|---|---|---|
| **D2-5** | 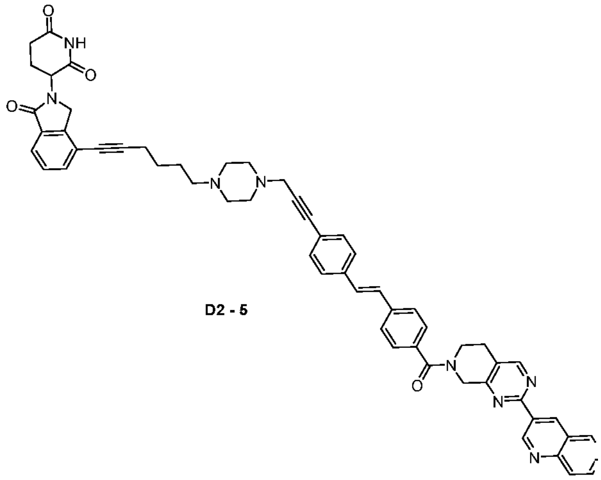 D2 - 5 <br> LC-MS: $C_{57}H_{53}N_8O_4$, $[M/2+H]^+$ 457.2; found 457.7. $^1H$ NMR (400 MHz, DMSO-$d_6$) δ 9.85-9.76 (m, 1H), 9.43-9.32 (m, 1H), 8.85 (s, 1H), 8.27-8.14 (m, 2H), 7.95 (s, 1H), 7.77-7.73 (m, 4H), 7.72-7.66 (m, 3H), 7.60-7.55 (m, 3H), 7.49 (d, *J* = 8.0 Hz, 2H), 7.41-7.38 (m, 2H), 5.12-5.09 (m, 1H), 4.97-4.81 (m, 2H), 4.52-4.48 (m, 1H), 4.37-4.33 (m, 1H), 3.88-3.76 (m, 4H), 3.56-3.37 (m, 3H), 3.34-2.86 (m, 11H), 2.68-2.54 (m, 2H), 2.46-2.41 (m, 2H), 2.09-2.06 (m, 1H), 1.82-1.78 (m, 2H), 1.68-1.62 (m, 2H). | 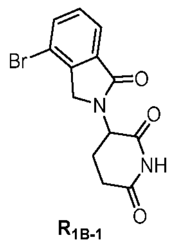 $R_{1B-1}$ | 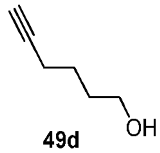 **49d** | 11.2 % |

According to the step 1 to step 6 of the method for Compound **D2-1,** Compound $\mathbf{R_{1B-1}}$ was used to replace Compound $\mathbf{R_{1B-2}}$, the raw material **1** in the following table was used to replace Compound $\mathbf{R_{2\text{-}D1}}$, the raw material **2** in the following table was used to replace Compound **49a,** and the other raw materials and operating methods remained unchanged to obtain Compounds **D2-6, D2-7, D2-8, D2-9** and **D2-10.**

| Compound No. | Structure and characterization data of compound | Raw material 1 | Rawmaterial 2 | Yield |
|---|---|---|---|---|
| **D2-6** | 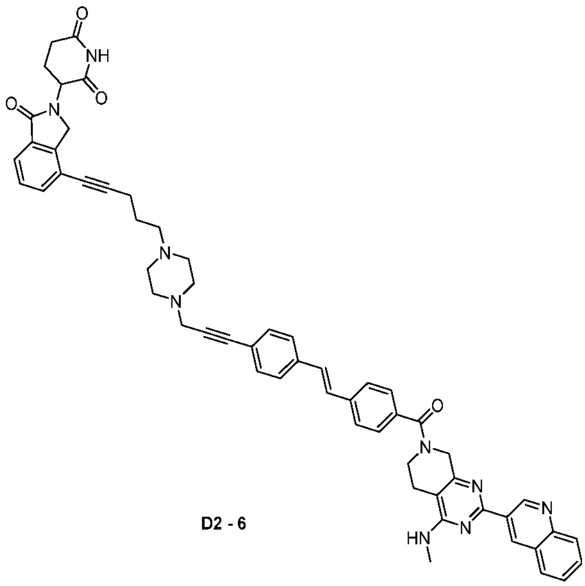<br>D2 - 6<br><br>LC-MS: $C_{57}H_{54}N_9O_4$, $[M/2+H]^+$ 465.1; found 465.1. $^1H$ NMR (400 MHz, DMSO-$d_6$) δ 9.76-9.69 (m, 1H), 9.36-9.23 (m, 1H), 8.24-8.15 (m, 2H), 8.14 (s, 1H), 7.77-7.75 (m, 4H), 7.71-7.66 (m, 3H), 7.57-7.52 (m, 3H), 7.50-7.48 (m, 2H), 7.37 (s, 2H), 5.14-5.09 (m, 1H), 4.79-4.61 (m, 2H), 4.54-4.50 (m, 1H), 4.39-4.35 (m, 1H), 3.72 (s, 4H), 3.52-3.23 (m, 6H), 3.20 (s, 4H), 3.12-2.87 (m, 4H), 2.69-2.60 (m, 5H), 2.46-2.41 (m, 2H), 2.11-1.95 (m, 3H). | 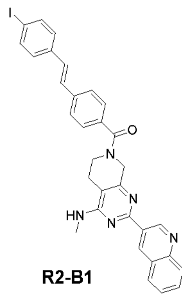<br>R2-B1 | 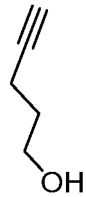<br>**49c** | 21.5 % |

(continued)

| Compound No. | Structure and characterization data of compound | Raw material 1 | Raw material 2 | Yield |
|---|---|---|---|---|
| D2-7 | 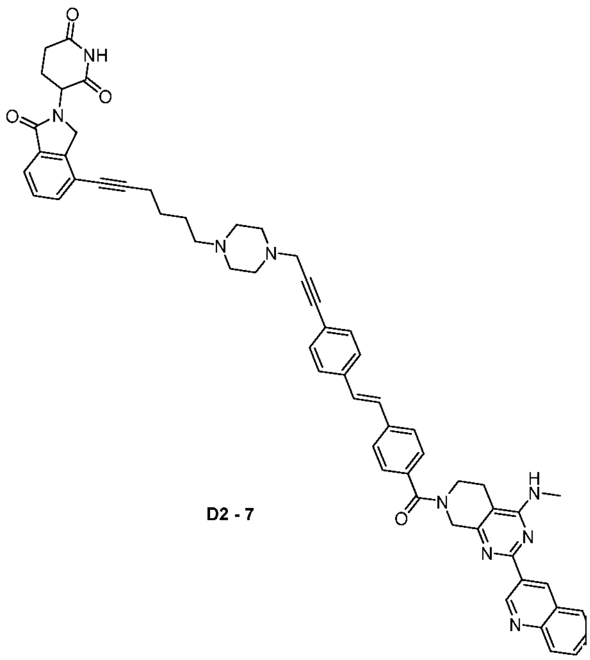 D2 - 7 | 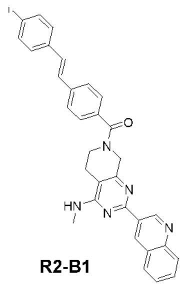 R2-B1 | 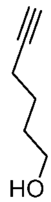 49d | 13.6 % |
| | LC-MS: $C_{58}H_{56}N_9O_4$, $[M+H]^+$943.1; found 472.2. $^1H$ NMR (400 MHz, DMSO-$d_6$) δ 9.76-9.68 (m, 1H), 9.37-9.24 (m, 1H), 8.29-8.24 (m, 2H), 8.17 (s, 1H), 7.97-7.75 (m, 4H), 7.67 (d, $J$ = 8.0 Hz, 3H), 7.57-7.53 (m, 3H), 7.50-7.48 (m, 2H), 7.37 (s, 2H), 5.14-5.09 (m, 1H), 4.72-4.63 (m, 2H), 4.52-4.48 (m, 1H), 4.37-4.33 (m, 1H), 3.73 (s, 4H), 3.57-3.35 (m, 4H), 3.16 (s, 5H), 3.13-2.89 (m, 4H), 2.68-2.58 (m, 5H), 2.46-2.43 (m, 2H), 2.09-2.05 (m, 1H), 1.82-1.79 (m, 2H), 1.69-1.59 (m, 2H). | | | |

(continued)

| Compound No. | Structure and characterization data of compound | Raw material 1 | Raw material 2 | Yield |
|---|---|---|---|---|
| D2-8 | 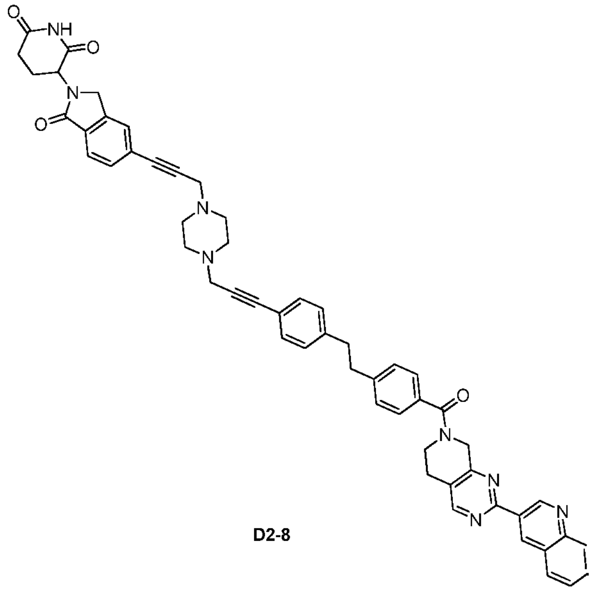<br>D2-8<br>LC-MS: $C_{54}H_{49}N_8O_4$, $[M/2+H]^+$ 437.2; found 437.6. $^1H$ NMR (600 MHz, DMSO-$d_6$) 611.01(s, 1H), 9.83-9.76 (m, 1H), 9.31-9.25 (m, 1H), 8.84 (m, 1H), 8.22 (br, 1H), 8.10 (br, 1H), 7.86 (br, 1H), 7.77-7.75 (m, 2H), 7.70-7.69 (m, 1H), 7.64-7.62 (m, 1H), 7.44-7.42 (m, 4H), 7.34 (d, *J* = 7.8 Hz, 2H), 7.29 (d, *J* = 7.8 Hz, 2H), 5.14-5.11 (m, 1H), 4.93 (br, 1H), 4.75 (br, 1H), 4.47 (d, *J* = 17.4 Hz, 1H), 4.35 (d, *J* = 17.4 Hz, 1H), 4.09 (s, 3H), 3.99 (m, 6H), 3.74 (m, 10H), 2.92-2.88 (m, 2H), 2.61 (d, *J* = 15.6 Hz, 1H), 2.44-2.36 (m, 1H), 2.03-2.01 (m, 1H). Purity > 85%. | 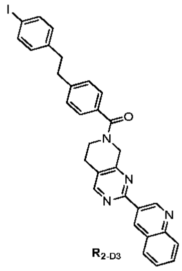<br>$R_{2\text{-}D3}$ | 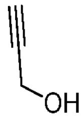<br>**49a** | 29.8 % |

(continued)

| Compound No. | Structure and characterization data of compound | Raw material 1 | Rawmaterial 2 | Yield |
|---|---|---|---|---|
| D2-9 | 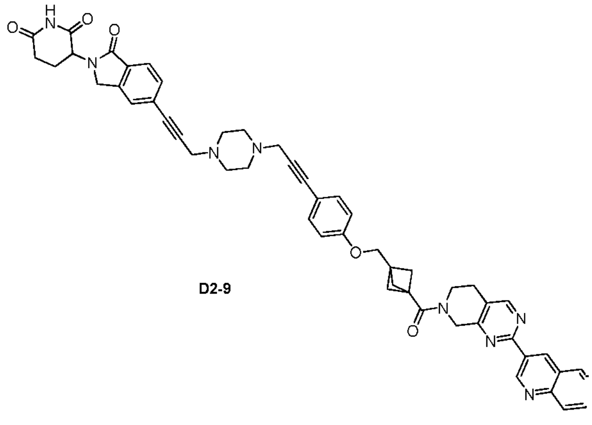<br>D2-9<br>LC-MS: $C_{52}H_{49}N_8O_5$, $[M+H]^+$ 865.4; found 865.1. $^1H$ NMR (600 MHz, DMSO-$d_6$) $\delta$ 11.00 (s, 1H), 9.82-9.80 (m, 1H), 9.27 (s, 1H), 8.81 (s, 1H), 8.21 (d, $J$ = 7.8 Hz, 1H), 8.10 (m, 1H), 7.85 (t, $J$ = 7.8 Hz, 1H), 7.72-7.68 (m, 3H), 7.56 (d, $J$ = 7.8 Hz, 1H), 7.37-7.36 (d, $J$ = 8.4 Hz,, 2H), 6.95-6.94 (d, $J$ = 8.4 Hz, 2H), 5.13-5.10 (m, 1H), 4.95-4.78 (m, 2H), 4.47-4.44 (d, $J$ = 18.0 Hz 1H), 4.35-4.32 (d, $J$ = 17.4 Hz, 1H), 4.08-4.04 (m, 3H), 3.95-3.80 (m, 2H), 3.58 (s, 2H), 3.46 (s, 2H), 3.34 (s, 2H), 2.94-2.86 (m, 3H), 2.61-2.59 (m, 6H), 2.42-2.38 (qd, $J$ = 13.2,4.8 Hz 1H), 2.27 (s, 6H), 2.02-2.00 (m, 1H). Purity > 99%. | 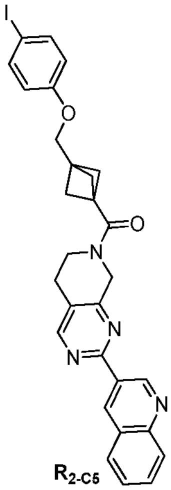<br>$R_{2\text{-}C5}$ | 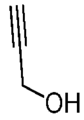<br>49a | 9.6% |
| D2-10 | 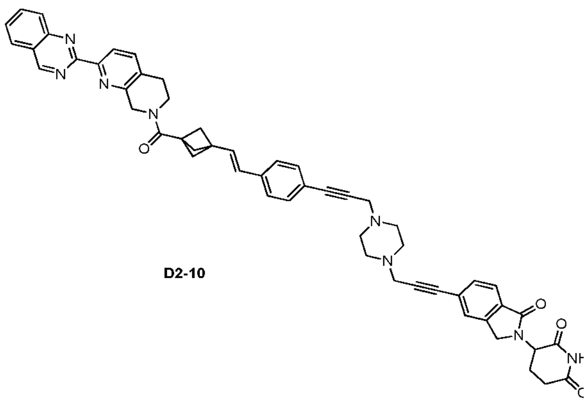<br>D2-10<br>LC-MS: $C_{53}H_{49}N_8O_4$, $[M/2+H]^+$ 431.5; found 431.7. $^1H$ NMR (600 MHz, DMSO-$d_6$) $\delta$ 11.00 (s, 1H), 9.82-9.80 (m, 1H), 9.28-9.27 (m, 1H), 8.84-8.80 (m, 1H), 8.22 (t, $J$ = 8.4 Hz, 1H), 8.11-8.09 (m, 1H), 7.87-7.85 (m, 1H), 7.84-7.82 (m, 2H), 7.81-7.78 (m, 2H), 7.51-7.47 (m, 2H), 7.32-7.28 (m, 2H), 6.60-6.53 (m, 1H), 5.81-5.76 (m, 1H), 5.13-5.11 (m, 1H), 4.87-4.80 (m, 1H), 4.74 (s, 1H), 4.48-4.33 (m, 4H), 4.09-4.01 (m, 3H), 3.88 (t, $J$ = 6.0 Hz, 1H), 2.91-2.88 (m, 4H), 2.62-2.59 (m, 2H), 2.41-2.39 (m, 2H), 2.28 (s, 2H), 2.21-2.15 (m, 5H), 2.11-2.07 (m, 2H), 2.04-2.01 (m, 3H). Purity > 80%. | 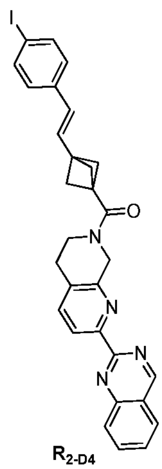<br>$R_{2\text{-}D4}$ | 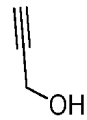<br>49a | 3.8% |

According to the step 1 to step 6 of the method for Compound **D2-1,** the raw material **1** in the following table

was used to replace Compound $R_{1B-2}$, and the raw material **2** in the following table was used to replace Compound **49a,** and the other raw materials and operating methods remained unchanged to obtain Compounds **D2-11, D2-12, D2-13** and **D2-14.**

| Compound No. | Structure and characterization data of compound | Raw material 1 | Raw material 2 | Yield |
|---|---|---|---|---|
| D2-11 | 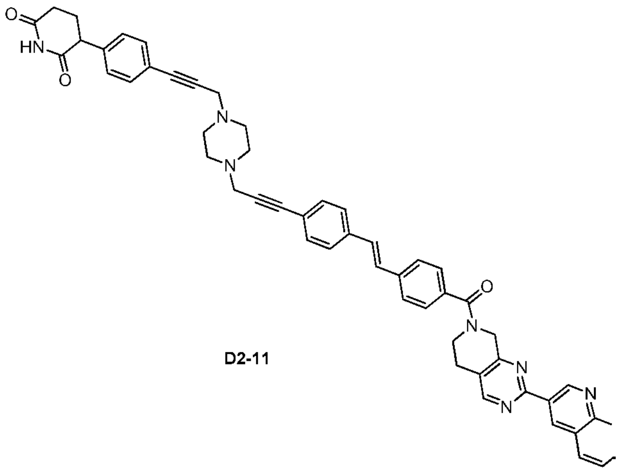 D2-11<br>LC-MS: $C_{52}H_{46}N_7O_3$, $[M/2+H]^+$ 409.2; found 409.1. $^1H$ NMR (600 MHz, DMSO-$d_6$) δ 10.88 (s, 1H), 9.84-9.76 (m, 1H), 9.31-9.24 (m, 1H), 8.86 (s, 1H), 8.23 (s, 1H), 8.10 (s, 1H), 7.86 (s, 1H), 7.75 (d, *J* = 7.8 Hz, 2H), 7.69 (d, *J* = 8.4 Hz, 3H), 7.61-7.53 (m, 5H), 7.48 (d, *J* = 7.8 Hz, 2H), 7.43-7.42 (m, 2H), 7.29 (s, 1H), 4.95-4.80 (m, 2H), 4.06 (br, 4H), 3.94-3.91 (dd, J = 12.0, 4.8 Hz 1H), 3.73 (br, 2H), 3.34-2.94 (m, 10H), 2.71-2.65 (m, 1H), 2.50 (s, 1H), 2.23-2.21 (m, 1H), 2.08-2.01 (m, 1H). Purity > 95%. | 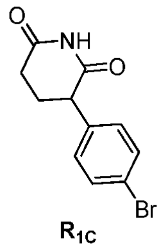<br>$R_{1C}$ | 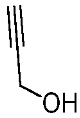<br>**49a** | 29.3 % |

(continued)

| Compound No. | Structure and characterization data of compound | Raw material 1 | Raw material 2 | Yield |
|---|---|---|---|---|
| **D2-12** | 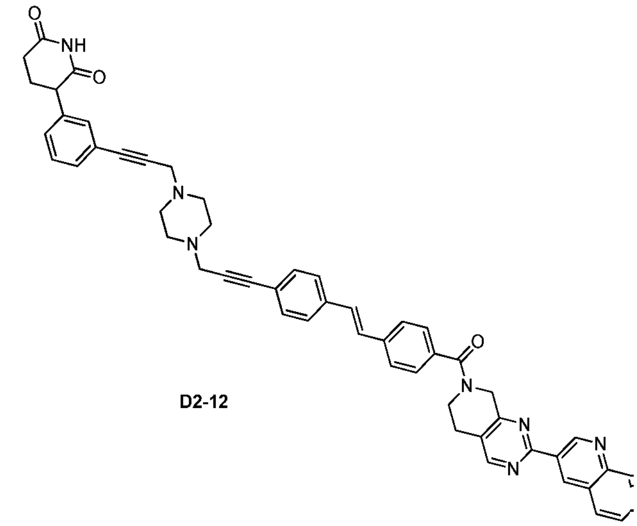<br>D2-12<br><br>LC-MS: $C_{52}H_{46}N_7O_3$, $[M/2+H]^+$ 409.2; found 409.0. $^1H$ NMR (600 MHz, DMSO-$d_6$) δ 10.88 (s, 1H), 9.84-9.77 (m, 1H), 9.33-9.25 (m, 1H), 8.85 (s, 1H), 8.23 (s, 1H), 8.11 (s, 1H), 7.87 (s, 1H), 7.75 (d, *J* = 8.4 Hz, 2H), 7.69 (d, *J* = 8.4 Hz, 3H), 7.57-7.53 (m, 4H), 7.43-7.38 (m, 5H), 7.32-7.31 (d, *J* = 7.8 Hz, 1H), 4.95-4.80 (m, 2H), 4.07 (s, 4H), 3.92-3.89 (dd, *J* = 12.0, 4.8 Hz, 1H), 3.73 (br, 2H), 3.18-2.98 (m, 10H), 2.72-2.66 (m, 1H), 2.55 (s, 1H), 2.27-2.21 (qd, *J* =12.6, 4.2 Hz, 1H), 2.06-2.02 (m, 1H). Purity > 99%. | 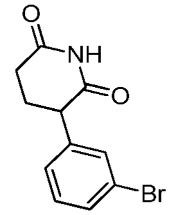<br>**$R_{2C}$** | 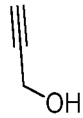<br>**49a** | 27.8 % |

(continued)

| Compound No. | Structure and characterization data of compound | Raw material 1 | Raw material 2 | Yield |
|---|---|---|---|---|
| D2-13 | 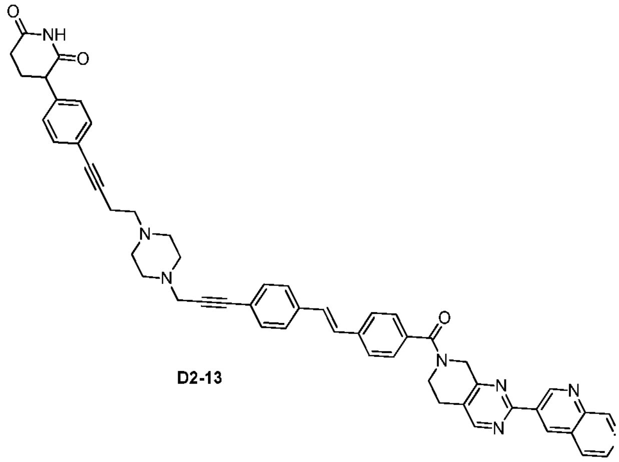<br>D2-13 | 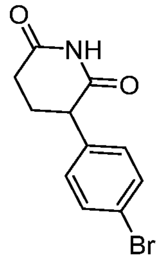<br>$R_{1C}$ | 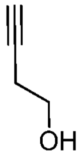<br>**49b** | 19.9 % |
| | LC-MS: $C_{53}H_{48}N_7O_3$, $[M+H]^+$ 830.4; found 830.6. $^1H$ NMR (600 MHz, DMSO-$d_6$) δ 10.87 (s, 1H), 9.86-9.78 (m, 1H), 9.37-9.29 (m, 1H), 8.86 (s, 1H), 8.26 (s, 1H), 8.12 (s, 1H), 7.88-7.76 (s, 1H), 7.74-7.69 (m, 3H), 7.66-7.60 (m, 2H), 7.57-7.55 (m, 2H), 7.54-7.53 (m, 2H), 7.51-7.44 (m, 4H), 7.43-7.42 (d, *J* = 6.0 Hz, 2H), 4.95 (br, 1H), 4.80 (br, 1H), 3.99-3.97 (m, 1H), 3.91-3.88 (m, 1H), 3.82 (s, 2H), 3.74 (s, 1H), 3.71-3.35 (m, 5H), 3.00-2.91 (m, 9H), 2.70-2.65 (m, 1H), 2.49 (s, 1H), 2.22-2.19 (m, 1H), 2.08-2.02 (m, 1H). Purity > 95%. | | | |

(continued)

| Compound No. | Structure and characterization data of compound | Raw material 1 | Raw material 2 | Yield |
|---|---|---|---|---|
| **D2-14** | 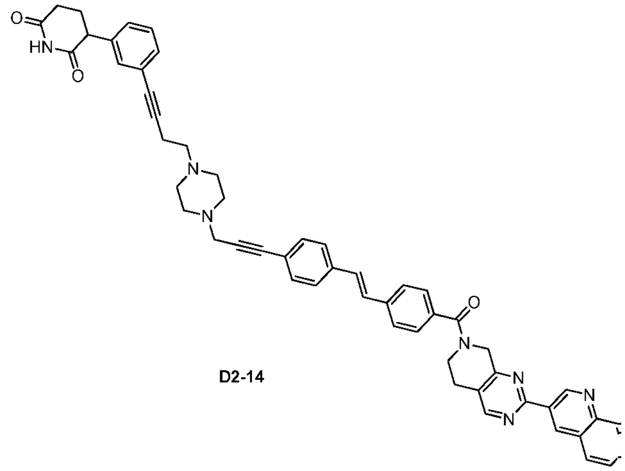 D2-14<br>LC-MS: $C_{53}H_{48}N_7O_3$, $[M+H]^+$ 830.4; found 830.6. $^1H$ NMR (600 MHz, DMSO-$d_6$) δ 10.87 (s, 1H), 9.85-9.77 (m, 1H), 9.32-9.25 (m, 1H), 8.86 (s, 1H), 8.23 (s, 1H), 8.14 (s, 1H), 7.86 (s, 1H), 7.77-7.75 (m, 2H), 7.74-7.73 (m, 3H), 7.68-7.66 (d, *J* = 7.8 Hz, 2H), 7.56-7.55 (d, *J* = 7.8 Hz, 2H), 7.50-7.49 (d, *J* = 8.4 Hz, 2H), 7.43-7.40 (m, 2H), 7.37-7.28 (m, 2H), 4.95-4.76 (m, 2H), 4.00 (s, 1H), 3.90-3.88 (dd, *J* = 12.0, 4.8 Hz, 1H), 3.78 (m, 3H), 3.60-2.91 (m, 14H), 2.83-2.73 (m, 1H), 2.70-2.64 (m, 1H), 2.23-2.21 (m, 1H), 2.04-2.02 (m, 1H). Purity > 99%. | 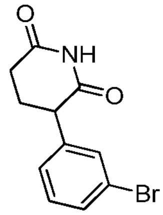<br>**$R_{2C}$** | 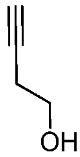<br>**49b** | 9.4% |

According to the step 1 to step 6 of the method for Compound **D2-1,** Compound **$R_{2C}$** was used to replace Compound **$R_{1B-2}$**, the raw material **1** in the following table was used to replace Compound **$R_{2-D1}$**, the raw material 2 in the following table was used to replace Compound **49a,** and the other raw materials and operations remained unchanged to obtain Compounds **D2-15** and **D2-16.**

| Compound No. | Structure and characterization data of compound | Raw material 1 | Raw materia 12 | Yield |
|---|---|---|---|---|
| D2-15 | 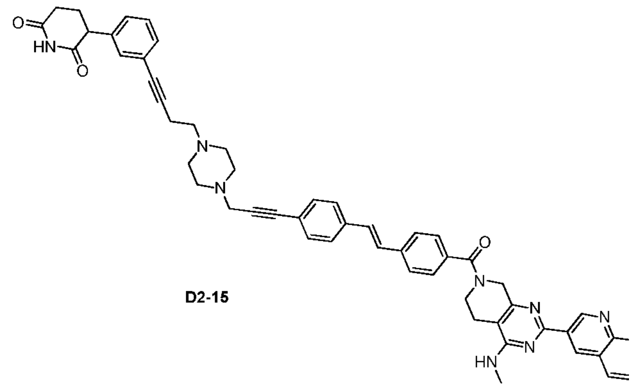 D2-15 | 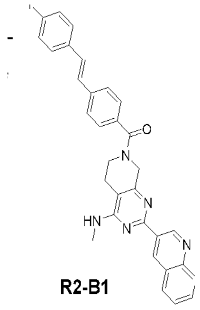 R2-B1 | 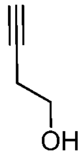 **49b** | 29.3 % |
| | LC-MS: $C_{54}H_{51}N_8O_3$, $[M+H]^+$ 859.4; found 859.4. $^1H$ NMR (600 MHz, DMSO-$d_6$) δ 9.78-9.70 (m, 1H), 9.34-9.23 (m, 1H), 8.24-8.20 (m, 1H), 8.13 (s, 1H), 7.91 (s, 1H), 7.74-7.73 (d, *J* = 7.8 Hz, 3H), 7.66-7.65 (d, *J* = 7.8 Hz, 2H), 7.53-7.48 (m, 4H), 7.37-7.27 (m, 4H), 7.25 (s, 1H), 7.24-7.19 (m, 1H), 4.77 (br, 1H), 4.59 (br, 1H), 3.95 (s, 1H), 3.88-3.85 (dd, *J* = 12.0, 4.8 Hz, 1H), 3.80 (s, 2H), 3.53 (s, 2H), 3.33 (t, *J* = 7.2 Hz, 6H), 3.10 (s, 3H), 2.96-2.89 (m, 6H), 2.68-2.64 (m, 1H), 2.57-2.51 (m, 3H), 2.20-2.17 (m, 1H), 2.03-2.01 (m, 1H). Purity > 95%. | | | |
| D2-16 | 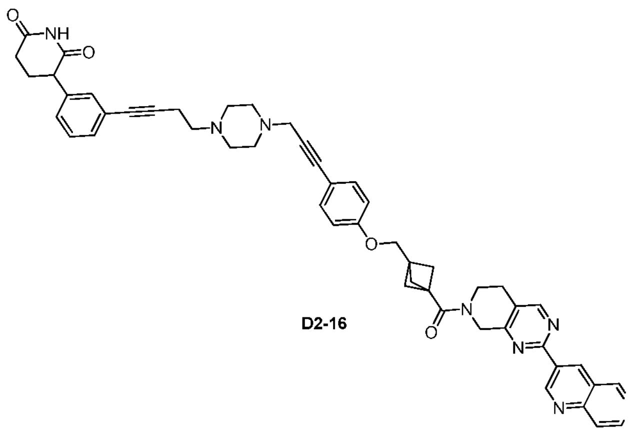 D2-16 | 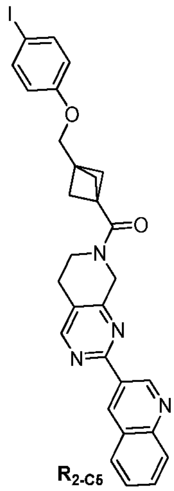 $R_{2\text{-}C5}$ | 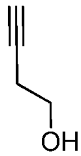 **49b** | 32.1 % |
| | LC-MS: $C_{51}H_{50}N_7O_4$, $[M+H]^+$ 824.4; found 824.4. $^1H$ NMR (600 MHz, DMSO-$d_6$) δ 10.87 (s, 1H), 9.82 (m, 1H), 9.31 (s, 1H), 8.83 (m, 1H), 8.24 (d, *J* = 8.4 Hz, 1H), 8.11 (t, *J* = 8.4 Hz, 1H), 7.88 (t, *J* = 7.2 Hz, 1H), 7.71 (t, *J* = 7.2 Hz, 1H), 7.41-7.40 (m, 2H), 7.35-7.32 (m, 2H), 7.28-7.25 (m, 2H), 6.97 (d, *J* = 9.0 Hz, 2H), 4.95 (s. 1H), 4.79 (s, 2H), 4.10 (s, 2H), 3.96-3.94 (m, 1H), 3.89-3.87 (m, 1H), 3.80-3.78 (m, 3H), 3.31-3.09 (m, 6H), 2.95-2.86 (m, 8H), 2.70-2.64 (m, 1H), 2.23-2.22 (m, 1H), 2.20 (s, 6H), 2.10-2.00 (m, 1H). Purity > 99%. | | | |

**Example 8.** Synthesis of Compounds **E1-1** and **E1-2** of the present invention

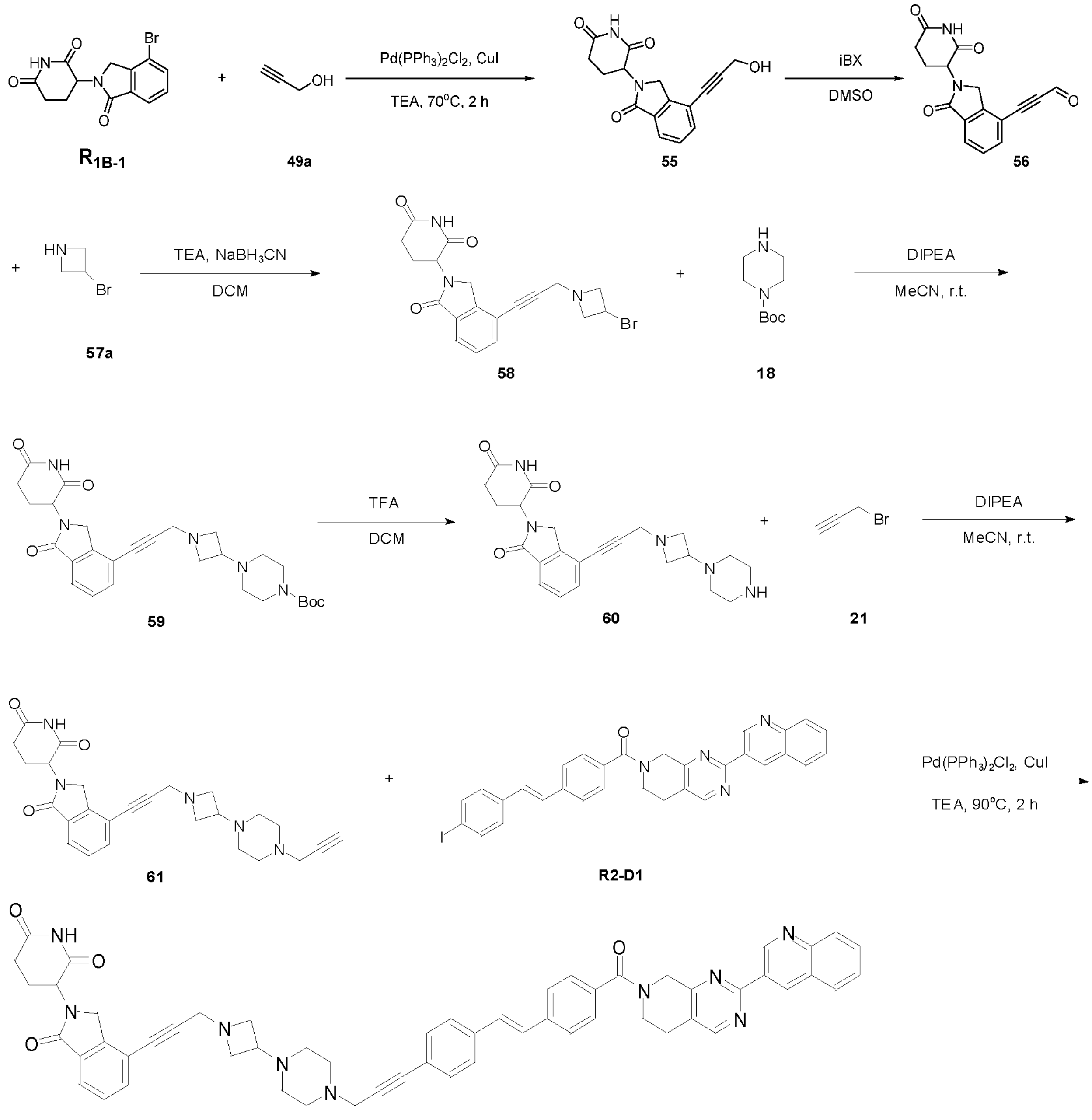


$R_{1B-1}$ 49a 55 56

57a 58 18

59 60 21

61 R2-D1

E1 - 1

**Step 1. Synthesis of Compound 55**

Under nitrogen protection, Compound $\mathbf{R_{1B-1}}$ (1.00 mmol), Compound **49a** (1.02 mmol), copper iodide (2.00 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 70°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1: 100 to 1:5), and the solvent was removed by concentration under reduced pressure to obtain Compound **55** ($C_{16}H_{15}N_2O_4$, $[M+H]^+$ 299.1; found 299.3, yield 63.4%).

**Step 2. Synthesis of Compound 56**

Compound **55** (0.60 mmol), 2-iodoxybenzoic acid (1.80 mmol) and DMSO (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **56** ($C_{16}H_{13}N_2O_4$, $[M+H]^+$ 297.1; found 297.3, yield 78.2%).

**Step 3. Synthesis of Compound 58**

Compound **56** (0.45 mmol), Compound **57a** (0.65 mmol), triethylamine (2 mL), sodium cyanoborohydride (0.90 mmol) and dichloroethane (5 mL) were added in sequence to a 50 mL reaction bottle, reacted under stirring at room temperature for 2 hours, then the reaction was quenched (LC-MS monitoring), the solvent was removed by evaporation to dryness, purification was carried out by medium-pressure preparative chromatography, and the solvent was removed by concentration under reduced pressure to obtain Compound **58** ($C_{19}H_{19}BrN_3O_3$, $[M+H]^+$ 416.1; found 416.2, yield 66.7%).

**Step 4. Synthesis of Compound 59**

Compound **58** (0.30 mmol), Compound **18** (0.65 mmol), *N,N*-diisopropylethylamine (1.20 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **59** ($C_{28}H_{36}N_5O_5$, $[M+H]^+$ 522.3; found 522.5, yield 63.8%).

**Step 5. Synthesis of Compound 60**

Compound **59** (0.20 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **60** ($C_{23}H_{28}N_5O_3$, $[M+H]^+$ 422.2; found 422.1, crude product).

**Step 6. Synthesis of Compound 61**

Compound **60** (0.20 mmol), Compound **21** (0.50 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to remove the solvent, purified by medium-pressure preparative chromatography, and concentrated under reduced pressure to remove the solvent to obtain Compound **61** ($C_{26}H_{30}N_5O_3$, $[M+H]^+$ 460.2; found 460.4, yield 75.2%).

**Step 7. Synthesis of Compound E1-1**

Under nitrogen protection, Compound $\mathbf{R_{2\text{-}D1}}$ (0.15 mmol), Compound **61** (0.15 mmol), copper iodide (0.35 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and the solvent was removed by concentration under reduced pressure to obtain Compound **E1-1** (10.6 mg, 10.79 μmol). LC-MS: $C_{57}H_{52}N_9O_4$ $[M/2+H]^+$ 464.1; found 464.2. $^1H$ NMR (400 MHz, Methanol-*d4*) δ 9.92 (s, 1H), 9.67 (s, 1H), 8.80 (s, 1H), 8.34-8.17 (m, 2H), 8.01 (s, 1H), 7.99 (s, 1H), 7.87-7.73 (m, 4H), 7.64-7.59 (m, 2H), 7.57-7.50 (m, 5H), 7.33 (s, 2H), 5.22-5.17 (m, 1H), 5.04-4.87 (m, 2H), 4.62-4.55 (m, 2H), 4.51-4.37 (m, 6H), 4.27-4.26 (m, 2H), 4.18-4.03 (m, 1H), 3.87-3.84 (m, 2H), 3.54-3.31 (m, 4H), 3.13-3.05 (m, 3H), 2.97-2.88 (m, 2H), 2.81-2.77 (m, 2H), 2.57-2.45 (m, 2H), 2.21-2.18 (m, 1H).

Compound **E1-2** could be obtained by following the step 1 to step 7 of the method for Compound **E1-1,** replacing Compound **57a** with the raw materials in the below table, and keeping other raw materials and operating methods

unchanged.

| Compound No. | Structure and characterization data of compound | Raw material | Yield |
|---|---|---|---|
| E1-2 | 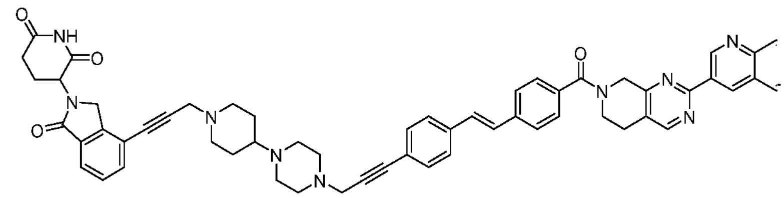<br>E1 - 2<br>LC-MS: $C_{59}H_{56}N_9O_4$ $[M/2+H]^+$ 478.5; found 478.1. $^1H$ NMR (400 MHz, Methanol-$d_4$) $\delta$ 9.95-9.88 (m, 1H), 9.72-9.61 (m, 1H), 8.81 (s, 1H), 8.41-8.25 (m, 2H), 8.19 - 8.01 (m, 1H), 7.98-7.89 (m, 1H), 7.86-7.73 (m, 4H), 7.68-7.55 (m, 5H), 7.48-7.42 (m, 2H), 7.32 (s, 2H), 5.22-5.17 (m, 1H), 5.05-4.87 (m, 2H), 4.63-4.51 (m, 2H), 4.36 (s, 2H), 4.14 (s, 3H), 3.83-3.77 (m, 4H), 3.31-3.06 (m, 12H), 2.97-2.92 (m, 1H), 2.89-2.77 (m, 1H), 2.53-2.49 (m, 1H), 2.48-2.27 (m, 2H), 2.22-2.19 (m, 1H), 2.02-1.99 (m, 2H). | 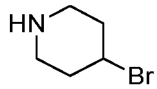<br>**57b** | 16.3 % |

**Example 9.** Synthesis of Compounds **F1A-1, F1A-2, F1A-3, F1A-4** and **F1A-5** of the present invention

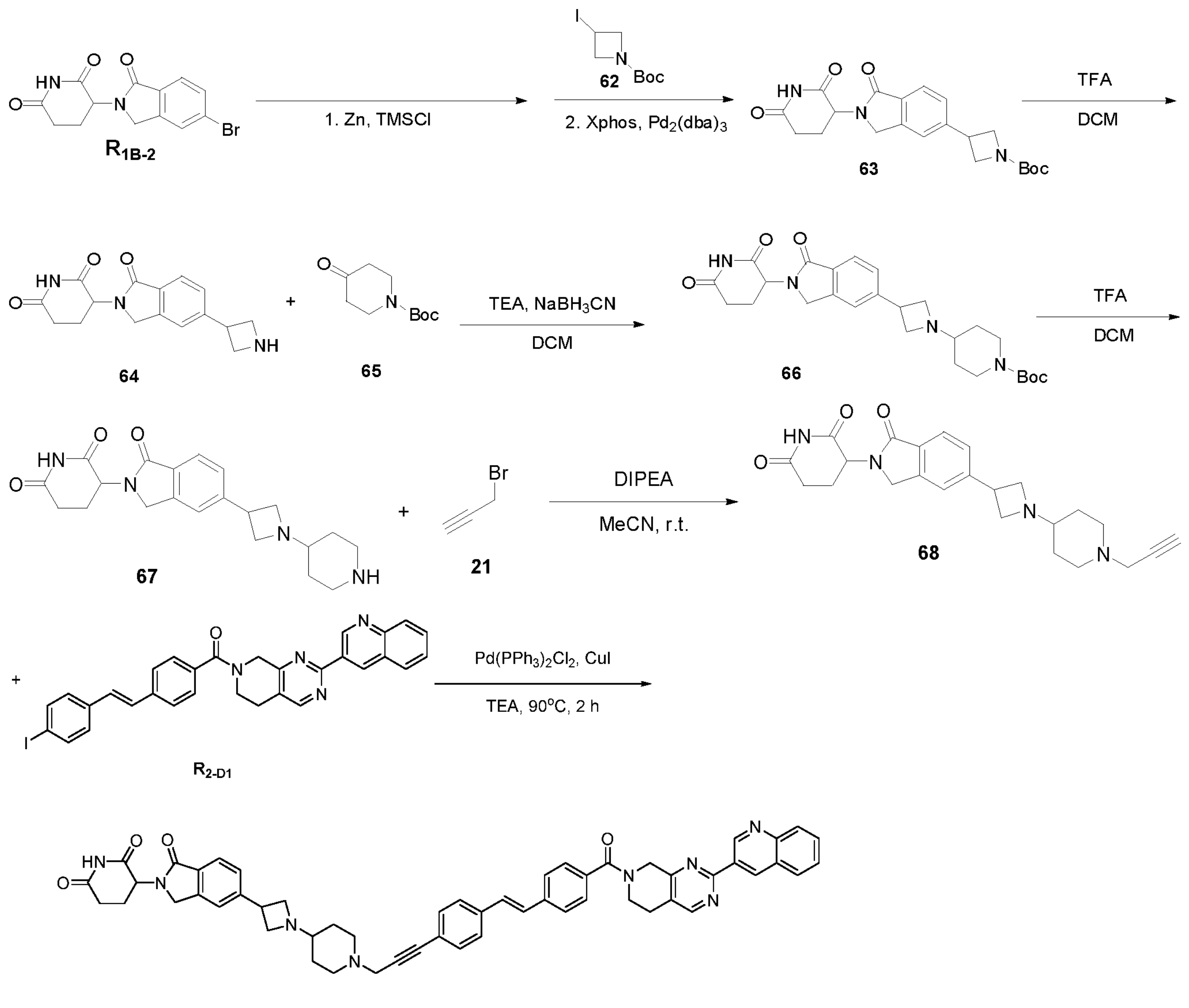


F1A - 1

**Step 1. Synthesis of Compound 63**

Compound $\mathbf{R_{1B\text{-}2}}$ (1.0 mmol), zinc powder and 1,4-dioxane (5 mL) were added in sequence to a 50 mL reaction bottle, TMSCl was added dropwise under ice bath conditions, slowly warmed to room temperature, reacted under stirring for 1 hour, and then the reaction was quenched (LC-MS monitoring). Extraction was carried out with saturated sodium chloride solution (20 mL) and ethyl acetate (3× 20 mL), the organic phases were combined, the combined organic phase was dried over anhydrous sodium sulfate, and evaporated to dryness to remove the solvent. Under nitrogen protection, Compound **62** (1.0 mmol), Xphos (0.2 mmol), $Pd_2(dba)_3$ (0.25 mol%), and 1,4-dioxane (5 mL) were added into the reaction bottle. The reaction system was sealed, the reaction was carried out under stirring at 80°C for 2 hours, and then the reaction was quenched (LC-MS monitoring). Extraction was carried out with saturated sodium chloride solution (20 mL) and ethyl acetate (3× 20 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1: 100 to 1:5), and the solvent was removed by concentration under reduced pressure to obtain Compound **63** ($C_{21}H_{26}N_3O_5$, $[M+H]^+$ 400.2; found 400.4, yield 56.8%).

**Step 2. Synthesis of Compound 64**

Compound **63** (0.55 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **64** ($C_{16}H_{18}N_3O_3$, $[M+H]^+$ 300.1; found 300.2, crude product).

**Step 3. Synthesis of Compound 66**

Compound **64** (0.55 mmol), Compound **65** (0.65 mmol), triethylamine (2 mL), sodium cyanoborohydride (0.85 mmol) and dichloroethane (5 mL) were added in sequence to a 50 mL reaction bottle, reacted under stirring at room temperature for 2 hours, then the reaction was quenched (LC-MS monitoring), the solvent was removed by evaporation to dryness, purification was carried out by medium-pressure preparative chromatography, and the solvent was removed by concentration under reduced pressure to obtain Compound **66** ($C_{26}H_{35}N_4O_5$, $[M+H]^+$ 483.3; found 483.4, yield 54.5%).

**Step 4. Synthesis of Compound 67**

Compound **66** (0.30 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **67** ($C_{21}H_{27}N_4O_3$, $[M+H]^+$ 383.2; found 383.2, crude product).

**Step 5. Synthesis of Compound 68**

Compound **67** (0.30mmol), Compound **21** (0.65 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to remove the solvent, purified by medium-pressure preparative chromatography, and concentrated under reduced pressure to remove the solvent to obtain Compound **68** ($C_{24}H_{29}N_4O_3$, $[M+H]^+$ 421.2; found 421.1, yield 83.3%).

**Step 6. Synthesis of Compound F1A-1**

Under nitrogen protection, Compound $\mathbf{R_{2\text{-}D1}}$ (0.25 mmol), Compound 68 (0.25 mmol), copper iodide (0.55 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and the solvent was removed by concentration under reduced pressure to obtain Compound **F1A-1** (11.9 mg, 13.40 μmol, 91.8% purity). LC-MS: $C_{55}H_{51}N_8O_4$, $[M/2+H]^+$ 444.5; found 444.5. $^1H$ NMR (600 MHz, DMSO-*d6*) δ 9.82-9.78 (m, 1H), 9.29-9.23 (m, 1H), 8.85 (s, 1H), 8.21 (s, 1H), 8.09 (s, 1H), 7.84 (s, 1H), 7.75 (d, *J* = 8.0 Hz, 2H), 7.67-7.64 (m, 4H), 7.58-7.55 (m, 3H), 7.49-7.45 (m, 3H), 7.39 (s, 2H), 5.10 (d, *J* = 5.2 Hz, 1H), 4.94 (s,1H), 4.80 (s, 1H), 4.44- 4.41 (m, 1H), 4.32-4.29 (m, 1H), 3.99 (s, 1H), 3.69-3.63 (m, 3H), 3.52 (s, 2H), 3.11 (s, 2H), 2.99 (s, 2H), 2.91-2.89 (m, 1H), 2.80 (s, 2H), 2.61-2.58 (m, 1H), 2.26 (s, 2H), 2.09 (s, 1H), 1.99-1.94 (m, 1H), 1.75 (s, 2H), 1.24 (s, 4H).

Compounds **F1A-4** and **F1A-5** could be obtained by following the steps 1 to 6 of the method for Compound **F1A-1,** replacing $\mathbf{R_{2\text{-}D1}}$ with raw material 1 in the below table, and keeping other raw materials and operating methods unchanged.

| Compound No. | Structure and characterization data of compound | Raw material | Yield |
|---|---|---|---|
| F1A-4 | 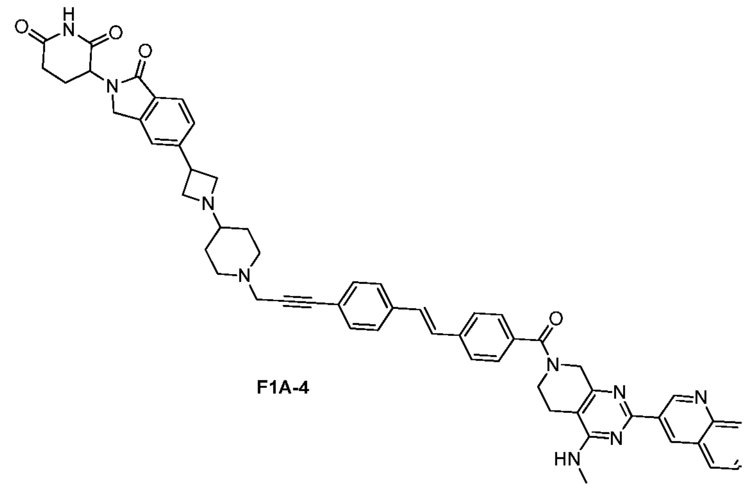<br>F1A-4<br>LC-MS: $C_{56}H_{54}N_9O_4$, $[M+H]^+$ 916.4; found 916.8. $^1H$ NMR (600 MHz, DMSO-$d_6$) δ 9.82-9.73 (m, 1H), 9.34-9.23 (m, 1H), 8.25-7.80 (m, 3H), 7.79-7.68 (m, 7H), 7.61-7.48 (m, 5H), 7.39 (s, 2H), 5.13-5.10 (m, 1H), 4.78-4.25 (m, 12H), 3.99 (s, 1H), 3.74 (s, 3H), 3.11-2.89 (m, 6H), 2.64-2.59 (m, 3H), 2.46-2.40 (m, 1H), 2.28-2.27 (m, 2H), 2.04-2.02 (m, 1H), 1.72 (s, 2H). Purity > 90%. | 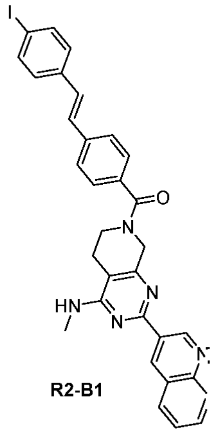<br>R2-B1 | 16.3 % |
| F1A-5 | 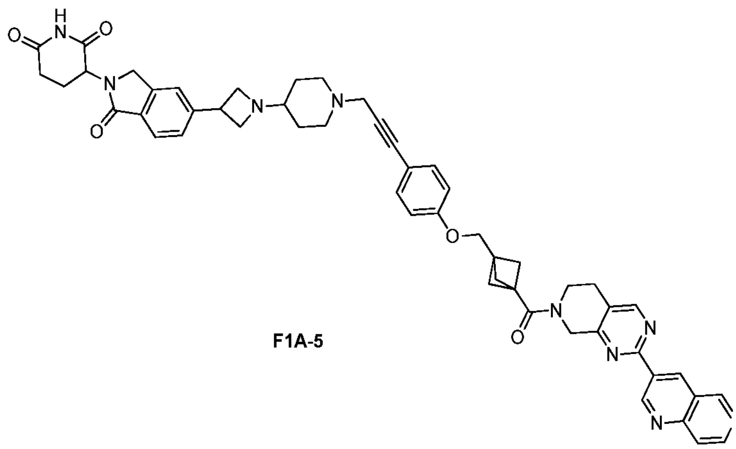<br>F1A-5<br>LC-MS: $C_{53}H_{53}N_8O_5$, $[M/2+H]^+$ 441.5; found 441.5. $^1H$ NMR (600 MHz, DMSO-$d_6$) δ 9.83 (s, 1H), 9.38-9.37 (m, 1H), 8.84-8.83 (m, 1H), 8.27 (d, *J* = 8.4 Hz, 1H), 8.15 (d, *J* = 8.4 Hz, 1H), 7.93 (t, *J* = 7.8 Hz, 1H), 7.80-7.74 (m, 2H), 7.67 (s, 1H), 7.60 (d, *J* = 7.2 Hz, 1H), 7.50 (d, *J* = 1.8 Hz, 2H), 7.03 (d, *J* = 8.4 Hz, 2H), 5.72 (s, 1H), 5.12-5.09 (m, 1H), 4.96 (s, 1H), 4.80 (s, 1H), 4.56-4.50 (m, 2H), 4.47 (s, 1H), 4.37 (s, 5H), 4.37 (s, 1H), 4.13 (s, 2H), 3.96 (s, 3H), 3.11 (s, 2H), 2.96-2.88 (m, 3H), 2.65-2.63 (m, 1H), 2.46-2.42 (m, 1H), 2.40-2.18 (m, 9H), 2.05-2.03 (m, 1H), 1.72-1.71 (m, 2H). Purity > 90%. | 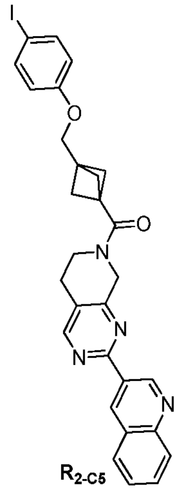<br>$R_{2-C5}$ | 23.2 % |

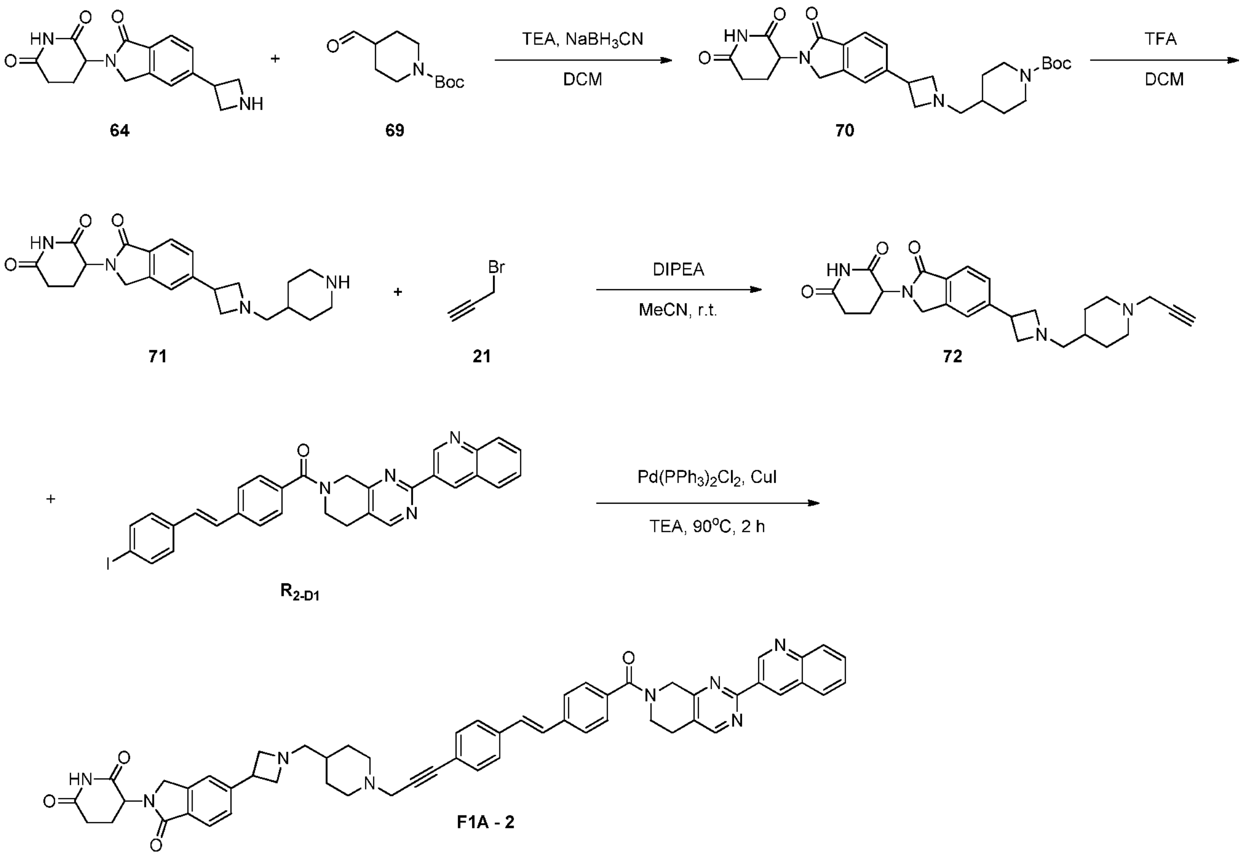


64 69 70 71 21 72 $R_{2-D1}$ F1A - 2

**Step 1. Synthesis of Compound 70**

Compound **64** (0.55 mmol), Compound **69** (0.65 mmol), triethylamine (2 mL), sodium cyanoborohydride (0.85 mmol) and dichloroethane (5 mL) were added in sequence to a 50 mL reaction bottle, reacted under stirring at room temperature for 2 hours, then the reaction was quenched (LC-MS monitoring), the solvent was removed by evaporation to dryness, purification was carried out by medium-pressure preparative chromatography, and the solvent was removed by concentration under reduced pressure to obtain Compound **70** ($C_{27}H_{37}N_4O_5$, $[M+H]^+$ 497.3; found 497.1, yield 63.6%).

**Step 2. Synthesis of Compound 71**

Compound **70** (0.35 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **71** ($C_{22}H_{28}N_4O_3$, $[M+H]^+$ 396.2; found 396.3, crude product).

**Step 3. Synthesis of Compound 72**

Compound **71** (0.35 mmol), Compound **21** (0.65 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **72** ($C_{25}H_{31}N_4O_3$, $[M+H]^+$ 435.2; found 435.1, yield 84.5%).

**Step 4. Synthesis of Compound F1A-2**

Under nitrogen protection, Compound $\mathbf{R_{2\text{-}D1}}$ (0.25 mmol), Compound **72** (0.25 mmol), copper iodide (0.55 mmol), triethylamine (5 mL), and $Pd(PPh_3)_2Cl_2$ (0.25 mol%) were added in sequence to a 50 mL reaction bottle. The

reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and the solvent was removed by concentration under reduced pressure to obtain Compound **F1A-2** (9.4 mg, 10.42 μmol, 84.9% purity). LC-MS: $C_{56}H_{53}N_8O_4$, $[M/2+H]^+$ 451.6; found 451.6. $^1$H NMR (600 MHz, DMSO-*d6*) δ 11.00 (s, 1H), 10.27 (s, 1H), 9.83 - 9.75 (m, 1H), 9.31 - 9.24 (m, 1H), 8.86 (s, 1H), 8.22 (s, 1H), 8.10 (s, 1H), 7.85 (s, 1H), 7.78 - 7.67 (m, 6H), 7.60 - 7.56 (m, 5H), 7.43 (s, 2H), 5.12 (d, *J* = 5.4 Hz, 1H), 4.95 (s, 1H), 4.79 (s, 1H), 4.55 (s, 2H), 4.45 - 4.33 (m, 6H), 4.24 (s, 3H), 3.26 (s, 2H), 3.09 - 2.96 (m, 4H), 2.95 - 2.90 (m, 1H), 2.61 - 2.60 (m, 2H), 2.46 - 2.39 (m, 2H), 2.02 - 2.01 (m, 3H), 2.00 - 1.91 (m, 1H), 1.47 - 1.45 (m, 2H).

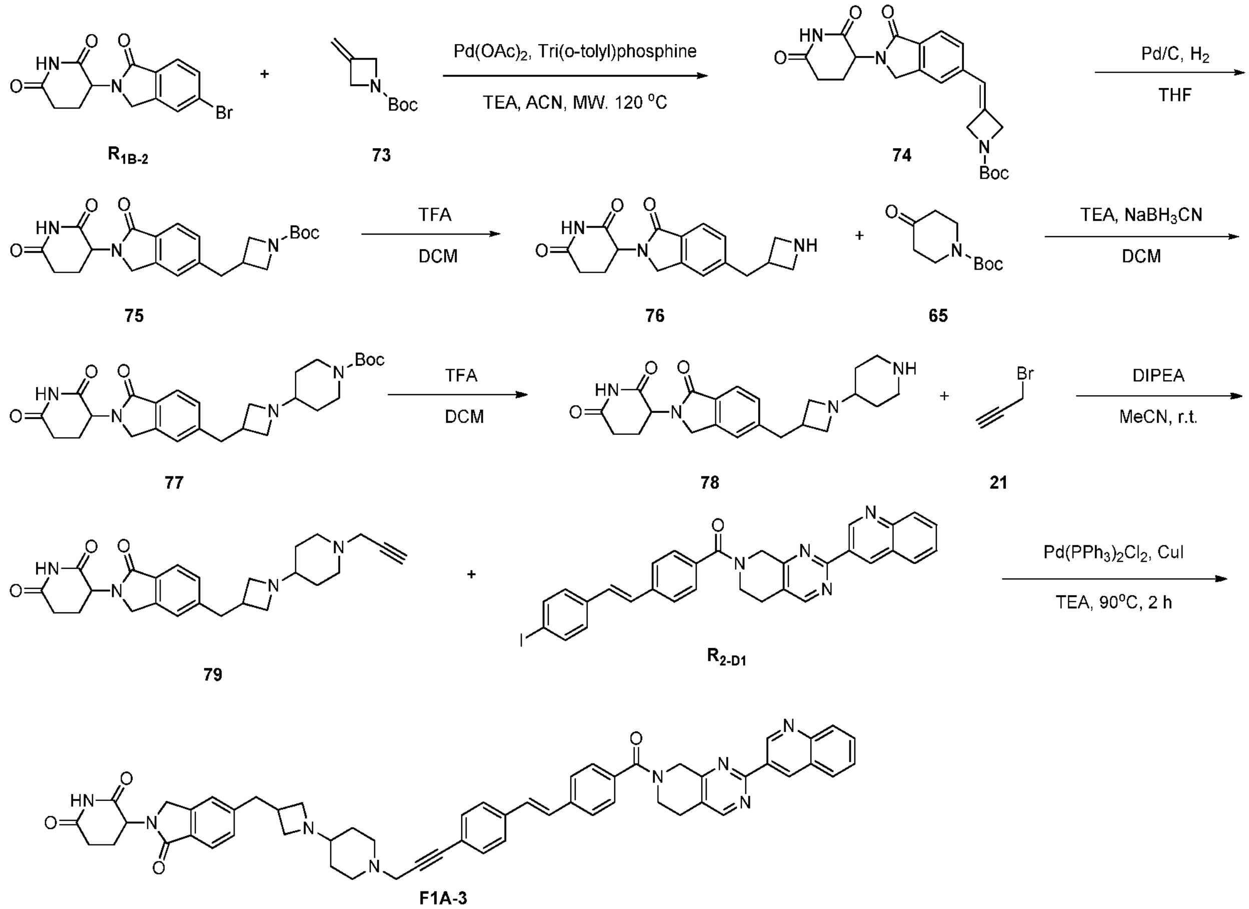

R1B-2, 73, 74, 75, 76, 65, 77, 78, 21, 79, R2-D1, F1A-3

**Step 1. Synthesis of Compound 74**

Under nitrogen protection, Compound $\mathbf{R_{1B-2}}$ (1.00 mmol), Compound **73** (1.20 mmol), tri(*o*-tolyl)phosphine (0.20 mmol), triethylamine (2 mL), and palladium acetate (0.25 mol%) were added in sequence to the 50 mL reaction bottle. The reaction system was sealed, the reaction was carried out under stirring in a microwave reactor at 120°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1: 100 to 1:5), and the solvent was removed by concentration under reduced pressure to obtain Compound **74** ($C_{22}H_{26}N_3O_5$, $[M+H]^+$ 412.2; found 412.1, yield 63.2%).

**Step 2. Synthesis of Compound 75**

Compound **74** (0.60 mmol), tetrahydrofuran (5 mL), and palladium-on-carbon (30.00 mg) were added in sequence to a 50 mL reaction bottle. The reaction system was replaced with hydrogen, maintained at an atmospheric pressure hydrogen atmosphere, and the reaction was carried out under stirring at room temperature for 3 hours (LC-MS monitoring). Filtration was carried out to remove palladium-on-carbon, washing was carried out with methanol (2×20 mL), the filtrate was collected, and concentrated under reduced pressure to remove the solvent to obtain Compound **75** ($C_{22}H_{28}N_3O_5$, $[M+H]^+$ 414.2; found 414.1, crude product).

**Step 3. Synthesis of Compound 76**

Compound **75** (0.55 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **76** ($C_{17}H_{20}N_3O_3$, $[M+H]^+$ 314.2; found 314.3, crude product).

**Step 4. Synthesis of Compound 77**

Compound **76** (0.55 mmol), Compound **65** (1.02 mmol), triethylamine (2 mL), sodium cyanoborohydride (64.10 mg, 1.02 mmol) and dichloroethane (5 mL) were added in sequence to a 50 mL reaction bottle, reacted under stirring at room temperature for 2 hours, then the reaction was quenched (LC-MS monitoring), the solvent was removed by evaporation to dryness, purification was carried out by medium-pressure preparative chromatography, and the solvent was removed by concentration under reduced pressure to obtain Compound **77** ($C_{27}H_{37}N_4O_5$, $[M+H]^+$ 497.3; found 497.1, yield 63.6%).

**Step 5. Synthesis of Compound 78**

Compound **77** (0.35 mmol) was dissolved in dichloromethane (5 mL) in a 50 mL reaction bottle, then trifluoroacetic acid (1.5 mL) was added dropwise, the reaction was carried out under stirring at room temperature for 0.5 hour, and the solvent was removed by concentration under reduced pressure to obtain Compound 78 ($C_{22}H_{29}N_4O_3$, $[M+H]^+$ 397.2; found 397.3, crude product).

**Step 6. Synthesis of Compound 79**

Compound **78** (0.35 mmol), Compound **21** (0.65 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, purified by medium-pressure preparative chromatography, and concentrated under reduced pressure to remove the solvent to obtain Compound **79** ($C_{25}H_{31}N_4O_3$, $[M+H]^+$ 435.2; found 435.4, yield 71.4%).

**Step 7. Synthesis of Compound F1A-3**

Under nitrogen protection, Compound $\mathbf{R_{2\text{-}D1}}$ (0.25 mmol), Compound **79** (0.25 mmol), copper iodide (0.55 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and the solvent was removed by concentration under reduced pressure to obtain Compound **F1A-3** (20.3 mg, 22.50 μmol). LC-MS: $C_{56}H_{53}N_8O_4$, $[M/2+H]^+$ 451.6; found 451.5. $^1$H NMR (600 MHz, DMSO-*d6*) δ 9.82 - 9.77 (m, 1H), 9.29 - 9.22 (m, 1H), 8.85 (s, 1H), 8.21 (s, 1H), 8.09 (s, 1H), 7.84 (s, 1H), 7.74 (d, *J* = 7.9 Hz, 2H), 7.63 - 7.54 (m, 4H), 7.45 (d, *J* = 7.7 Hz, 2H), 7.40 - 7.32 (m, 5H), 7.31 (d, *J* = 8.1 Hz, 1H), 5.10 (d, *J* = 5.6 Hz, 1H), 4.94 (s, 1H), 4.80 (s, 1H), 4.42 - 4.40 (m, 1H), 4.29 - 4.27 (m, 1H), 3.99 (s, 1H), 3.73 (s, 1H), 3.49 (s, 2H), 3.24 (s, 2H), 2.98 - 2.91 (m, 6H), 2.78 (s, 4H), 2.21 (s, 2H), 1.98 (s, 2H), 1.63 (s, 2H), 1.23 - 1.17 (m, 3H).

**Example 10.** Synthesis of Compounds **F1B-1** and **F1B-2** of the present invention

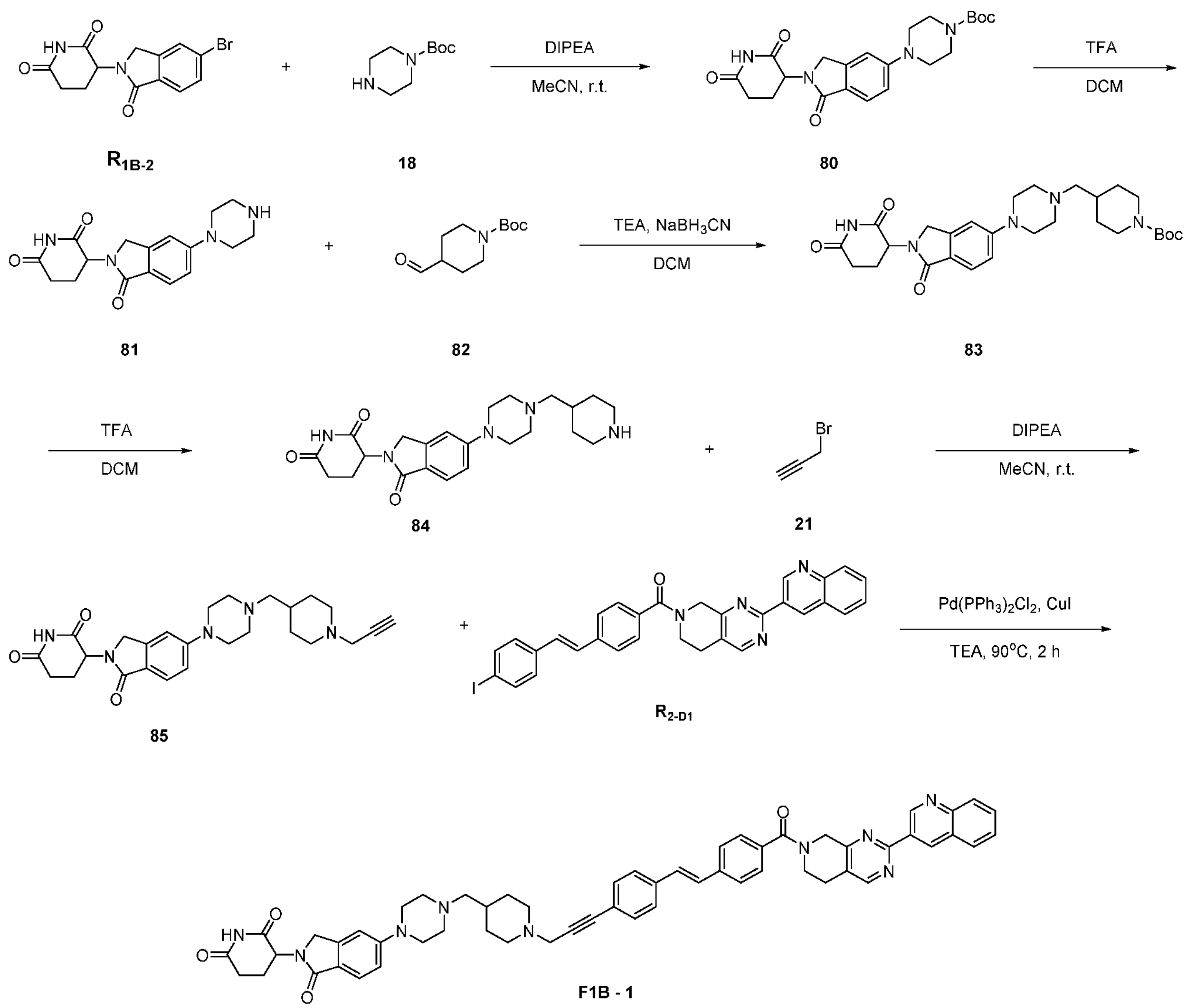


$R_{1B-2}$ 18 80

81 82 83

84 21

85 $R_{2-D1}$

F1B - 1

**Step 1. Synthesis of Compound 80**

Compound $\mathbf{R_{1B-2}}$ (1.00 mmol), Compound **18** (1.15 mmol), *N,N*-diisopropylethylamine (2.10 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **80** ($C_{22}H_{29}N_4O_5$, $[M+H]^+$ 429.1; found 429.3, yield 73.2%).

**Step 2. Synthesis of Compound 81**

Compound **80** (0.35 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **81** ($C_{17}H_{21}N_4O_3$, $[M+H]^+$ 329.2; found 329.3, crude product).

**Step 3. Synthesis of Compound 83**

Compound **81** (0.25 mmol), Compound **82** (0.45 mmol), triethylamine (2 mL), sodium cyanoborohydride (0.55 mmol) and dichloromethane (5 mL) were added in sequence to a 50 mL reaction bottle, reacted under stirring at room temperature for 2 hours, then the reaction was quenched (LC-MS monitoring), the solvent was removed by evaporation to dryness, purification was carried out by medium-pressure preparative chromatography, and the solvent was removed

by concentration under reduced pressure to obtain Compound **83** ($C_{28}H_{40}N_5O_5$, $[M+H]^+$ 526.3; found 526.5, yield 81.6%).

**Step 4. Synthesis of Compound 84**

Compound **83** (0.20 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **84** ($C_{23}H_{32}N_5O_3$, $[M+H]^+$ 426.2; found 426.3, crude product).

**Step 5. Synthesis of Compound 85**

Compound **84** (0.15 mmol), Compound **21** (0.35 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **85** ($C_{26}H_{34}N_5O_3$, $[M+H]^+$ 464.3; found 464.5, yield 66.7%).

**Step 6. Synthesis of Compound F1B-1**

Under nitrogen protection, Compound $\mathbf{R_{2\text{-}D1}}$ (0.10 mmol), Compound 85 (0.10 mmol), copper iodide (0.25 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and concentrated under reduced pressure to remove the solvent to obtain Compound **F1B-1** (7.2 mg, 7.73 μmol). LC-MS: $C_{57}H_{56}N_9O_4$, $[M/2+H]^+$ 466.0; found 466.0. $^1H$ NMR (400 MHz, Methanol-*d4*) δ 9.99-9.89 (m, 1H), 9.53-9.44 (m, 1H), 8.80 (s, 1H), 8.25-8.14 (m, 2H), 8.09 (s, 1H), 7.94-7.75 (m, 2H), 7.73-7.71 (m, 1H), 7.66-7.64 (m, 2H), 7.57- 7.53 (m, 5H), 7.34 (s, 2H), 7.18-7.16 (m, 2H), 5.22-5.10 (m, 2H), 4.49-4.42 (m, 2H), 4.42-4.38 (m, 2H), 3.87-3.47 (m, 6H), 3.34-3.22 (m, 4H), 3.13-3.05 (m, 2H), 2.93-2.90 (m, 1H), 2.89-2.86 (m, 1H), 2.79-2.75 (m, 1H), 2.52-2.47 (m, 1H), 2.45-2.03 (m, 4H), 1.73-1.67 (m, 2H).

According to the step 1 to step 6 of the method for Compound **F1B-1,** the raw material 1 in the following table was used to replace Compound $\mathbf{R_{1B\text{-}2}}$, the raw material 2 was used to replace Compound $\mathbf{R_{2\text{-}D1}}$, and other raw materials and operating methods remained unchanged, to obtain Compounds **F1B-2, F1B-3** and **F1B-4.**

| Compound No. | Structure and characterization data of compound | Raw material 1 | Raw material 2 | Yield |
|---|---|---|---|---|
| F1B-2 | 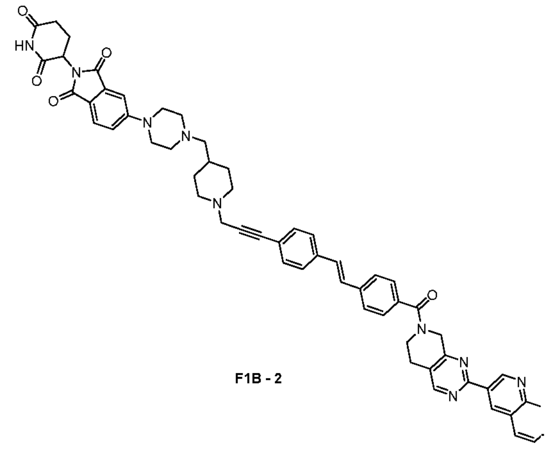 F1B - 2 | 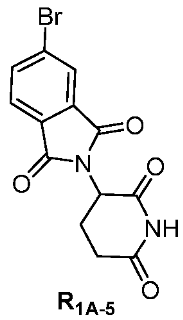 $R_{1A-5}$ | 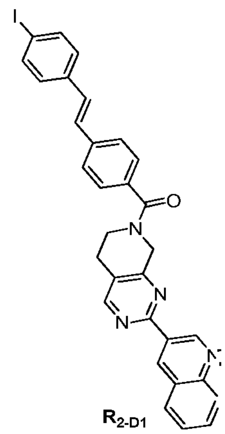 $R_{2-D1}$ | 13.6 % |
| | LC-MS: $C_{57}H_{54}N_9O_5$, $[M+H]^+$ 944.1; found 944.3. $^1H$ NMR (400 MHz, DMSO-$d_6$) δ 11.01 (s, 1H), 9.83-9.79 (m, 1H), 9.31-9.28 (m, 1H), 8.85 (s, 1H), 8.20 (s, 1H), 8.10 (s, 1H), 7.85 (s, 1H), 7.76-7.68 (m, 2H), 7.66-7.54 (m, 4H), 7.47-7.45 (m, 2H), 7.42-7.39 (m, 2H), 7.34 (s, 2H), 7.33-7.26 (m, 1H), 7.24-7.23 (m, 1H), 5.05-5.03 (m, 1H), 4.95-4.81 (m, 2H), 4.10-4.01 (m, 1H), 3.99-3.73 (m, 1H), 3.52 (s, 2H), 3.43 (s, 4H), 3.00-2.97 (m, 2H), 2.88-2.85 (m, 3H), 2.67-2.52 (m, 3H),2.34-2.33 (m, 1H), 2.29-2.17 (m, 5H), 2.01-2.00 (m, 1H), 1.76-1.74 (m, 2H), 1.52 (s, 1H), 1.23-1.14 (m, 3H). | | | |
| F1B-3 | 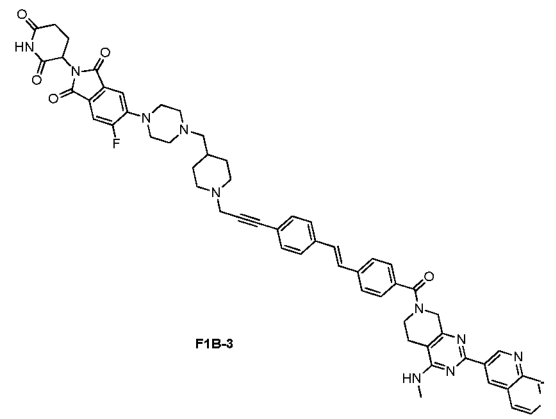 F1B-3 | 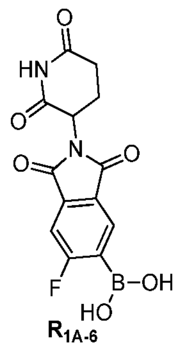 $R_{1A-6}$ | 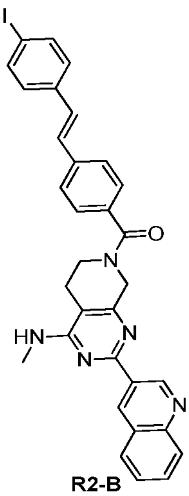 R2-B | 48.9 % |
| | LC-MS: $C_{58}H_{56}N_{10}O_5F$, $[M+H]^+$ 991.4; found 991.4. $^1H$ NMR (600 MHz, DMSO-$d_6$) δ 11.12 (s, 1H), 9.94-9.75 (m, 2H), 9.24-9.16 (m, 1H), 8.18-8.08 (m, 2H), 7.85-7.82 (m, 2H), 7.76-7.71 (m, 4H), 7.67-7.51 (m, 5H), 7.47 (s, 2H), 7.22 (s, 1H), 5.14-5.11 (m, 1H), 4.56 (s, 2H), 4.47 (s, 4H), 3.98-3.79 (m, 4H), 3.66-3.64 (m, 4H), 3.48-3.09 (m, 10H), 2.94-2.87 (m, 1H), 2.68-2.54 (m, 3H), 2.16 (s, 1H), 2.08-2.03 (m, 3H), 1.51-1.48 (m, 2H). Purity > 90%. | | | |

(continued)

| Compound No. | Structure and characterization data of compound | Raw material 1 | Raw material 2 | Yield |
|---|---|---|---|---|
| F1B-4 | 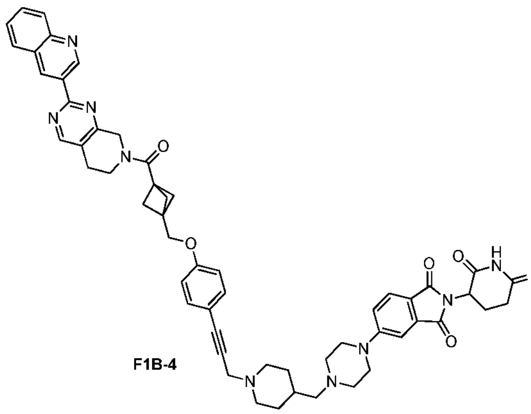 F1B-4<br>LC-MS: $C_{55}H_{56}N_9O_6$, $[M/2+H]^+$ 470.0; found 470.1. $^1H$ NMR (600 MHz, DMSO-$d_6$) $\delta$ 11.10 (s, 1H), 9.83-9.82 (m, 1H), 9.29 (s, 1H), 8.84 (s, 1H), 8.23 (d, *J* = 7.8 Hz, 1H), 8.11 (d, *J* = 8.4 Hz, 1H), 7.87 (t, *J* = 7.8 Hz, 1H), 7.76 (t, *J* = 8.4 Hz, 1H), 7.70 (t, *J* = 7.2 Hz, 1H), 7.51-7.48 (m, 3H), 7.36-7.34 (m, 1H), 7.03-7.02 (m, 2H), 5.11-5.08 (m, 1H), 4.95 (s, 1H), 4.79 (s, 2H), 4.34 (s, 2H), 4.22 (s, 2H), 4.14 (s, 2H), 3.96-3.80 (m, 2H), 3.64-3.62 (m, 4H), 3.47 (s, 3H), 3.33-3.08 (m, 6H), 2.96-2.87 (m, 3H), 2.61-2.50 (m, 2H), 2.20-2.17 (m, 7H), 2.07-2.03 (m, 3H). Purity > 99%. | 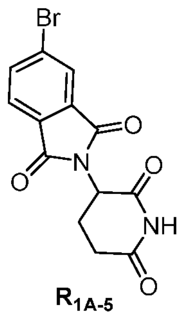 $R_{1A-5}$ | 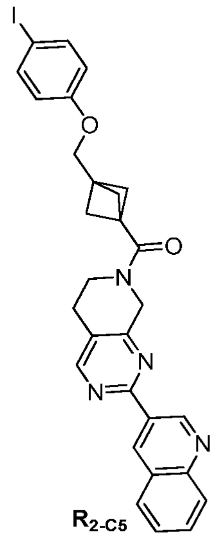 $R_{2-C5}$ | 31.2 % |

**Example 11.** Synthesis of Compounds **F2-1**, **F2-2**, **F2-2-A**, **F2-3** and **F2-4** of the present invention

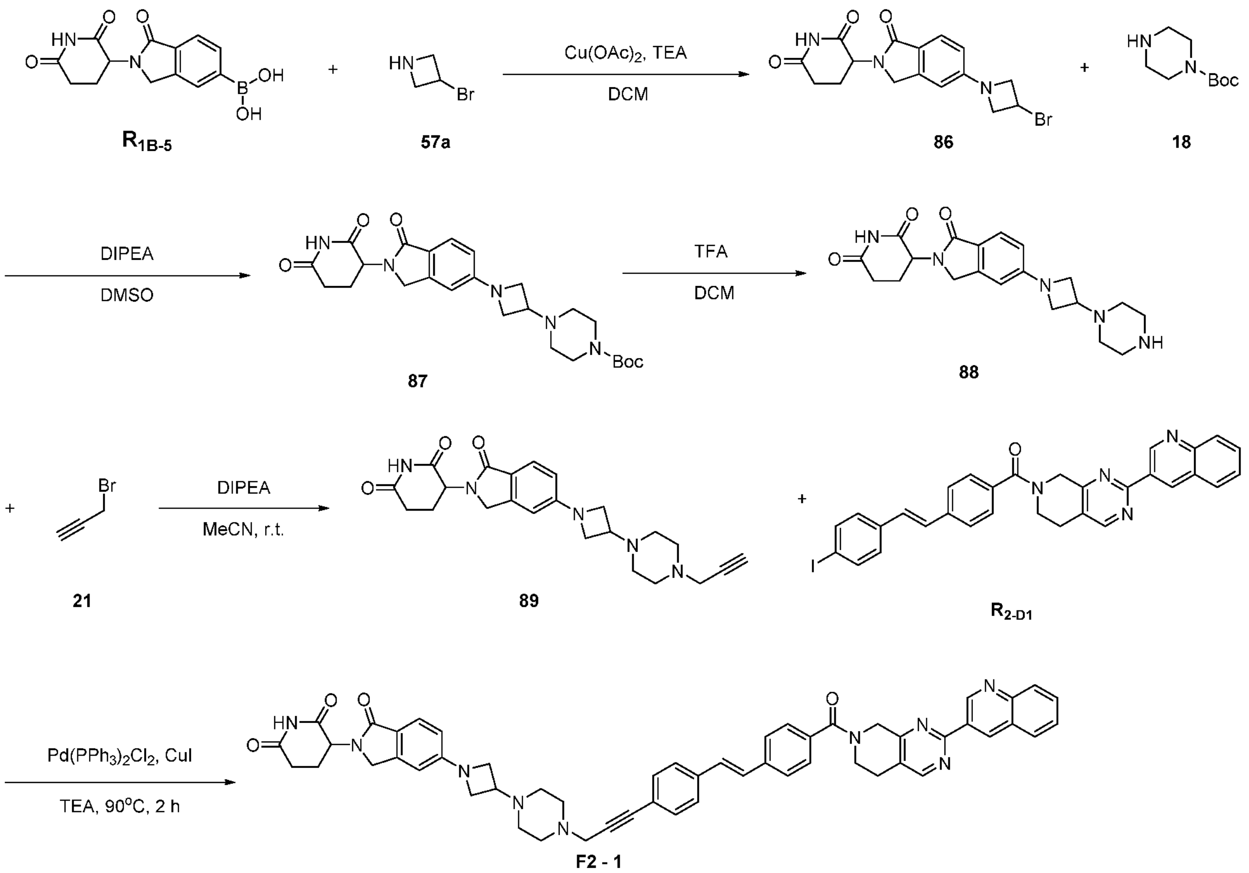


$R_{1B-5}$ 57a 86 18 87 88 21 89 $R_{2-D1}$ F2 - 1

**Step 1. Synthesis of Compound 86**

Under nitrogen protection, Compound $\mathbf{R_{1B-5}}$ (1.00 mmol), Compound **57a** (1.05 mmol), $Cu(OAc)_2$ (2.00 mmol), and triethylamine (5 mL) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3 × 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and concentrated under reduced pressure to remove the solvent to obtain Compound **86** ($C_{16}H_{17}BrN_3O_3$, $[M+H]^+$ 378.0; found 378.2, yield 48.2%).

**Step 2. Synthesis of Compound 87**

Compound **86** (0.45 mmol), Compound **18** (0.75 mmol), *N,N*-diisopropylethylamine (1.02 mmol) and DMSO (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×5 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **87** ($C_{25}H_{34}N_5O_5$, $[M+H]^+$ 484.3; found 484.2, yield 66.7%).

**Step 3. Synthesis of Compound 88**

Compound **87** (0.30 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **88** ($C_{20}H_{26}N_5O_3$, $[M+H]^+$ 384.2; found 384.2, crude product).

**Step 4. Synthesis of Compound 89**

Compound **88** (0.30 mmol), Compound **21** (0.65 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3 × 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **89** ($C_{23}H_{28}N_5O_3$, $[M+H]^+$ 422.2; found 422.3, yield 33.3%).

**Step 5. Synthesis of Compound F2-1**

Under nitrogen protection, Compound $\mathbf{R_{2\text{-}D1}}$ (0.10 mmol), Compound 89 (0.10 mmol), copper iodide (0.45 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3 × 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and concentrated under reduced pressure to remove the solvent to obtain Compound **F2-1** (4.7 mg, 5.29 μmol). LC-MS: $C_{54}H_{50}N_9O_4$, $[M/2+H]^+$ 445.0; found 445.1. $^1$H NMR (400 MHz, DMSO-*d6*) δ 10.94 (s, 1H), 9.83-9.78 (m, 1H), 9.31-9.28 (m, 1H), 8.86 (s, 1H),8.57-7.86 (m, 4H), 7.85-7.41 (m, 10H), 7.18-6.54 (m, 4H), 5.3-4.80 (m, 3H), 4.31- 3.99 (m, 6H), 3.17-2.50 (m, 7H), 1.46-0.85 (m, 12H).

According to the step 1 to step 5 of the method for Compound F2-1, the raw material 1 in the following table was used to replace Compound $\mathbf{R_{1B\text{-}5}}$, the raw material 2 in the following table was used to replace Compound **57a**, and the other raw materials and operating methods remained unchanged to obtain Compounds **F2-2, F2-2-A, F2-3, F2-4**.

| Compound No. | Structure and characterization data of compound | Raw material 1 | Raw material 2 | Yield |
|---|---|---|---|---|
| **F2-2** | 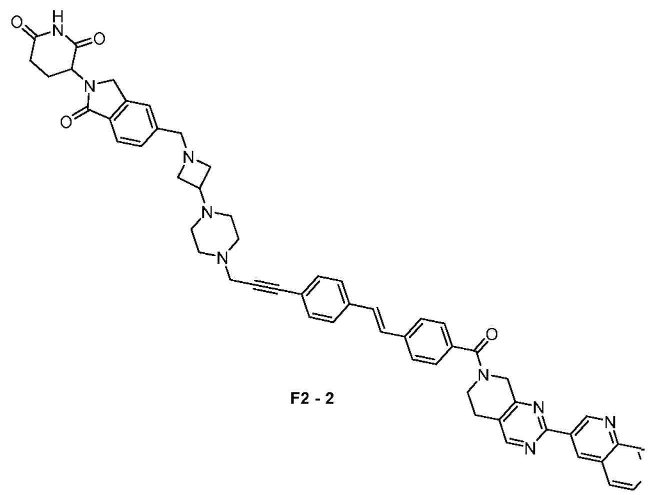 F2 - 2 <br> LC-MS: $C_{55}H_{52}N_9O_4$, $[M/2+H]^+$ 452.0; found 452.2. $^1$H NMR (400 MHz, DMSO-$d_6$) δ 9.85-9.78 (m, 1H), 9.40-9.31 (m, 1H), 8.86 (s, 1H),8.23 (s, 1H), 8.10 (s, 1H), 7.86-7.82 (m, 2H), 7.77-7.70 (m, 7H), 7.63 (d, *J* = 8.0 Hz, 1H), 7.57-7.55 (m, 4H), 7.44 (s, 2H), 5.16-5.12 (m, 1H), 4.94-4.88 *m, 2H), 4.53 (s, 2H), 4.38-4.34 (m, 4H), 4.16-4.13 (m, 2H), 4.05-4.04 (m, 2H), 3.43 (t, *J* = 8.0 Hz, 2H), 3.29-3.17 (m, 3H), 3.10 (t, *J* = 8.0 Hz, 3H), 2.99-2.94 (m, 1H), 2.89-2.81 (m, 3H), 2.68-2.51 (m, 2H), 2.50-2.31 (m, 2H), 2.03-2.01 (m, 1H). | 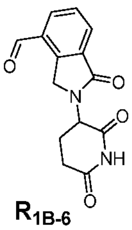 $\mathbf{R_{1B\text{-}6}}$ | 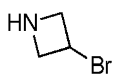 **57a** | 23.5 % |

(continued)

| Compound No. | Structure and characterization data of compound | Raw material 1 | Raw material 2 | Yield |
|---|---|---|---|---|
| F2-2-A | 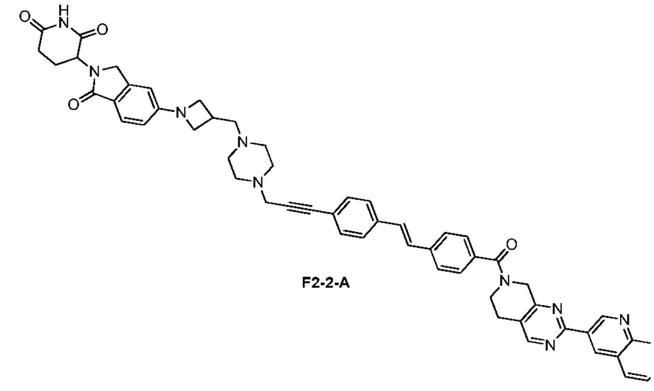 F2-2-A | 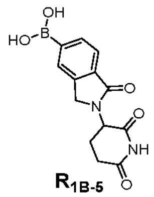 $R_{1B-5}$ | 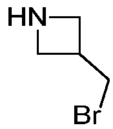 57b | 12.3 % |
| | LC-MS: $C_{55}H_{52}N_9O_4$, $[M/2+H]^+$ 452.0 found 451.9. $^1H$ NMR (600 MHz, $CDCl_3$) δ 9.81 (br, 1H), 9.08 (br, 1H), 8.61 (s, 1H), 8.10 (d, *J* = 7.8 Hz, 2H), 7.96 (d, *J* = 7.2 Hz, 1H), 7.88 (br, 1H), 7.70 (t, *J* = 7.2 Hz, 1H), 7.61 (d, *J* = 8.4 Hz, 1H), 7.54-7.50 (m, 3H), 7.48-7.45 (m, 2H), 7.41-7.37 (m, 5H), 7.88 (m, 2H), 5.13-5.10 (m, 1H), 4.31-4.29 (m, 1H), 4,16-4.13 (m, 1H), 4.03-3.99 (m, 3H), 3.85 (s, 1H), 3.57 (t, *J* = 6.0 Hz, 2H), 3.51-3.49 (m, 3H), 3.42 (s, 1H), 2.95 (br, 4H), 2.83-2.80 (m, 2H), 2.77-2.76 (m, 1H), 2.75 (s, 6H), 2.27-2.22 (m, 2H), 2.15-2.10 (m, 2H). Purity > 75%. | | | |
| F2-3 | 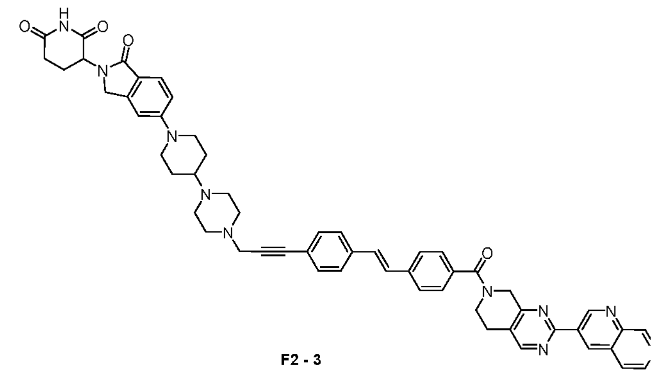 F2 - 3 | 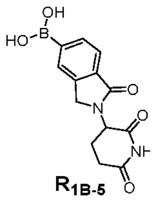 $R_{1B-5}$ | 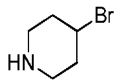 57b | 21.2 % |
| | LC-MS: $C_{56}H_{54}N_9O_4$, $[M/2+H]^+$ 458.7; found 458.7. $^1H$ NMR (600 MHz, DMSO-$d_6$) δ 9.79 (s, 1H), 9.35 (s, 1H), 8.85 (s, 1H), 8.35-7.85 (m, 2H), 7.88-7.29 (m, 11H), 7.16-7.11 (m, 2H), 5.33 (s, 1H), 5.20-4.69 (m, 3H), 4.49-4.15 (m, 2H), 3.74 (s, 3H), 3.65-3.40 (m, 5H), 3.16-2.80 (m, 6H), 2.50 (s, 3H), 2.24-1.93 (m, 5H), 1.23 (s, 6H). | | | |

(continued)

| Compound No. | Structure and characterization data of compound | Raw material 1 | Raw material 2 | Yield |
|---|---|---|---|---|
| F2-4 | 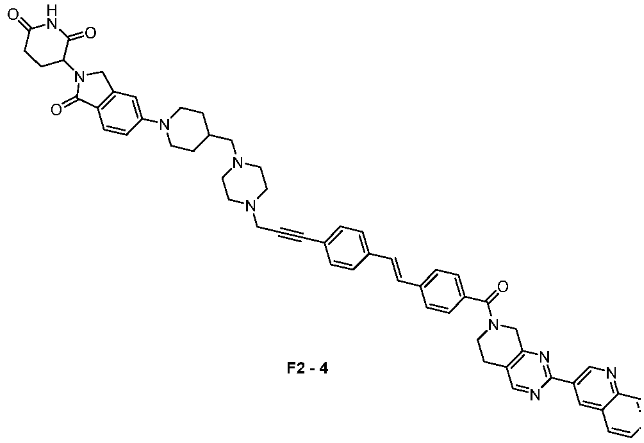 F2 - 4<br>LC-MS: $C_{57}H_{56}N_9O_4$, $[M/2+H]^+$ 466.1; found 466.0. $^1H$ NMR (400 MHz, DMSO-$d_6$) δ 10.95 (s, 1H), 9.83-9.77 (m, 1H), 9.31-9.28 (m, 1H), 8.86 (s, 1H), 8.21 (s, 1H), 8.10 (s, 1H), 7.85 (s, 1H), 7.76-7.68 (m, 2H), 7.66-7.57 (m, 3H), 7.54-7.51 (m, 2H), 7.50-7.48 (m, 3H), 7.40 (s, 2H), 7.08-7.06 (m, 2H), 5.03-5.02 (m, 1H), 4.95-4.81 (m, 2H), 4.32-4.30 (m, 1H), 4.22-4.18 (m, 1H), 3.92-3.90 (m, 2H), 3.74 (s, 4H), 3.12-3.07 (m, 8H), 3.00-2.99 (m, 3H), 2.89-2.78 (m, 2H), 2.38-2.33 (m, 1H), 2.07-1.97 (m, 4H), 1.84-1.81 (m, 2H), 1.33-1.16 (m, 3H). | 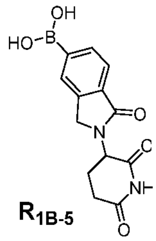 $R_{1B-5}$ | 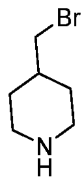 57c | 19.6 % |

According to the step 1 to step 5 of the method for Compound **F2-1**, the raw material **1** in the following table was used to replace compound $\mathbf{R_{2D-1}}$, the raw material **2** in the following table was used to replace Compound **57a**, and the other raw materials and operating methods remained unchanged, to obtain Compounds **F2-7** to **F2-10**.

| Compound No. | Structure and characterization data of compound | Raw material 1 | Raw materia 12 | Yield |
|---|---|---|---|---|
| F2-7 | 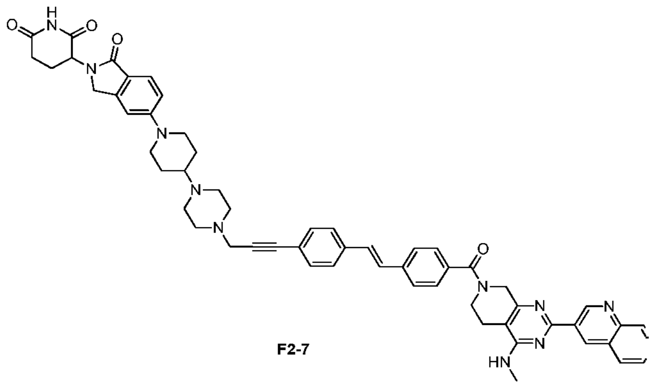<br>F2-7<br>LC-MS: $C_{57}H_{57}N_{10}O_4$, $[M/2+H]^+$ 473.6; found 473.7. $^1H$ NMR (600 MHz, DMSO-$d_6$) δ 10.97-10.95 (m, 1H), 9.82-9.73 (m, 1H), 9.24-9.13 (m, 2H), 8.16-8.08 (m, 2H), 7.82 (s, 1H), 7.23 (d, $J$ = 8.4 Hz, 2H), 7.65 (t, $J$ = 7.2 Hz, 3H), 7.55-7.52 (m, 3H), 7.48-7.45 (m, 2H), 7.39 (s, 2H), 7.22-7.19 (m, 1H), 7.13-7.11 (m, 1H), 5.10-5.04 (m, 1H), 4.73 (s, 1H), 4.57 (s, 1H), 4.36-4.31 (m, 1H), 4.22-4.20 (m, 1H), 4.08 - 3.96 (m, 4H), 3.71-3.68 (m, 2H), 3.09-3.08 (m, 8H), 2.89-2.77 (m, 4H), 2.65-2.58 (m, 6H), 2.39-2.37 (m, 1H), 2.12 (s, 2H), 1.99-1.98 (m, 1H), 1.68 (s, 2H). Purity > 90%. | 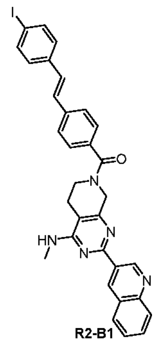<br>R2-B1 | 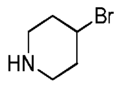<br>57b | 15.8 % |
| F2-8 | 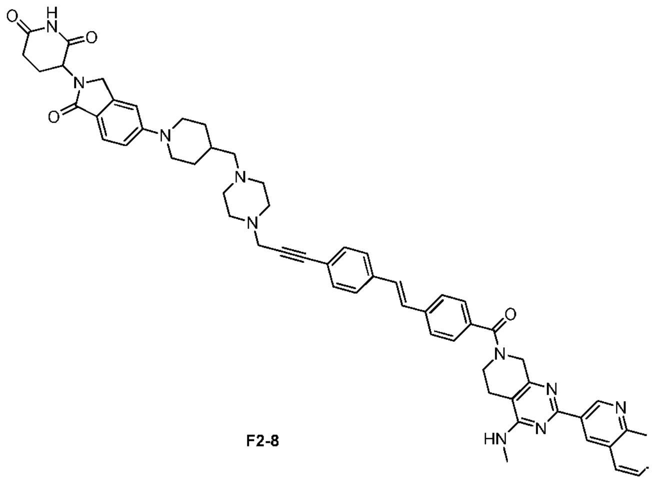<br>F2-8<br>LC-MS: $C_{58}H_{59}N_{10}O_4$, $[M+H]^+$ 959.5; found 958.1. $^1H$ NMR (600 MHz, DMSO-$d_6$) δ 11.00 (s, 1H), 9.82-9.80 (m, 1H), 9.27 (s, 1H), 8.81 (s, 1H), 8.21 (d, $J$ = 8.4 Hz, 1H), 8.10 (d, $J$ = 3.0 Hz, 1H), 7.85 (t, $J$ = 8.4 Hz, 1H), 7.72-7.68 (m, 3H), 7.56 (d, $J$ = 5.8 Hz, 1H), 7.37-7.36 (m, 2H), 6.95-6.94 (m, 2H), 5.13-5.10 (m, 1H), 4.95-4.78 (m, 2H), 4.47-4.44 (m, 1H), 4.35-4.32 (m, 1H), 4.08-4.04 (m, 3H), 3.95-3.80 (m, 2H), 3.65 (s, 2H), 3.58 (s, 2H), 3.34 (s, 2H), 2.94-2.86 (m, 3H), 2.61-2.59 (m, 6H), 2.42-2.38 (m, 1H), 2.27 (s, 6H), 2.02-2.00 (m, 1H). Purity > 90%. | 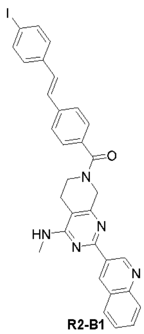<br>R2-B1 | 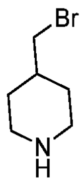<br>57c | 16.7 % |

(continued)

| Compound No. | Structure and characterization data of compound | Raw material 1 | Raw materia 12 | Yield |
|---|---|---|---|---|
| F2-9 | 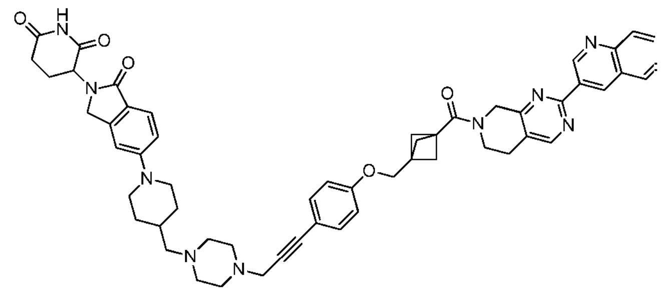<br>F2-9<br>LC-MS: $C_{55}H_{58}N_9O_5$, $[M/2+H]^+$ 463.1; found 462.8. $^1$H NMR (600 MHz, DMSO-$d_6$) δ 9.87 (s, 1H), 9.46-9.44 (m, 1H), 8.84 (s, 1H), 8.32-8.31 (m, 1H), 8.18-8.17 (m, 1H), 7.97-7.96 (m, 1H), 7.80-7.78 (m, 1H), 7.56-7.55 (m, 1H), 7.44-7.43 (m, 2H), 7.10-7.09 (m, 2H), 7.01-6.99 (m, 2H), 5.05-4.80 (m, 3H), 4.36-4.33 (m, 1H), 4.24-4.21 (m, 1H), 4.11 (s, 2H), 3.96-3.90 (m, 6H), 3.38 (s, 3H), 3.14-2.88 (m, 12H), 2.63-2.56 (m, 1H), 2.41-2.34 (m, 1H), 2.18 (s, 6H), 2.07-1.98 (m, 2H), 1.84-1.83 (m, 2H), 1.31-1.30 (m, 2H). Purity > 99%. | 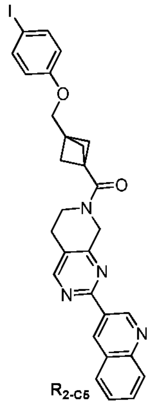<br>$R_{2-C6}$ | 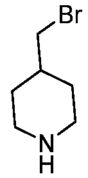<br>**57c** | 35.2 % |
| F2-10 | 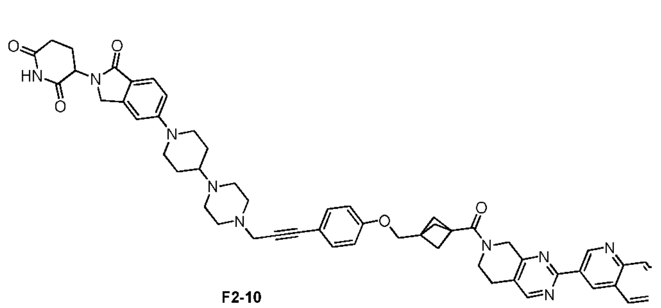<br>F2-10<br>LC-MS: $C_{54}H_{56}N_9O_5$, $[M/2+H]^+$ 456.0; found 455.8. $^1$H NMR (600 MHz, DMSO-$d_6$) δ 10.97-10.95 (m, 1H), 9.82 (s, 1H), 9.29 (s, 1H), 8.84 (s, 1H), 8.23 (d, *J* = 6.6 Hz, 1H), 8.11 (d, *J* = 8.4 Hz, 1H), 7.87 (t, *J* = 7.2 Hz, 1H), 7.70 (t, *J* = 7.2 Hz, 1H), 7.55-7.54 (m, 1H), 7.46-7.45 (m, 2H), 7.38-7.37 (m, 2H), 7.02-6.96 (m, 2H), 5.08-5.04 (m, 1H), 4.95-4.79 (m, 2H), 4.36-4.20 (m, 2H), 4.09-3.95 (m, 6H), 3.80 (s, 1H), 3.68-3.64 (m, 3H), 3.17 (s, 4H), 3.06-2.77 (m, 5H), 2.64-2.62 (m, 3H), 2.39-2.36 (m, 1H), 2.16-2.03 (m, 9H), 2.00-1.95 (m, 1H), 1.67 (s, 2H). Purity > 95%. | 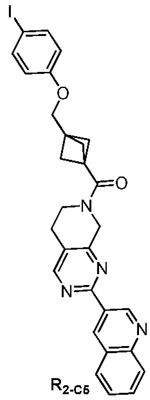<br>$R_{2-C6}$ | 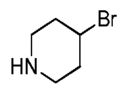<br>**57b** | 21.2 % |

According to the step 1 to step 5 of the method for Compound **F2-1**, Compound $\mathbf{R_{1B-5}}$ was replaced with Compound $\mathbf{R_{1A-5}}$, Compound $\mathbf{R_{2-D1}}$ was replaced with the raw material **1** in the following table, Compound **57a** was replaced with the raw material **2** in the following table, and the other raw materials and operating method remained unchanged to obtain Compound **F2-11**.

| Compound No. | Structure and characterization data of compound | Raw material 1 | Raw material 2 | Yield |
|---|---|---|---|---|
| **F2-11** | 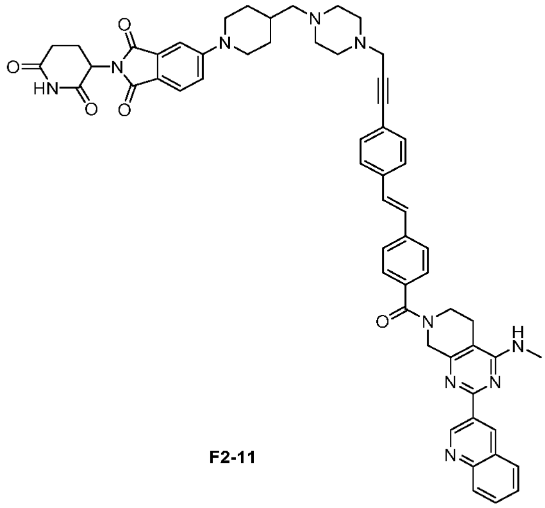 F2-11 | 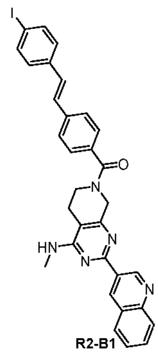 R2-B1 | 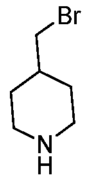 **57c** | |
| | LC-MS: $C_{58}H_{57}N_{10}O_5$, $[M+H]^+$ 973.44; found 973.5. $^1H$ NMR (400 MHz, DMSO-$d_6$) δ 11.01 (s, 1H), 9.87-9.75 (m, 1H), 9.19-9.12 (m, 1H), 8.15-8.06 (m, 2H), 7.80 (m, 1H), 7.73 (d, *J* = 2.6 Hz, 2H), 7.68-7.63 (m, 4H), 7.54-7.52 (m, 2H), 7.47-7.45 (m, 2H), 7.38 (s, 2H), 7.33 (m, 1H), 7.24 (m, 1H), 7.17 (s, 1H), 5.09-5.06 (m, 1H), 4.71-4.57 (m, 2H), 3.95-3.70 (m, 2H), 3.53 (s, 2H), 3.42 (s, 4H), 3.34 (m, 2H), 3.18-3.17 (m, 3H), 2.94-2.86 (m, 3H), 2.64-2.53 (m, 4H), 2.49-2.42 (m, 4H), 2.23-2.17 (m, 4H), 2.03-1.99 (m, 1H), 1.74 (m, 2H), 1.52 (s, 1H). Purity > 99%. | | | |

**Example 12.** Synthesis of Compounds **F2-5** and **F2-6** of the present invention

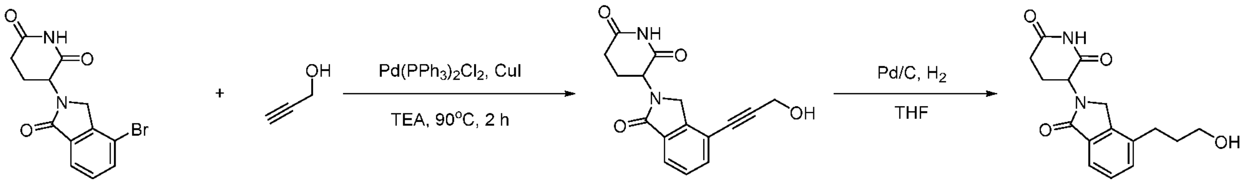


$R_{1B-1}$ **90** **91** **92**

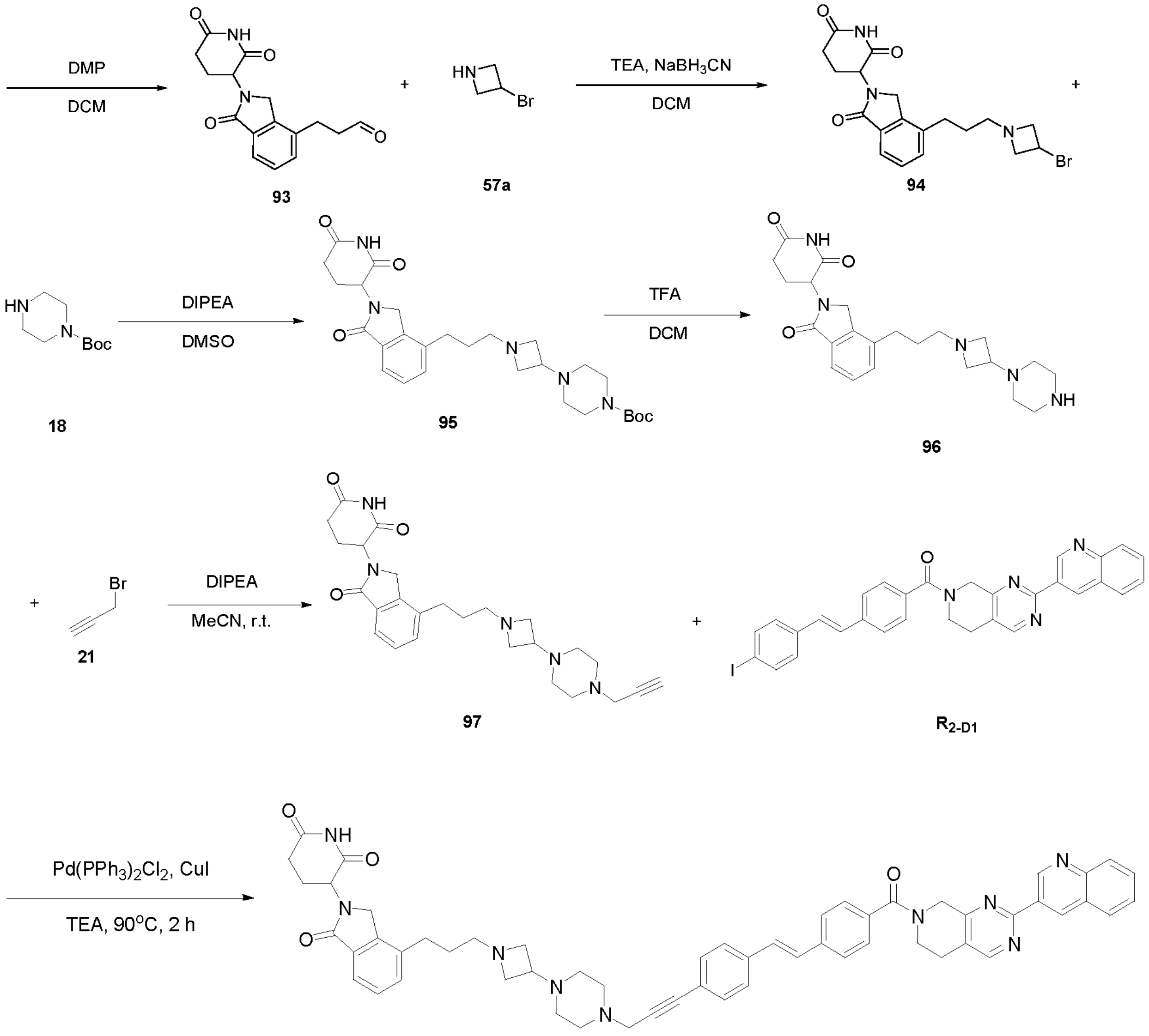


93 + 57a → 94 + 18 → 95 → 96 + 21 → 97 + $R_{2\text{-}D1}$ →

F2 - 5

**Step 1. Synthesis of Compound 91**

Under nitrogen protection, Compound $\mathbf{R_{1B\text{-}1}}$ (1.00 mmol), compound 90 (1.05 mmol), copper iodide (2.00 mmol), triethylamine (5 mL), and $Pd(PPh_3)_2Cl_2$ (1.12 mg, 0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3 × 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and concentrated under reduced pressure to remove the solvent to obtain Compound 91 ($C_{16}H_{15}N_2O_4$, $[M+H]^+$ 299.1; found 299.2, yield 58.6%).

**Step 2. Synthesis of Compound 92**

Compound **91** (0.55 mmol), tetrahydrofuran (5 mL), and palladium-on-carbon (30.00 mg) were added in sequence to a 50 mL reaction bottle. The reaction system was replaced with hydrogen, maintained at an atmospheric pressure hydrogen atmosphere, and the reaction was carried out under stirring at room temperature for 3 hours (LC-MS monitoring). Filtration was carried out to remove palladium-on-carbon, washing was carried out with methanol (2×20 mL), the filtrate was collected, and concentrated under reduced pressure to remove the solvent to obtain Compound 92 ($C_{16}H_{19}N_2O_4$, $[M+H]^+$ 303.1; found 303.2, crude product).

**Step 3. Synthesis of Compound 93**

Compound **92** (0.55 mmol), Dess-Martin Periodinane (1.02 mmol) and dichloromethane (5 mL) were added to a 50 mL reaction bottle, reacted under stirring at room temperature for 1 hour, and then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound 93 ($C_{16}H_{17}N_2O_4$, $[M+H]^+$ 301.1; found 301.2, yield 81.8%).

**Step 4. Synthesis of Compound 94**

Compound **93** (0.45 mmol), Compound **57a** (0.65 mmol), triethylamine (2 mL), sodium cyanoborohydride (0.85 mmol) and dichloroethane (5 mL) were added in sequence to a 50 mL reaction bottle, reacted under stirring at room temperature for 2 hours, then the reaction was quenched (LC-MS monitoring), the solvent was removed by evaporation to dryness, purification was carried out by medium-pressure preparative chromatography, and concentrated under reduced pressure to remove the solvent to obtain Compound **94** ($C_{19}H_{23}BrN_3O_3$, $[M+H]^+$ 420.1; found 420.2, yield 77.8%).

**Step 5. Synthesis of Compound 95**

Compound **94** (0.35 mmol), Compound **18** (0.65 mmol), *N,N*-diisopropylethylamine (1.02 mmol) and DMSO (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **95** ($C_{28}H_{40}N_5O_5$, $[M+H]^+$ 526.3; found 526.5, yield 85.7%).

**Step 6. Synthesis of Compound 96**

Compound **95** (0.30 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **96** ($C_{23}H_{32}N_5O_3$, $[M+H]^+$ 426.2; found 426.4, crude product).

**Step 7. Synthesis of Compound 97**

Compound **96** (0.20 mmol), Compound **21** (0.55 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **97** ($C_{26}H_{34}N_5O_3$, $[M+H]^+$ 464.3; found 464.5, yield 51.8%).

**Step 8. Synthesis of Compound F2-5**

Under nitrogen protection, Compound $\mathbf{R_{2\text{-}D1}}$ (0.10 mmol), Compound **97** (0.09 mmol), copper iodide (0.25 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3 × 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and concentrated under reduced pressure to remove the solvent to obtain Compound **F2-5** (7.2 mg, 7.73 μmol). LC-MS: $C_{57}H_{56}N_9O_4$, $[M/2+H]^+$ 465.7; found 466.2. $^1H$ NMR (400 MHz, Methanol-*d4*) δ 9.96-9.90 (m, 1H), 9.58-9.51 (m, 1H), 8.79 (s, 1H), 8.22-8.15 (m, 2H), 7.98 (s, 1H), 7.97-7.73 (m, 3H), 7.71-7.70 (m, 1H), 7.68 (d, *J*= 4.0 Hz, 2H), 7.63-7.45 (m, 6H), 7.33 (s, 2H), 5.21-5.16 (m, 1H), 5.09-4.98 (m, 2H), 4.57-4.44 (m, 2H), 4.37 (s, 2H), 4.32-3.68 (m, 6H), 3.59-3.30 (m, 5H), 3.29-3.05 (m, 4H), 2.96-2.91 (m, 2H), 2.89-2.78

(m, 3H), 2.68-2.66 (m, 1H), 2.55-2.44 (m, 2H), 2.21-2.16 (m, 2H), 1.96-1.71 (m, 2H).

Compound **F2-6** could be obtained by following the step 1 to step 8 of the method for Compound **F2-5**, replacing Compound **57a** with the raw materials in the following table, and keeping other raw materials and operating methods unchanged.

| Compound No. | Structure and characterization data of compound | Raw material | Yield |
|---|---|---|---|
| F2-6 | 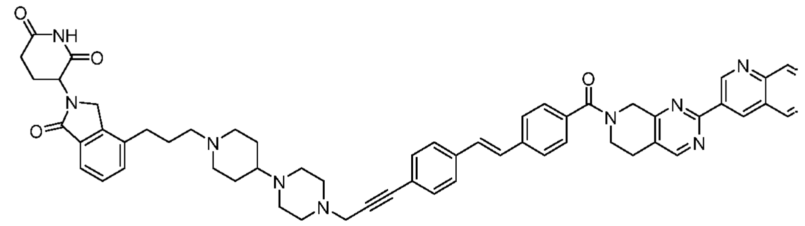 F2 - 6 | 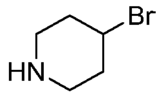 **57b** | 20.8 % |
| | LC-MS: $C_{59}H_{60}N_9O_4$, $[M/2+H]^+$ 479.8; found 480.1. $^1H$ NMR (400 MHz, Methanol-$d_4$) δ 10.01-9.91 (m, 1H), 9.80-9.74 (m, 1H), 8.80 (d, *J* = 4.0 Hz, 1H), 8.34-8.33 (m, 2H), 8.08 (s, 1H), 7.89 (s, 1H), 7.74-7.69 (m, 3H), 7.62-7.59 (m, 2H), 7.56-7.47 (m, 6H), 7.30 (d, *J* = 4.0 Hz, 2H), 5.21-5.16 (m, 1H), 5.03-4.98 (m, 2H), 4.57-4.38 (m, 3H), 4.12-4.08 (m, 3H), 3.85-3.71 (m, 3H), 3.31-2.97 (m, 12H), 2.95-2.88 (m, 2H), 2.80-2.78 (m, 3H), 2.57-2.46 (m, 2H), 2.26-1.95 (m, 7H). | | |

**Example 13.** Synthesis of Compounds **F3A-1**, **F3A-2**, **F3A-3**, and **F3A-4** of the present invention

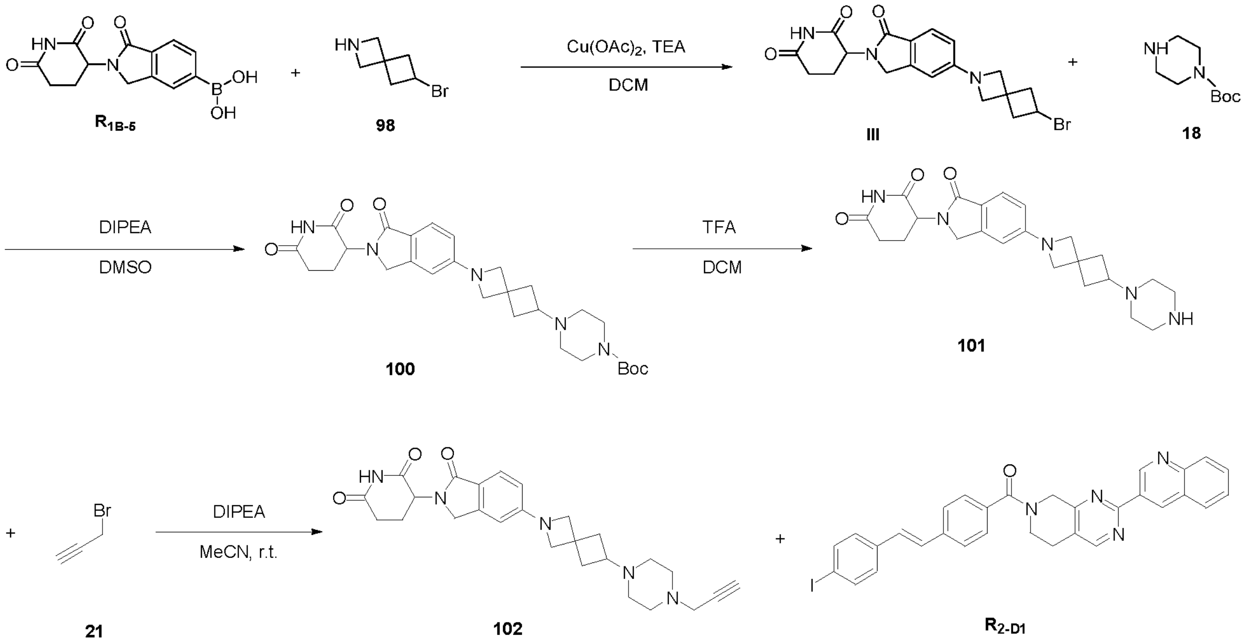

R1B-5 98 III 18 100 101 21 102 R2-D1

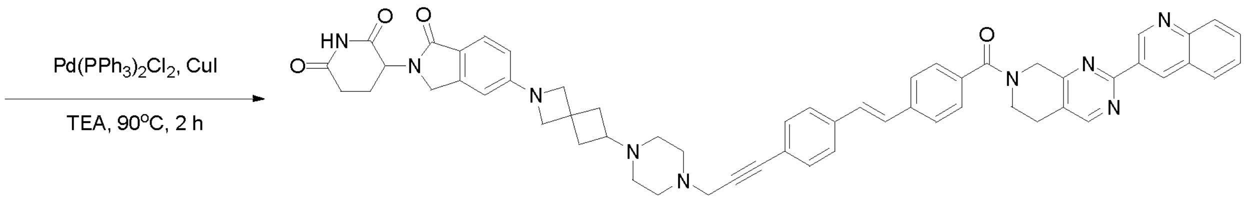


F3A - 3

**Step 1. Synthesis of** Compound **III**

Under nitrogen protection, Compound $\mathbf{R_{1B-5}}$ (1.0 mmol), Compound **98** (1.0 mmol), $Cu(OAc)_2$ (2.0 mmol), and triethylamine (5 mL) were added to a 50 mL reaction bottle in sequence. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and concentrated under reduced pressure to remove the solvent to obtain Compound **III** ($C_{19}H_{21}BrN_3O_3$, $[M+H]^+$ 418.1; found 418.2, yield 78.9%).

**Step 2. Synthesis of Compound 100**

Compound **III** (0.60 mmol), Compound **18** (0.85 mmol), *N,N*-diisopropylethylamine (1.02 mmol) and DMSO (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **100** ($C_{28}H_{38}N_5O_5$, $[M+H]^+$ 524.3; found 524.5, yield 76.2%).

**Step 3. Synthesis of Compound 101**

Compound **100** (0.45 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound 101 ($C_{23}H_{30}N_5O_3$, $[M+H]^+$ 424.2; found 424.1, crude product).

**Step 4. Synthesis of Compound 102**

Compound **101** (0.40 mmol), Compound **21** (0.85 mmol), *N,N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **102** ($C_{26}H_{32}N_5O_3$, $[M+H]^+$ 462.2; found 462.3, yield 37.5%).

**Step 5. Synthesis of Compound F3A-3**

Under nitrogen protection, Compound $\mathbf{R_{2-D1}}$ (0.15 mmol), Compound **102** (0.15 mmol), copper iodide (0.45 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and concentrated under reduced pressure to remove the solvent to obtain Compound **F3A-3** (2.4 mg, 2.70 μmol). LC-MS: $C_{54}H_{50}N_9O_4$, $[M/2+H]^+$ 464.7; found 465.0. $^1H$

NMR (400 MHz, DMSO-*d6*) δ 10.93 (s, 1H), 9.83-9.61 (m, 1H), 9.30-9.29 (m, 1H), 8.86 (s, 1H), 8.21-7.40 (m, 13H), 7.28-7.02 (m, 2H), 6.77-6.48 (m, 2H), 5.32-4.80 (m, 3H), 4.31- 3.72 (m, 8H), 3.10-2.99 (m, 8H), 1.46-0.85 (m, 13H).

Compounds **F3A-1**, **F3A-2** and **F3A-4** could be obtained by following the step 2 to step 5 of the method for Compound **F3A-3**, replacing Compound **III** with the raw materials in the following table, and keeping the other raw materials and operating methods unchanged.

The preparation of Compounds **IA**, **IB** and **II** was as follows:

**Synthesis of Compound I**

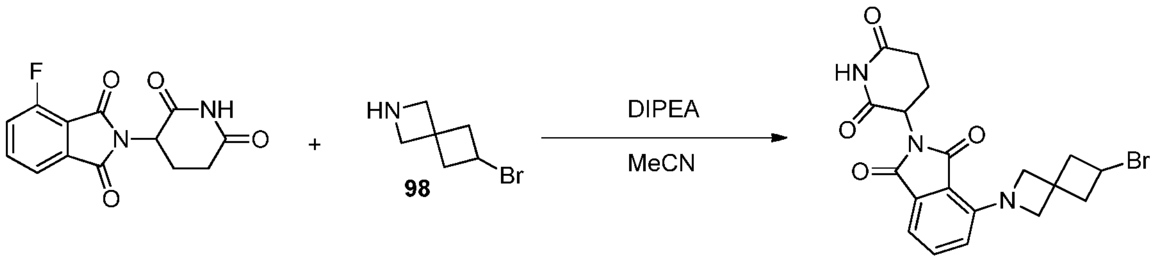


$R_{1A-1}$ 98 I

Compound $R_{1A-1}$ (1.0 mmol), Compound **98** (1.15 mmol), *N,N*-diisopropylethylamine (2.80 mmol) and acetonitrile (10 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Intermediate **I** ($C_{19}H_{19}BrN_3O_4$, $[M+H]^+$ 432.1; found 432.3, yield 86.9%).

**Synthesis of Compound II**

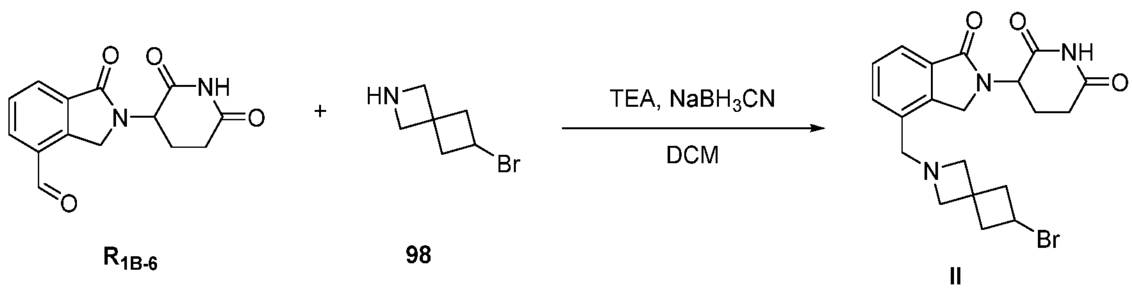


$R_{1B-6}$ 98 II

Compound $R_{1B-6}$ (1.00 mmol), Compound **98** (1.05 mmol), triethylamine (2 mL), sodium cyanoborohydride (1.55 mmol) and dichloromethane (5 mL) were added in sequence to a 50 mL reaction bottle, reacted under stirring at room temperature for 2 hours, then the reaction was quenched (LC-MS monitoring), the solvent was removed by evaporation to dryness, purified by medium-pressure preparative chromatography, and concentrated under reduced pressure to remove the solvent to obtain Intermediate **II** ($C_{20}H_{23}BrN_3O_3$, $[M+H]^+$ 432.1; found 432.3, yield 85.2%).

**Synthesis of Compound IB**

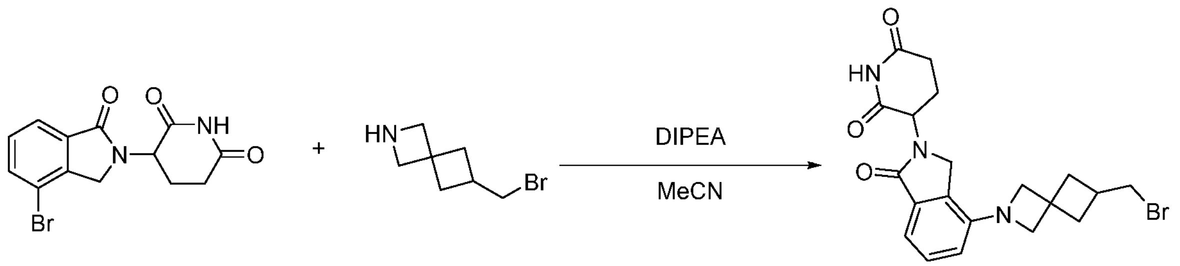


$R_{1B-1}$ 99 IB

Compound $\mathbf{R_{1B-1}}$ (1.0 mmol), Compound **99** (1.15 mmol), *N,N*-diisopropylethylamine (2.80 mmol) and acetonitrile (10 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Intermediate **IB** ($C_{20}H_{23}BrN_3O_3$, $[M+H]^+$ 433.3; found 433.3, yield 79.2%).

| Compound No. | Structure and characterization data of compound | Raw material | Yield |
|---|---|---|---|
| F3A-1 | 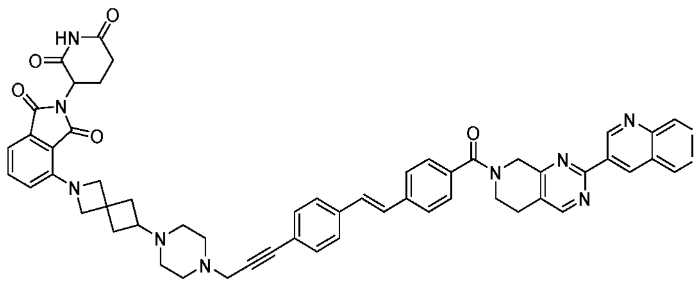<br>F3A - 1<br>LC-MS: $C_{57}H_{52}N_9O_5$, $[M/2+H]^+$ 472.0; found 472.1. $^1$H NMR (400 MHz, DMSO-$d_6$) δ 9.85-9.75 (m, 1H), 9.45-9.34 (m, 1H), 8.85 (s, 1H), 8.29-8.24 (m, 2H), 8.16 (s, 1H), 7.97-7.79 (m, 3H), 7.78-7.76 (m, 2H), 7.68 (d, *J* = 8.0 Hz, 1H), 7.61 (d, *J* = 4.0 Hz, 2H), 7.58-7.52 (m, 2H), 7.38 (s, 2H), 7.17 (d, *J* = 7.2 Hz, 1H), 6.80 (d, *J* = 8.0 Hz, 1H), 5.10-4.97 (m, 3H), 4.25-4.18 (m, 3H), 3.99-3.65 (m, 5H), 3.16-2.81 (m, 12H), 2.73-2.62 (m, 3H), 2.58-2.41 (m, 3H), 2.05-2.04 (m, 1H). | 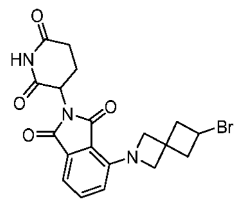<br>I | 20.1 % |
| | 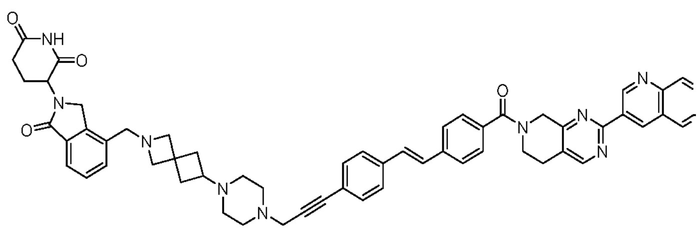<br>F3A - 2 | 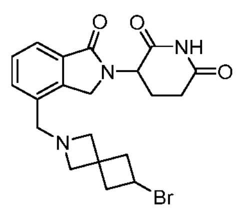<br>II | 18.6 % |

(continued)

| Compound No. | Structure and characterization data of compound | Raw material | Yield |
|---|---|---|---|
| F3A-2 | LC-MS: $C_{58}H_{56}N_9O_4$, $[M/2+H]^+$ 472.0; found 471.9. $^1H$ NMR (400 MHz, DMSO-$d_6$) δ 9.79-9.73 (m, 1H), 9.33-9.31 (m, 1H), 8.85 (s, 1H), 8.21-8.10 (m, 2H), 7.86 - 7.84 (m, 2H), 7.77-7.65 (m, 7H), 7.57-7.47 (m, 4H), 7.38 (s, 2H), 5.33-5.14 (m, 2H), 4.95-4.92 (m, 1H), 4.80-4.77 (m, 1H), 4.62-4.58 (2H), 4.49-4.45 (m, 3H), 4.28-4.21 (m, 3H), 4.08-4.02 (m, 3H), 3.58-3.52 (m, 2H), 3.19-2.89 (m, 7H), 2.03-1.97 (m, 2H), 1.23 (s, 6H). | | |
| | 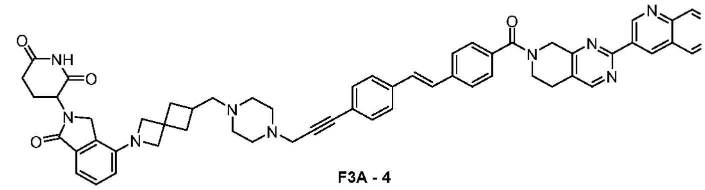 F3A - 4 | 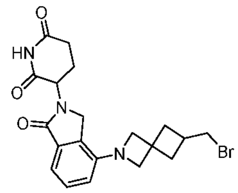 IB | 12.7 % |
| F3A-4 | LC-MS: $C_{58}H_{56}N_9O_4$, $[M+H]^+$ 472.0; found 472.0. $^1H$ NMR (400 MHz, DMSO-$d_6$) δ 9.84-9.77 (m, 1H), 9.40-9.33 (m, 1H), 8.86 (s, 1H), 8.25-8.13 (m, 2H), 7.93 (s, 1H), 7.77-7.69 (m, 3H), 7.67-7.63 (m, 2H), 7.60-7.48 (m, 4H), 7.38 (s, 2H), 7.34-7.30 (m, 1H), 7.09 (d, $J$ = 8.0 Hz, 1H), 6.57 (d, $J$ = 7.8 Hz, 1H), 5.12-5.06 (m, 1H), 4.96-4.81 (m, 2H), 4.47-4.42 (m, 1H), 4.31-4.27 (m, 1H), 4.03-4.01 (m, 2H), 3.91-3.87 (m, 2H), 3.72 (s, 4H), 3.58-3.49 (m, 1H), 3.38-3.32 (m, 2H), 3.19-3.17 (m, 3H), 3.12-3.08 (m, 3H), 3.00-2.88 (m, 5H), 2.71-2.68 (m, 2H), 2.44-2.35 (m, 3H), 2.08-2.06 (m, 2H). | | |

**Example 14.** Synthesis of Compounds **F3B-1**, **F3B-2**, and **F3B-3** of the present invention

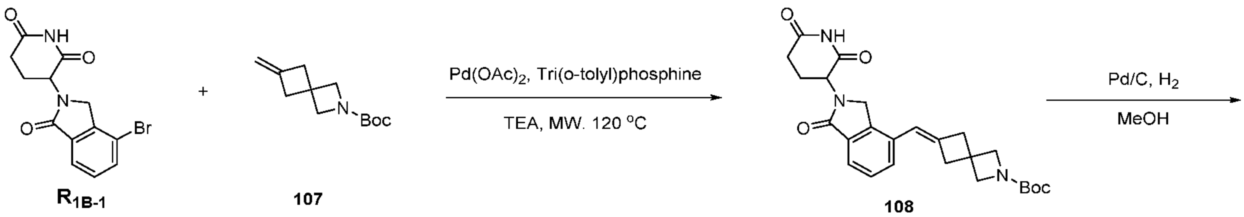


$R_{1B-1}$ 107 108

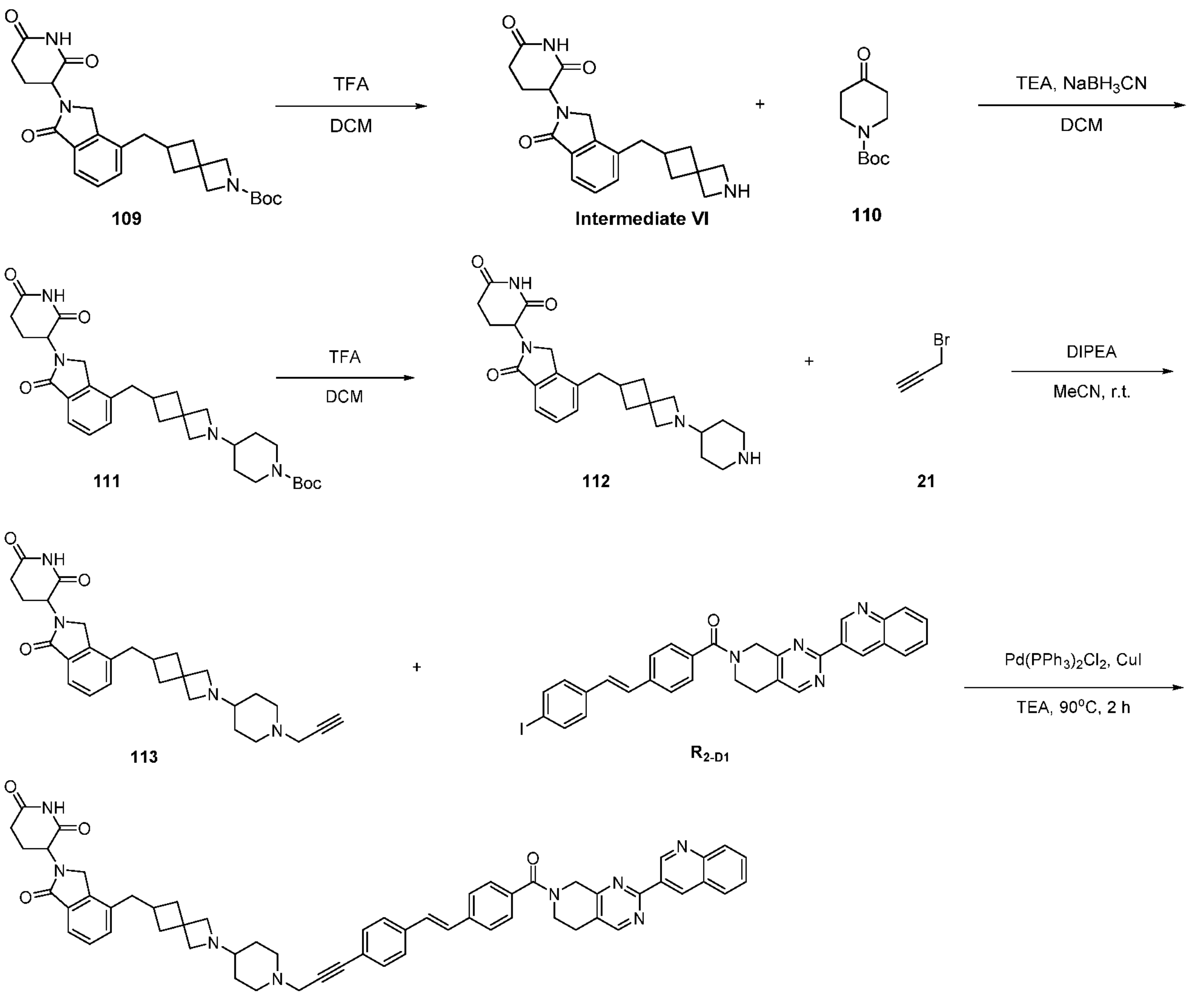


109 Intermediate VI 110

111 112 21

113 R2-D1

F3B - 1

### Step 1-3, Synthesis of Intermediate VI

Under nitrogen protection, Compound $\mathbf{R_{1B-1}}$ (1.00 mmol), Compound **107** (1.05 mmol), tri(o-tolyl)phosphine (1.05 mmol), triethylamine (2 mL), and palladium acetate (0.25 mol%) were added in sequence to a 50 mL reaction bottle. The reaction system was sealed, the reaction was carried out under stirring in a microwave reactor at 120°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1: 100 to 1:5), and concentrated under reduced pressure to remove the solvent to obtain Compound **108** ($C_{25}H_{30}N_3O_5$, $[M+H]^+$ 452.2; found 452.3, yield 48.2%).

Compound **108** (0.45 mmol), methanol (5 mL), and palladium-on-carbon (30.00 mg) were added in sequence to a 50 mL reaction bottle. The reaction system was replaced with hydrogen, maintained at an atmospheric pressure hydrogen atmosphere, and the reaction was carried out under stirring at room temperature for 3 hours (LC-MS monitoring). The palladium-on-carbon was removed by filtration, and washing was carried out with methanol (2 × 20 mL). The filtrate was collected and concentrated under reduced pressure to remove the solvent to obtain Compound **109** ($C_{25}H_{32}N_3O_5$, $[M+H]^+$ 454.2; found 454.1, crude product).

Compound **109** (0.30 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound Intermediate **VI** ($C_{20}H_{24}N_3O_3$, $[M+H]^+$ 354.2; found 354.1, crude product).

**Step 4. Synthesis of Compound 111**

Intermediate **VI** (0.30 mmol), Compound **110** (0.55 mmol), triethylamine (2 mL), sodium cyanoborohydride (0.75 mmol) and dichloromethane (5 mL) were added in sequence to a 50 mL reaction bottle, reacted under stirring at room temperature for 2 hours, then the reaction was quenched (LC-MS monitoring), the solvent was removed by evaporation to dryness, purified by medium-pressure preparative chromatography, and concentrated under reduced pressure to remove the solvent to obtain Compound **111** ($C_{30}H_{41}N_4O_5$, $[M+H]^+$ 537.3; found 537.5, yield 71.8%).

**Step 5. Synthesis of Compound 112**

Compound **111** (0.21 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound **112** ($C_{25}H_{33}N_4O_3$, $[M+H]^+$ 437.3; found 437.5, crude product).

**Step 6. Synthesis of Compound 113**

Compound **112** (0.21 mmol), Compound **21** (0.44 mmol), *N,N*-diisopropylethylamine (0.95 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **113** ($C_{28}H_{35}N_4O_3$, $[M+H]^+$ 457.3; found 457.5, yield 71.8%).

**Step 7. Synthesis of Compound F3B-1**

Under nitrogen protection, Compound $\mathbf{R_{2\text{-}D1}}$ (0.15 mmol), Compound **113** (0.15 mmol), copper iodide (0.35 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and concentrated under reduced pressure to remove the solvent to obtain Compound **F3B-1** (9.1 mg, 9.66 μmol,). LC-MS: $C_{59}H_{57}N_8O_4$, $[M/2+H]^+$ 471.6; found 471.7. $^1H$ NMR (400 MHz, DMSO-*d6*) δ 9.87-9.77 (m, 1H), 9.36-9.24 (m, 1H), 8.86 (s, 1H), 8.22-8.12 (m, 2H), 7.90 (s, 1H), 7.78-7.72 (m, 5H), 7.62-7.51 (m, 6H), 7.49-7.47 (m, 1H), 7.44- 7.42 (m, 3H), 5.10-5.09 (m, 1H), 4.97 (s, 1H), 4.80-4.77 (m, 1H), 4.48-4.44 (m, 1H), 4.36-4.28 (m, 3H), 4.18-4.15 (m, 2H), 4.09 (s, 2H), 3.51 (s, 2H), 3.44-3.41 (m, 1H), 3.10-2.95 (m, 4H), 2.92-2.89 (m, 1H), 2.75-2.73 (m, 2H), 2.68-2.63 (m, 1H), 2.44-2.41 (m, 3H), 2.20-2.16 (m, 4H), 2.06-1.96 (m, 4H), 1.62-1.55 (m, 2H).

According to the step 4 to step 7 of the method for Compound **F3B-1,** the raw materials in the following table were used to replace Intermediate **VI**, and the other raw materials and operating methods remained unchanged to obtain Compounds **F3B-2** and **F3B-3**.

The synthesis of Compounds **IV** and **V** was as follows:

**Synthesis of Intermediate IV**

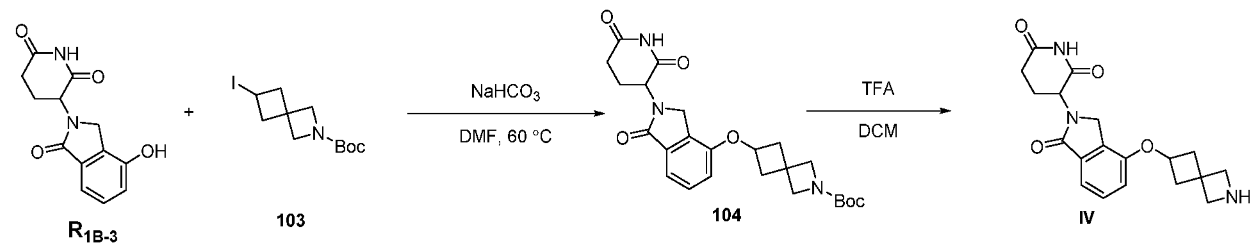


$R_{1B\text{-}3}$ 103 104 IV

Compound $\mathbf{R_{1B-3}}$ (1.00 mmol), Compound **103** (1.05 mmol), sodium bicarbonate (1.15 mmol) and *N,N*-dimethylformamide (5 mL) were added into a 50 mL reaction bottle, heated and reacted at 60°C. After 1 hour, the reaction was quenched by adding water (LC-MS monitoring). Extraction was carried out with saturated sodium chloride solution (25 mL) and ethyl acetate (3×25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **104** ($C_{24}H_{30}N_3O_6$, $[M+H]^+$ 456.2; found 456.4, yield 37.5%).

Compound **104** (0.35 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound Intermediate **IV** ($C_{19}H_{22}N_3O_4$, $[M+H]^+$ 356.2; found 356.3, crude product).

**Synthesis of intermediate V**

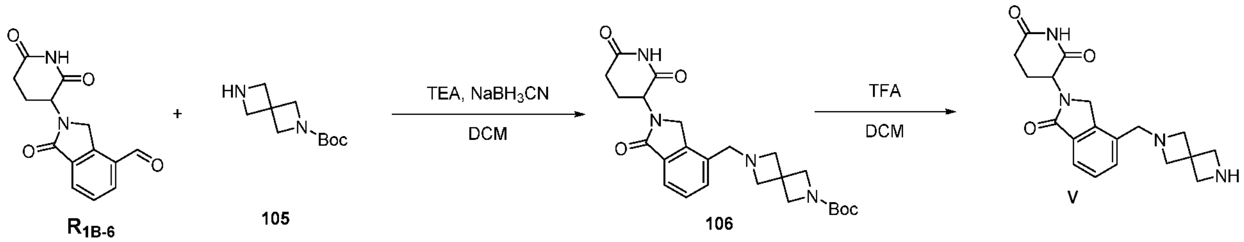


$\mathbf{R_{1B-6}}$ **105** **106** **V**

Compound $\mathbf{R_{1B-6}}$ (1.00 mmol), Compound **105** (1.05 mmol), triethylamine (2 mL), sodium cyanoborohydride (1.15 mmol) and dichloromethane (5 mL) were added in sequence to a 50 mL reaction bottle, reacted under stirring at room temperature for 2 hours, and then the reaction was quenched (LC-MS monitoring). The solvent was removed by evaporation to dryness, purified by medium-pressure preparative chromatography, and concentrated under reduced pressure to remove the solvent to obtain Compound **106** ($C_{24}H_{31}N_4O_5$, $[M+H]^+$ 455.2; found 455.5, yield 58.9%).

Compound **106** (0.55 mmol) was dissolved in dichloromethane (5 mL) in a 50 mL reaction bottle, then trifluoroacetic acid (1.5 mL) was added dropwise, and the reaction was carried out under stirring at room temperature for 0.5 hour. The solvent was removed by concentration under reduced pressure to obtain Compound Intermediate V ($C_{19}H_{23}N_4O_3$, $[M+H]^+$ 355.2; found 355.4, crude product).

| Compound No. | Structure and characterization data of compound | Raw material | Yield |
|---|---|---|---|
| F3B-2 | 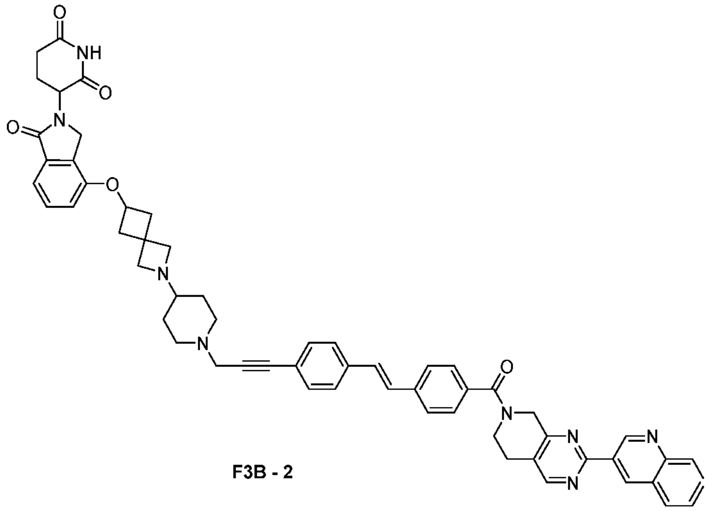<br>F3B - 2<br>LC-MS: $C_{58}H_{55}N_8O_5$, $[M/2+H]^+$ 472.2; found 472.5. $^1$H NMR (400 MHz, DMSO-$d_6$) δ 9.85-9.77 (m, 1H), 9.44-9.33 (m, 1H), 8.86 (s, 1H), 8.27-8.15 (m, 2H), 7.95 (s, 1H), 7.78-7.72 (m, 5H), 7.60-7.53 (m, 4H), 7.51-7.49 (m, 1H), 7.42-7.38 (m, 2H), 7.36-7.34 (m, 1H), 7.07 (d, *J* = 4.0 Hz, 1H), 5.07-5.06 (m, 1H), 4.86 (s, 1H), 4.84-4.82 (m, 2H), 4.41-4.36 (m, 3H), 4.26-4.21 (m, 4H), 3.73 (s, 2H), 3.50-3.45 (m, 2H), 3.16 (s, 5H), 3.14-3.08 (m, 2H), 3.01 (s, 2H), 2.93-2.89 (m, 3H), 2.71-2.66 (m, 2H), 2.48-2.47 (m, 2H), 2.44-2.41 (m, 1H), 2.37-2.36 (m, 2H), 2.23-2.03 (m, 1H), 1.66-1.64 (m, 2H), 1.28-1.25 (m, 1H). | 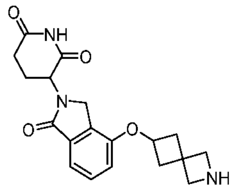<br>IV | 18.6 % |

(continued)

| Compound No. | Structure and characterization data of compound | Raw material | Yield |
|---|---|---|---|
| F3B-3 | 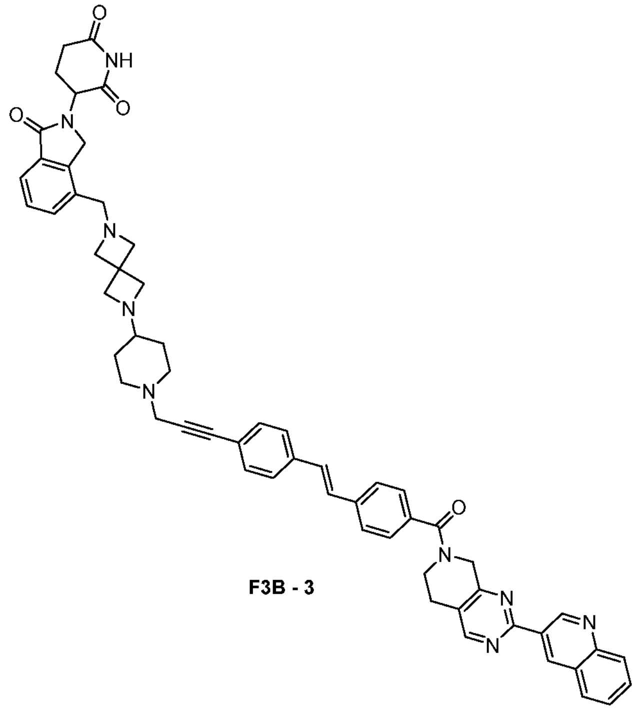 F3B - 3 | 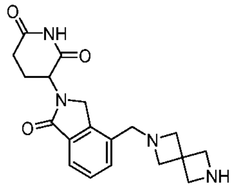 V | 19.5 % |
| | LC-MS: $C_{58}H_{56}N_9O_4$, $[M/2+H]^+$ 472.0; found 472.2. $^1H$ NMR (400 MHz, DMSO-$d_6$) δ 9.83-9.75 (m, 1H), 9.34-9.25 (m, 1H), 8.86 (s, 1H), 8.22-8.14 (m, 2H), 8.12 - 7.89 (s, 2H), 7.87-7.66 (m, 7H), 7.58-7.55 (m, 4H), 7.42 (s, 2H), 5.19-5.15 (m, 2H), 4.96-4.92 (m, 1H), 4.80-4.77 (m, 1H), 4.62-4.57 (2H), 4.48-4.36 (m, 10H), 4.03-3.91 (m, 2H), 3.44-3.38 (m, 2H), 3.19-3.00 (m, 5H), 2.65-2.55 (m, 3H), 2.22-2.07 (m, 4H), 1.62 (s, 2H). | | |

**Example 15.** Synthesis of Compounds **G1-1, G1-2, G1-3, G1-4,** and **G1-5** of the present invention

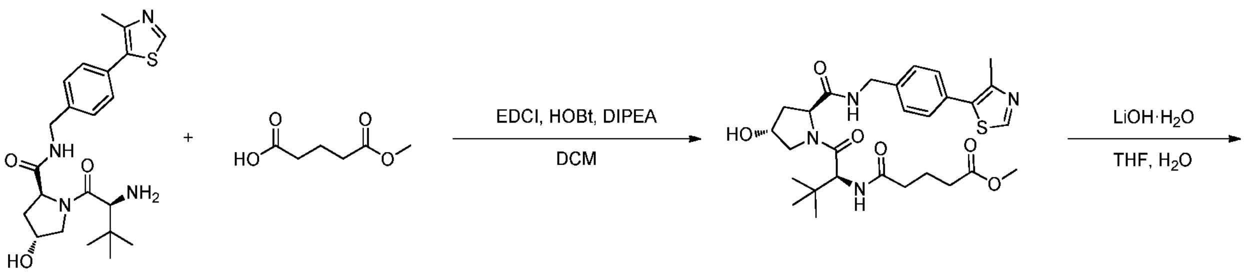


113 114c 115

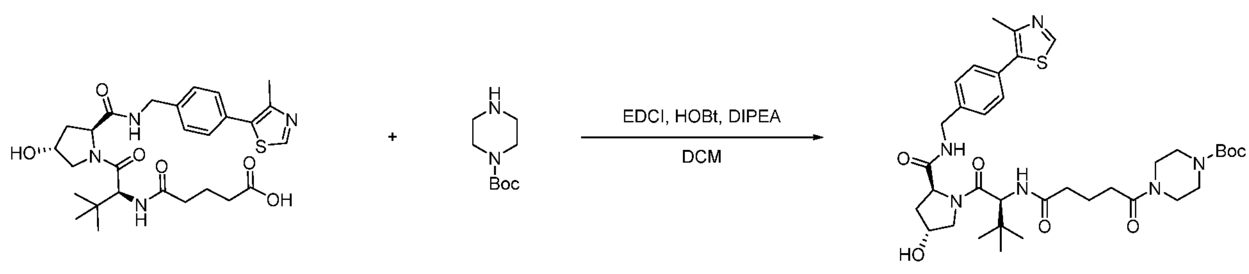


116 18 117

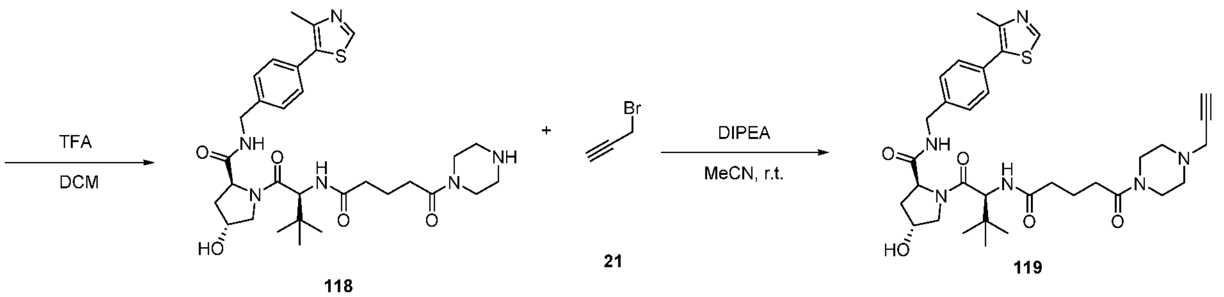


118 21 119

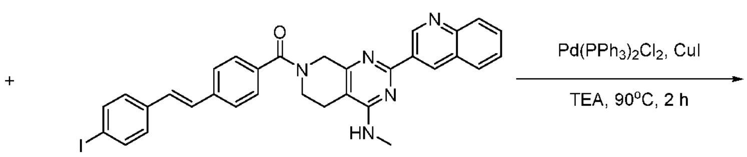


$R_{2\text{-}B1}$

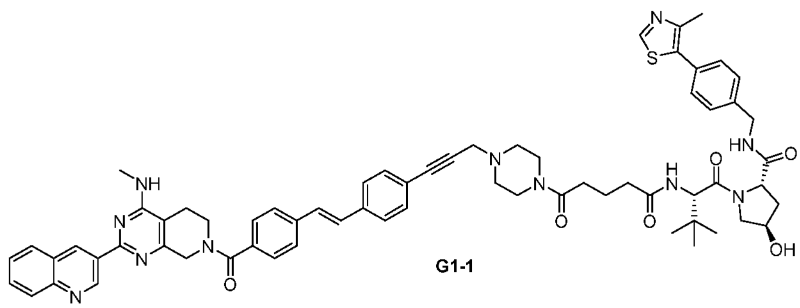

G1-1

**Step 1. Synthesis of Compound 115**

Compound **114a** (1.00 mmol), EDCI (1.05 mmol), HOBt (1.05 mmol) and dichloromethane (2 mL) were added in sequence to a 50 mL reaction bottle. After the temperature of the reaction system dropped to 0°C, Compound **113** (1.05 mmol) was added. The reaction was carried out under stirring and ice bath conditions for 1 hour, and then the reaction was quenched (LC-MS monitoring). Extraction was carried out with saturated sodium chloride solution (10 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **115** ($C_{28}H_{39}N_4O_6S$, $[M+H]^+$ 559.3; found 559.5, yield 78.2%).

**Step 2. Synthesis of Compound 116**

Compound **115** (0.75 mmol), lithium hydroxide monohydrate (8.10 mmol), tetrahydrofuran (4 mL) and water (2 mL) were added in sequence to a 50 mL reaction bottle, reacted under stirring at room temperature for 8 hours, and then the reaction was quenched (LC-MS monitoring). 1N hydrochloric acid solution was used to adjust the pH value of the system to 6-7, and the reaction solution was concentrated to obtain Compound **116** ($C_{27}H_{37}N_4O_6S$, $[M+H]^+$ 545.2; found 545.5, crude product).

**Step 3. Synthesis of Compound 117**

Compound **116** (0.70 mmol), EDCI (0.75 mmol), HOBt (0.75 mmol) and dichloroethane (2 mL) were added in sequence to a 50 mL reaction bottle. After the temperature of the reaction system dropped to 0°C, Compound 18 (0.95 mmol) was added, reacted under stirring and ice bath conditions for 1 hour, and then the reaction was quenched (LC-MS monitoring). Extraction was carried out with saturated sodium chloride solution (10 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **117** ($C_{36}H_{53}N_6O_7S$, $[M+H]^+$ 713.4; found 713.2, yield 64.3%).

**Step 4. Synthesis of Compound 118**

Compound **117** (0.45 mmol) was dissolved in dichloromethane (5 mL) in a 50 mL reaction bottle, then trifluoroacetic acid (1.5 mL) was added dropwise, and reacted under stirring at room temperature for 0.5 hour. The solvent was removed by concentration under reduced pressure to obtain Compound **118** ($C_{31}H_{45}N_6O_5S$, $[M+H]^+$ 613.3; found 613.1, crude product).

**Step 5. Synthesis of Compound 119**

Compound **118** (0.40 mmol), Compound **21** (0.80 mmol), *N*,*N*-diisopropylethylamine (1.02 mmol) and acetonitrile (5 mL) were added into a 50 mL reaction bottle, stirred and reacted at room temperature for 1 hour, then water was added to quench the reaction (LC-MS monitoring). Extraction was carried out with saturated sodium chloride solution (25 mL) and ethyl acetate (3× 25 mL), the organic phases were combined, the combined organic phase was dried over anhydrous sodium sulfate, evaporated to dryness to remove the solvent, purified by medium-pressure preparative chromatography, and concentrated under reduced pressure to remove the solvent to obtain Compound **119** ($C_{34}H_{47}N_6O_5S$, $[M+H]^+$ 651.3; found 651.5, yield 51.2%).

**Step 6. Synthesis of Compound G1**

Under nitrogen protection, Compound $\mathbf{R_{2\text{-}B1}}$ (0.20 mmol), Compound **119** (0.20 mmol), copper iodide (0.60 mmol), triethylamine (5 mL), and $Pd(PPh_3)_2Cl_2$ (0.25 mol%) were added in sequence to a 50 mL reaction bottle. The reaction system was sealed, the reaction was carried out under stirring at 90°C for 2 hours, and then the reaction was quenched (LC-MS monitoring). Extraction was carried out with saturated sodium chloride solution (20 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and concentrated under reduced pressure to remove the solvent to obtain Compound G1 (3.8 mg, 3.31 μmol, 97.2% purity). LC-MS: $C_{66}H_{72}N_{11}O_6S$, $[M/2+H]^+$ 574.2; found 574.2. $^1H$ NMR (600 MHz, Methanol-*d4*) δ 9.78 (s, 1H), 9.51 (s, 1H), 8.89 (d, *J* = 6.3 Hz, 1H), 8.27-8.20 (m, 2H), 8.17 (s, 1H), 7.86 (s, 1H), 7.72 (t, *J* = 6.9 Hz, 2H), 7.63 (dd, *J* = 8.0, 5.7 Hz, 2H), 7.55-7.48 (m, 4H), 7.45-7.33 (m, 4H), 7.32 (s, 2H), 4.86 (s, 1H), 4.61-4.51 (m, 4H), 4.48 (s, 1H), 4.38-4.33 (m, 2H), 4.12 (s, 1H), 3.93-3.79 (m, 7H), 3.49-3.42 (m, 5H), 3.25 (s, 3H), 2.67 (s, 2H), 2.54-2.46 (m, 5H), 2.36 (t, *J* = 7.2 Hz, 2H), 2.22-2.13 (m, 1H), 2.10-2.03 (m, 1H), 1.93-1.90 (m, 2H), 1.03 -1.01 (m, 9H).

Compounds **G1-2**, **G1-3**, **G1-4**, and **G1-5** could be obtained by following the step 1 to step 6 of the method for Compound **G1-1**, replacing Compound **114a** with the raw materials in the following table, and keeping the other raw materials and operating methods unchanged.

| Compound No. | Structure and characterization data of compound | Raw material | Yield |
|---|---|---|---|
| G1-2 | 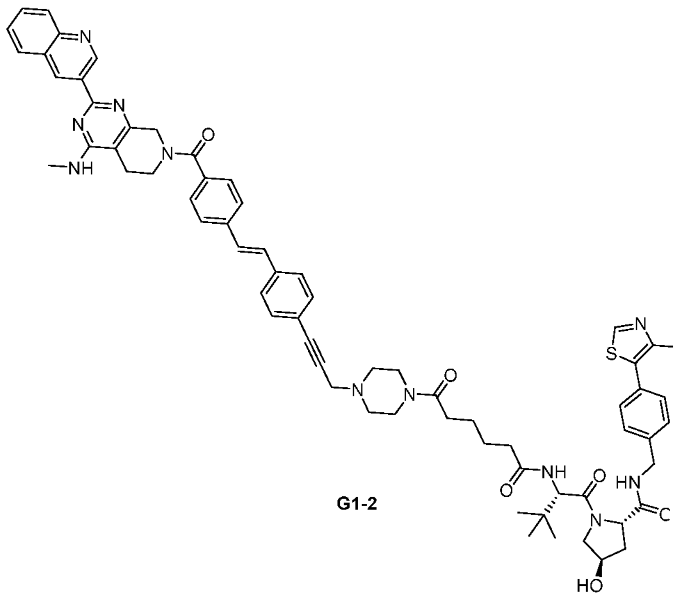<br>G1-2<br>LC-MS: $C_{67}H_{74}N_{11}O_6S$, $[M/2+H]^+$ 581.2; found 581.4. $^1$H NMR (600 MHz, Methanol-$d_4$) δ 9.77 (s, 1H), 9.54 (s, 1H), 8.96 (s, 1H), 8.29 (s, 1H), 8.28-8.21 (m, 1H), 8.07 (s, 1H), 7.88 (s, 1H), 7.72 (d, $J$ = 7.9 Hz, 2H), 7.61 (d, $J$ = 7.9 Hz, 2H), 7.55-7.45 (m, 4H), 7.44-7.30 (m, 4H), 7.28 (s, 2H), 4.89 (s, 1H), 4.63 (s, 1H), 4.57-4.49 (m, 3H), 4.38-4.35 (m, 3H), 4.33 (s, 1H), 3.91-3.81 (m, 4H), 3.80-3.79 (m, 2H), 3.51-3.31 (m, 5H), 3.25 (s, 3H), 2.66 (s, 2H), 2.49 (s, 5H), 2.48-2.47 (m, 1H), 2.34-2.31 (m, 1H), 2.21-2.20 (m, 1H), 2.10-2.08 (m, 1H), 1.67-1.62 (m, 4H), 1.06-1.01 (m, 9H). | 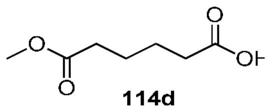<br>114d | 30.6 % |
| G1-3 | 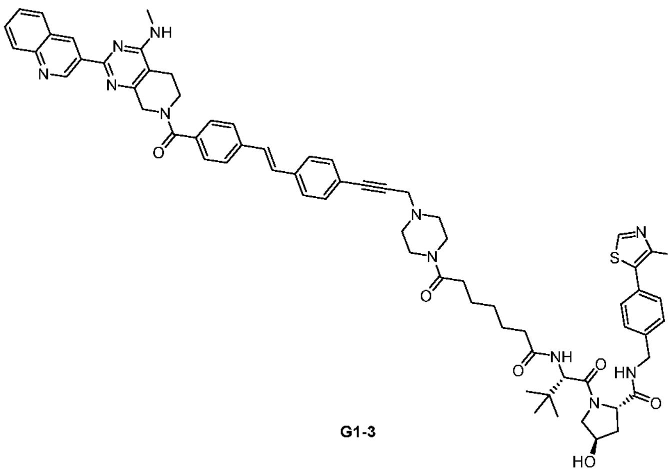<br>G1-3<br>LC-MS: $C_{68}H_{76}N_{11}O_6S$, $[M/2+H]^+$ 588.2; found 588.2. $^1$H NMR (600 MHz, Methanol-$d_4$) δ 9.87 - 9.76 (m, 1H), 9.42-9.35 (m, 1H), 8.87 (s, 1H), 8.31-8.15 (m, 2H), 8.00 (m, 1H), 7.97-7.80 (m, 1H), 7.71 (d, $J$ = 8.0 Hz, 2H), 7.61 (d, $J$ = 8.0 Hz, 2H), 7.54-7.49 (m, 4H), 7.49-7.30 (m, 4H), 7.28 (s, 2H), 4.86 (s, 1H), 4.62-4.49 (m, 4H), 4.37-4.34 (m, 1H), 4.29 (s, 2H), 4.11 (s, 1H), 3.90-3.78 (m, 7H), 3.40-3.31 (m, 5H), 3.21 (s, 3H), 2.63 (s, 2H), 2.46-2.44 (m, 5H), 2.31-2.25 (m, 2H), 2.23-2.20 (m, 1H), 2.10-2.03 (m, 1H), 1.66-1.61 (m, 4H), 1.41-1.37 (m, 2H), 1.03 (s, 9H). | 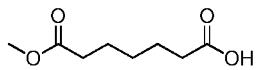<br>114e | 29.8 % |

(continued)

| Compound No. | Structure and characterization data of compound | Raw material | Yield |
|---|---|---|---|
| G1-4 | 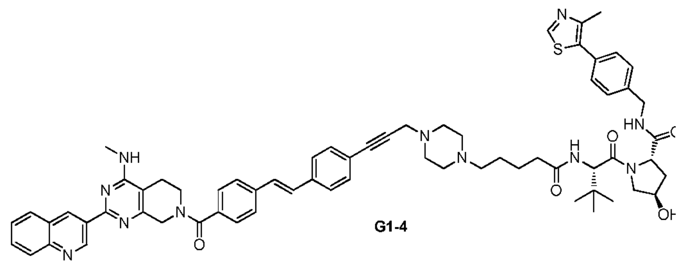<br>LC-MS: $C_{66}H_{74}N_{11}O_5S$, $[M/2+H]^+$ 567.3; found 567.1. $^1H$ NMR (600 MHz, DMSO-$d_6$) δ 9.83-9.74 (m, 1H), 9.28-9.13 (m, 1H), 8.98 (s, 1H), 8.55 (d, $J$ = 6.0 Hz, 1H), 8.20-8.07 (m, 2H), 7.94 (d, $J$ = 9.6 Hz, 1H), 7.83 (s, 1H), 7.72 (d, $J$ = 4.8 Hz, 3H), 7.66 (d, $J$ = 4.8 Hz, 3H), 7.53 (d, $J$ = 4.8 Hz, 2H), 7.47 (d, $J$ = 4.8 Hz, 2H), 7.41 (d, $J$ = 4.8 Hz, 2H), 7.39-7.37 (m, 6H), 7.26 (s, 1H), 4.73 (s, 1H), 4.54 (d, $J$ = 9.3 Hz, 2H), 4.45-4.41 (m, 2H), 4.35 (s, 1H), 4.24-4.21 (m, 2H), 3.97 (s, 2H), 3.68-3.62 (m, 5H), 3.57 (s, 3H), 3.01 (s, 4H), 2.63-2.57 (m, 5H), 2.44 (s, 3H), 2.34-2.29 (m, 1H), 2.23-2.18 (m, 2H), 2.17-2.07 (m, 1H), 1.9-1.89 (m, 1H), 1.62-1.56 (m, 2H), 1.53 (t, $J$ = 4.8 Hz, 2H), 0.95 (s, 9H). | 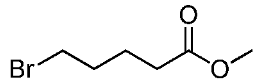<br>120a | 39.1 % |
| G1-5 | 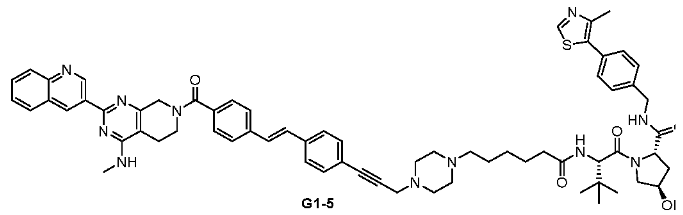<br>LC-MS: $C_{67}H_{76}N_{11}O_5S$, $[M+H]^+$ 1146.6; found 1146.6. $^1H$ NMR (600 MHz, DMSO-$d_6$) δ 9.82-9.73 (m, 1H), 9.20-9.10 (m, 1H), 8.98 (s, 1H), 8.55 (t, $J$ = 6.0 Hz, 1H), 8.17-8.06 (m, 2H), 7.84 (d, $J$ = 9.6 Hz, 1H), 7.73 (s, 1H), 7.72 (d, $J$ = 4.8 Hz, 2H), 7.63 (d, $J$ = 4.8 Hz, 3H), 7.52 (d, $J$ = 4.8 Hz, 3H), 7.43 (d, $J$ = 4.8 Hz, 2H), 7.36 (d, $J$ = 4.8 Hz, 2H), 7.34-7.32 (m, 4H), 7.18-7.16 (m, 1H), 5.14 (s, 1H), 4.89 - 4.75 (m, 1H), 4.52 (d, $J$ = 9.3 Hz, 1H), 4.44-4.41 (m, 2H), 4.35 (s, 1H), 4.25 - 4.18 (m, 2H), 3.97 (s, 1H), 3.68-3.62 (m, 5H), 3.57 (s, 3H), 3.08 (s, 3H), 2.61 (s, 2H), 2.44 (d, $J$ = 4.8 Hz, 3H), 2.39 (s, 1H), 2.29-2.25 (m, 2H), 2.11 (t, $J$ = 6.0 Hz, 1H), 2.07 (t, $J$= 9.9 Hz, 1H), 1.52-1.42 (m, 5H), 1.40-1.23 (s, 3H), 0.93 (s, 9H). | 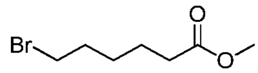<br>120b | 37.2 % |

According to the step 1 to step 6 of the method for Compound **G1-1,** the raw material **1** in the following table was used to replace Compound **113**, the raw material **2** in the following table was used to replace Compound **114a**, and the other raw materials and operating methods remained unchanged to obtain Compounds **G2-1**, **G2-2**, **G2-3**, **G2-4**, **G2-5**, **G2-6**, **G2-7**.

| Compound No. | Structure and characterization data of compound | Raw material 1 | Raw material 2 | Yield |
|---|---|---|---|---|
| G2-1 | 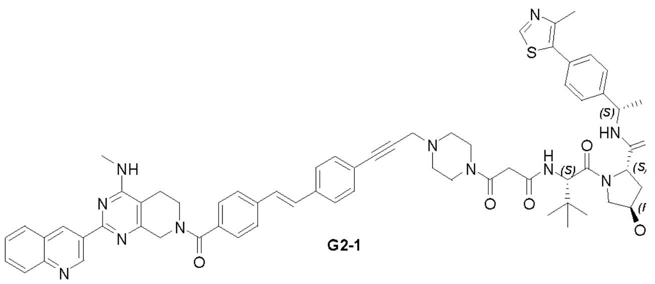 G2-1<br>LC-MS: $C_{65}H_{70}N_{11}O_6S$, $[M/2+H]^+$ 567.3; found 567.2. $^1H$ NMR (600 MHz, DMSO-$d_6$) δ 9.82-9.73 (m, 1H), 9.20-9.10 (m, 1H), 8.97 (d, *J* = 6.6 Hz, 1H), 8.39 (d, *J* = 7.8 Hz, 1H), 8.17 (d, *J* = 9.4 Hz, 1H), 8.06 (s, 2H), 7.80 (s, 1H), 7.73 (d, *J* = 8.0 Hz, 2H), 7.64 (d, *J*= 8.1 Hz, 3H), 7.52 (d, *J*= 7.9 Hz, 2H), 7.46-7.42 (m, 4H), 7.38-7.20 (m, 4H), 7.18-1.76 (m, 2H), 5.11 (d, *J* = 3.5 Hz, 1H), 4.91 (t, *J* = 7.4 Hz, 1H), 4.71 (s, 1H), 4.52 (d, *J* = 9.3 Hz, 1H), 4.43 (t, *J* = 8.1 Hz, 1H), 4.28 (s, 1H), 3.97 (s, 1H), 3.69 (s, 1H), 3.50-3.50 (m, 4H), 3.48-3.46 (m, 4H), 3.08 (d, *J* = 4.4 Hz, 3H), 2.61 (s, 4H), 2.45 (s, 4H), 2.23 (s, 3H), 2.07-2.02 (m, 1H), 1.81-1.78 (m, 1H), 1.37 (d, *J*= 7.0 Hz, 3H), 0.94 (s, 9H). | 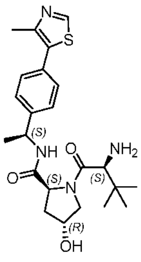<br>121 | 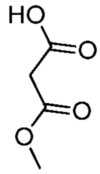<br>**114a** | 42.1 % |
| G2-2 | 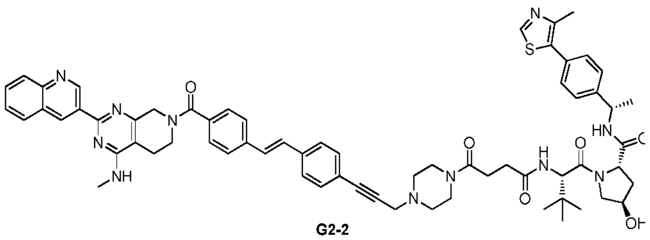 G2-2<br>$C_{66}H_{72}N_{11}O_6S$ LC-MS 1146.5; $[M/2+H]^+$ found 574.2. $^1H$ NMR (600 MHz, DMSO-$d_6$) δ 9.82-9.73 (m, 1H), 9.25-9.14 (m, 1H), 8.98 (s, 1H), 8.37 (d, *J* = 7.8 Hz, 1H), 8.15-8.08 (m, 2H), 7.88 (d, *J* = 9.0 Hz, 1H), 7.87 (s, 1H), 7.78 (d, *J* = 8.0 Hz, 2H), 7.74 (d, *J* = 8.1 Hz, 2H), 7.71 (d, *J* = 7.9 Hz, 1H), 7.69-7.61 (m, 4H), 7.56 (d, *J* = 8.4 Hz, 1H), 7.54-7.44 (m, 4H), 7.42-7.34 (m, 2H), 7.29 (s, 1H), 5.11 (d, *J* = 3.5 Hz, 1H), 4.93-4.90 (m, 1H), 4.50 (d, *J* = 2.4 Hz, 1H), 4.43 (t, *J* = 8.1 Hz, 1H), 4.40-4.35 (m, 1H), 4.28 (s, 2H), 3.97 (s, 1H), 3.60(s, 1H), 3.57-3.55 (m, 4H), 3.16-3.08 (m, 6H), 2.79 (s, 1H), 2.61-2.55 (m, 5H), 2.53 (s, 3H), 2.45-2.35 (m, 2H), 2.01-1.99 (m, 1H), 1.79-1.77 (m, 1H), 1.37 (d, *J* = 7.0 Hz, 3H), 1.27-1.23 (m, 1H), 0.93 (s, 9H). | 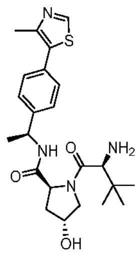<br>121 | 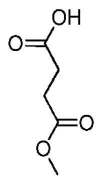<br>114b | 40.9 % |

(continued)

| Compound No. | Structure and characterization data of compound | Raw material 1 | Raw material 2 | Yield |
|---|---|---|---|---|
| G2-3 | 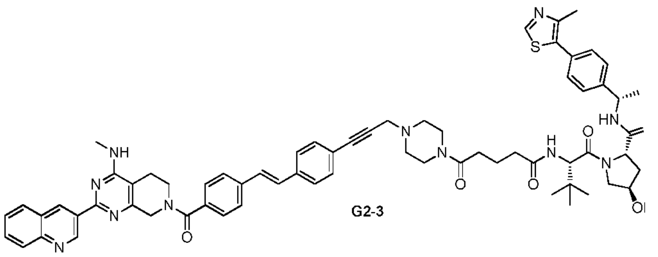 G2-3<br>LC-MS: $C_{67}H_{74}N_{11}O_6S$, $[M/2+H]^+$ 581.3; found 581.2. 1H NMR (600 MHz, DMSO-d6) δ 9.83-9.81 (m, 1H), 9.28-9.17 (m, 1H), 8.98 (s, 1H), 8.36 (d, J = 7.7 Hz, 1H), 8.22-8.10 (m, 2H), 7.84 (d, J = 9.0 Hz, 2H), 7.74 (d, J = 8.4 Hz, 2H), 7.70 (d, J = 2.4 Hz, 2H), 7.68-7.67 (m, 3H), 7.57-7.53 (m, 3H), 7.44-7.40 (m, 4H), 7.38-7.33 (m, 3H), 4.93-4.90 (m, 1H), 4.74-4.69 (m, 1H), 4.57 (s, 1H), 4.51 (d, J = 9.3 Hz, 1H), 4.42 (t, J = 8.1 Hz, 1H), 4.40-4.35 (m, 4H), 3.97 (s, 3H), 3.69 (s, 3H), 3.09 (d, J = 3.0 Hz, 4H), 2.62-2.58 (m, 5H), 2.45 (s, 3H), 2.37 (t, J = 7.5 Hz, 3H), 2.26-2.20 (m, 1H), 2.13 (t, J = 6.0 Hz, 1H), 2.02 (t, J = 9.9 Hz, 1H), 1.84-1.73 (m, 1H), 1.75-1.71 (m, 2H), 1.37 (d, J = 7.0 Hz, 3H), 0.93 (s, 9H). | 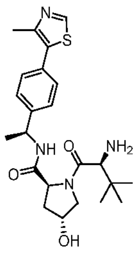 121 | 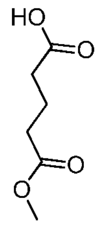 114c | 38.5 % |
| G2-4 | 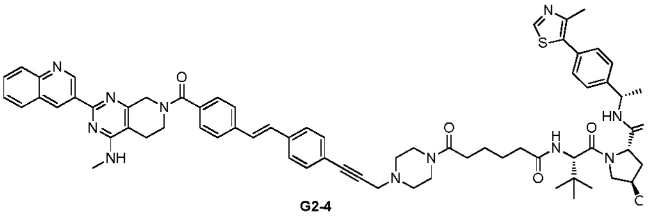 G2-4<br>LC-MS: $C_{68}H_{76}N_{11}O_6S$, $[M/2+H]^+$ 588.3; found 588.5. $^1H$ NMR (600 MHz, DMSO-$d_6$) δ 9.83-9.73 (m, 1H), 9.27-9.16 (m, 1H), 8.98 (s, 1H), 8.36 (d, *J* = 7.7 Hz, 1H), 8.20 - 8.09 (m, 2H), 7.84 (s, 1H), 7.80 (d, *J* = 3.6 Hz, 1H), 7.79-7.69 (m, 6H), 7.57-7.53 (m, 4H), 7.46-7.42 (m, 4H), 7.36-7.35 (m, 2H), 7.34 (s, 1H), 4.98-4.90 (m, 1H), 4.89 - 4.75 (m, 1H), 4.57 (s, 1H), 4.52 (d, *J* = 9.3 Hz, 1H), 4.42 (t, *J* = 8.1 Hz, 1H), 4.40 - 4.35 (m, 3H), 3.98 (s, 2H), 3.09 (s, 3H), 2.61-2.57 (m, 9H), 2.45 (s, 3H), 2.36 (t, *J* = 7.5 Hz, 3H), 2.26-2.20 (m, 1H), 2.13 (t, *J* = 6.0 Hz, 1H), 2.02 (t, *J* = 9.9 Hz, 1H), 1.84-1.73 (m, 1H), 1.75-1.70 (m, 5H), 1.37 (d, *J* = 7.0 Hz, 3H), 0.94 (s, 9H). | 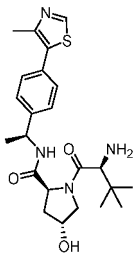 121 | 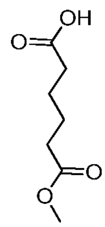 114d | 35.7 % |

(continued)

| Compound No. | Structure and characterization data of compound | Raw material 1 | Raw material 2 | Yield |
|---|---|---|---|---|
| G2-5 | 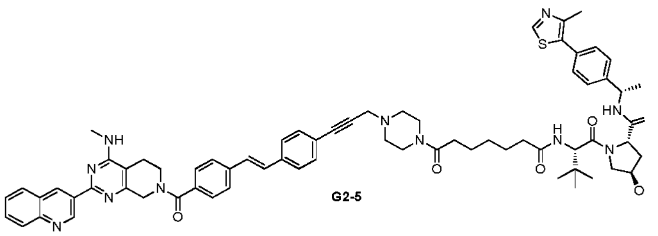 G2-5<br>LC-MS: $C_{69}H_{78}N_{11}O_6S$, $[M+H]^+$ 1188.6; found 1188.2. $^1H$ NMR (600 MHz, DMSO-$d_6$) δ 9.86-9.73 (m, 1H), 9.20-9.10 (m, 1H), 8.97 (s, 1H), 8.36 (d, *J* = 7.7 Hz, 1H), 8.16 - 8.06 (m, 2H), 7.78 (d, *J* = 6.6 Hz, 2H), 7.72 (d, *J* = 3.6 Hz, 2H), 7.64 (d, *J* = 9.8 Hz, 3H), 7.52 (d, *J* = 9.8 Hz, 2H), 7.46-7.42 (m, 4H), 7.38-7.33 (m, 4H), 7.19-7.14 (m, 2H), 5.10 (s, 1H), 4.98-4.90 (m, 1H), 4.89 - 4.75 (m, 1H), 4.62 - 4.50 (m, 2H), 4.42 (t, *J* = 8.1 Hz, 1H), 4.40 (s, 1H), 3.98 (s, 1H), 3.85-3.62 (m, 1H), 3.62 - 3.55 (m, 4H), 3.48 (s, 4H), 3.09 (s, 3H), 2.56-2.53 (m, 2H), 2.47-2.45 (m, 5H), 2.36 (t, *J* = 7.5 Hz, 3H), 2.26-2.20 (m, 1H), 2.13 (t, *J* =6.0 Hz, 1H), 2.02 (t, *J* = 9.9 Hz, 1H), 1.81-1.77 (m, 1H), 1.52-1.47 (m, 4H), 1.37 (d, *J* = 7.0 Hz, 3H), 1.28-1.24 (m, 3H), 0.94 (s, 9H). | 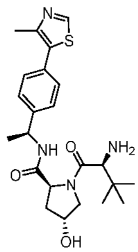 121 | 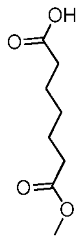 114e | 19.2 % |
| G2-6 | 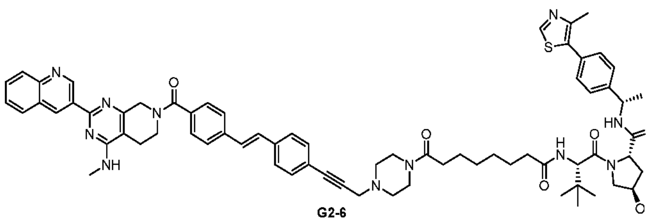 G2-6<br>LC-MS: $C_{70}H_{80}N_{11}O_6S$, $[M/2+H]^+$ 602.3; found 602.3. $^1H$ NMR (600 MHz, DMSO-$d_6$) δ 9.82-9.73 (m, 1H), 9.21-9.10 (m, 1H), 8.97 (s, 1H), 8.36 (d, *J* = 7.7 Hz, 1H), 8.17 - 8.06 (m, 2H), 7.78 (d, *J* = 6.6 Hz, 2H), 7.72 (d, *J* = 3.6 Hz, 2H), 7.64 (d, *J* = 9.8 Hz, 3H), 7.52 (d, *J* = 9.8 Hz, 2H), 7.45-7.42 (m, 4H), 7.38-7.32 (m, 5H), 7.19-7.15 (m, 1H), 5.10 (s, 1H), 4.98-4.90 (m, 1H), 4.89 - 4.75 (m, 1H), 4.57 (s, 1H), 4.52 (d, *J* = 9.3 Hz, 1H), 4.42 (t, *J* = 8.1 Hz, 1H), 4.27 (s, 1H), 3.97 (s, 1H), 3.85-3.68 (m, 1H), 3.62 - 3.58 (m, 4H), 3.48 (s, 4H), 3.07 (s, 3H), 2.61 (s, 1H), 2.56 - 2.45 (m, 5H), 2.36 (t, *J* = 7.5 Hz, 2H), 2.26-2.20 (m, 1H), 2.11 (t, *J* = 6.0 Hz, 1H), 2.07 (t, *J* = 9.9 Hz, 1H), 1.81-1.77 (m, 1H), 1.52-1.47 (m, 4H), 1.37 (d, *J* = 7.0 Hz, 3H), 1.37-1.24 (m, 6H), 0.93 (s, 9H). | 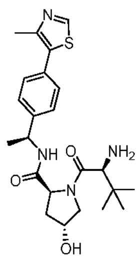 121 | 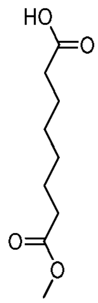 114f | 33.7 % |

(continued)

| Compound No. | Structure and characterization data of compound | Raw material 1 | Raw material 2 | Yield |
|---|---|---|---|---|
| G2-7 | 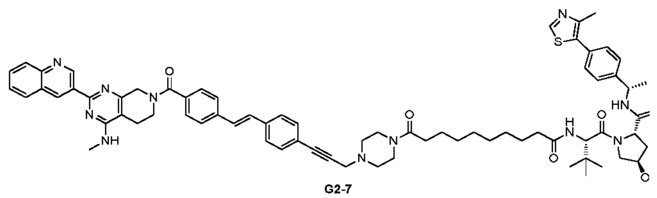 G2-7<br>LC-MS: $C_{72}H_{84}N_{11}O_6S$, $[M/2+H]^+$ 616.3; found 616.3. $^1H$ NMR (600 MHz, DMSO-$d_6$) $\delta$ 9.83-9.73 (m, 1H), 9.20-9.15 (m, 1H), 8.97 (s, 1H), 8.36 (d, *J* = 7.7 Hz, 1H), 8.17 - 8.07 (m, 2H), 7.78 (d, *J* = 6.6 Hz, 2H), 7.72 (d, *J* = 3.6 Hz, 2H), 7.64 (d, *J* = 9.8 Hz, 3H), 7.52 (d, *J* = 9.8 Hz, 2H), 7.45-7.42 (m, 4H), 7.38-7.32 (m, 4H), 7.19-7.15 (m, 2H), 5.10 (s, 1H), 4.98-4.90 (m, 1H),4.89 - 4.75 (m, 1H), 4.57 (s, 1H), 4.52 (d, *J* = 9.3 Hz, 1H), 4.42 (t, *J* = 8.1 Hz, 1H), 4.40 (s, 1H), 3.97 (s, 1H), 3.85-3.68 (m, 1H), 3.62 - 3.58 (m, 4H), 3.48 (s, 4H), 3.08 (s, 3H), 2.57-2.53 (m, 3H), 2.47-2.45 (m, 5H), 2.36 (t, *J* = 7.5 Hz, 3H), 2.11 (t, *J* = 6.0 Hz, 1H), 2.07 (t, *J* = 9.9 Hz, 1H), 1.81-1.78 (m, 1H), 1.52-1.47 (m, 4H), 1.37 (d, *J* = 7.0 Hz, 3H), 1.28-1.24 (m, 8H), 0.93 (s, 9H). | 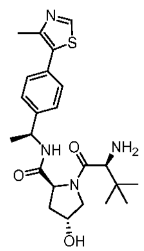<br>121 | 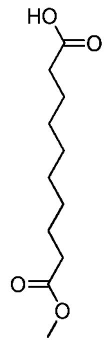<br>114g | 19.8 % |

**Example 16.** Synthesis of Compound **G3-1** of the present invention

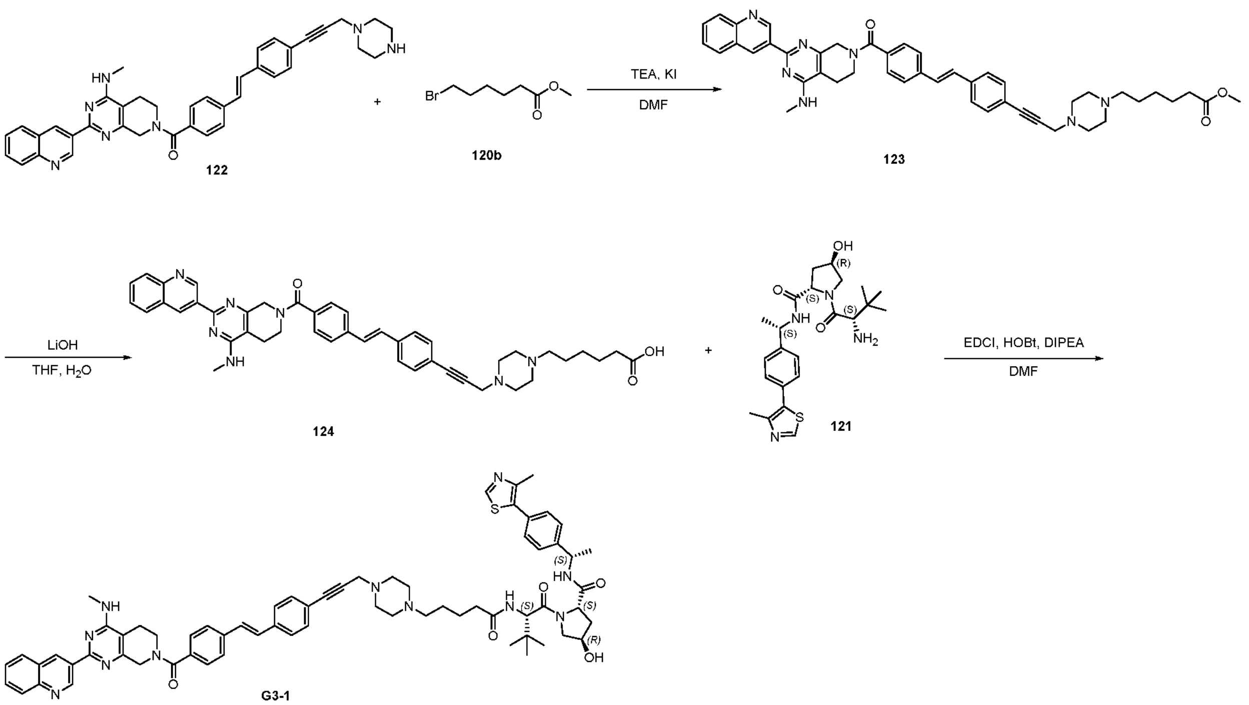

122 120b 123 124 121 G3-1

**Step 1. Synthesis of Compound 123**

Compound **122** (1.00 mmol), Compound **120b** (2.25 mmol), potassium iodide (1.05 mmol), triethylamine (2 mL) and *N,N*-dimethylformamide (5 mL) were added into a 50 mL reaction bottle, and reacted at room temperature.

After the reaction was completed, water was added to quench the reaction (LC-MS monitoring). Extraction was carried out with saturated sodium chloride solution (25 mL) and ethyl acetate (3× 25 mL), the organic phases were combined, the organic phase was dried over anhydrous sodium sulfate, evaporated to dryness to remove the solvent, purified by medium-pressure preparative chromatography, and concentrated under reduced pressure to remove the solvent to obtain Compound 123 ($C_{42}H_{42}N_7O_3$, $[M+H]^+$ 692.3; found 692.2, yield 59.8%).

**Step 2. Synthesis of Compound 124**

Compound **123** (0.75 mmol), lithium hydroxide monohydrate (8.10 mmol), tetrahydrofuran (4 mL) and water (2 mL) were added in sequence to a 50 mL reaction bottle, stirred and reacted at room temperature for 8 hours, and then the reaction was quenched (LC-MS monitoring). 1N hydrochloric acid solution was used to adjust the pH value of the system to 6-7, and the reaction solution was concentrated to obtain Compound **124** ($C_{41}H_{40}N_7O_3$, $[M+H]^+$ 678.3; found 678.5).

**Step 3. Synthesis of Compound G3-1**

Compound **124** (crude product), EDCI (0.75 mmol), HOBt (0.75 mmol) and dichloromethane (2 mL) were added in sequence to a 50 mL reaction bottle. After the temperature of the reaction system dropped to 0°C, Compound **121** (0.95 mmol) was added, reacted under stirring and ice bath conditions for 1 hour, and then the reaction was quenched (LC-MS monitoring). Extraction was carried out with saturated sodium chloride solution (10 mL) and ethyl acetate (3× 25 mL), the organic phases were combined, the combined organic phase was dried over anhydrous sodium sulfate, evaporated to dryness to remove the solvent, and separated by column chromatography (the volume ratio of the eluents used was petroleum ether/ethyl acetate = 1:100 to 1:5), and concentrated under reduced pressure to remove the solvent to obtain Compound **G3-1** (6.5 mg, 5.67 μmol, 90.9% purity). LC-MS: $C_{67}H_{76}N_{11}O_5S$, $[M/2+H]^+$ 574.2; found 574.2. $^1H$ NMR (600 MHz, DMSO-*d6*) δ 9.82-9.74 (m, 1H), 9.21-9.11 (m, 1H), 8.98 (s, 1H), 8.36 (d, *J* = 6.0 Hz, 1H), 8.17-8.07 (m, 2H), 7.95 (s, 1H), 7.78 (d, *J* = 4.8 Hz, 2H), 7.72 (d, *J* = 4.8 Hz, 2H), 7.66 (d, *J* = 4.8 Hz, 3H), 7.52 (d, *J* = 4.8 Hz, 2H), 7.46-7.42 (m, 4H), 7.38-7.36 (m, 4H), 7.19 -7.15 (m, 1H), 5.10 (s, 1H), 4.91 (t, *J* = 5.4 Hz, 1H), 4.73 (s, 1H), 4.50 (d, *J* = 9.3 Hz, 2H), 4.42 (t, *J*= 4.8 Hz, 1H), 4.31-4.28 (m, 1H), 3.97 (s, 1H), 3.69 (s, 1H), 3.62 (t, *J* = 6.6 Hz, 2H), 3.58 (s, 2H), 3.07 (s, 3H), 2.89 (s, 4H), 2.73 (s, 4H), 2.57 (s, 3H), 2.45 (s, 3H), 2.25 (s, 3H), 2.18-2.10 (m, 1H), 2.02-1.99 (m, 1H), 1.80-1.77 (m, 1H), 1.49-1.47 (m, 2H), 1.38-1.36 (m, 4H), 0.93 (s, 9H).

**Synthesis of Intermediate 129**

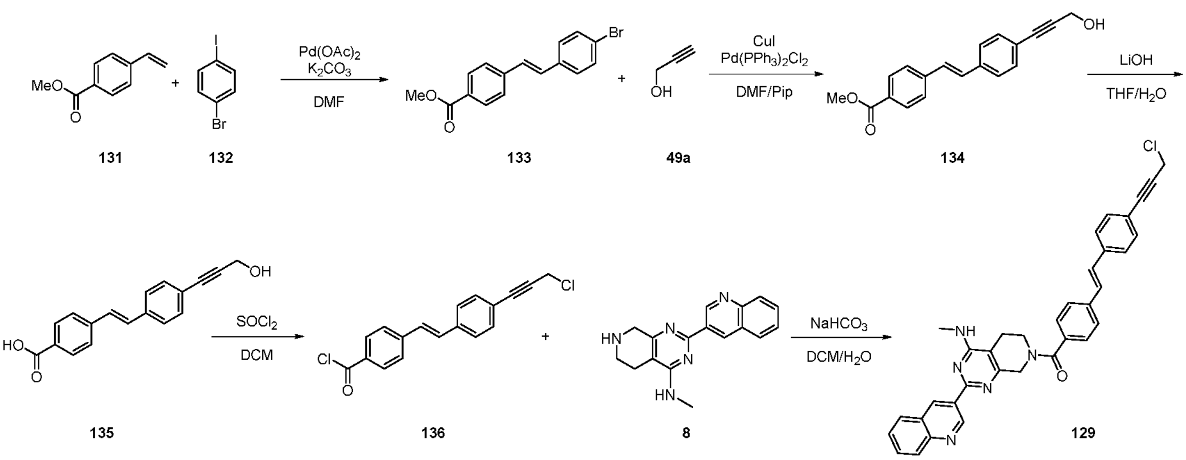


131 132 133 49a 134

135 136 8 129

**Step 1. Synthesis of Compound 133**

Compound **131** (1.00 g, 6.16 mmol, 1.0 eq) and Compound **132** (2.09 g, 7.41 mmol, 1.2 eq) were added into a 50 mL reaction bottle, then added with *N,N*-dimethylformamide (26 mL), potassium carbonate (1.71 g, 12.34 mmol, 2.0 eq), and palladium acetate (0.05 g, 0.31 mmol, 0.05 eq) under the protection of nitrogen gas, heated and reacted at 80°C overnight, and purified by MPLC to obtain Compound **133** (0.78 g, 2.46 mmol, yield 40.00%).

**Step 2. Synthesis of Compound 134**

Compound **133** (0.78 g, 2.46 mmol, 1.0 eq) and Compound **49a** (0.43 g, 7.37 mmol, 3.0 eq) were added into a 50 mL reaction bottle, then added with *N,N*-dimethylformamide (10 mL), copper iodide (46.80 mg, 0.25 mmol, 0.1 eq.), and $Pd(PPh_3)_2Cl_2$ (0.086 g, 0.12 mmol, 0.05 eq.) under the protection of nitrogen gas, heated and reacted at 80°C overnight, and purified by MPLC to obtain Compound 134 (0.57 g, 1.95 mmol, yield 80.00%).

**Step 3. Synthesis of Compound 135**

Compound **134** (0.57 g, 1.95 mmol, 1 eq.), LiOH (0.17 g, 3.9 mmol, 2.0 eq.), and THF/$H_2O$ (10 mL, 2:1) were added in sequence to a 50 mL reaction bottle, and reacted under stirring at room temperature for 4 hours, and then the reaction was quenched (LC-MS monitoring), the pH value of the system was adjusted to 6-7 with 1M hydrochloric acid solution, and the reaction solution was concentrated to obtain Compound 135 (0.57 g, crude product).

**Step 4. Synthesis of Compound 136**

Compound **135** (0.57 g, 2.03 mmol, 1 eq.), DCM (10 mL), and $SOCl_2$ (2 mL) were added in sequence to a 50 mL reaction bottle, heated and reacted at 50°C for 2 hours, and the reaction solution was concentrated to obtain Compound **136** (0.66 g, crude product).

**Step 5. Synthesis of Intermediate 129**

Compound **136** (0.66 g, 2.10 mmol, 1 eq.), Compound **8** (0.55 g, 2.10 mmol, 1 eq.), $NaHCO_3$ (0.35g, 4.20 mmol, 2 eq.) and DCM/$H_2O$ (9 mL, 2:1) were added into a 50 ML reaction bottle, heated and reacted at 50°C for half an hour, then the reaction solution was filtered and concentrated to obtain Compound **129** (1.10 g, crude product).

Referring to the synthesis method of intermediate Compound **129**, Compound 12 in the following table was used to replace Compound **8**, and the other raw materials and operating methods remained unchanged to obtain intermediate Compound **130**.

| Intermediate Compound No. | Intermediate compound structure | Raw material |
|---|---|---|
| 130 | 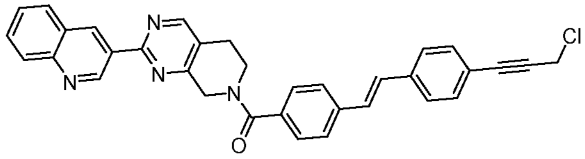 130 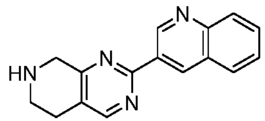 12 | 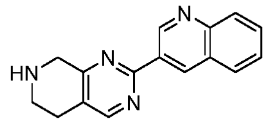 12 |

**Example 17.** Synthesis of Compound **H1-1** of the present invention

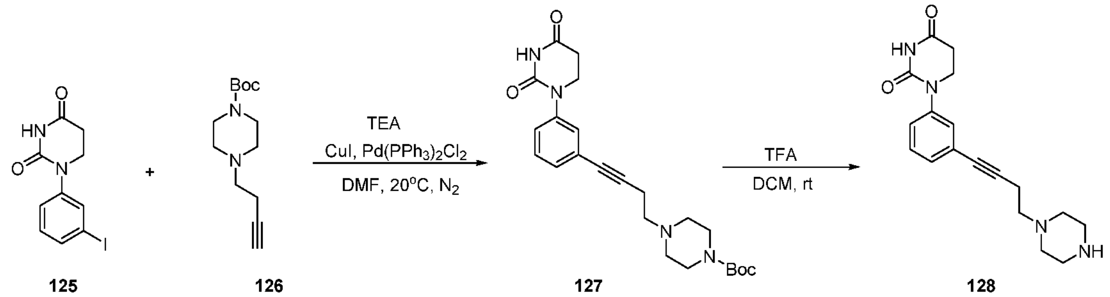


**125** **126** **127** **128**

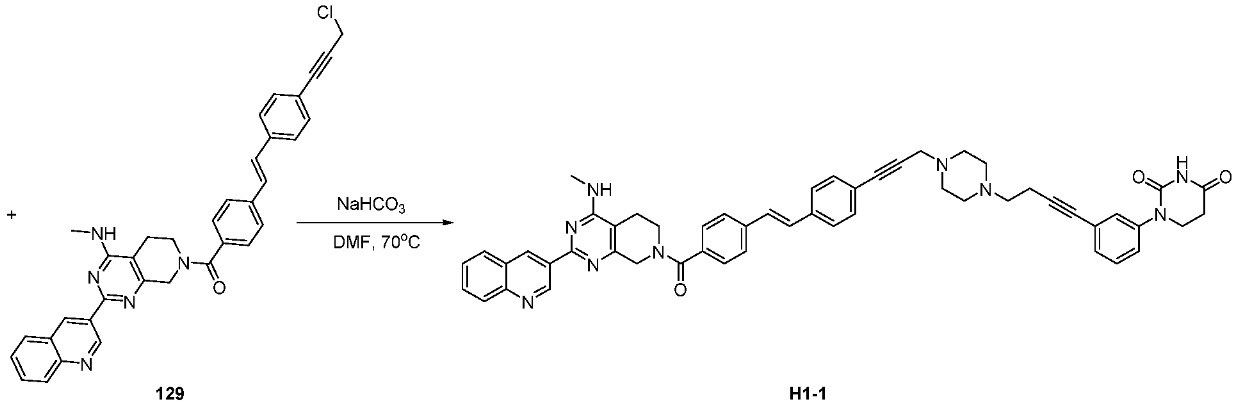


**129** **H1-1**

### Step 1. Synthesis of Compound 127

Compound **125** (317.00 mg, 1.00 mmol), Compound **126** (286.81 mg, 1.20 mmol), copper iodide (19.10 mg, 100.29 μmol), and bis(triphenylphosphine)palladium dichloride (35.15 mg, 50.14 μmol) and triethylamine (725.50 mg, 7.17 mmol, 1 mL) were added into a 25 mL round-bottom flask, then added with DMF (4 mL) under nitrogen protection, stirred at room temperature for 2 hours, water was added to quench the reaction, and extracted with ethyl acetate. The obtained organic phase was washed with saturated brine, dried over anhydrous sodium sulfate, and then the solvent was rotary evaporated to obtain Compound **127** (600.00 mg, 1.41 mmol).

### Step 2. Synthesis of Compound 128

Compound **127** (600 mg, 1.41 mmol), DCM (5 mL), and triethylamine (5 mL) were added into a 25 mL round-bottom flask. After stirring at room temperature for half an hour, the solvent was evaporated under reduced pressure, and purification was carried out by MPLC to obtain Compound **128** (220.00 mg, 674.04 μmol, yield 47.91%).

### Step 3. Synthesis of Compound H1-1

Compound **128** (50.00 mg, 153.19 μmol), $NaHCO_3$ (64.34 mg, 765.95 μmol), Compound **129** (87.33 mg, 153.19 μmol) and DMF (3 mL) were added into a 25 mL round-bottomed flask, reacted under stirring at 70°C for half an hour, then water was added to quench the reaction, and extraction was carried out with ethyl acetate. The obtained organic phase was washed with saturated brine, dried over $Na_2SO_4$, evaporated under reduced pressure to remove the solvent, and purified by MPLC to obtain Compound **H1-1** (15.00 mg, 17.44 μmol, yield 11.39%, purity 95.9%). LC-MS: $C_{53}H_{50}N_9O_3$, $[M+H]^+$: 861.4, found: 860.8. $^1H$ NMR (600 MHz, DMSO-*d6*) δ 10.42 (s, 1H), 9.85-9.72 (m, 1H), 9.24-9.21(m, 1H), 8.15- 8.13 (m, 2H), 7.85 (s, 1H), 7.76-7.73 (m, 2H), 7.68-7.65 (m, 3H), 7.54-7.52 (m, 4H), 7.43-7.38 (m, 4H), 7.36-7.34 (m, 2H), 7.31-7.30 (m, 1H), 4.74 (s, 1H), 4.57 (s, 1H), 4.10-4.05 (m, 2H), 3.64-3.59 (m, 2H), 3.37-3.33 (m, 4H), 3.20-3.03 (m, 7H), 2.96 (s, 3H), 2.81 (s, 2H), 2.70 (t, *J*= 6.6 Hz, 2H), 2.58 (s, 3H).HPLC>95%.

According to the synthesis method of Compound **H1-1,** the raw material **130** in the following table was used to replace Compound **129**, and the other raw materials and operating methods remained unchanged to obtain Compound **H1-2.**

| Compound No. | Structure and characterization data of compound | Raw material | Yield |
| --- | --- | --- | --- |
| **H1-2** | 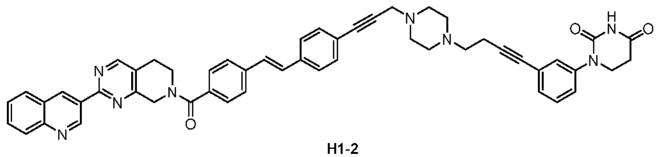 H1-2 **H1-2** (9.30 mg, 10.81 μmol, yield 21.64%, purity 97.6%). LC-MS: $C_{52}H_{47}N_8O_3$, $[M+1]^+$ 831.4, found: 831.6. <br><br> $^1H$ NMR (600 MHz, DMSO-$d_6$) δ 10.43 (s, 1H), 9.80-9.76 (m, 1H), 9.26-9.23 (m, 1H), 8.17-8.14 (m, 2H), 7.85 (s, 1H), 7.81-7.79 (m, 2H), 7.68-7.64 (m, 3H), 7.50-7.48 (m, 4H), 7.43-7.38 (m, 4H), 7.36-7.34 (m, 2H), 7.30-7.28 (m, 1H), 4.74 (s, 1H), 4.57 (s, 1H), 4.10-4.05 (m, 2H), 3.63-3.60 (m, 2H), 3.36-3.30 (m, 4H), 3.18-3.03 (m, 7H), 2.96 (s, 3H), 2.81 (s, 2H), 2.70 (t, $J$ = 6.6 Hz, 2H). HPLC > 95%. | 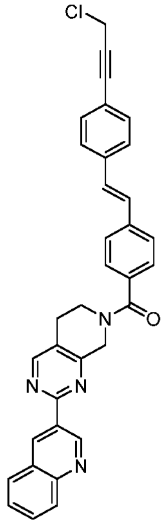 130 | 21.64% |

The technical effects of the compounds of the present invention were illustrated below through experimental examples:
NaVc: sodium vitamin C; His-Tag Labeling Kit-RED-tris-NTA: RED-Tris-NTA protein labeling kit; Dianthus 384 well plates: Dianthus 384-well plate; ZebaTM Spin Desalting Columns 7K: Zeba desalting spin column; DMSO/Dimethyl Sulfoxide: dimethyl sulfoxide; BCL-XL/BAK Binding Assay Kit: BCLXL/BAK binding kit; S series NTA sensor chip: S series NTA chip; NaOH 50: sodium hydroxide; EDTA: ethylenediaminetetraacetic acid; $NiCl_2$: nickel chloride; EDC: 1-ethyl-(3-dimethylaminopropyl)carbodiimide; NHS: N-Hydroxysulfosuccinimide; Ethanolamine-HCl (pH 8.5): ethanolamine-hydrochloride (pH 8.5); HuMan BCL2L1BCL-Xl, Protein: human BCL2L1/BCL-XL protein; AlphaLISA®Nikel Chelate Acceptor beads: AlphaLISA nickel receptor magnetic beads; Strep-Tactin® Alpha Donor beads: Strep-Tactin Alpha donor magnetic beads; RPMI medium modified: RPMI 1640 medium; FBS: fetal bovine serum; P/S: penicillin-streptomycin double antibody; CellTiter-Glo Luminescent Cell Viability Assay: CellTiter-Glo® luminescent viable cell detection system; Diluent buffer: dilution buffer salt; Glycine: glycine; Imidazole: imidazole.

**Experimental Example 1,** BCL-Xl, TRIC (temperature-dependent fluorescence intensity change method) binding detection

1. Experimental materials and reagents

$Na_2HPO_4$ (Sigma), $NaH_2PO_4$ (Sigma), sodium chloride (Sigma), Tween 20 (Sigma), His-Tag Labeling Kit-RED-tris-NTA (NanoTemper), Dianthus 384 well plates (NanoTemper), deionized water (NanoTemper), BCL-XL protein (Sino Biological), ZebaTM Spin Desalting Columns 7K (Thermo scientific).

2. Experimental methods

2.1 RED-tris-NTA protein labeling

2.1.1 Protein stock buffer replacement:

ZebaTM Spin Desalting Columns 7K MWCO desalting column was used to replace the stock buffer of BCL-XL with 10 mM $NaH_2PO_4$, 40 mM $Na_2HPO_4$, 150 mM sodium chloride, 0.03% Tween 20, 10% glycerol, pH7.4, so as to remove the imidazole and hydroxymethylaminomethane ingredients in the stock buffer.

2.1.2 Testing of affinity between dye and protein

To a 5×PBST (from RED-tris-NTA protein labeling kit) bottle, 8.0 mL of $ddH_2O$ was added for dilution to obtain 1×PBST, 25.0 μL of PBST was added to dilute the dye to 5 μM, 2.0 μL of 5 μM dye and 198.0 μL of PBST were mixed to obtain 200.0 μL of 50 nM dye; 30.0 μL of 4 μM histidine-tagged BCL-XL protein diluted with PBST was prepared and subjected to a 2-fold gradient dilution with 16 points. 10 μL of protein with different concentrations was taken and thoroughly mixed with 10.0 μL of 50 nM dye, incubated at room temperature for 30 minutes, and read with DI. Kd was calculated by DI.SA, and the calculated Kd was <10nM.

2.1.3 Affinity of dye and protein

2.0 μL of 5 μM dye was taken and mixed with 98.0 μL of PBST to obtain 100.0 μL of 100 nM dye. Histidine-tagged BCL-XL protein was diluted to 200 nM. 90.0 μL of 200 nM histidine-tagged BCL-XL protein was taken and thoroughly mixed with 90.0 μL of 100 nM dye, and incubate at room temperature for 30 minutes. After centrifugation at 15000g for 10 minutes at 4°C, the supernatant was taken and placed in a new tube.

2.2. Testing of compound affinity

The compound was diluted with buffer PBST by a dilution with a total of 16 concentration gradient. 10.0 μL of the compound of each concentration was taken and thoroughly mixed with 10.0 μL of the labeled protein (the final concentration of the RED-tris-NTA-labeled protein used was not less than 20 nM.), and read by DI. Kd was calculated by DI.SA.

3. Data analysis

DI.SA was used to determine the binding of ligand/compound to target and the specific Kd.

**Experimental Example 2.** Detection of BCL-XL inhibitory activity of compound

1. Experimental instruments and reagents:

Microplate reader (Tecan Spark), ECHO (LABCYTE Echo 665), microplate constant temperature oscillator (Hangzhou Ruicheng Instrument Co., Ltd.), BCL-XL/BAK Binding Assay Kit (CISbIO), 384-well plates

2. Experimental method:

DMSO was used to dissolve the dry powder of compound into a 10.00 mM solution. The instrument ECHO was used to perform gradient dilution of the compound, which was added to a 384-well reaction plate, so that the final concentration of DMSO in the entire reaction system (12.0 μL) was less than 0.5%. At the same time, an equal amount of DMSO was added as a control.

The Diluent buffer (Lot 06A) in BCL-XL/BAK Binding Assay Kit was used to dilute BCL-XL and Tag2-BAK to 4 times the required final concentration, and 3.0 μL of each was pipetted and added to the 384-well reaction plate where the compound had been added. After centrifugation at 1000 rpm for 1 minute, the plate was placed on a microplate constant-temperature shaker and pre-incubated at 25°C, 280 rpm for 15 minutes. Then the detection Buffer (Lot 10A) in BCL-XL/BAK Binding Assay Kit was used to dilute 100X Anti-tag1-$Eu^{3+}$ (Lot 06A) and Anti-tag2-XI,665 (Lot 104A) to 1x, respectively, and prepare Anti-tag1-$Eu^{3+}$/Anti-tag2-XL665 mixture at a ratio of 1:1. 6.0 μL of Anti-tag1-$Eu^{3+}$/Anti-tag2-XL665 mixture was pipetted and added to the 384-well reaction plate, centrifuged at 1000 rpm for 1 minute and placed on a microplate constant-temperature shaker, and incubated at 25°C, 280 rpm for 2 hours. After the reaction, the microplate reader was used to read the fluorescence signal value in the 384-well reaction plate (Ex = 320 nm, Em = 665/620 nm).

3. Data analysis

The vehicle group (containing 1xTag1-Bcl-XL, 1xTag2-BAK, 1X Anti-Tag1-$Eu^{3+}$ and 1X Anti-Tag2-XL665, 0.5% DMSO) was used as a negative control, and the reaction buffer group (containing 1X Anti-Tag1-$Eu^{3+}$ and 1X Anti-Tag2-XI,665, 0.5% DMSO) was used as a blank control;

The remaining activity percentage of each concentration was calculated by the following formula:

Remaining activity (%) = 100% × ($Flu_{\text{compound group}}$ - $Flu_{\text{blank control}}$)/($Flu_{\text{negative control}}$ - $Flu_{\text{blank control}}$)

Then GraphPad 6.0 was used to fit the dose-effect curve to calculate $IC_{50}$ value.

**Experimental Example 3,** Binding detection of BCL-XL by surface plasmon resonance (SPR)

1. Experimental instruments and reagents:

Biacore T200 (GE Healthcare), S series NTA sensor chip (GE Healthcare), NaOH 50 (GE Healthcare), DMSO (MP), $Na_2HPO_4$ (Sigma), $NaH_2PO_4$ (Sigma), EDTA (Sigma), $NiCl_2$ (Sigma), sodium chloride (Sigma), Tween 20 (Sigma), EDC (GE Healthcare), NHS (GE Healthcare), Ethanolamine-hydrochloric acid (pH 8.5) (GE Healthcare), 96-well plate, HuMan BCL2L1BCL-XL Protein (Sino Biological), ZebaTM Spin Desalting Columns 7K (Thermo scientific).

2. Test method of SPR

Preparation of running buffers: The protein fixation buffer had the same ingredients as running buffer A, in which the concentration of $NaH_2PO_4$ was 10 mM, the concentration of $Na_2HPO_4$ was 40 mM, the concentration of sodium chloride was 150 mM, the content of Tween 20 was 0.03%, and the pH was adjusted to 7.4; in the running buffer B, the concentration of $NaH_2PO_4$ was 10 mM, the concentration of $Na_2HPO_4$ was 40 mM, the concentration of sodium chloride was 150 mM, the content of Tween 20 was 0.03%, the content of DMSO was 5.00%, and the pH was adjusted to 7.4. After the running buffers were prepared, they were filtered with a 0.22 μm filter.

Replacement of protein stock buffer: ZebaTM Spin Desalting Columns 7K MWCO desalting column was used to replace the BCL-XL stock buffer with 10 mM $NaH_2PO_4$, 40 mM $Na_2HPO_4$, 150 mM sodium chloride, 0.03% Tween 20, 10% glycerol, pH 7.4, so as to remove the imidazole and Tris ingredients in the stock buffer.

Immobilization of BCL-XL protein: Protein fixation buffer was used to immobilize BCL-XL on the S series NTA chip through His capture and amino coupling. The surface of the NTA chip was cleaned with 50 mM NaOH and 350 mM

EDTA respectively, with a flow rate of 60.0 μL/min, 60 seconds each time; then activated with 10 mM $NiCl_2$ for 1100 seconds, and activated with a mixture of EDC (75.00 mg/mL) and NHS (11.50 mg/mL) at a volume ratio of 1:1 for 650 seconds, with an activation flow rate of 10.0 μL/min; then, BCL-XL (0.04 mg/mL) was injected at 4.0 μL/min for 850 seconds. After injection of BCL-XL, a mixture of EDC (75.00 mg/mL) and NHS (11.50 mg/mL) with a volume ratio of 1:1 was cross-linked at a speed of 10.0 μL/min for 200 seconds, and finally 1M ethanolamine (pH 8.5) was injected at a speed of 6.0 μL/min for 7 minutes to block the chip surface. The final fixation amount of BCL-XL was 4690.00 RU.

Dilution of compound: The test compound was diluted with 100% DMSO to 100 times the required final concentration; after evenly mixing, 4.0 μL was taken and added to 396 μL of running buffer A, and centrifuged at 15000 rpm for 5 minutes to obtain 1× compound solution containing 1% DMSO for subsequent dilution. The compound was serially diluted by 3-fold to 8 concentrations starting from 100 μM with the running buffer B. The diluted compound was transferred to a 96-well plate for sample injection.

Running program: The experiment was run at 25°C. When running the program, the running buffer B was used at a flow rate of 30.0 μL/min. After running buffer B was injected 8 times to complete the equilibrium, the compound was injected sequentially from the lowest concentration to the highest concentration. The binding time and dissociation time were both 120 seconds. After each injection, the injection needle was cleaned with 50% DMSO. The solvent differences caused by DMSO were corrected with 0.50%, 0.75%, 1.00%, 1.25% and 1.50% DMSO.

3. Data analysis

The response value of the compound binding to BCL-XL was analyzed after subtracting the reference channel and 0 concentration. The affinity Kd was fitted by Biacore T200 Evaluation Software using a steady state affinity model (1: 1 binding model).

**Experimental Example 4,** Detection of compound-induced formation of ternary complex between BCL-XL and CRBN-DDB1 by AlphaLISA method

1. Experimental instruments and reagents:

$Na_2HPO_4$(Sigma), $NaH_2PO_4$(Sigma), sodium chloride (Sigma), Tween-20 (Sigma), AlphaLISA®Nikel Chelate Acceptor beads (PerkinElmer), Strep-Tactin® Alpha Donor beads (PerkinElmer), Assay plate (Corning), Strep-Bcl-XI, (HitGen), His-CRBN/DDB1 (HitGen).

2. Experimental methods

DMSO was used to dissolve the dry powder of the test compound to obtain a 10.00 mM solution. The instrument ECHO was used to perform gradient dilution of the compound, which was then added to a 384-well reaction plate (Corning, Cat#3824), so that the final concentration of DMSO in the entire reaction system (10.0 μL) was less than 1%, and the same amount of DMSO was added and used as a control.

Strep-Bcl-XI, and His-CRBN/DDB1 proteins were dissolved in buffer (10mM $NaH_2PO_4$, 40mM $Na_2HPO_4$, 150mM sodium chloride, 0.03% Tween20, pH=7.4) to obtain a protein mixture of 60nM Strep-BCL-XI, and 60nM His-CRBN/DDB1, the protein mixture (5ul/well) was added to the 384-well plate in which the compound had been added, and incubated at room temperature for 30 minutes, then 5μl of a detection mixture with 50μg/ml AlphaLISA®Nikel Chelate Acceptor beads and 50μg/ml Strep-Tactin® Alpha Donor beads was added, and incubated at room temperature for 2 hours; and then a microplate reader was used to detect AlphaLISA signal.

3. Data analysis

Fifth order polynomial or Sixth order polynomial in Polynomial of Graphpad 6.0 was used for data fitting.

Table 1: PROTAC ternary complex signal intensity table

| No. | AlphaLISA signal | No. | AlphaLISA signal | No. | AlphaLISA signal |
|---|---|---|---|---|---|
| A | ++ | C3-1 | + | F3B-3 | ++ |
| B3 | + | C3-2 | + | F2-4 | ++ |
| C2-1 | ++ | C3-14 | + | F1B-2 | + |
| C2-2 | ++++ | C3-13 | + | G2-1 | ++ |

(continued)

| No. | AlphaLISA signal | No. | AlphaLISA signal | No. | AlphaLISA signal |
|---|---|---|---|---|---|
| C2-3 | +++++ | C3-10 | ++ | G2-2 | + |
| C2-4 | ++++ | C3-6 | + | G2-3 | + |
| C2-5 | +++ | C3-4 | + | G2-4 | + |
| C3-17 | ++++ | C1-1 | + | G2-5 | + |
| B2 | + | D2-4 | + | G2-7 | + |
| B1 | + | C3-5 | ++ | G2-6 | + |
| C3-15 | + | D2-6 | ++ | G1-5 | ++ |
| C3-16 | ++++ | F3B-2 | ++ | G1-4 | + |
| C3-18 | +++ | E1-1 | + | G3-1 | + |
| C3-19 | +++ | E1-2 | ++ | | |
| C3-3 | + | D2-1 | + | | |

Wherein, the number of + indicates the intensity of AlphaLISA peak signal.

**Experimental Example 5,** Evaluation experiment of inhibitory activity of compound on MOLT4 cell proliferation by Cell Titer-Glo method

1. Experimental instruments and reagents:

RPMI medium modified (Hyclone), FBS (Corning), Dimethyl Sulfoxide, P/S, CellTiter-Glo Luminescent Cell Viability Assay (Promega), MOLT-4 cell (ATCC).

2. Experimental methods

MOLT-4 cells were cultured in monolayer in vitro in an incubator containing 5% $CO_2$ air at 37°C. The culture medium was RPMI 1640 with 10% FBS and 1% P/S. 15 μL of MOLT4 cells, containing $5\times10^4$ cells per ml, was inoculated to a 384-well plate (Corning, 3707), 750 cells/well, and incubated at 37°C and 5% $CO_2$ for 30 minutes. Then the compound that had been diluted in advance was added, with a total of 9 concentration points, and 2 parallel repetitions for each concentration point. The cell growing group without adding the compound was used as a negative control (maximum signal control), and the culture medium group was used as a blank control (minimum signal control). At the same time, it was ensured that the final DMSO content in each reaction well was 0.1%. The compound and the cells were incubated in a cell culture incubator at 37°C, 5% $CO_2$ for 3 days (72 hours).

The 384-well plate was taken out from the cell culture incubator and allowed to stand at room temperature, equilibrated for 20 to 30 minutes, and then 25 μl of Cell Titer-Glo detection reagent was added to each reaction well, lysed on a shaker for 2 minutes, incubated for 10 minutes, and BMG PHERAStar (Luminescence) was used for reading.

3. Data analysis

The inhibition rate was calculated based on the luminescence signals: the average value of the negative control (maximum signal control) and the blank control (minimum signal control) was first calculated, then the inhibition rates of the compound at different concentrations for the cells were calculated as follows: Inhibition% = 1 - $\frac{\text{S(compound signal value)} - \text{S(blank control signal value)}}{\text{S(negative control signal value)} - \text{S(blank control signal value)}}) \times 100\%$. The $IC_{50}$ of the compound to inhibit cell activity was calculated by fitting with GraphPad Prism 6 using log(inhibitor) vs. response-Variable slope mode. The fitting equation was: Y=Bottom+(Top-Bottom)/(1+10^((Log$IC_{50}$-X)*HillSlope)), wherein Y represented the inhibition rate and X represented the known compound concentration after Log.

Table 2: $IC_{50}$ concentrations for inhibiting MOLT-4 cells

| No. | $IC_{50}$ concentration | No. | $IC_{50}$ concentration | No. | $IC_{50}$ concentration |
|---|---|---|---|---|---|
| A | +++ | D2-2 | +++ | F3A-4 | ++ |
| C1-1 | ++ | D2-3 | + | F3B-1 | ++ |
| C1-2 | + | D2-4 | ++ | F3B-2 | +++ |
| C1-3 | ++++ | D2-5 | ++ | F3B-3 | ++++ |
| C1-4 | ++++ | D2-6 | ++ | F2-1 | ++ |
| C2-1 | +++ | D2-7 | ++ | F2-2 | +++ |
| C2-2 | +++ | D2-15 | +++++ | F2-3 | ++++ |
| C2-3 | ++ | D2-16 | ++++ | F2-4 | ++++ |
| C2-6 | ++++ | E1-1 | ++ | F2-5 | + |
| C3-2 | ++ | E1-2 | +++ | F2-6 | ++ |
| C3-4 | ++ | F1A-1 | +++++ | F2-7 | ++++ |
| C3-5 | ++ | F1A-2 | +++++ | F2-8 | ++++ |
| C3-6 | +++ | F1A-3 | ++++ | F2-9 | +++ |
| C3-7 | + | F1A-5 | ++++ | F2-10 | ++++ |
| C3-8 | + | F1B-1 | +++ | F2-11 | +++ |
| C3-11 | ++ | F1B-2 | +++ | G1-3 | + |
| C3-12 | +++ | F1B-3 | +++ | G2-1 | + |
| C3-16 | +++ | F1B-4 | ++++ | G1-5 | + |
| C3-17 | +++ | F3A-1 | + | H1-1 | +++++ |
| D1-1 | ++ | F3A-2 | ++ | | |
| D2-1 | ++ | F3A-3 | + | | |

Wherein, + represented $IC_{50}$ > 200 μM, ++ represented 200 μM > $IC_{50}$ > 100 μM, +++ represented 100 μM > $IC_{50}$ > 50 μM, ++++ represented 10 μM > $IC_{50}$ > 1 μM, +++++ represented $IC_{50}$ < 1 μM.

**Experimental Example 6.** Experiment to evaluate degradation effect of compound on BCL-XL protein in MOLT-4 cells by Western blot method

1. Experimental instruments and reagents:

SDS-PAGE gel (4-12%) (Genscript), PMSF 100mM (Biyotime), Cocktail 100× (Biyotime), P/S, RPMI medium modified (Hyclone), FBS (Corning), 4× loading buffer (Thermo), Bcl-xL (54H6) Rabbit mAb (CST), Anti-rabbit IgG-HRP (CST), Anti-β-actin-HRP (Abcam), RIPA (Biyotime), Tween-20, MOLT-4 cell (ATCC), BCA Protein Assay Kit (TIANGEN), Immobilon Western Chemilunescent HRP Substrate (Millipore).

2. Experimental methods

1) Incubation of compound and cells: 1mL of $10^6$ MOLT-4 cells (culture medium: 89% RPMI, 10% FBS, 1% P/S) was inoculated in a 6-well cell culture plate, the compound was subjected to gradient dilution with DMSO, then the compound diluted in DMSO in gradient was diluted again into the MOLT-4 medium, and 1 mL of the diluted compound was directly added to the 6-well plate with the cultured cells and incubated for 24 hours.

2) Protein extraction: After the compound and the cells were co-incubated for 24 hours, the cells were collected and

placed in a 1.5mL centrifuge tube, washed twice with ice-cold PBS, the supernatant was discarded, then 150 μL of RIPA lysis buffer was added and lysis was carried out on ice for 30 minutes; then the cell lysate was centrifuged in a low-temperature centrifuge at 15,000 rpm for 10 minutes, and the supernatant was collected; and BCA Protein Assay Kit was used to quantify the protein in the resulting cell lysate supernatant. 4X loading buffer was added to the cell lysate supernatant and incubated at 85°C for 10 minutes to obtain a sample. The obtained sample was stored in -80°C refrigerator for the use on the next day. 20 μg of the sample was taken to perform SDS-Page and transmembrane, 5% skim milk was used to block the membrane for 1 hour, and then Bcl-xL (54H6) rabbit mAb, Anti-rabbit IgG-HRP, Anti-β-actin-HRP were used for incubation. After incubation, Immobilon Western Chemiluminescent HRP Substrate was used for color development, and a gel analyzer was used for analysis.

3. Data analysis

Analysis was performed with a grayscale analysis software, the amount of BCL-XL protein was corrected by the amount of β-actin, and then Graphpad 6 was used to calculate $DC_{50}$.

Table 3: $DC_{50}$ concentration for BCL-XL protein degradation

| No. | $DC_{50}$ concentration | No. | $DC_{50}$ concentration | No. | $DC_{50}$ concentration |
|---|---|---|---|---|---|
| C2-2 | +++ | C3-13 | ++ | F1B-3 | ++++ |
| C2-3 | +++ | C3-10 | +++ | F2-4 | ++++ |
| C3-17 | ++++ | C3-6 | +++++ | F2-8 | +++++ |
| C3-16 | ++++ | E1-2 | ++++ | F2-9 | ++++ |
| C3-1 | ++ | D2-1 | +++++ | F2-11 | ++++ |
| C3-2 | ++++ | D2-15 | ++++ | F3B-3 | +++++ |
| C3-14 | ++ | F1B-2 | +++ | H1-1 | +++++ |
| Wherein, + represented $DC_{50}$ > 200 μM, ++ represented 200 μM > $DC_{50}$ > 100 μM, +++ represented 100 μM > $DC_{50}$ > 50 μM, ++++ represented 10 μM > $DC_{50}$ > 1 μM, +++++ represented $DC_{50}$ < 1 μM. | | | | | |

**Claims**

1. A compound represented by Formula I, or deuterated compound thereof, or stereoisomer thereof, or tautomer thereof, or polymorph thereof, or solvate thereof, or N-oxide thereof, or isotopically labeled compound thereof, or metabolite thereof, or prodrug thereof, or pharmaceutically acceptable salt thereof:

$$X—Y—Z$$

Formula I

wherein,

X represents a group binding to a BCL-XL protein;
Y represents a connecting group;
Z represents a group binding to an E3 ubiquitin-protein ligase.

2. The compound according to claim 1, wherein X is selected from

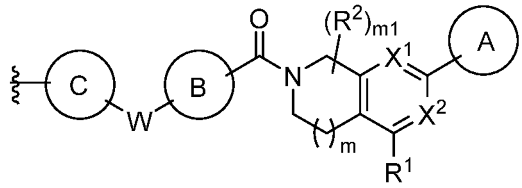

wherein,

Ring A is selected from $C_{6\sim10}$ aromatic ring or 6- to 10-membered aromatic heterocycle; wherein the aromatic ring and the aromatic heterocycle can be further substituted by one, two or three $R^{A1}$ groups;
each $R^{A1}$ is independently selected from hydrogen, halogen, cyano, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{A2}$ or $-C_{0\sim4}$ alkylene-$NR^{A2}R^{A3}$;
$R^{A2}$ and $R^{A3}$ are each independently selected from hydrogen, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl or halogen-substituted $-C_{2\sim6}$ alkynyl;
$X^1$ and $X^2$ are each independently selected from Nor $CR^{X1}$;
each $R^{X1}$ is independently selected from hydrogen, halogen, cyano, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{X2}$ or $-C_{0\sim4}$ alkylene-$NR^{X2}R^{x3}$,
$R^{X2}$ and $R^{X3}$ are each independently selected from hydrogen, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl or halogen-substituted $-C_{2\sim6}$ alkynyl;
$R^1$ is selected from hydrogen, halogen, cyano, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{11}$ or $-C_{0\sim4}$ alkylene-$NR^{11}R^{12}$;
$R^{11}$ and $R^{12}$ are each independently selected from hydrogen, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl or halogen-substituted $-C_{2\sim6}$ alkynyl;
each $R^2$ is independently selected from hydrogen, halogen, cyano, =O, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{21}$ or $-C_{0\sim4}$ alkylene-$NR^{21}R^{22}$,
$R^{21}$ and $R^{22}$ are each independently selected from hydrogen, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl or halogen-substituted $-C_{2\sim6}$ alkynyl;
m1 is selected from 0, 1, 2 or 3;
m is selected from 0, 1 or 2;
Ring B is selected from $C_{3\sim10}$ cycloalkane, 3- to 10-membered heterocycloalkane, benzene ring, 5- to 6-membered aromatic heterocycle or 5- to 12-membered bridged ring; wherein, the cycloalkane, heterocycloalkane, benzene ring, aromatic heterocycle and bridged ring can be further substituted by one, two or three $R^{B1}$;
each $R^{B1}$ is independently selected from hydrogen, halogen, cyano, =O, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{B2}$ or $-C_{0\sim4}$ alkylene-$NR^{B2}R^{B3}$;
$R^{B2}$ and $R^{B3}$ are each independently selected from hydrogen, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl or halogen-substituted $-C_{2\sim6}$ alkynyl;
W is selected from $-C_{1\sim6}$ alkylene-, $-C_{1\sim6}$ alkylene-O-, $-O-C_{1\sim6}$ alkylene-, $-C_{2\sim6}$ alkenylene-, $- C_{2\sim6}$ alkenylene-O-, $-O-C_{2\sim6}$ alkenylene-, $-C_{2\sim6}$ alkynylene-, $-C_{2\sim6}$ alkynylene-O- or $-O-C_{2\sim6}$ alkynylene-;
Ring C is selected from benzene ring or 5- to 6-membered aromatic heterocycle; wherein, benzene ring and aromatic heterocycle can be further substituted by one, two or three $R^{C1}$;
each $R^{C1}$ is independently selected from hydrogen, halogen, cyano, $-C_{1\sim6}$ alkyl, $-C_{2\sim6}$ alkenyl, $-C_{2\sim6}$ alkynyl, halogen-substituted $C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{C2}$ or $-C_{0\sim4}$ alkylene-$NR^{C2}R^{C3}$;
$R^{C2}$ and $R^{C3}$ are each independently selected from hydrogen, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl or halogen-substituted $-C_{2\sim6}$ alkynyl.

**3.** The compound according to claim 2, wherein Ring A is selected from naphthalene ring or 8- to 10-membered aromatic heterocycle; wherein, the naphthalene ring and aromatic heterocycle can be further substituted by one, two or three $R^{A1}$, each $R^{A1}$ is independently selected from hydrogen, halogen, cyano or $-C_{1\sim6}$ alkyl; preferably, Ring A is selected from naphthalene ring, benzopyrimidine ring or quinoline ring; more preferably, Ring A is selected from quinoline ring.

4. The compound according to any one of claims 2 to 3, wherein $X^1$ and $X^2$ are each independently selected from N or CH; preferably, $X^1$ and $X^2$ are selected from N.

5. The compound according to any one of claims 2 to 4, wherein $R^1$ is selected from hydrogen, halogen, cyano, $-C_{1\sim6}$ alkyl or $-NR^{11}R^{12}$, and $R^{11}$ and $R^{12}$ are each independently selected from hydrogen or $-C_{1\sim6}$ alkyl; preferably, $R^1$ is selected from hydrogen or $-NR^{11}R^{12}$, and $R^{11}$ and $R^{12}$ are each independently selected from hydrogen or $-C_{1\sim6}$ alkyl; more preferably, $R^1$ is selected from hydrogen

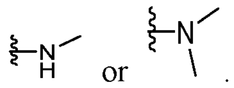

6. The compound according to any one of claims 2 to 5, wherein m is selected from 1.

7. The compound according to any one of claims 2 to 6, wherein Ring B is selected from benzene ring or 6-membered nitrogen-containing heterocycloalkane, 6-membered nitrogen-containing aromatic heterocycle, 5- to 6-membered cycloalkane or 5- to 8-membered bridged ring; wherein, the benzene ring, heterocycloalkane, aromatic heterocycle, cycloalkane and bridged ring can be further substituted by one, two or three $R^{B1}$; each $R^{B1}$ is independently selected from hydrogen, halogen, cyano or $-C_{1\sim6}$ alkyl; preferably, Ring B is selected from benzene ring, pyridine ring or

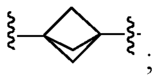

more preferably, Ring B is selected from benzene ring.

8. The compound according to any one of claims 2 to 7, wherein W is selected from $-C_{1\sim4}$ alkylene-, $-C_{1\sim4}$ alkylene-O-, $-O-C_{1\sim4}$ alkylene-, $-C_{2\sim4}$ alkenylene-, $-C_{2\sim4}$ alkenylene-O-, $-O-C_{2\sim4}$ alkenylene-, $-C_{2\sim4}$ alkynylene-, $-C_{2\sim4}$ alkynylene-O- or $-O-C_{2\sim4}$ alkynylene-; preferably, W is selected from $-C_{1\sim4}$ alkylene-, $-C_{1\sim4}$ alkylene-O-, $-O-C_{1\sim4}$ alkylene-, $-C_{2\sim4}$ alkenylene-, $-C_{2\sim4}$ alkenylene-O- or $-O-C_{2\sim4}$ alkenylene-; more preferably, W is selected from $-C_{1\sim3}$ alkylene-, $-C_{1\sim3}$ alkylene-O-, $-O-C_{1\sim3}$ alkylene- or $-C_{2\sim3}$ alkenylene-; further preferably, W is selected from ethylene,

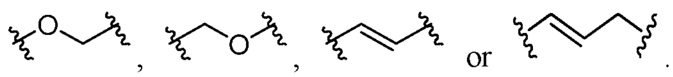

9. The compound according to any one of claims 2 to 8, wherein Ring C is selected from benzene ring or 6-membered nitrogen-containing aromatic heterocycle; wherein, the benzene ring and the aromatic heterocycle can be further substituted by one, two or three $R^{C1}$, each $R^{C1}$ is independently selected from hydrogen, halogen, cyano or $-C_{1\sim6}$ alkyl; preferably, Ring C is selected from benzene ring or pyridine ring.

10. The compound according to any one of claims 2 to 9, wherein each $R^2$ is independently selected from halogen, cyano, =O, $-C_{1\sim6}$ alkyl, $-C_{2\sim6}$ alkenyl, $-C_{2\sim6}$ alkynyl, halogen-substituted - $C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{21}$ or $-C_{0\sim4}$ alkylene-$NR^{21}R^{22}$; preferably, each $R^2$ is independently selected from halogen, cyano, $-C_{1\sim6}$ alkyl, halogen-substituted $-C_{1\sim6}$ alkyl, halogen-substituted $-C_{2\sim6}$ alkenyl, $-C_{0\sim4}$ alkylene-$OR^{21}$ or $-C_{0\sim4}$ alkylene-$NR^{21}R^{22}$, further preferably, each $R^2$ is independently selected from $-C_{0\sim4}$ alkylene-$NR^{21}R^{22}$,

preferably, m1 is selected from 0 or 1 or 2; further preferably, m1 is selected from 0;
preferably, $R^{21}$ and $R^{22}$ are each independently selected from hydrogen, $-C_{1\sim6}$ alkyl, or halogen-substituted $-C_{1\sim6}$ alkyl; further preferably, $R^{21}$ and $R^{22}$ are each independently selected from hydrogen.

11. The compound according to claim 2, wherein

Ring A is selected from naphthalene ring or 8- to 10-membered aromatic heterocycle; wherein, the naphthalene ring or aromatic heterocycle can be further substituted by one, two or three $R^{A1}$;
$X^1$ and $X^2$ are each independently selected from N or CH;
$R^1$ is selected from hydrogen or $-NR^{11}R^{12}$,

m is selected from 0 or 1;

Ring B is selected from 6-membered nitrogen-containing heterocycloalkane, benzene ring, 6-membered nitrogen-containing aromatic heterocycle, 5-membered bridged cycloalkane, or 5-membered cycloalkane; wherein, the benzene ring, aromatic heterocycle, cycloalkane, and bridged cycloalkane can be further substituted by one, two or three $R^{B1}$;

W is selected from -$C_{1\sim4}$ alkylene-, -$C_{1\sim4}$ alkylene-O-, -O-$C_{1\sim4}$ alkylene-, -$C_{2\sim4}$ alkenylene-, - $C_{2\sim4}$ alkenylene-O-, -O-$C_{2\sim4}$ alkenylene-, -$C_{2\sim4}$ alkynylene-, -$C_{2\sim4}$ alkynylene-O- or -O-$C_{2\sim4}$ alkynylene-;

Ring C is selected from benzene ring or 6-membered nitrogen-containing aromatic heterocycle; wherein, the benzene ring and aromatic heterocycle can be further substituted by one, two or three $R^{C1}$.

**12.** The compound according to any one of claims 2 to 11, wherein

Ring A is selected from

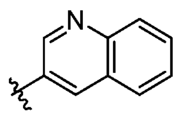,
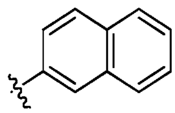 or
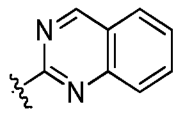;

$X^1$ and $X^2$ are selected from N or CH;

$R^1$ is selected from hydrogen,

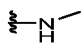 or
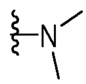;

m1 is selected from 0;

m is selected from 1;

Ring B is selected from

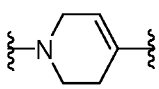,
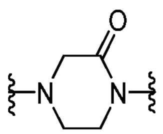,
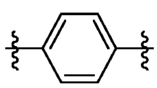,
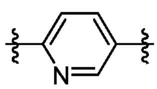,
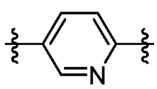 or
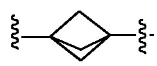;

preferably, Ring B is selected from

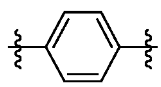,
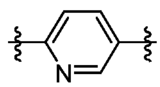

or

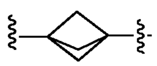;

W is selected from ethylene,

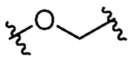, 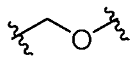, 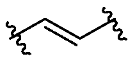 or 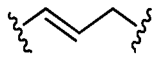;

preferably, W is selected from

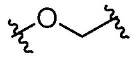, 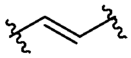 or 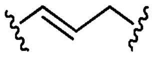;

Ring C is selected from

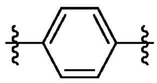, 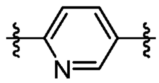 or 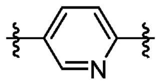.

**13.** The compound according to any one of claims 2 to 12, wherein X is selected from

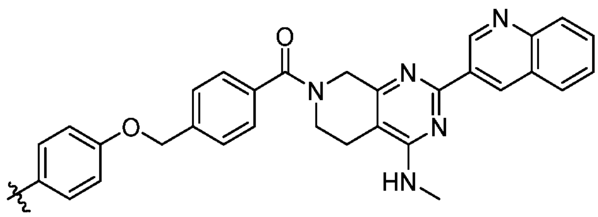, 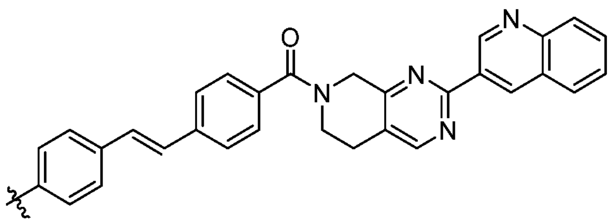,

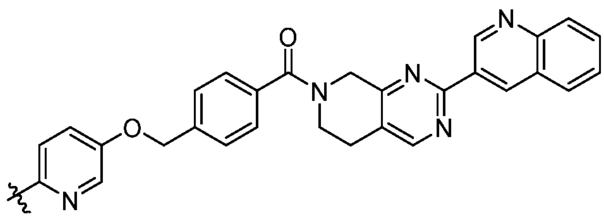, 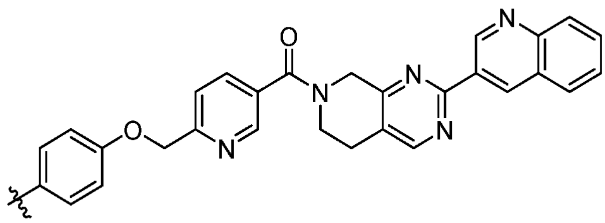,

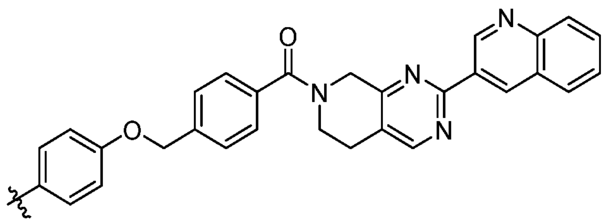, 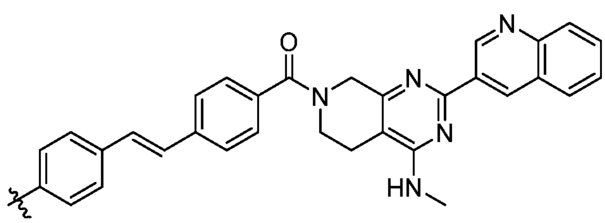,

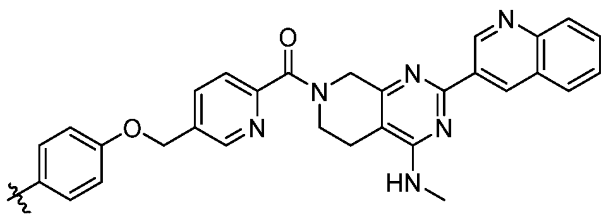, 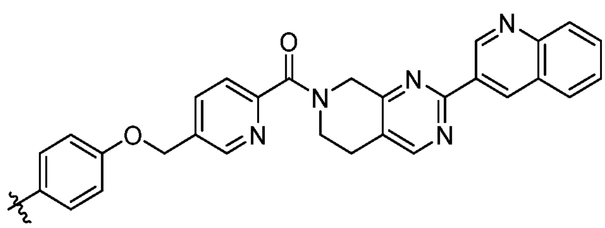,

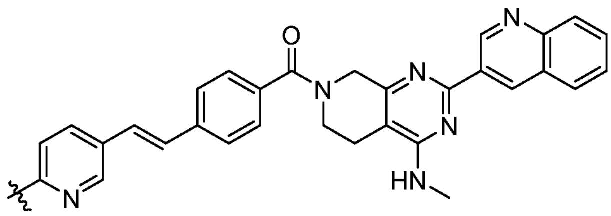
,
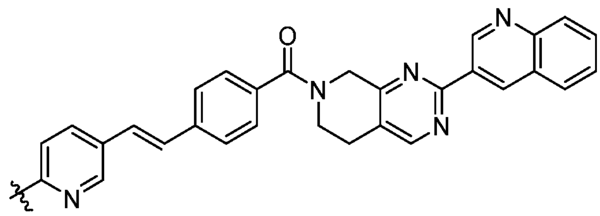
,
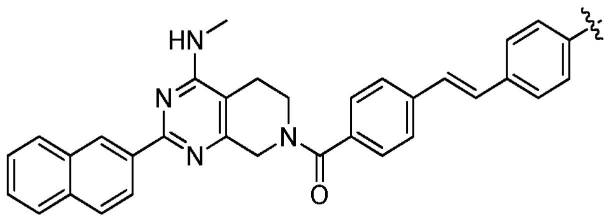
,
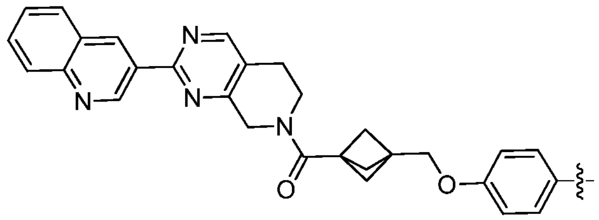
,
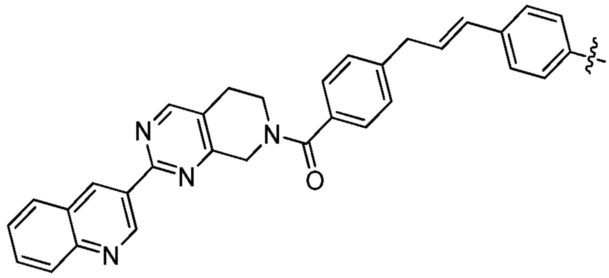
,
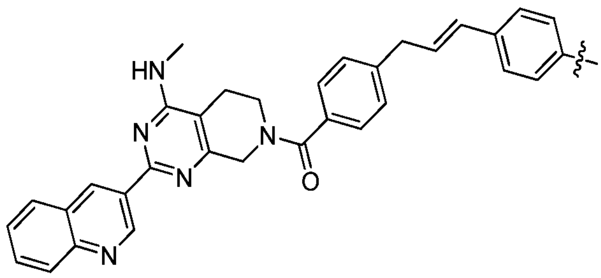
,
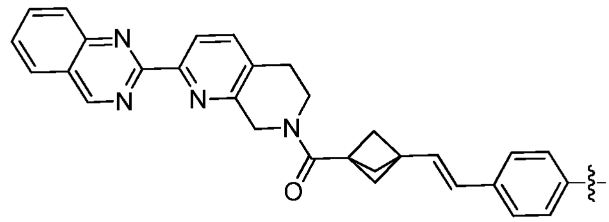
,
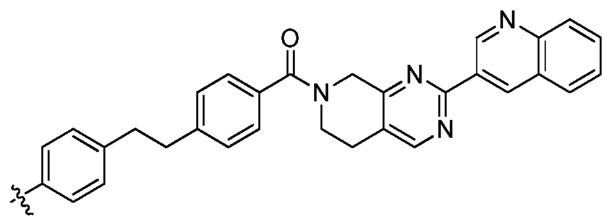
.

or

**14.** The compound according to any one of claims 1 to 13, wherein X is optionally and independently substituted by one or more (e.g., 2, 3, 4, 5, 6, 7, 8, 9 or 10) $R^{A1'}$.

preferably, each $R^{A1'}$ is independently selected from halogen, cyano, $-C_{1\sim6}$ alkyl, $-C_{2\sim6}$ alkenyl, $-C_{2\sim6}$ alkynyl, halogen-substituted $-C_{1\sim6}$ alkyl, halogen-substituted $-C_{2\sim6}$ alkenyl, halogen-substituted $-C_{2\sim6}$ alkynyl, $-C_{0\sim4}$ alkylene-$OR^{A2'}$ or $-C_{0\sim4}$ alkylene-$NR^{A2'}R^{A3'}$;

$R^{A2'}$ and $R^{A3'}$ are each independently selected from hydrogen, $-C_{1\sim3}$ alkyl, $-C_{2\sim6}$ alkenyl, $-C_{2\sim6}$ alkynyl, halogen-substituted $-C_{1\sim3}$ alkyl, halogen-substituted $-C_{2\sim6}$ alkenyl, or halogen-substituted $-C_{2\sim6}$ alkynyl;

preferably, each $R^{A1'}$ is independently selected from halogen, cyano, $-C_{1\sim3}$ alkyl, $-C_{2\sim4}$ alkenyl, halogen-substituted $-C_{1\sim3}$ alkyl, halogen-substituted $-C_{2\sim4}$ alkenyl, $-C_{0\sim4}$ alkylene-$OR^{A2'}$ or - $C_{0\sim4}$alkylene-$NR^{A2'}R^{A3'}$;

$R^{A2'}$ and $R^{A3'}$ are each independently selected from hydrogen, $-C_{1\sim3}$ alkyl, $-C_{2\sim4}$ alkenyl, halogen-substituted

$-C_{1\sim3}$ alkyl or halogen-substituted $-C_{2\sim4}$ alkenyl.

**15.** The compound according to any one of claims 1 to 14, wherein

the Y is selected from $-(L^Y)_q-$;
q is an integer selected from 1 to 30;
each $L^Y$ is independently selected from structural fragments consisting of any one or more members selected from the group consisting of $C(R)_2$, C(O), O, S, S(O), $S(O)_2$, NR, -CR=CR-, - C=C-, $C_{3\sim10}$ cycloalkane, 3- to 10-membered heterocycloalkane, $C_{6\sim10}$ aromatic ring, 5- to 10-membered aromatic heterocycle, 5- to 12-membered spiro ring, 5- to 12-membered spiro heterocycle, 5- to 12-membered bridged ring, and 5- to 12-membered bridged heterocycle; wherein the cycloalkane, heterocycloalkane, aromatic ring, aromatic heterocycle, spiro ring, spiro heterocycle, bridged ring, and bridged heterocycle can be further substituted by one, two, or three kYL ;
each $R^{YL}$ is independently selected from the group consisting of hydrogen, halogen, cyano, nitro, $C_{1\sim6}$ alkyl, halogen-substituted $C_{1\sim6}$ alkyl, -OR, and $-N(R)_2$;
each R is independently selected from the group consisting of hydrogen, halogen, $-C_{1\sim6}$ alkyl, halogen-substituted $-C_{1\sim6}$ alkyl, $-C_{0\sim2}$ alkylene-($C_{3\sim10}$ carbocyclic group), and $-C_{0\sim2}$ alkylene-(3-to 10-membered heterocycloalkyl).

**16.** The compound according to any one of claims 1 to 15, wherein Y is selected from

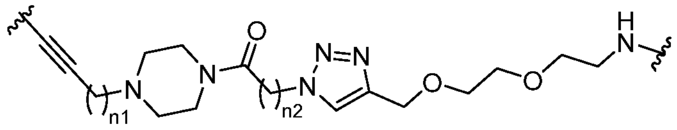
,

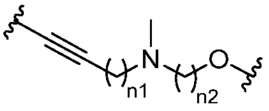
,
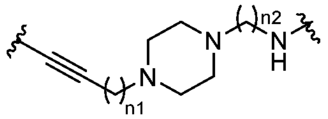
,
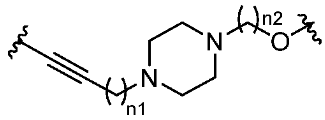
,

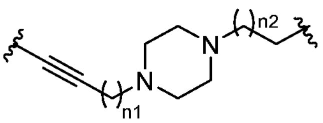
,
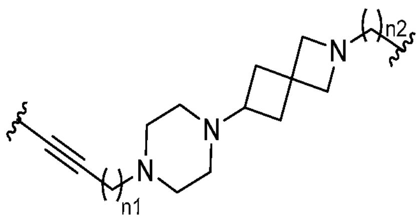
,
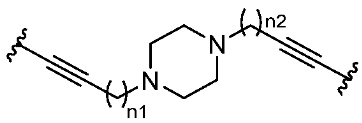
,

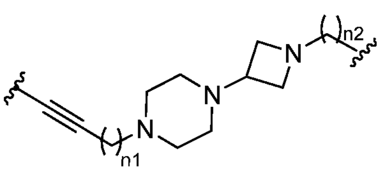
,
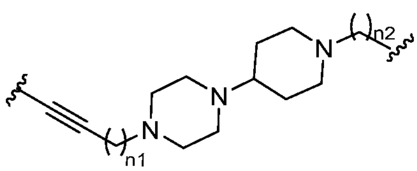
,

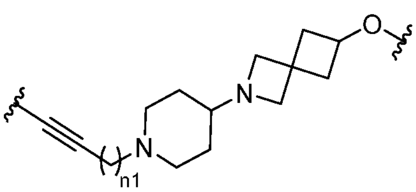
,
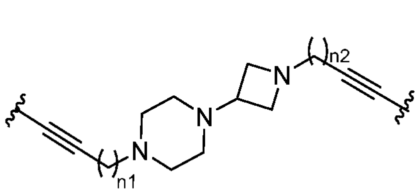
,

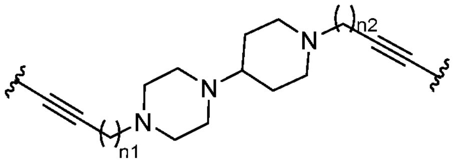
,
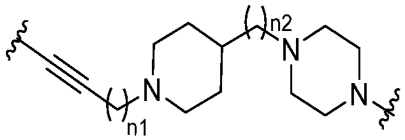
,

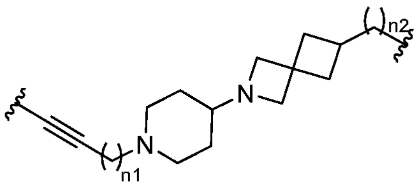
,
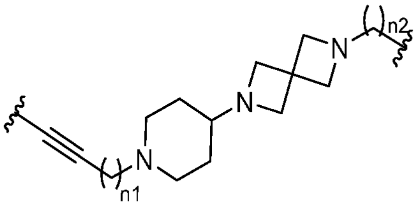
,

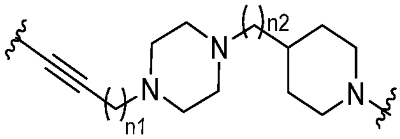
,
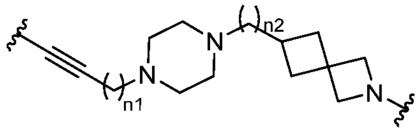
,
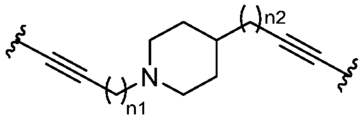
,

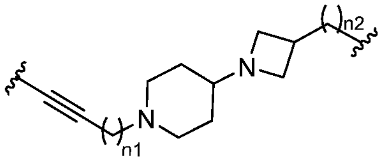
,
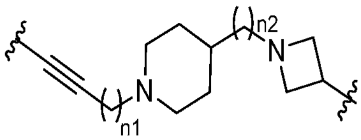
,
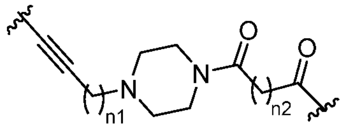
,

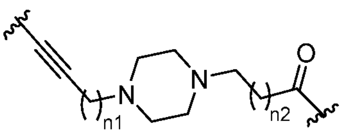
,
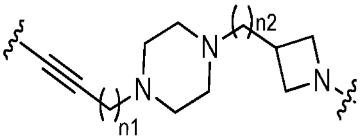
,

or

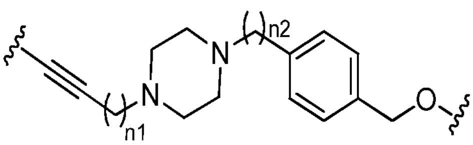
;

wherein, n1 in each structural fragment is independently an integer selected from 0 to 10, n2 in each structural fragment is independently an integer selected from 0 to 10; preferably, n1 in each structural fragment is independently selected from 0, 1, 2, 3, 4, 5, 6, 7, 8, and n2 in each structural fragment is independently selected from 0, 1, 2, 3, 4, 5, 6, 7, 8.

**17.** The compound according to any one of claims 1 to 16, wherein: Y is selected from

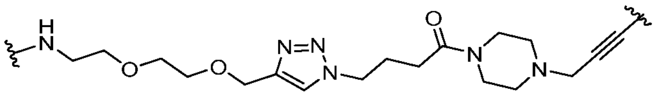
,

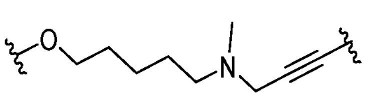 , 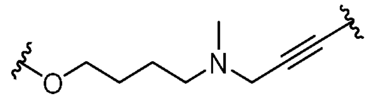 , 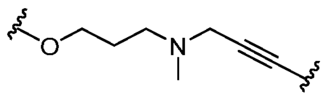 ,

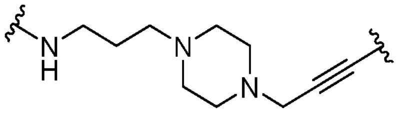 , 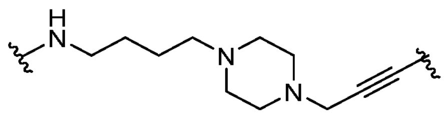 ,

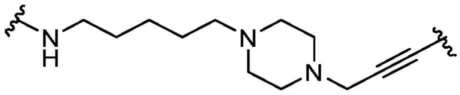 , 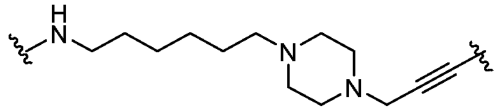 ,

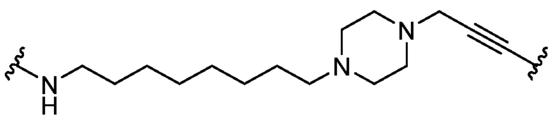 , 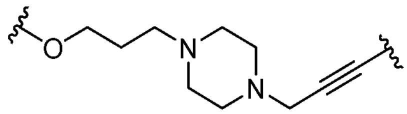 ,

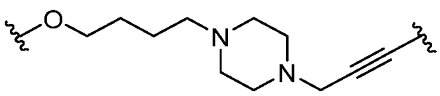 , 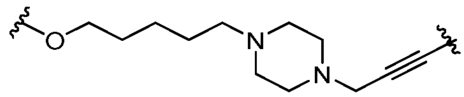 ,

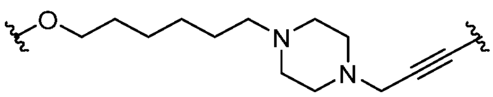 , 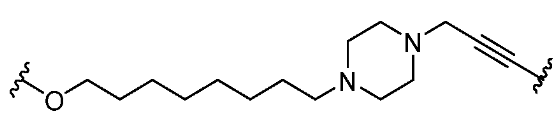 ,

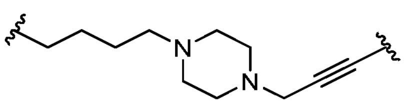 , 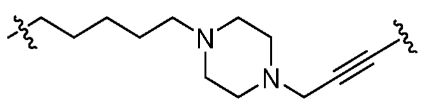 ,

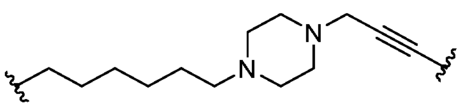 , 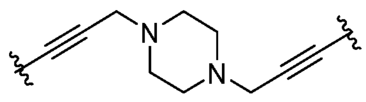 ,

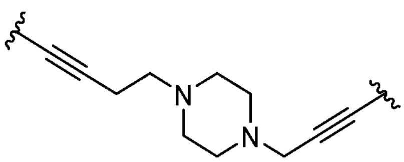 , 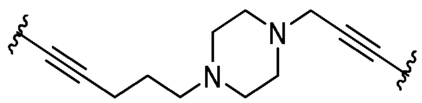 ,

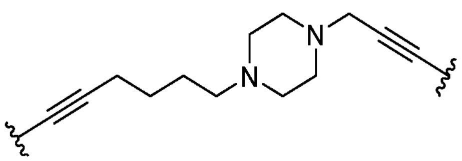 , 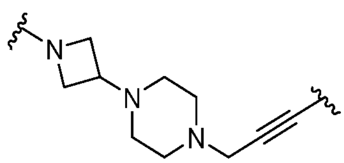 , 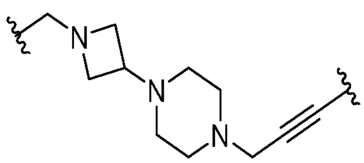 ,

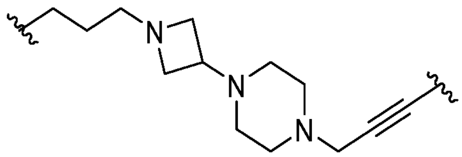,
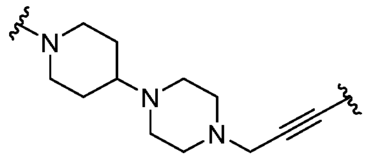,

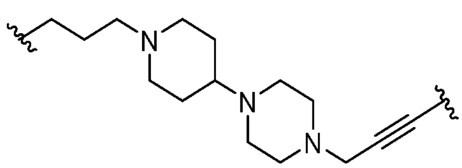,
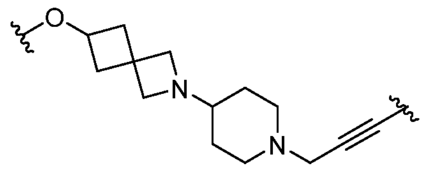,

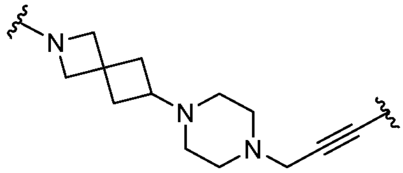,
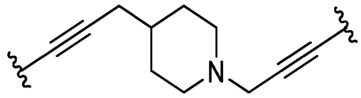,
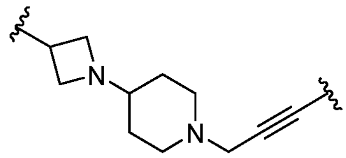,

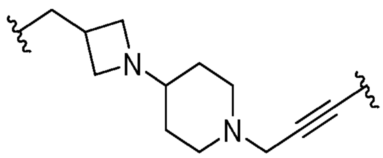,
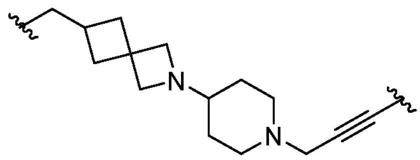,

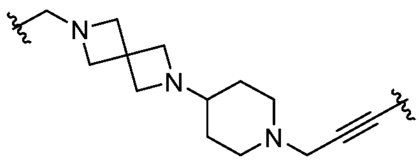,
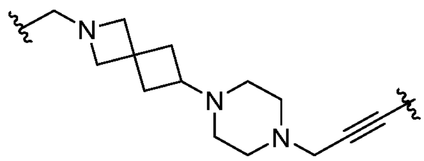,

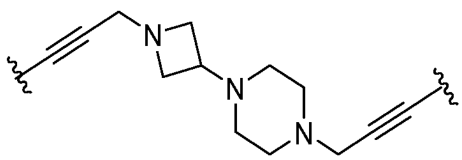,
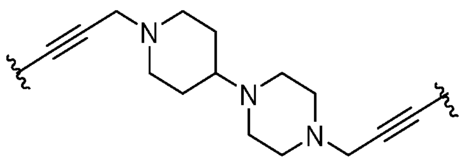,

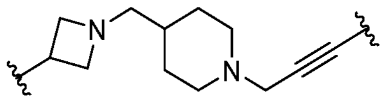,
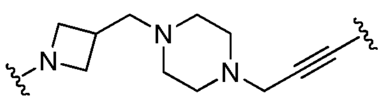,

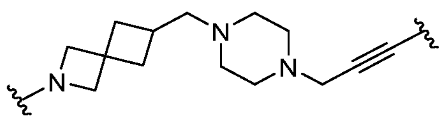,
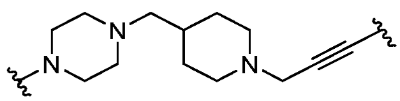,

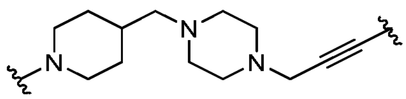,
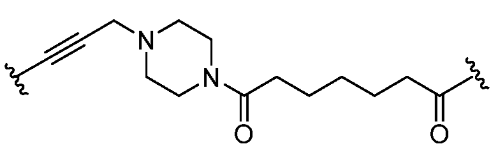,

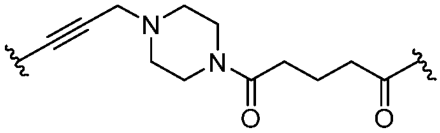,
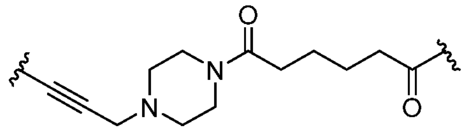,
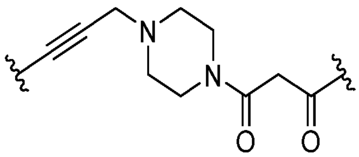,
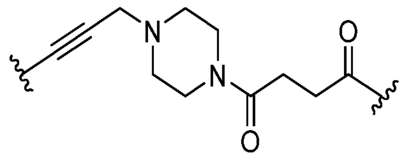,
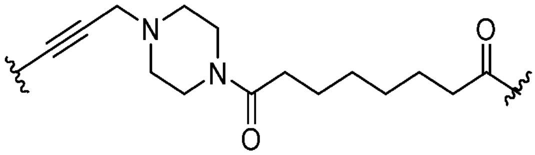,
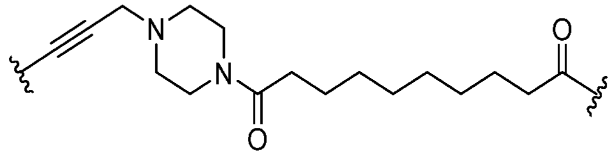,
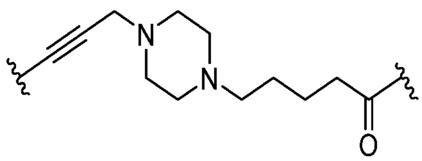,
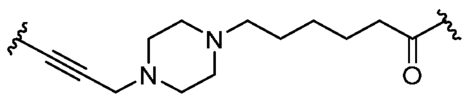,

or

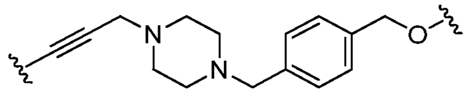.

**18.** The compound according to any one of claims 1 to 17, wherein the E3 ubiquitin-protein ligase is selected from the group consisting of CRBN, von Hippel-Lindau (VHL), XIAP, MDM2, and cIAP-1.

**19.** The compound according to any one of claims 1 to 18, wherein
Z is selected from

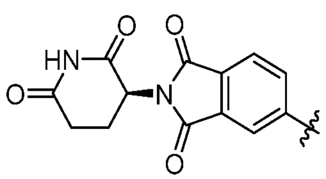,
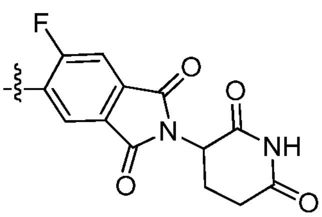,
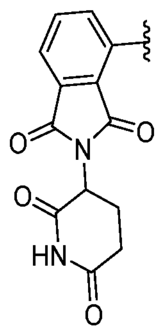,

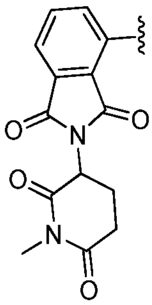, 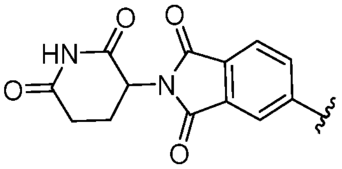, 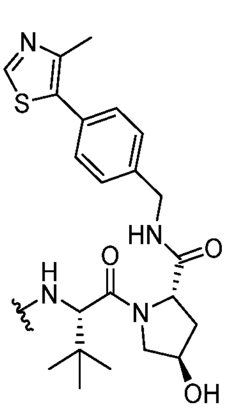, 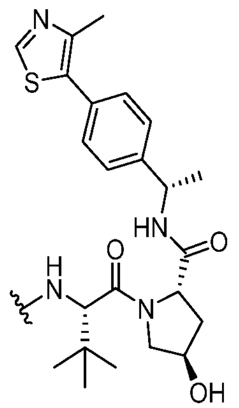, 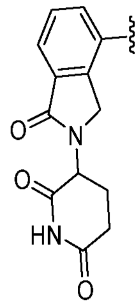,

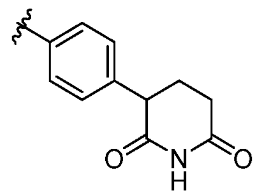, 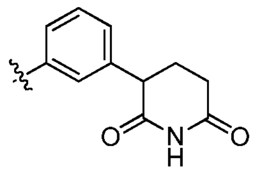, 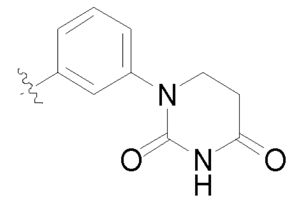, 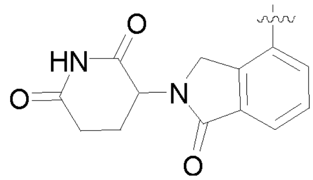,

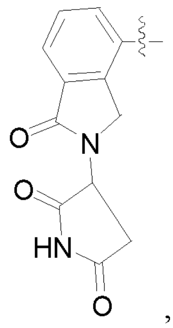, 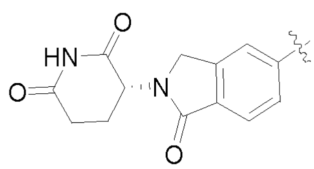, 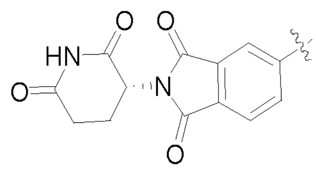 or 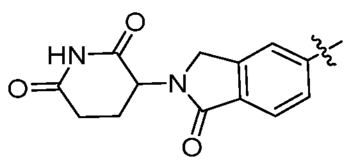.

**20.** The compound according to any one of claims 1 to 19, wherein the compound is specifically:

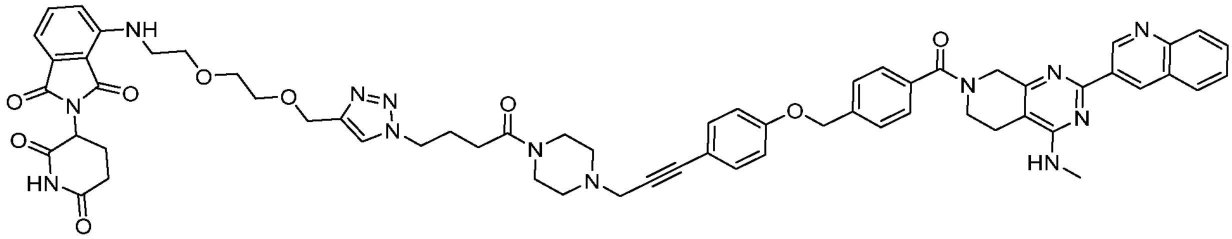,

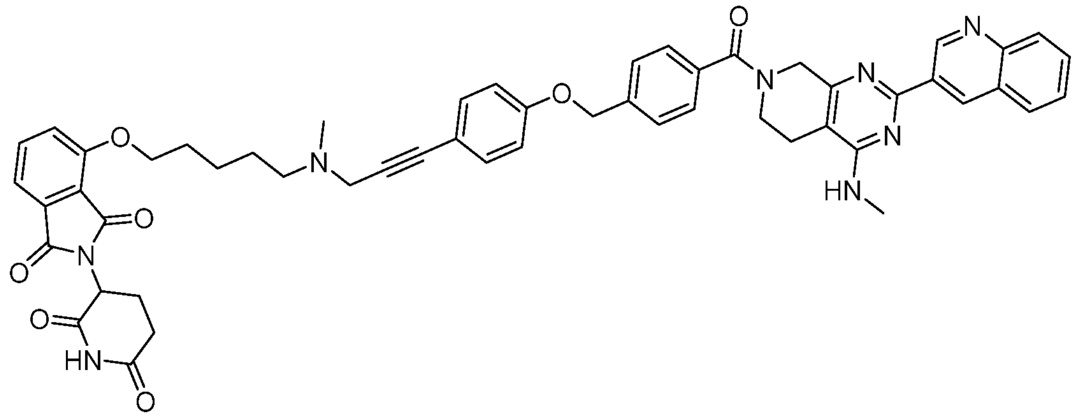

,

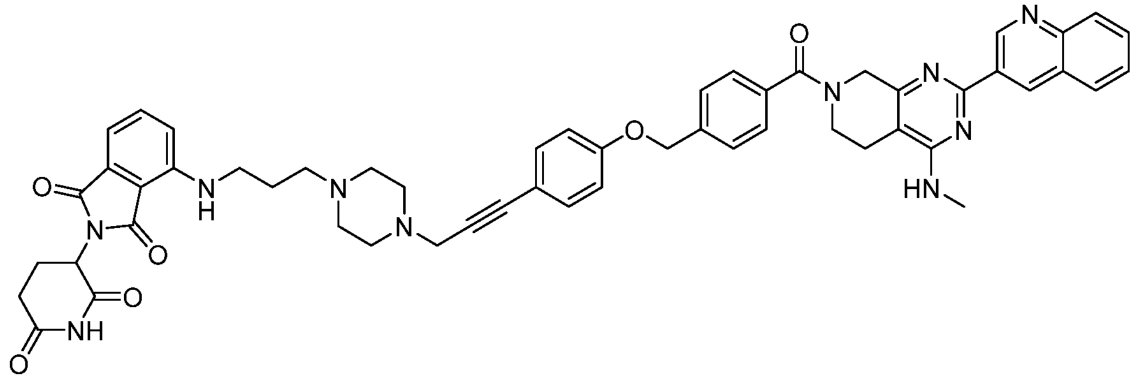

,

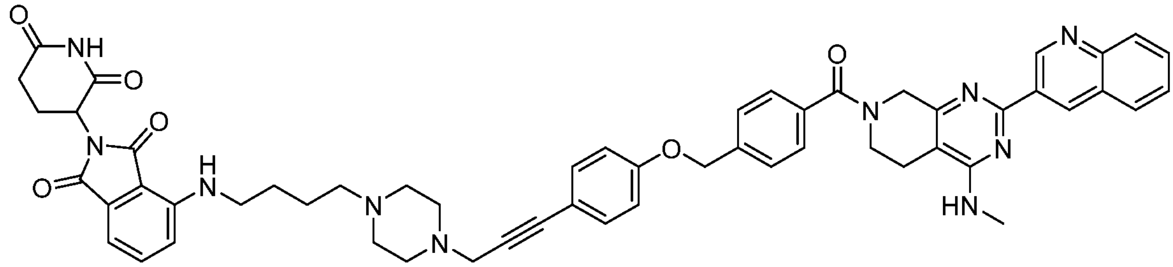

,

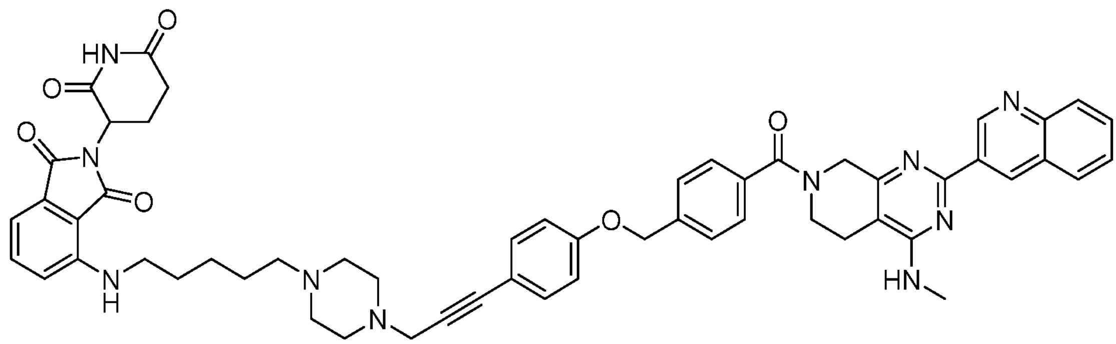

,

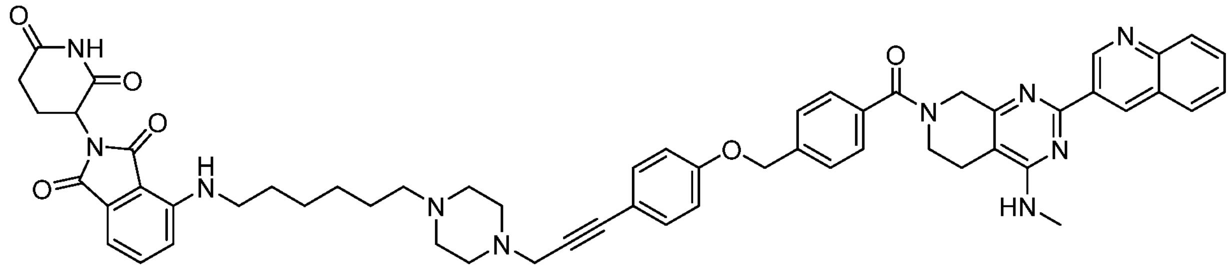

,

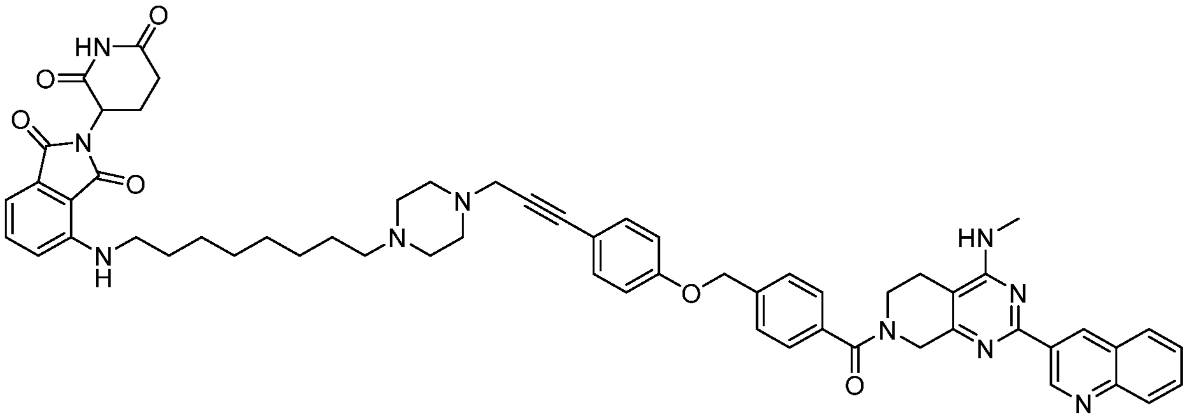

,

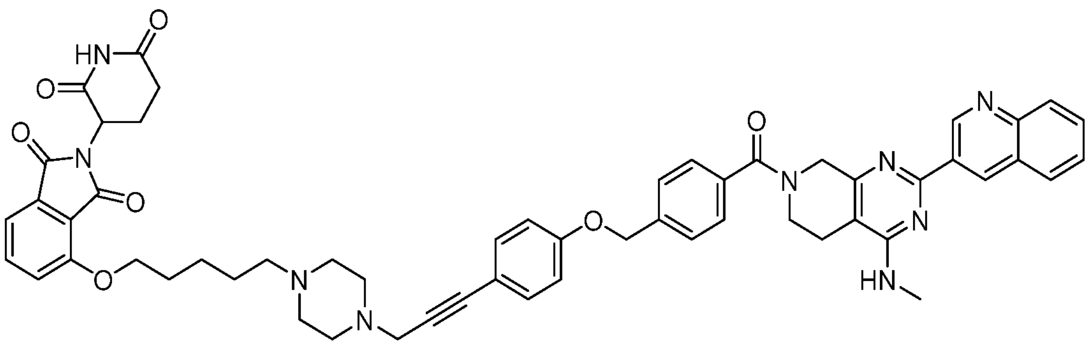

,

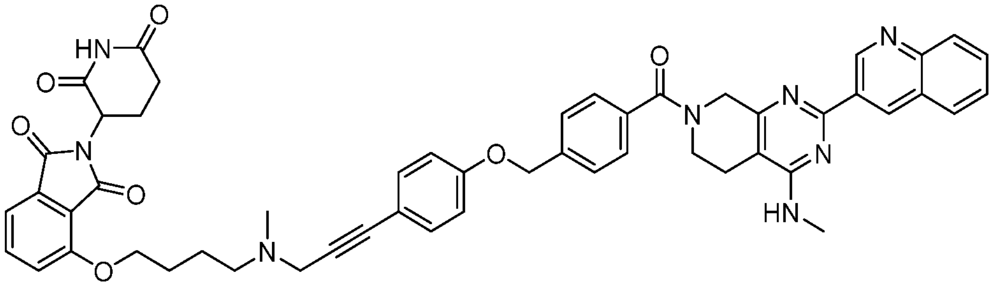

,

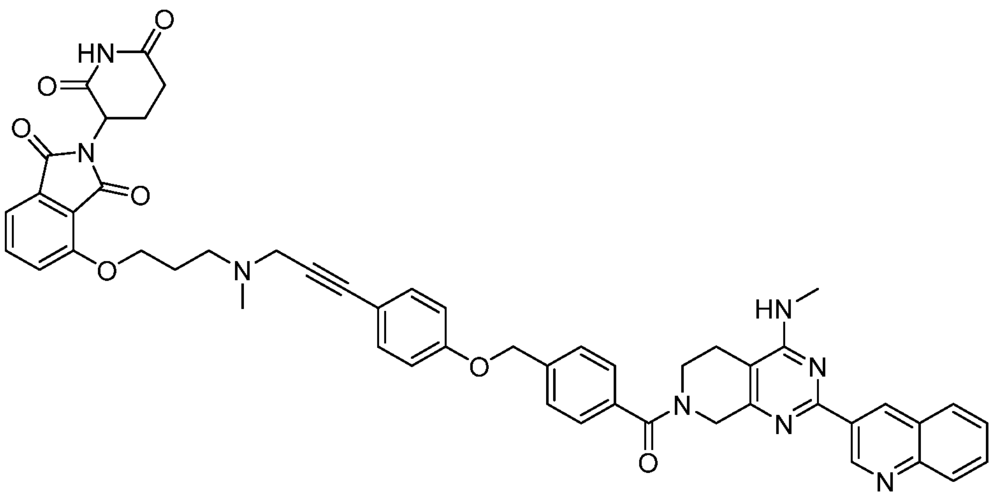

,

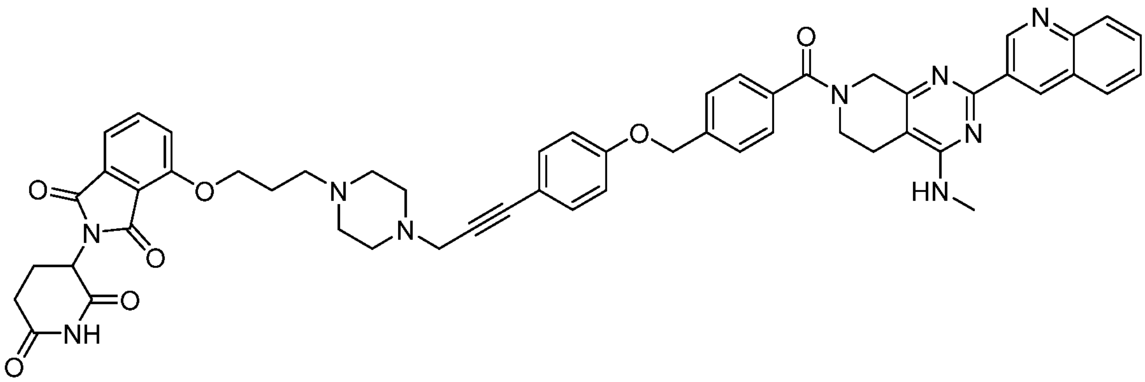

,

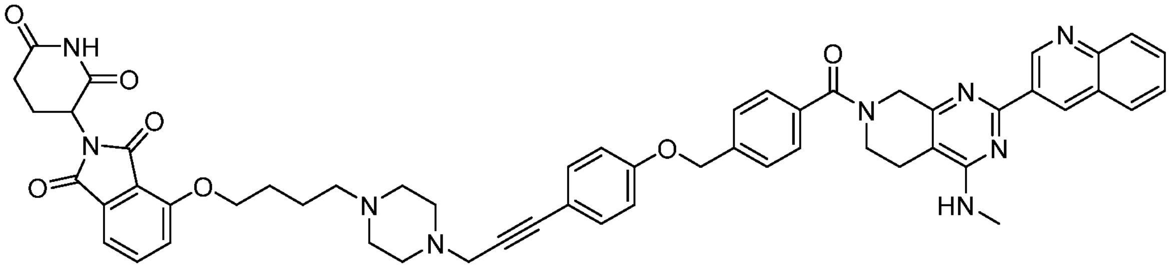

,

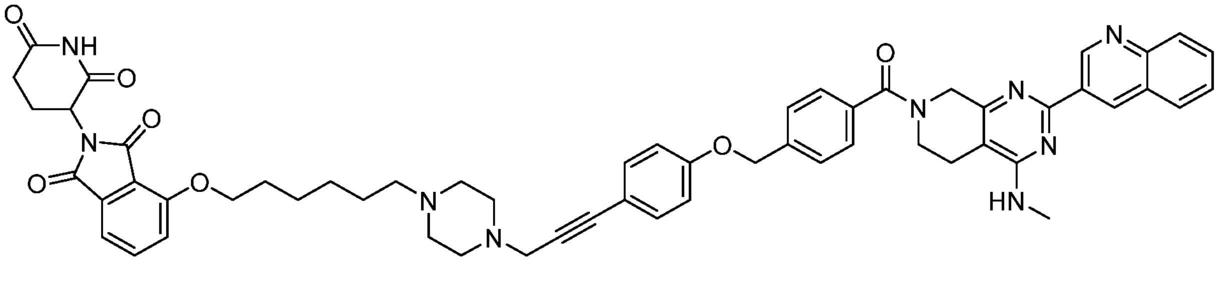

,

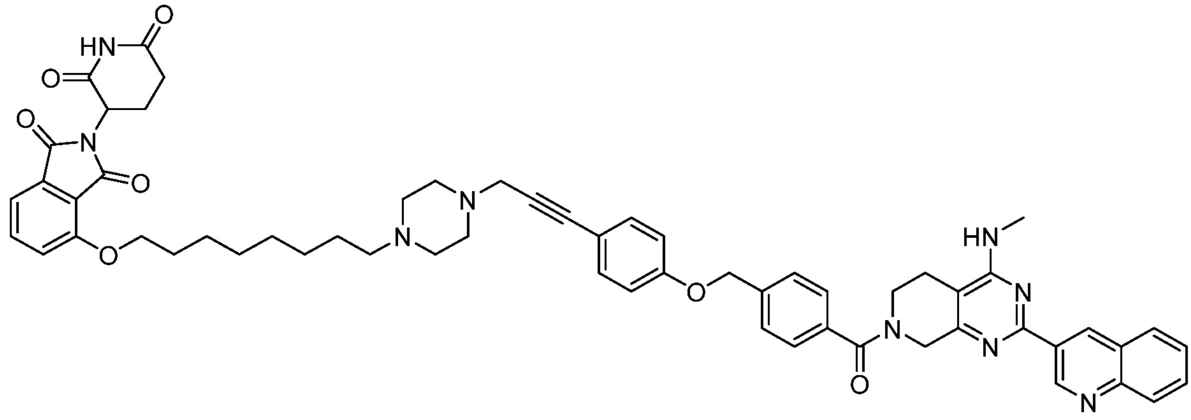

,

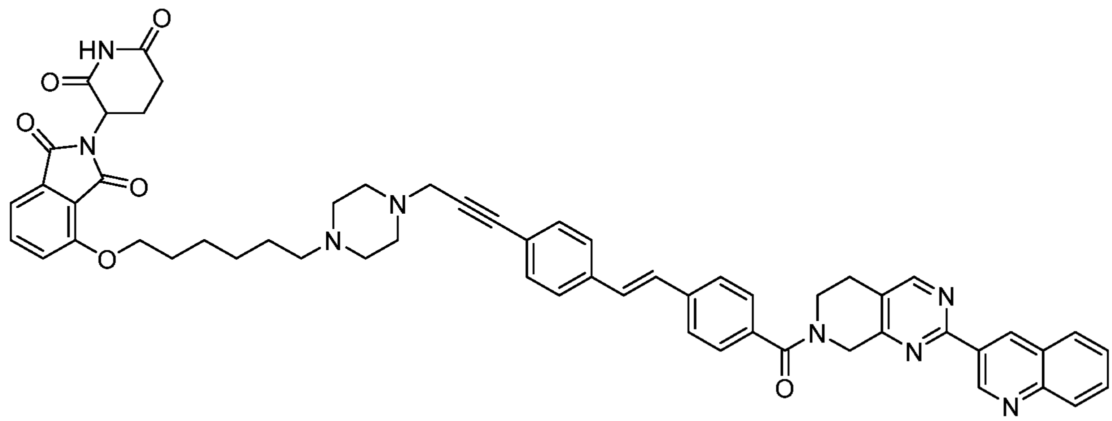

,

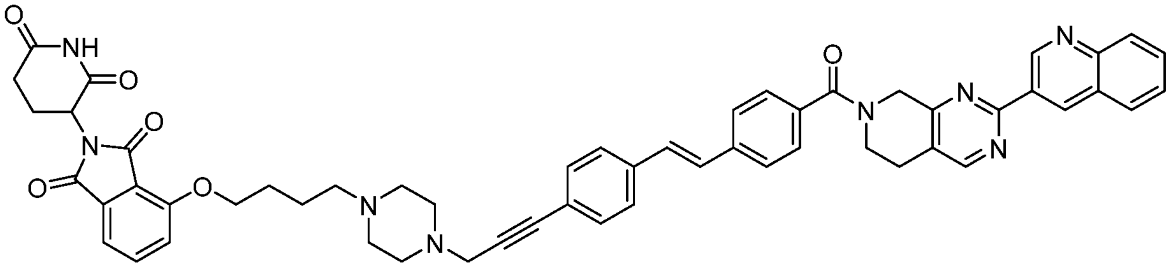

,

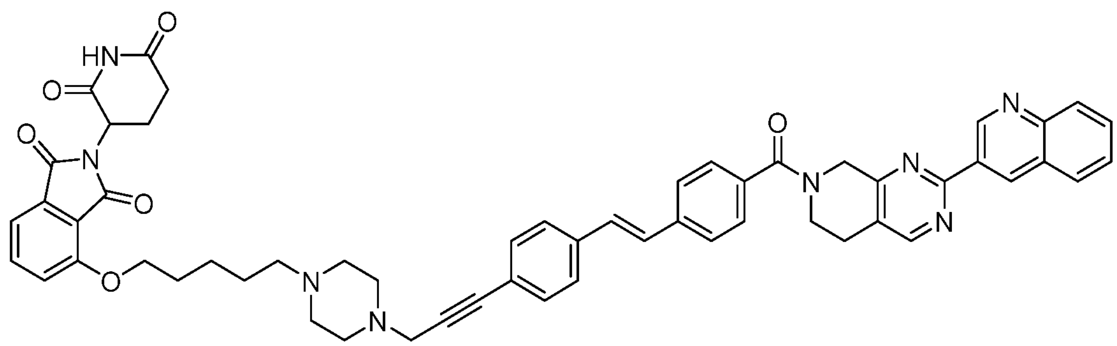

,

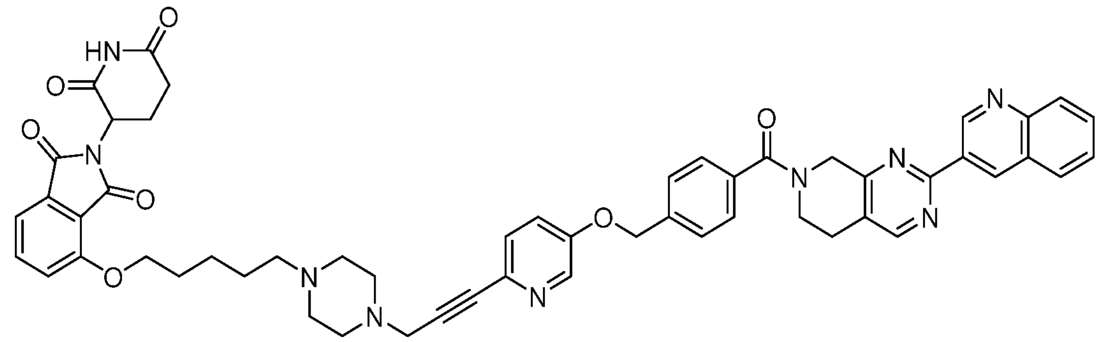

,

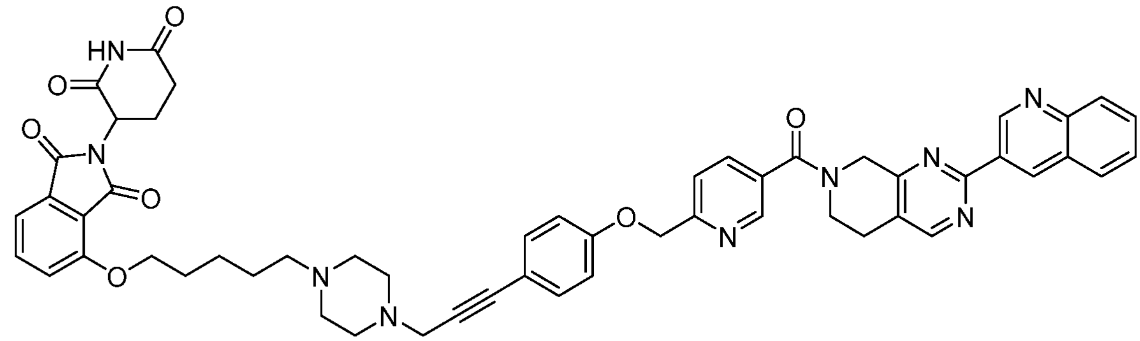

,

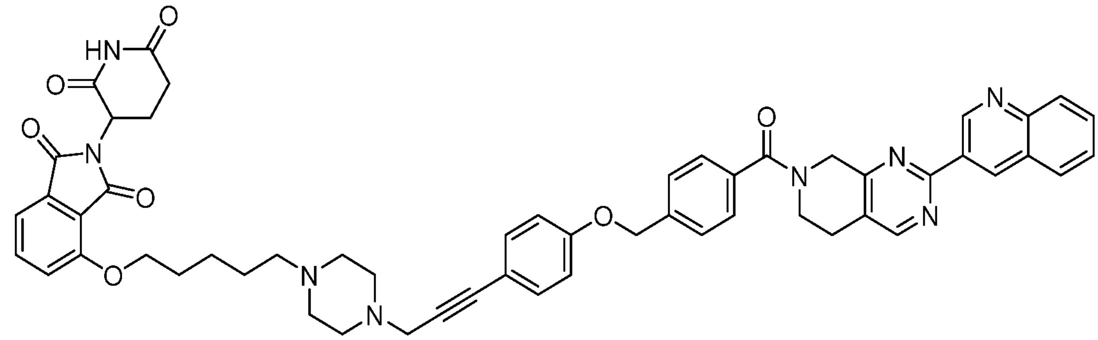

,

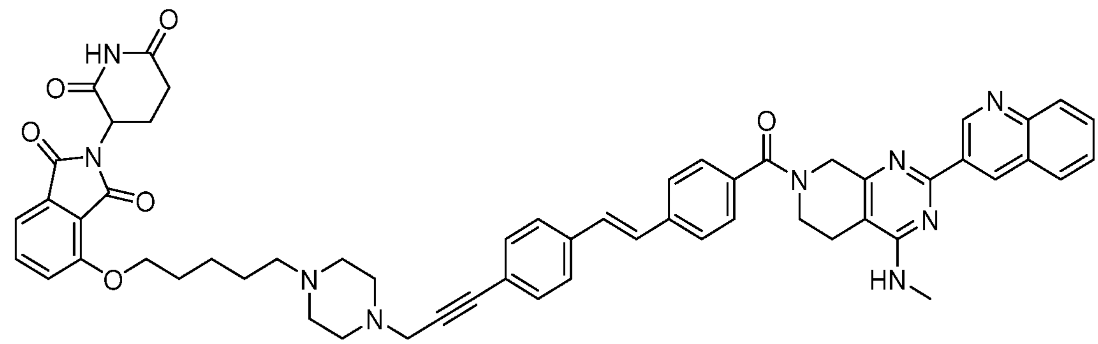

,

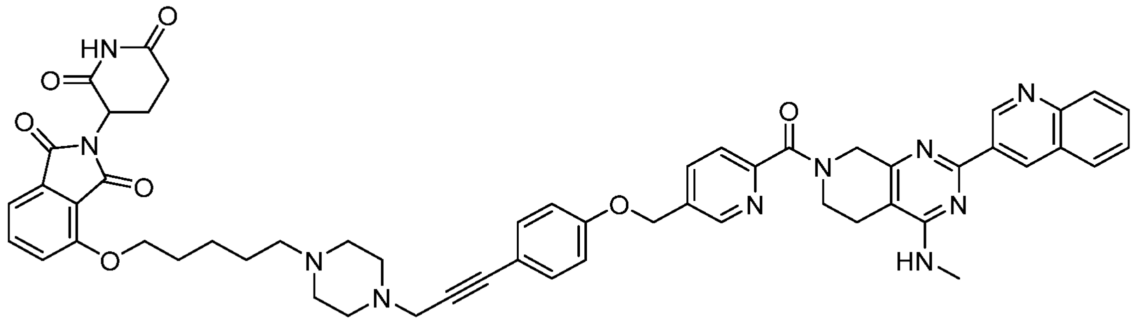

,

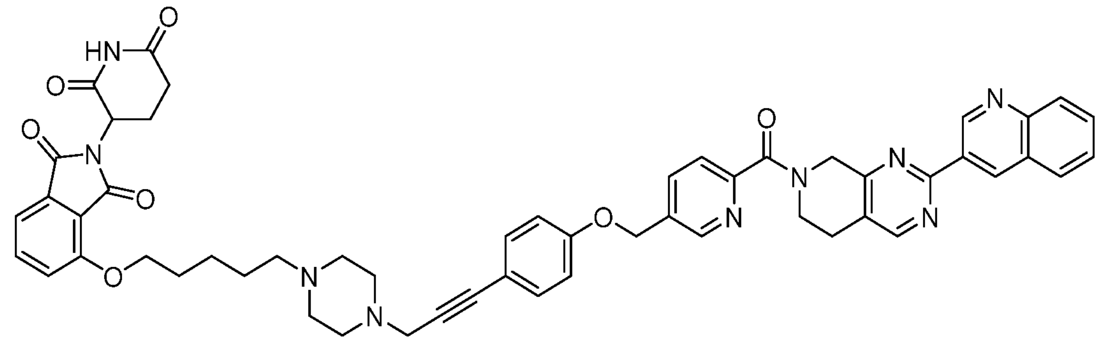

,

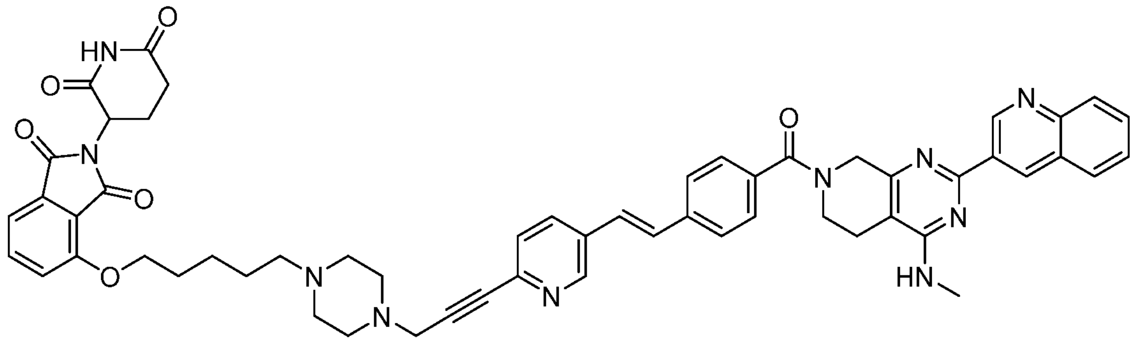

,

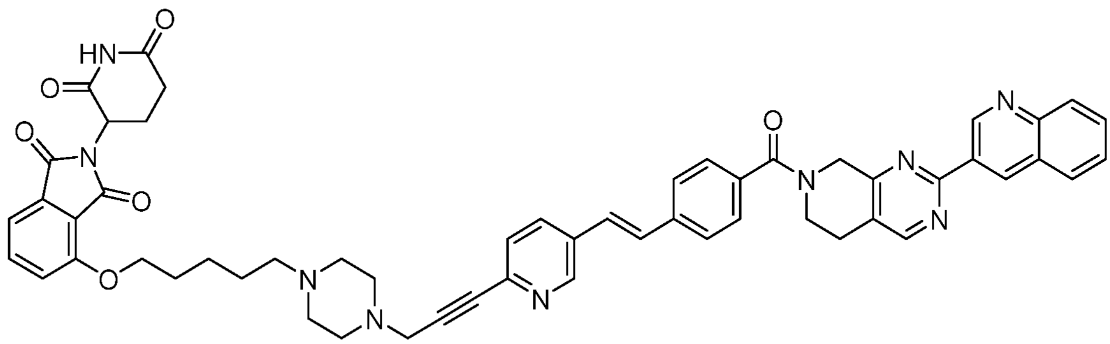

,

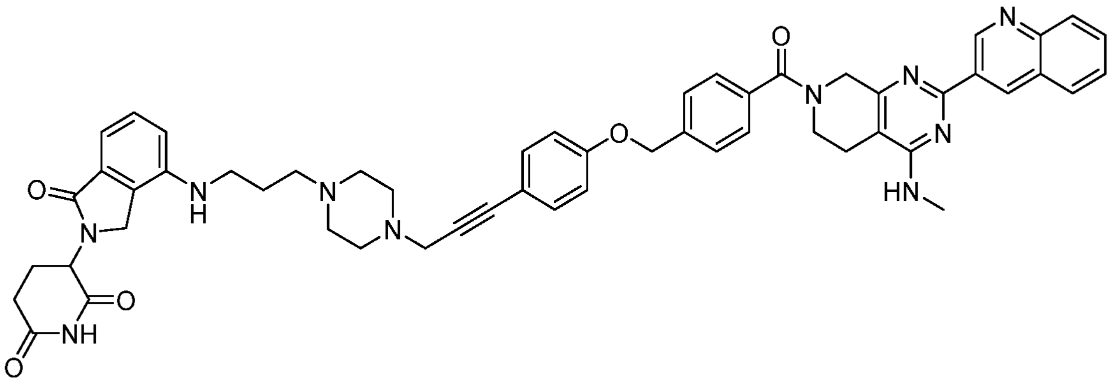

,

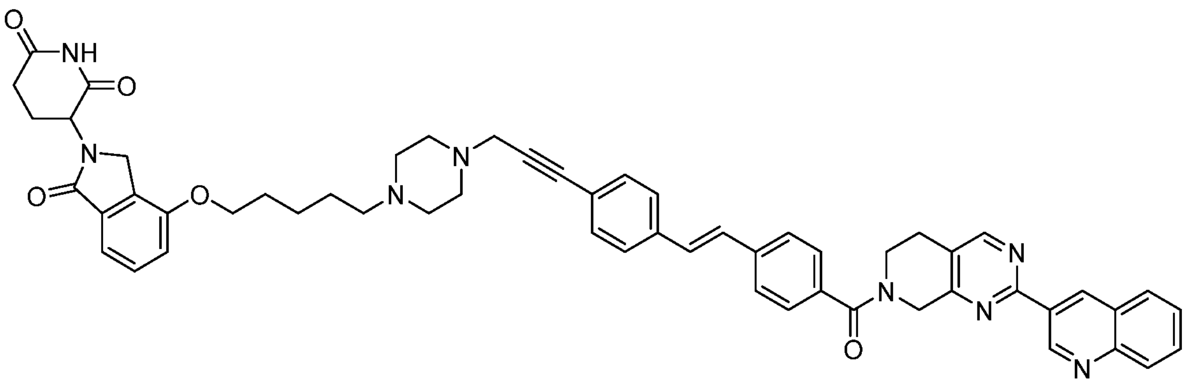

,

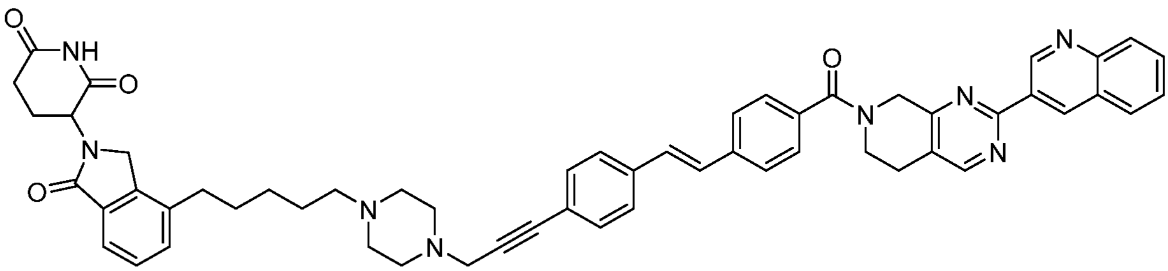

,

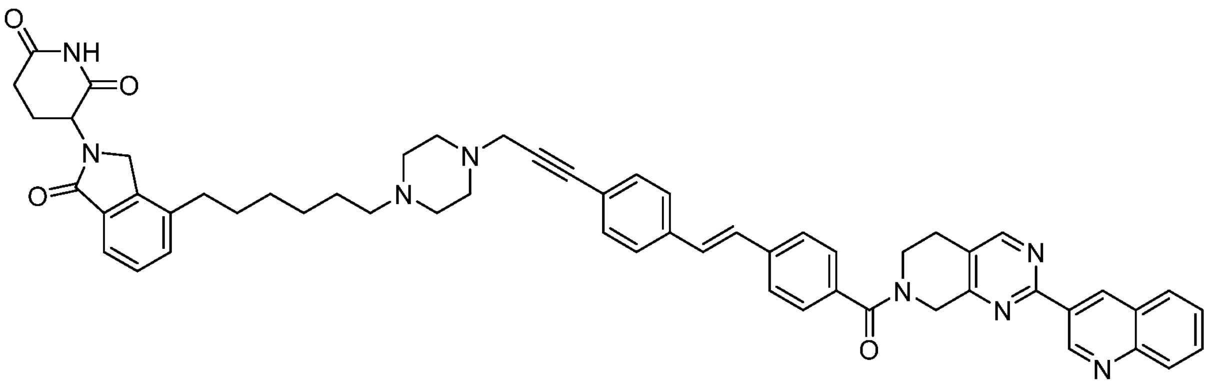

,

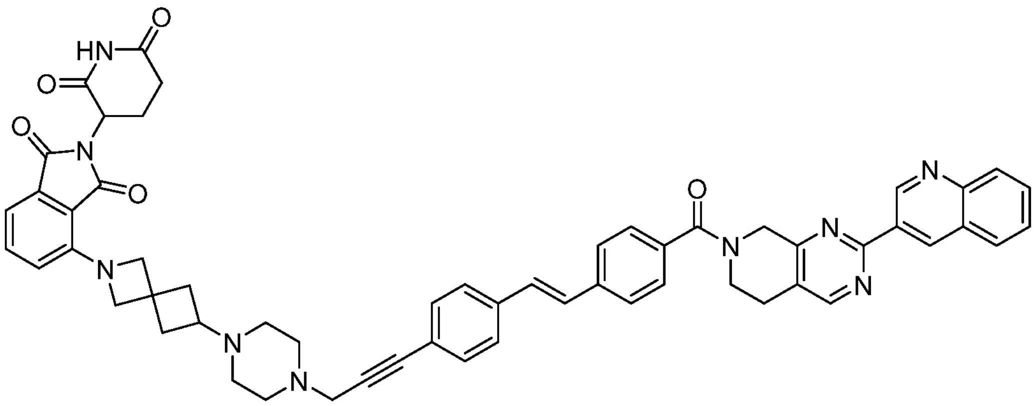

,

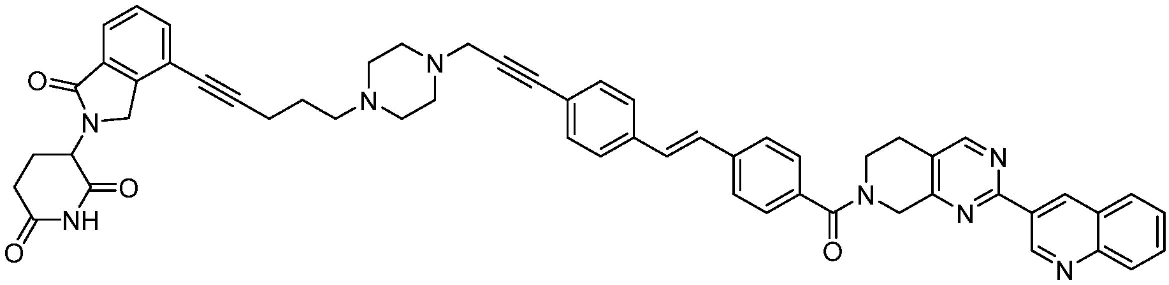

,

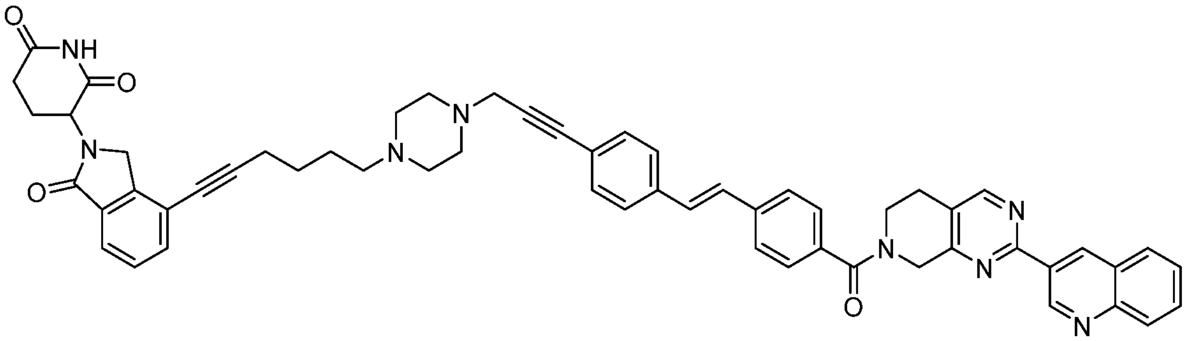

,

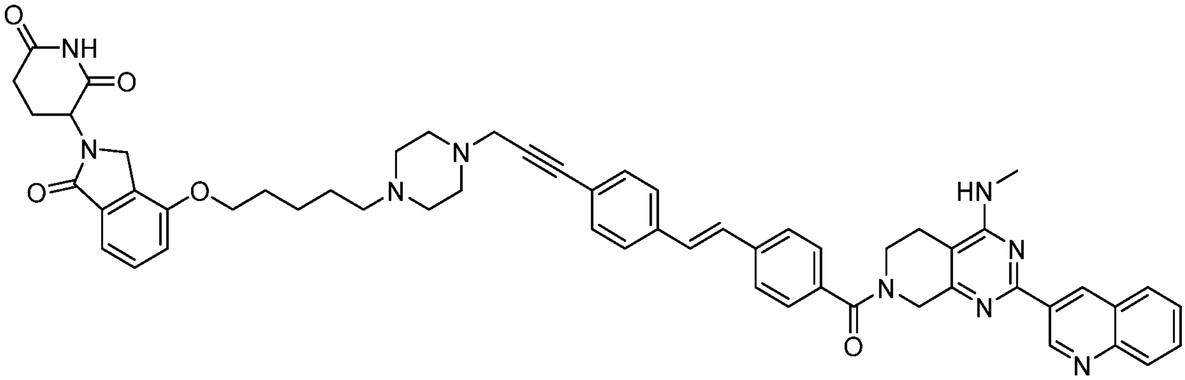

,

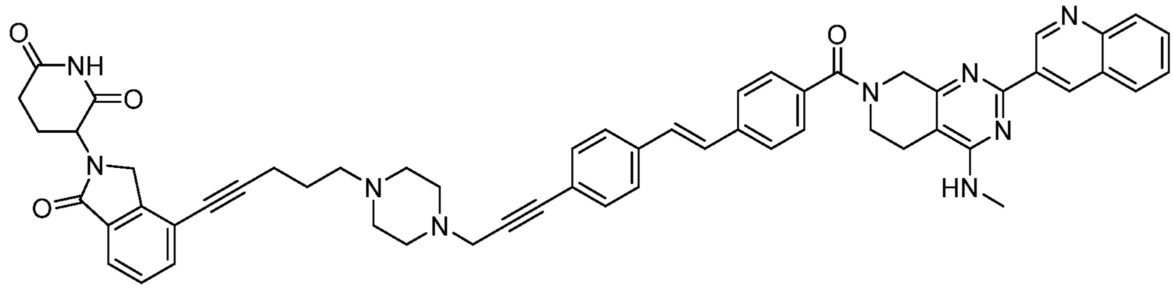

,

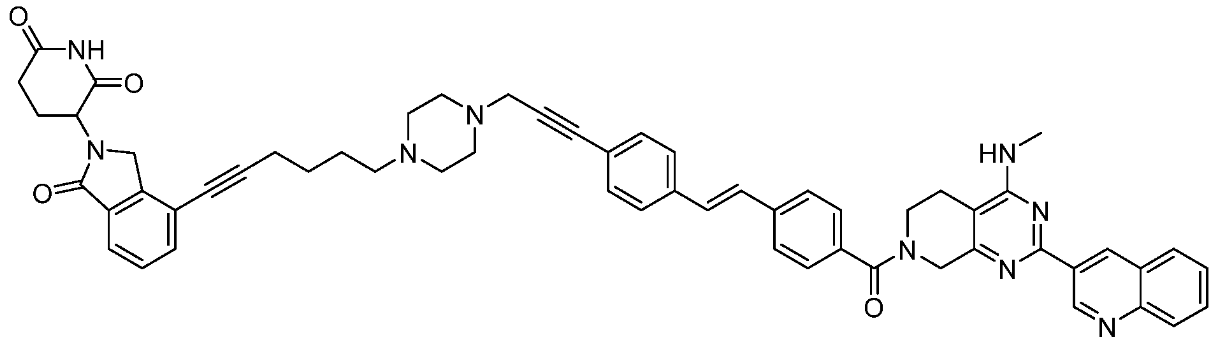

,

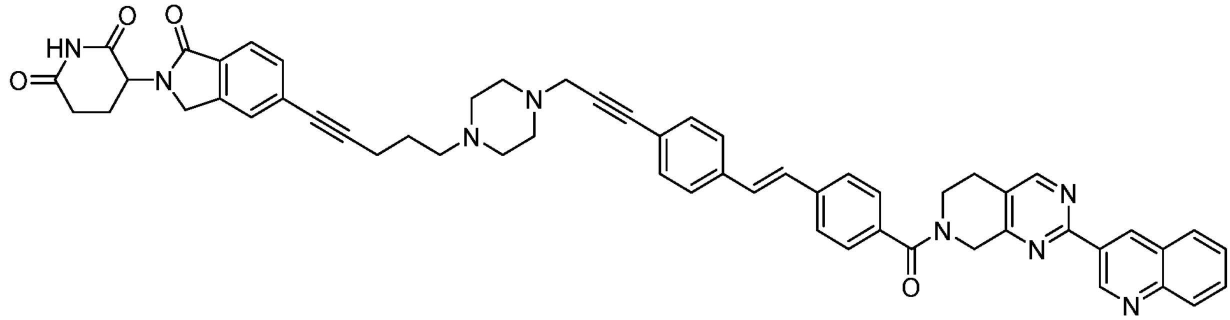

,

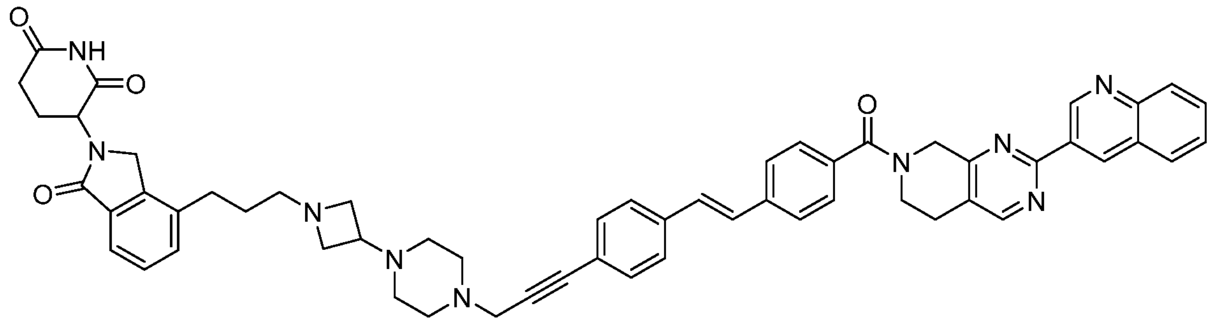

,

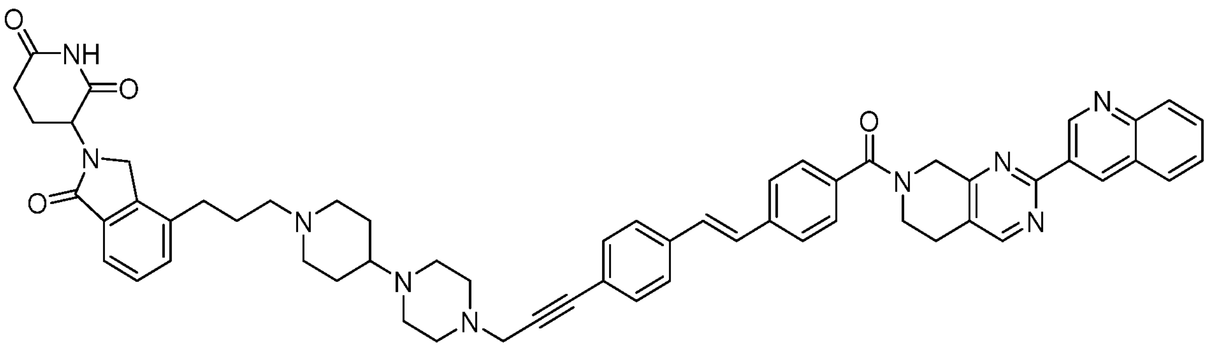

,

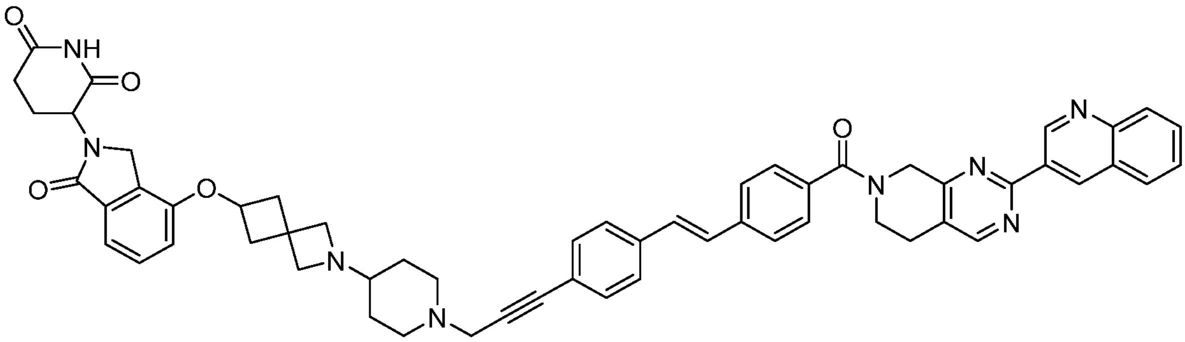

,

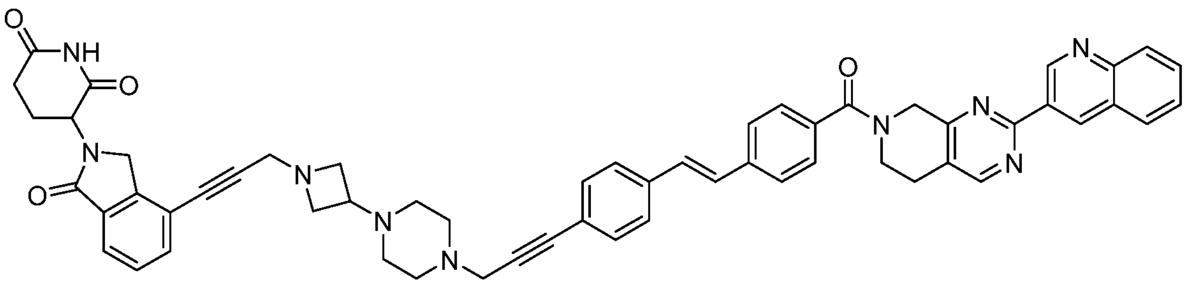

,

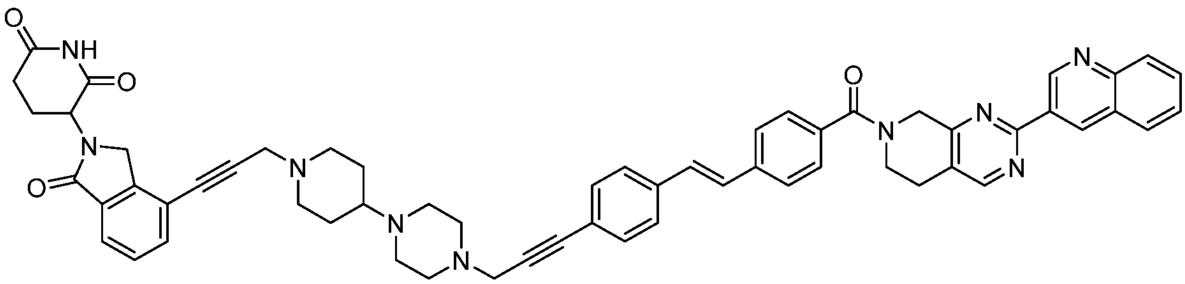

,

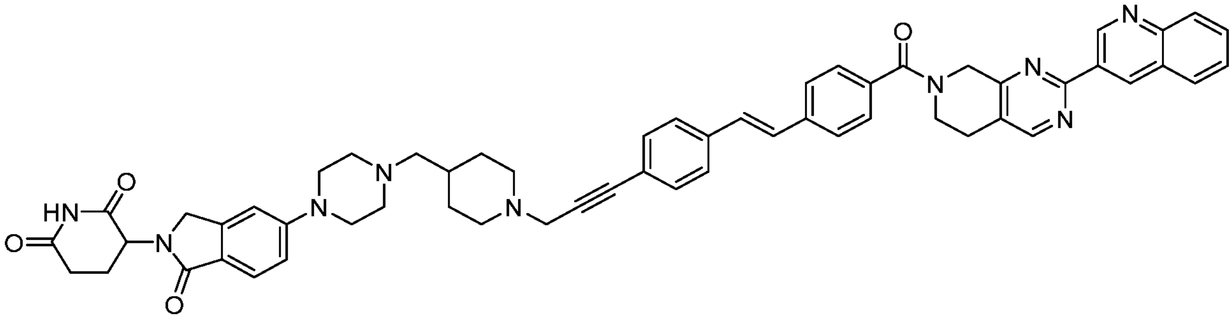

,

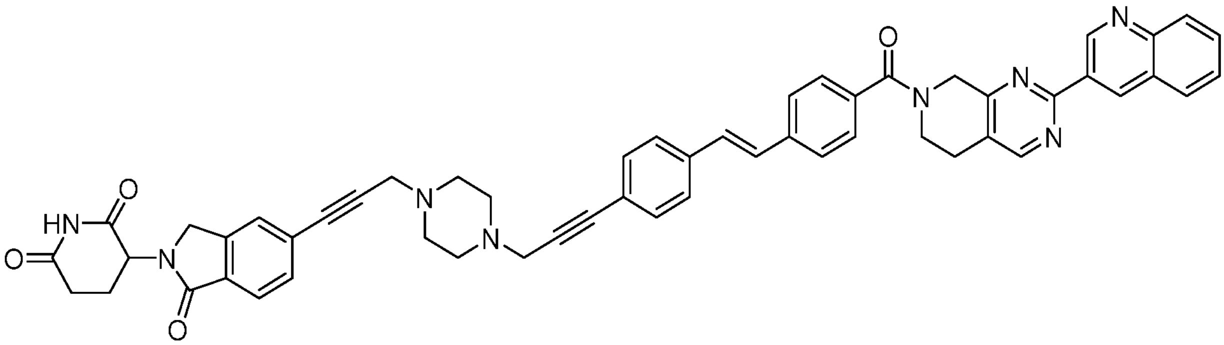

,

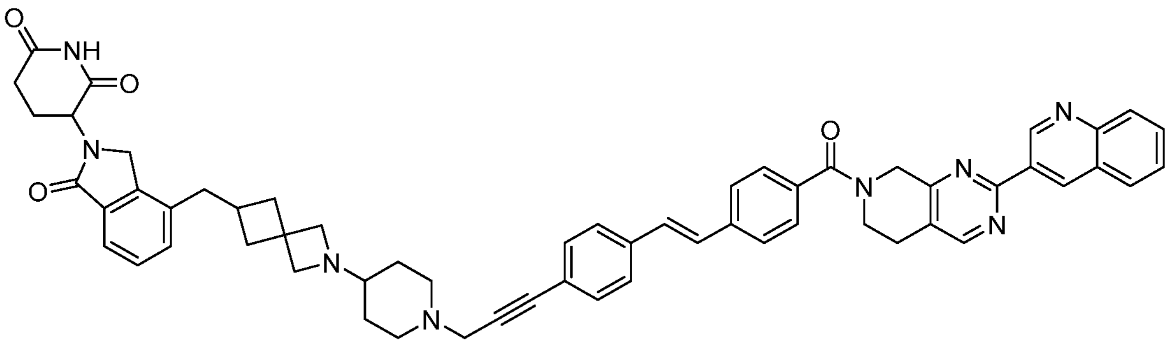

,

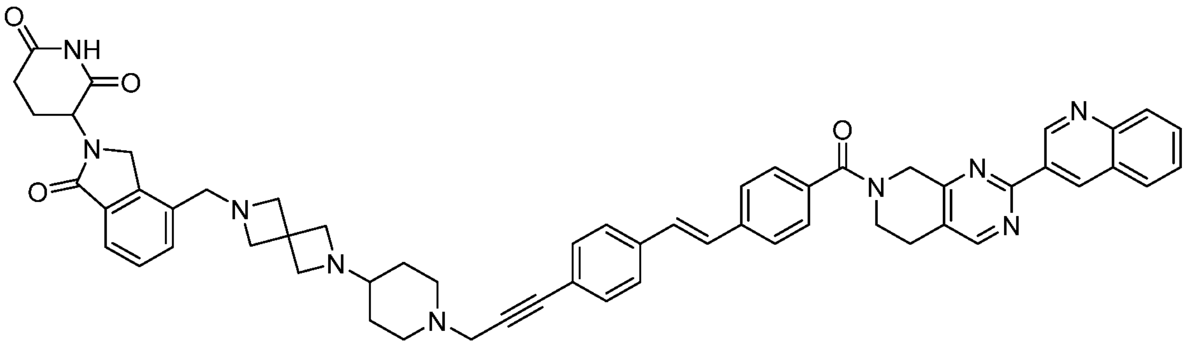

,

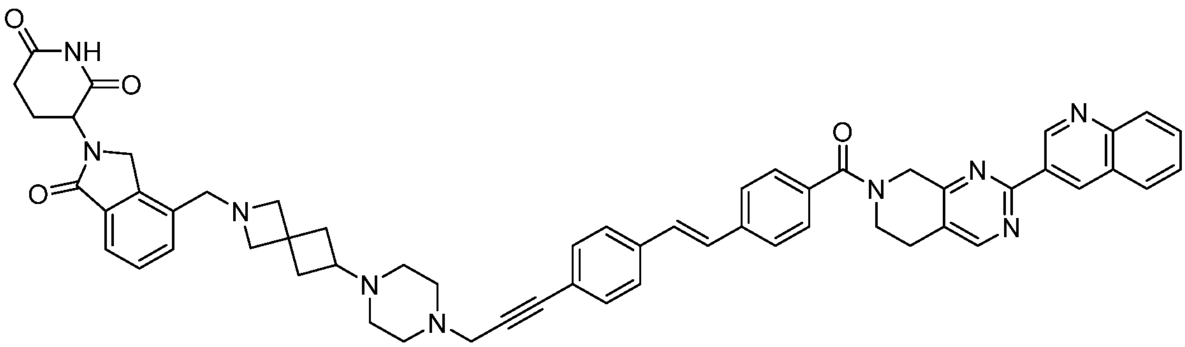

,

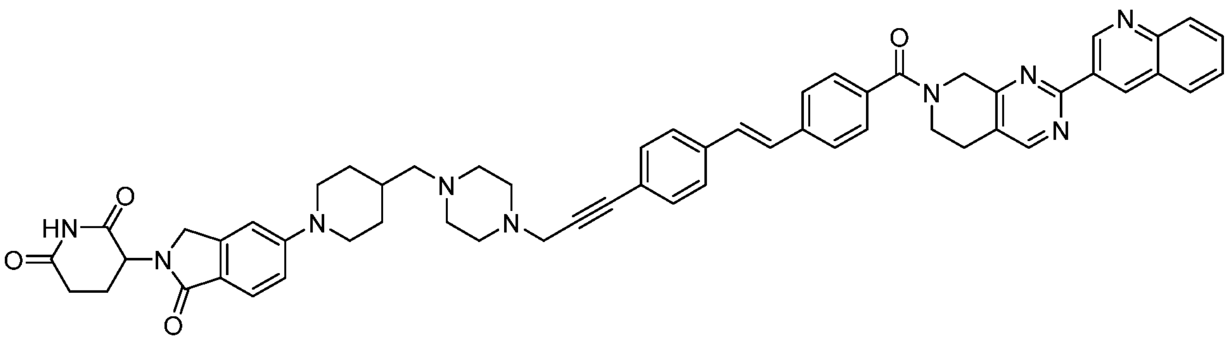

,

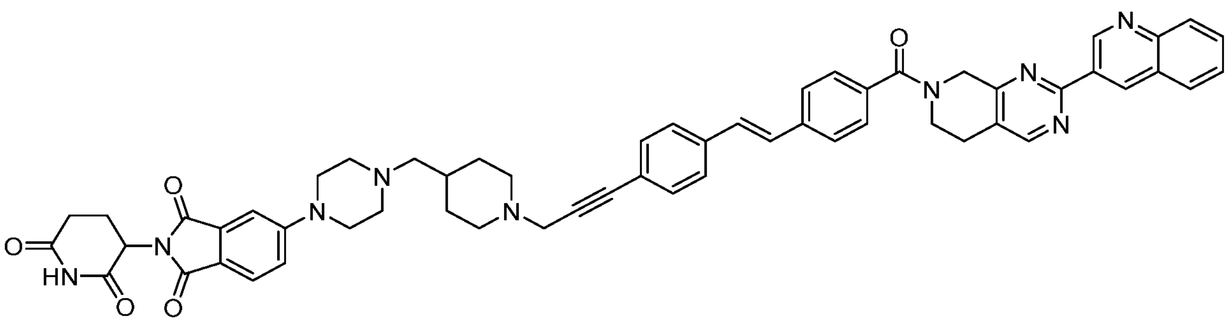

,

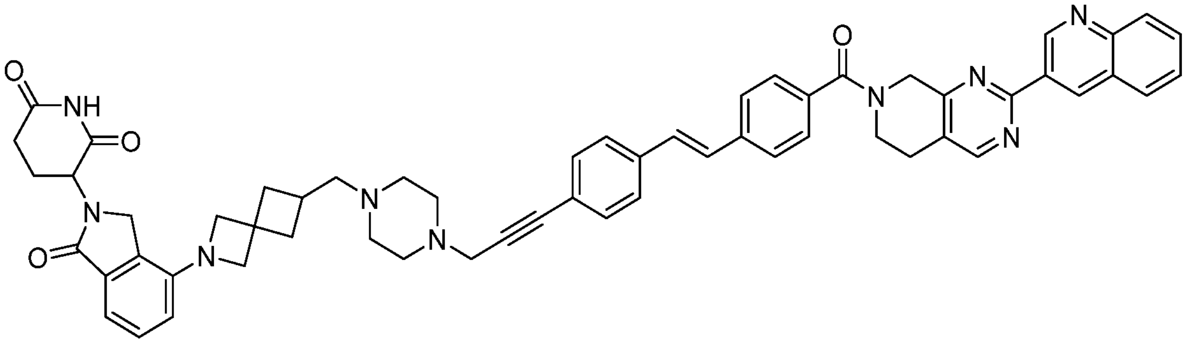

,

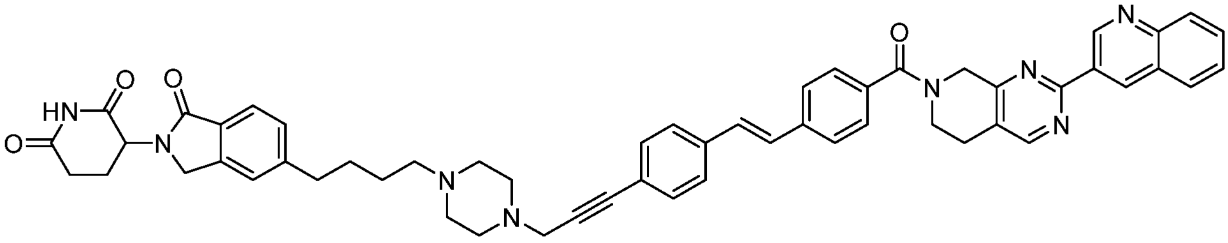

,

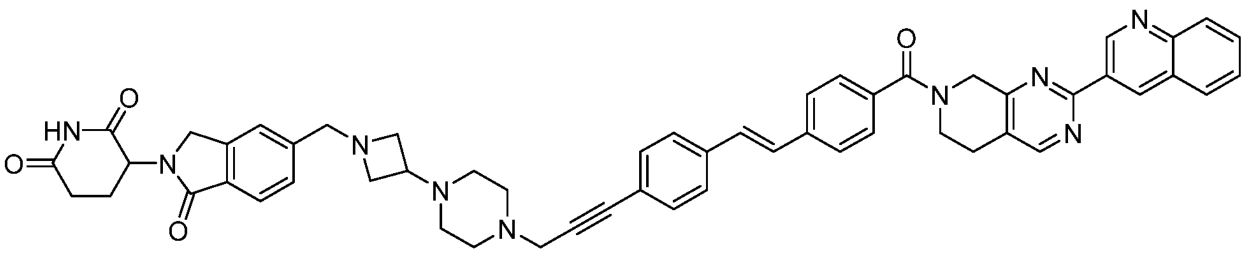

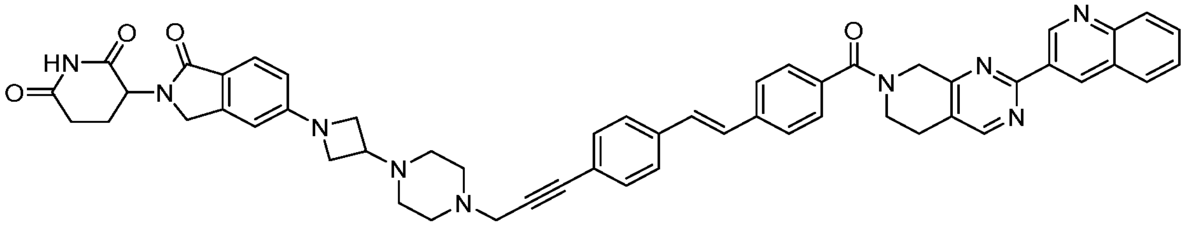

, ,

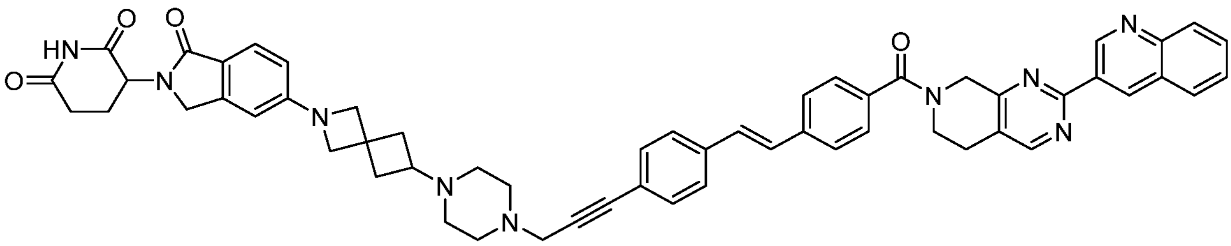

,

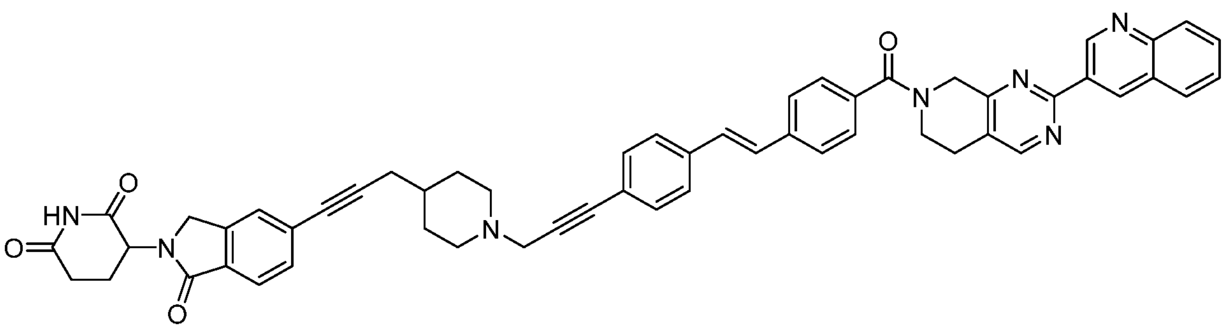

,

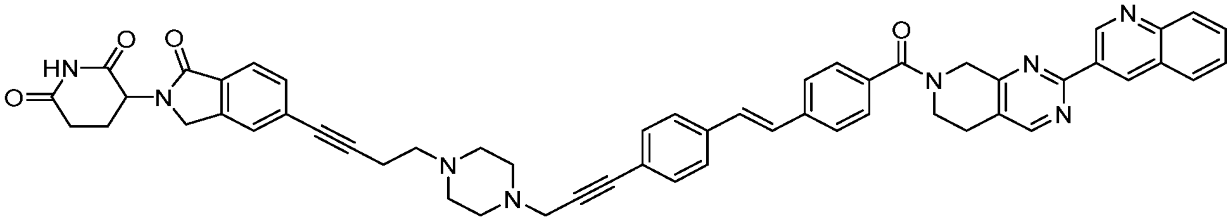

,

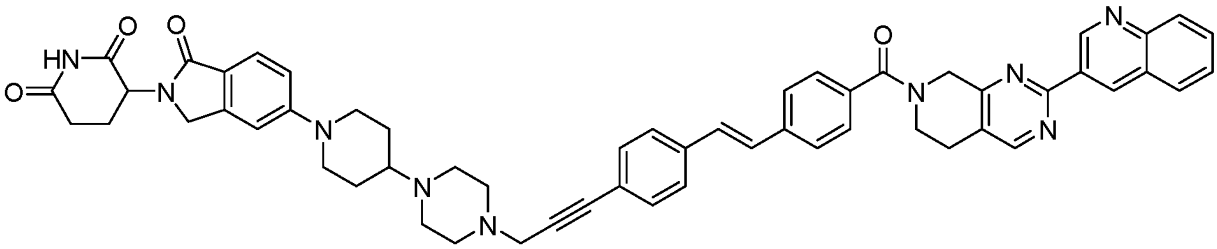

,

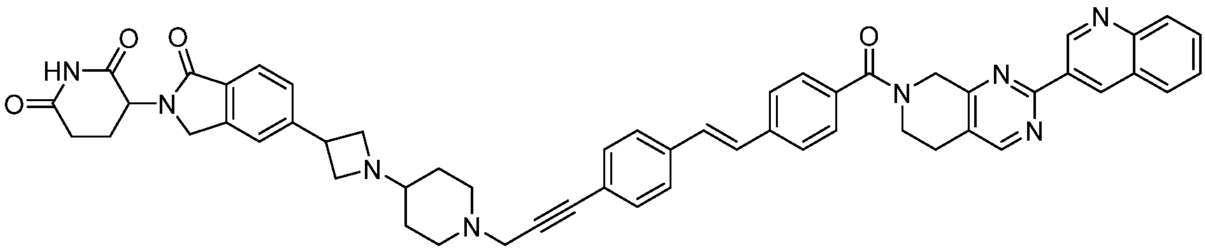

,

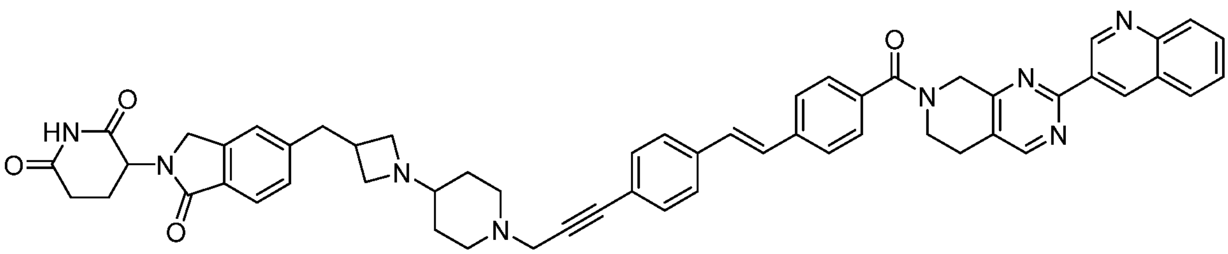

,

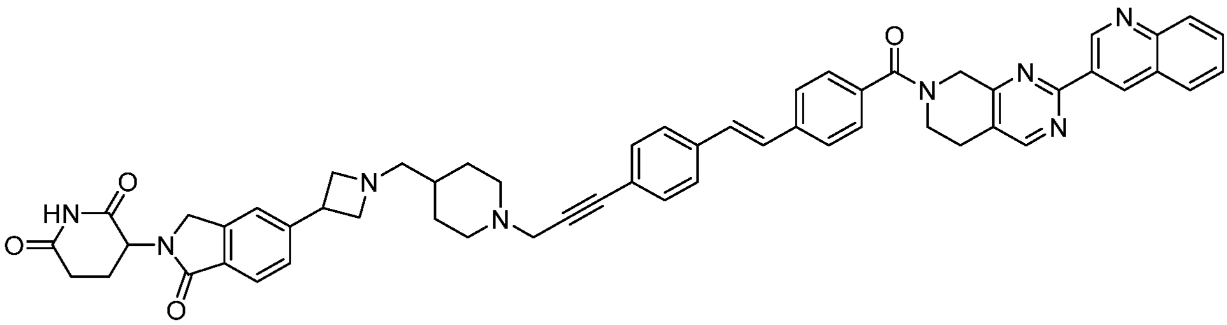

,

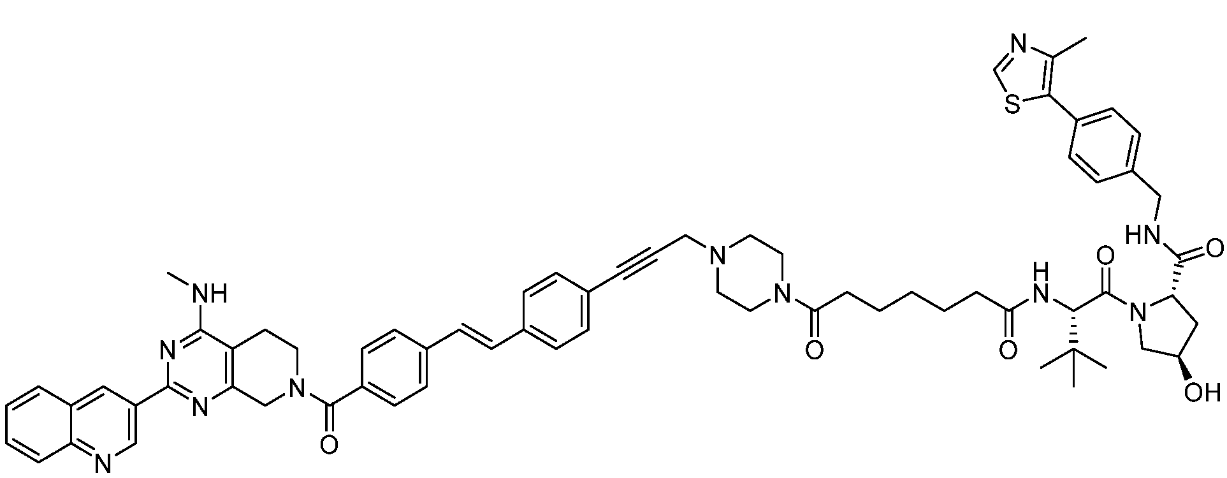

,

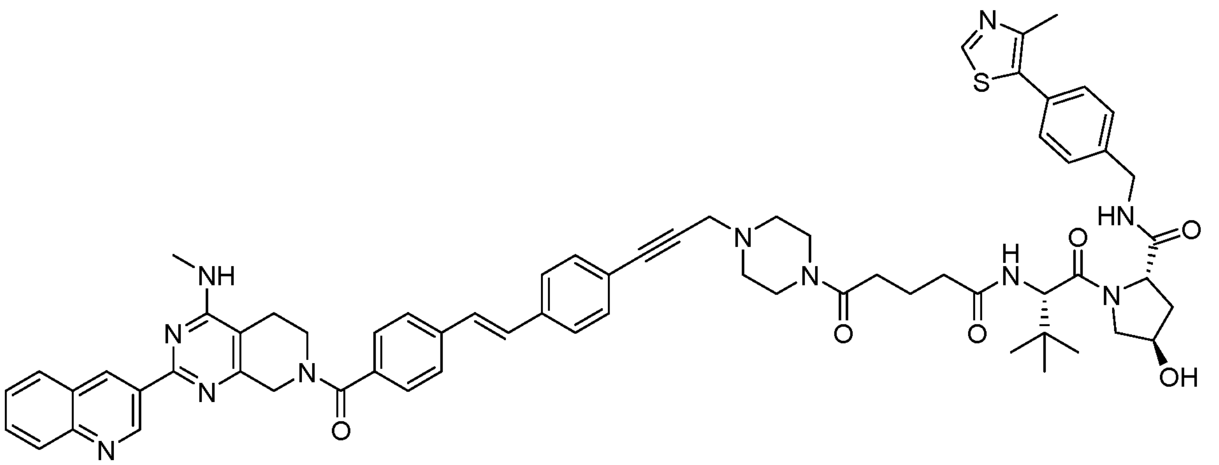

,

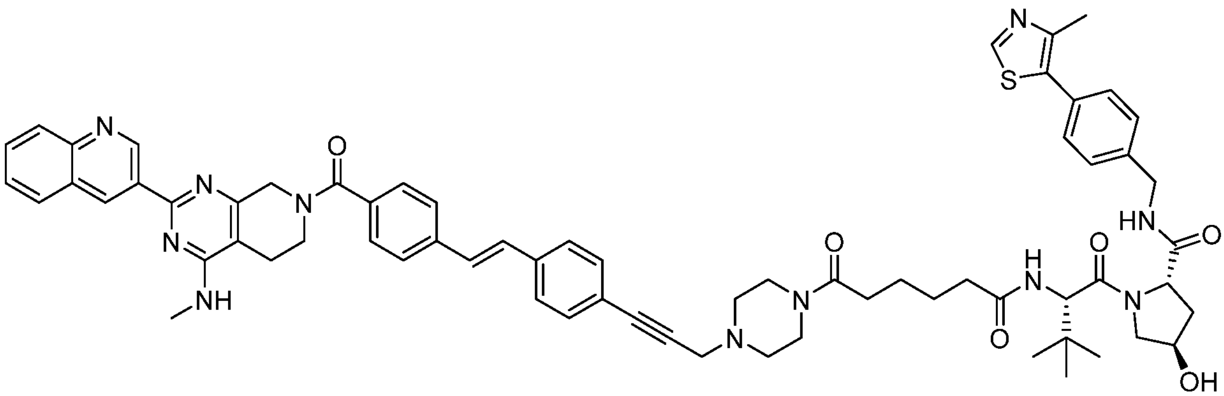

,

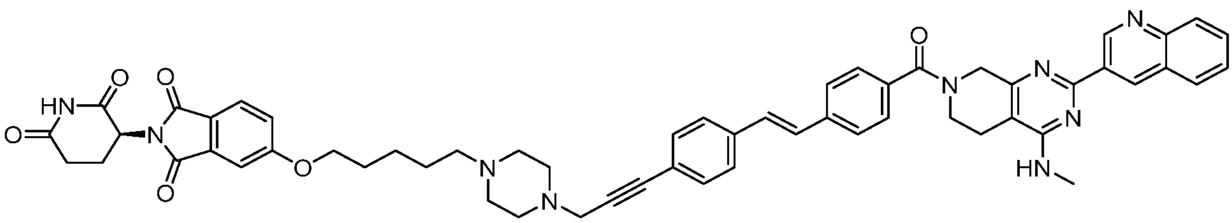

,

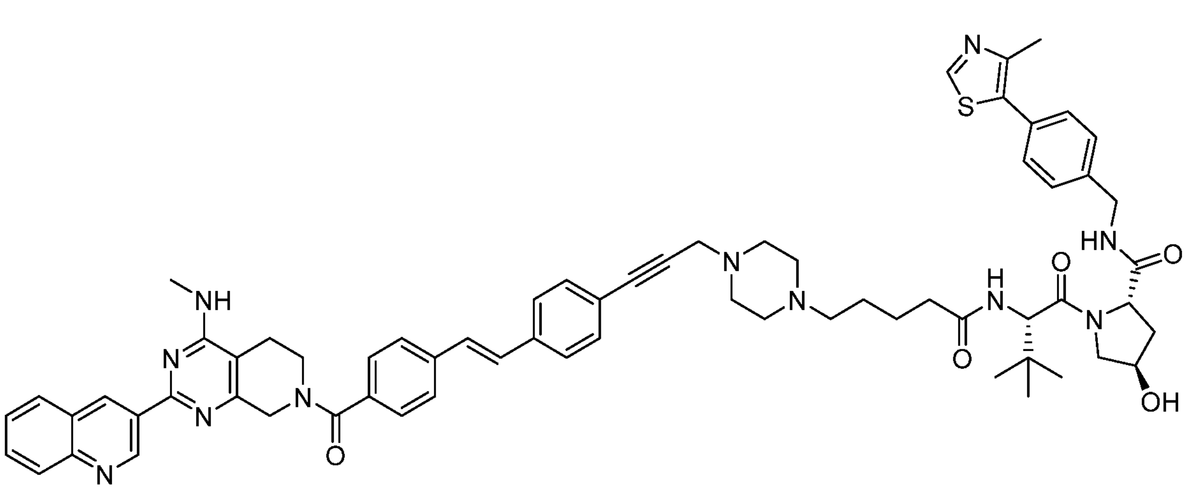

,

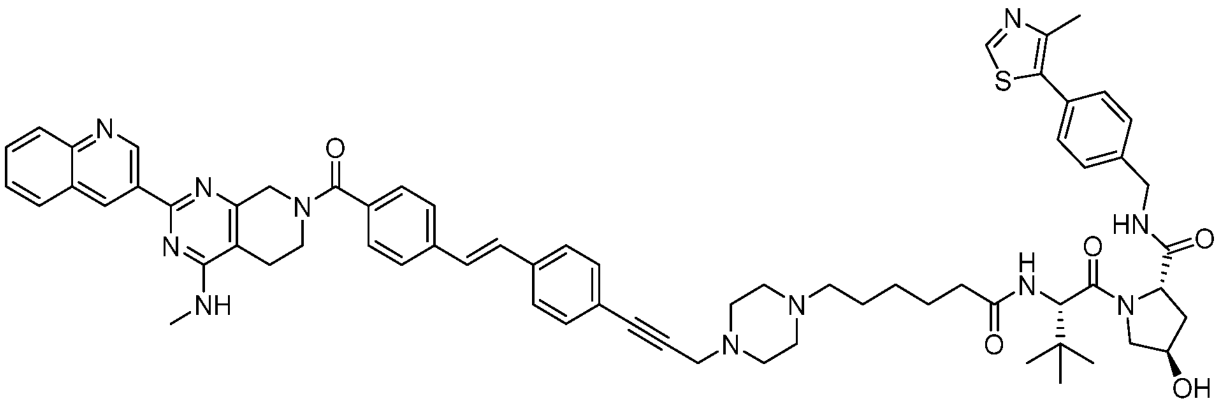

,

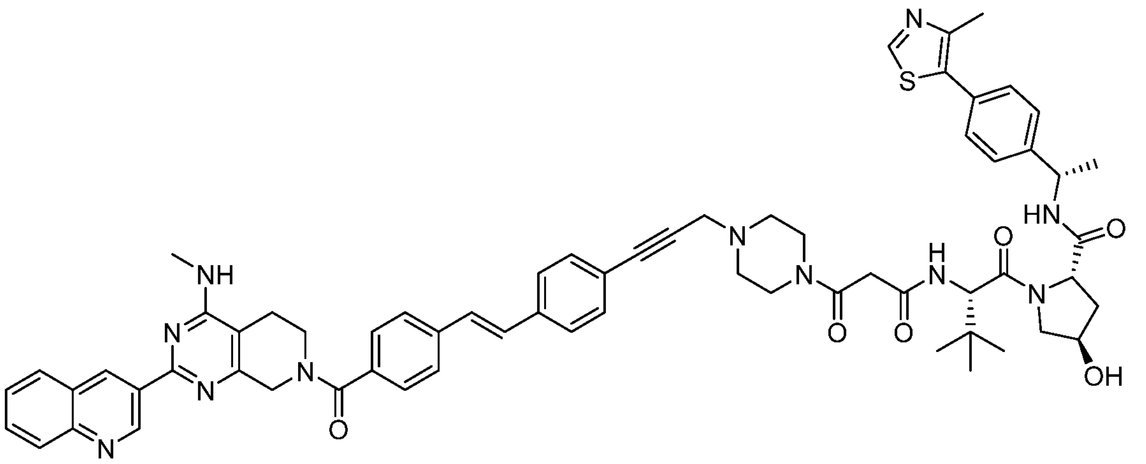

,

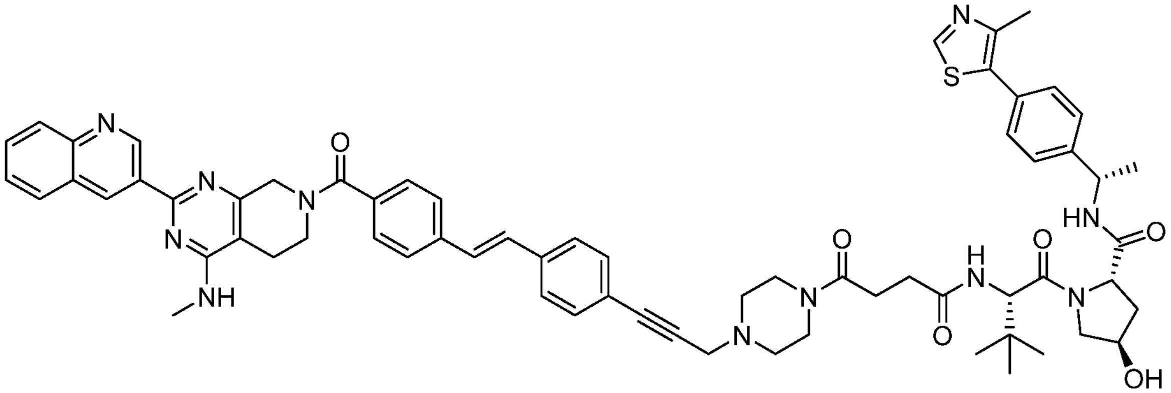

,

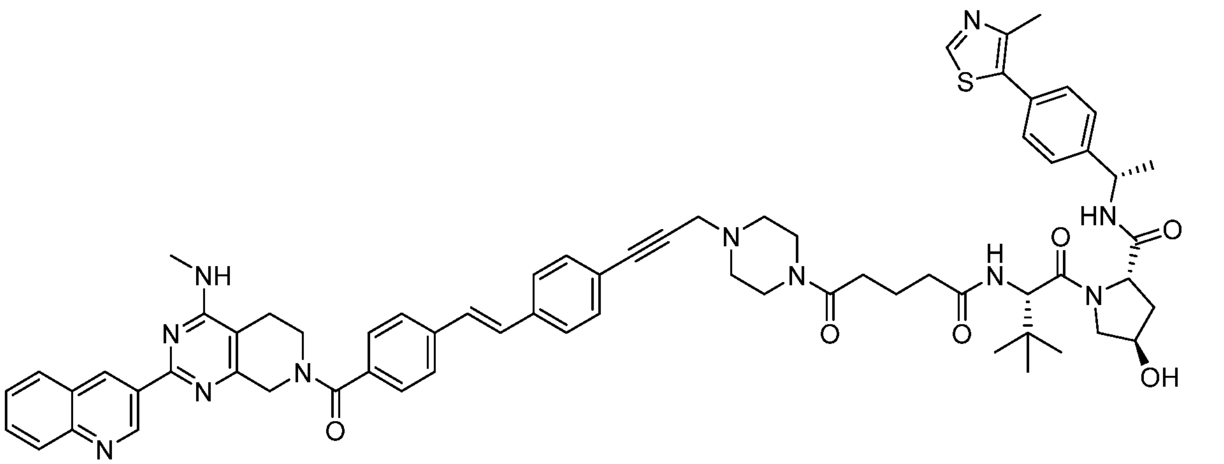

,

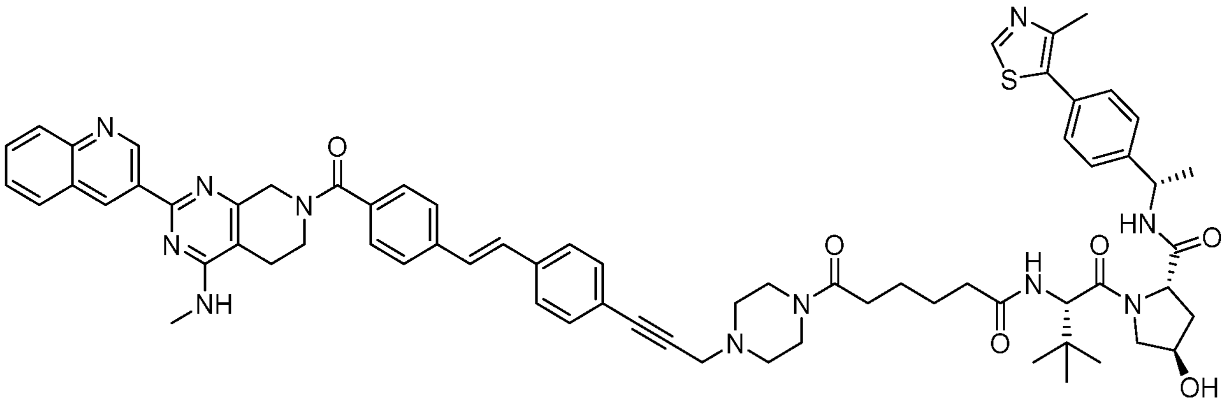

,

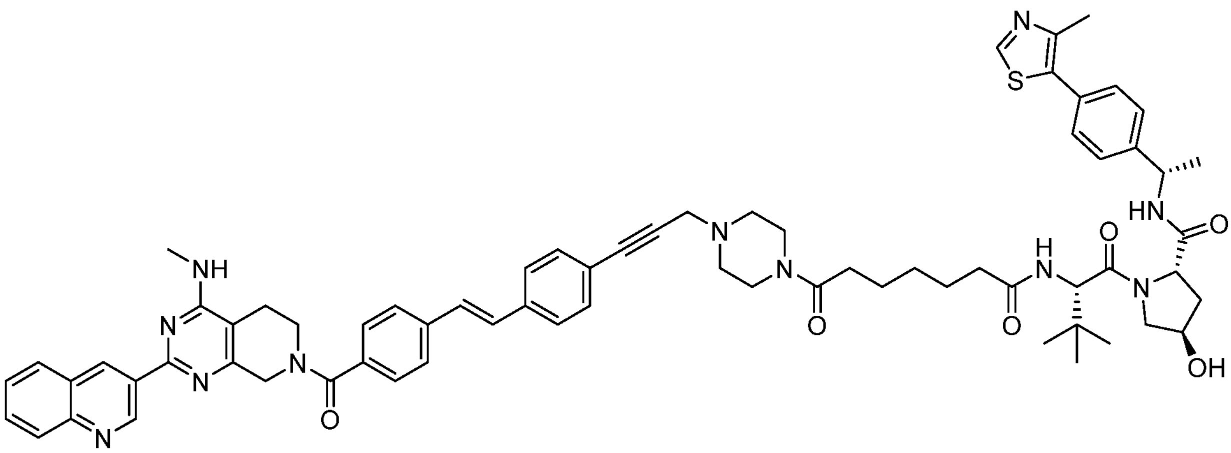

,

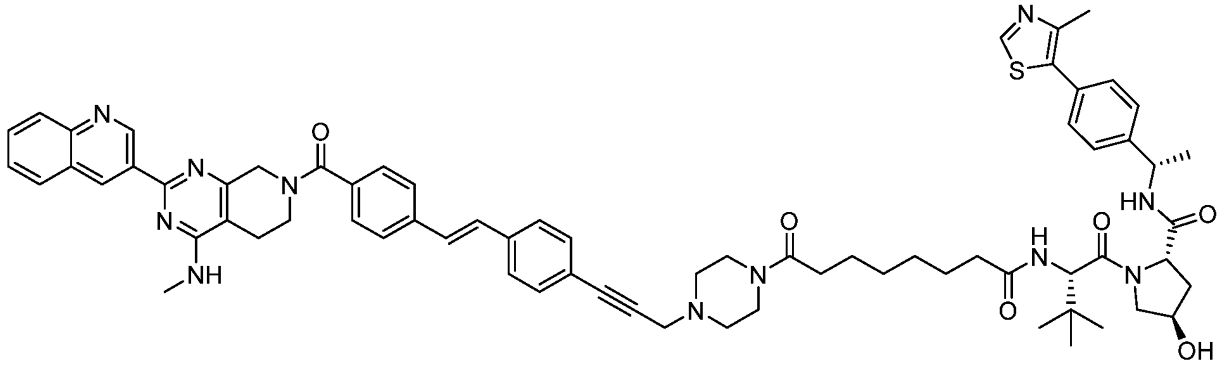,

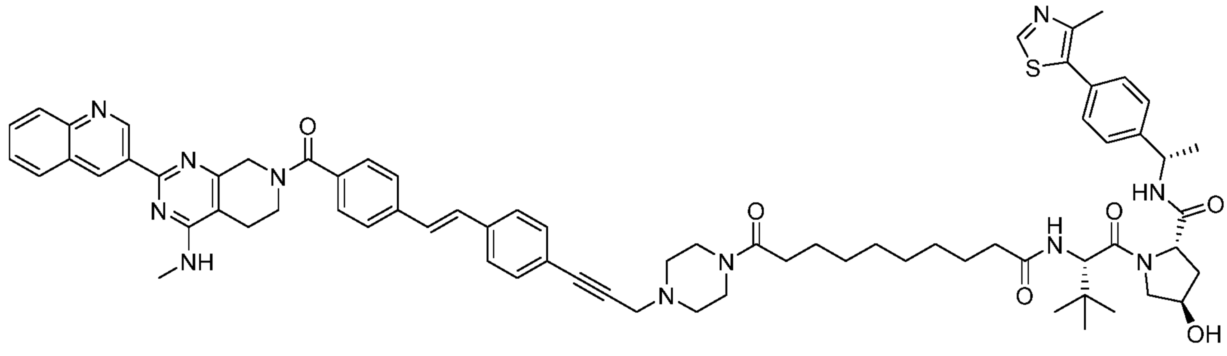,

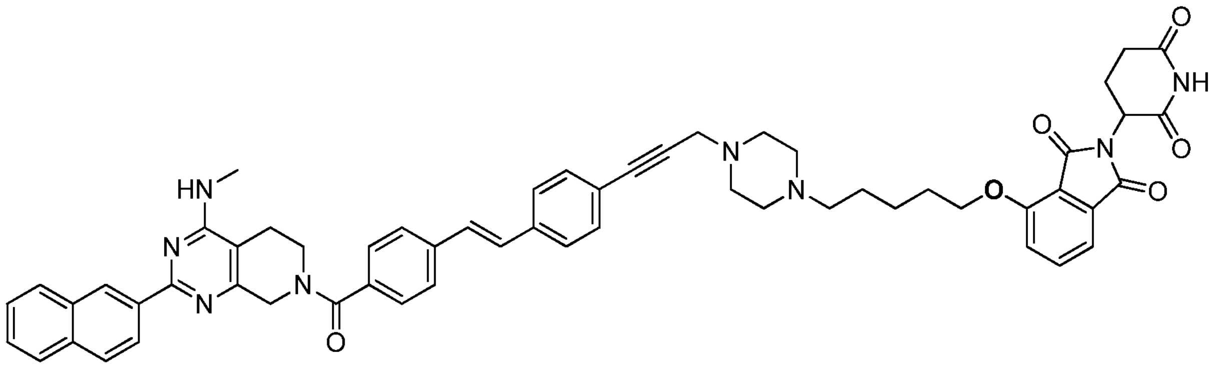,

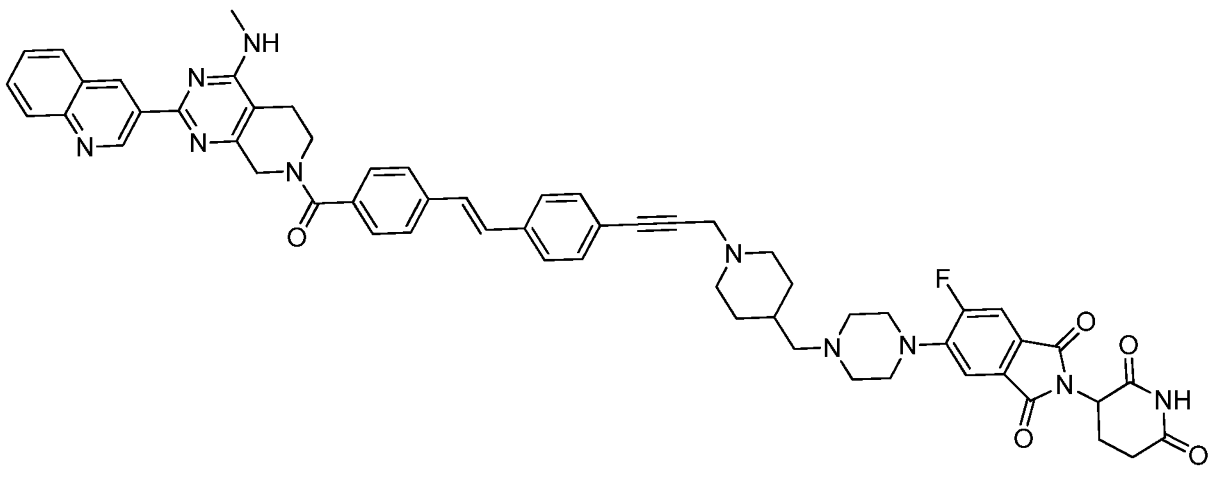,

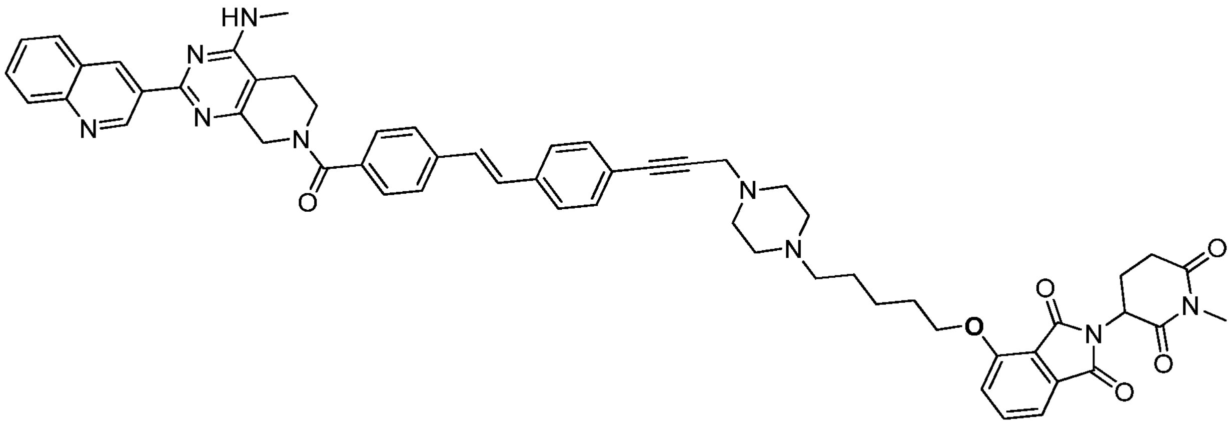

,

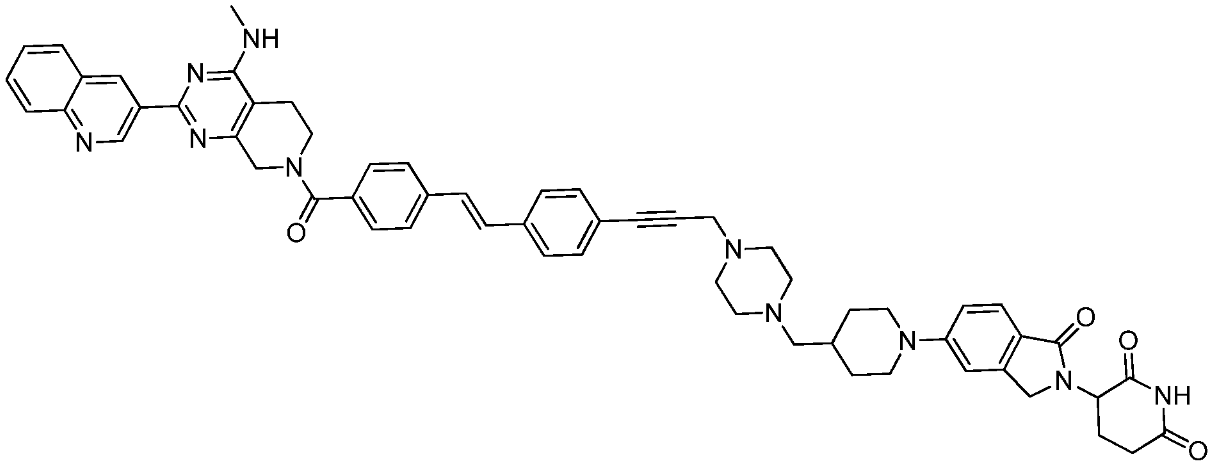

,

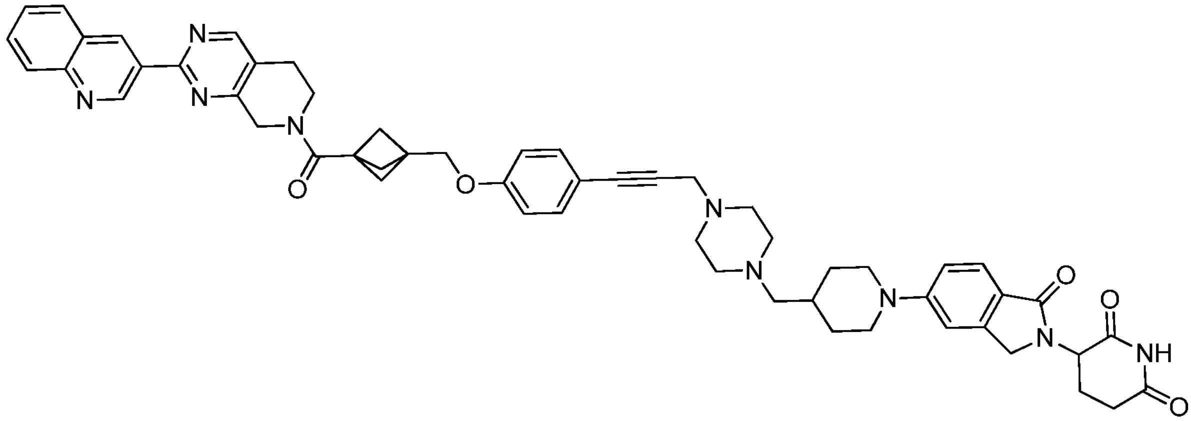

,

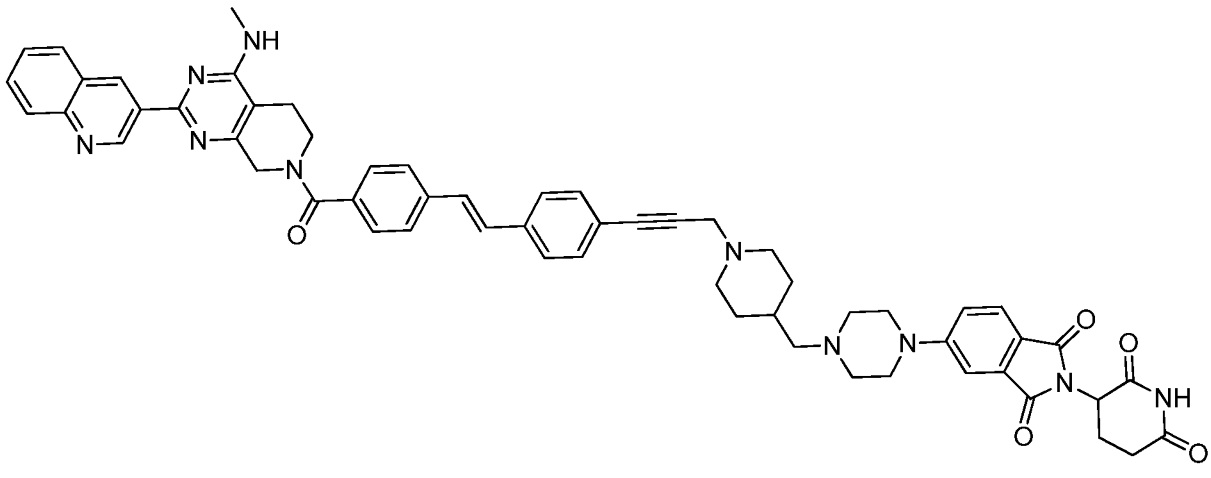,

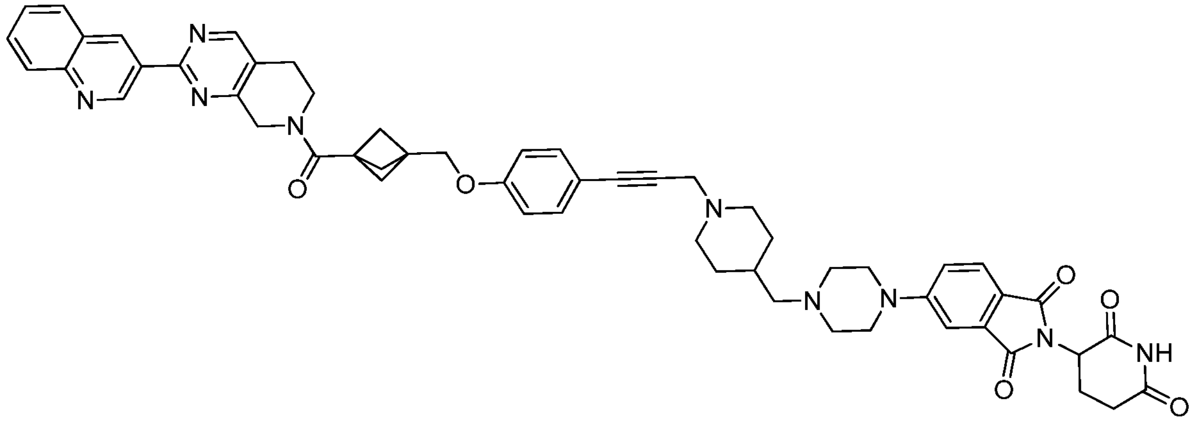,

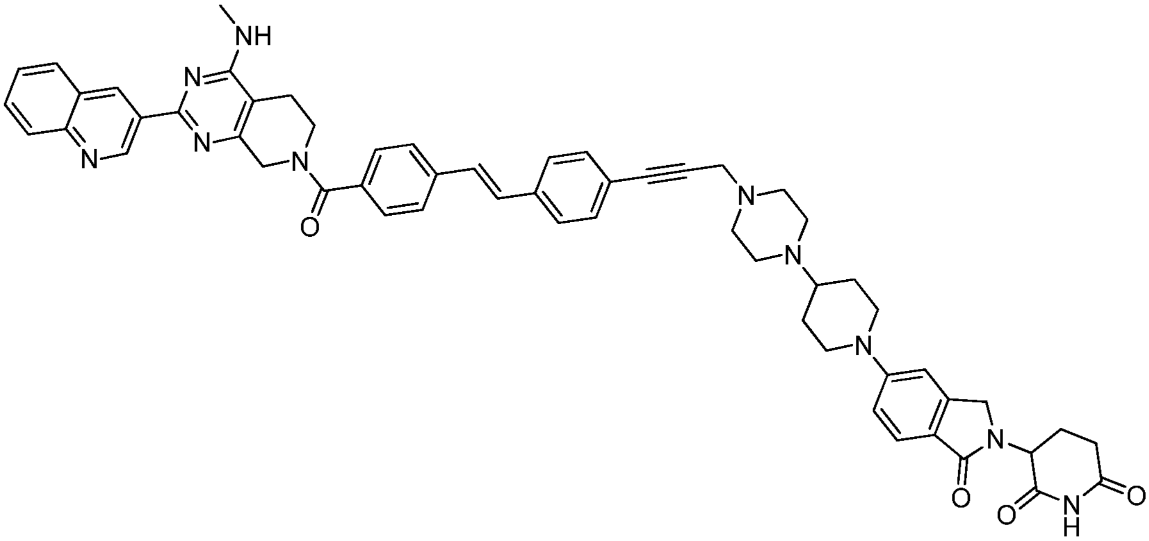,

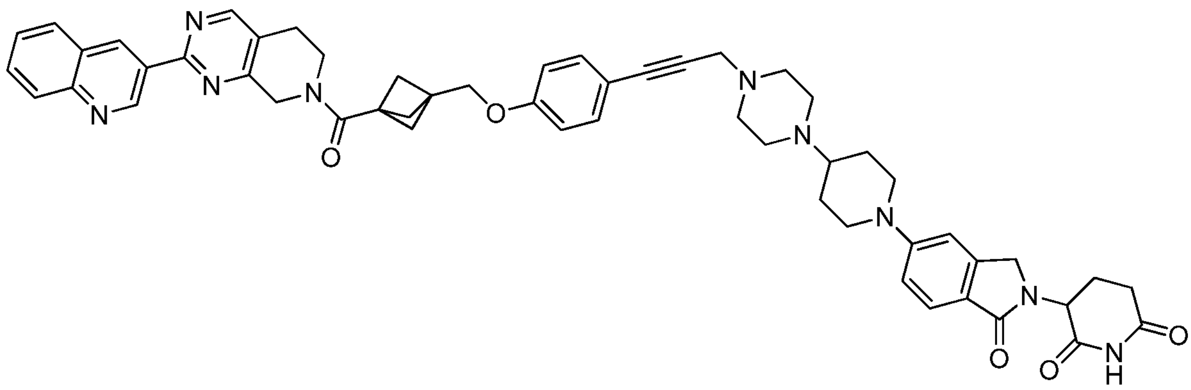

,

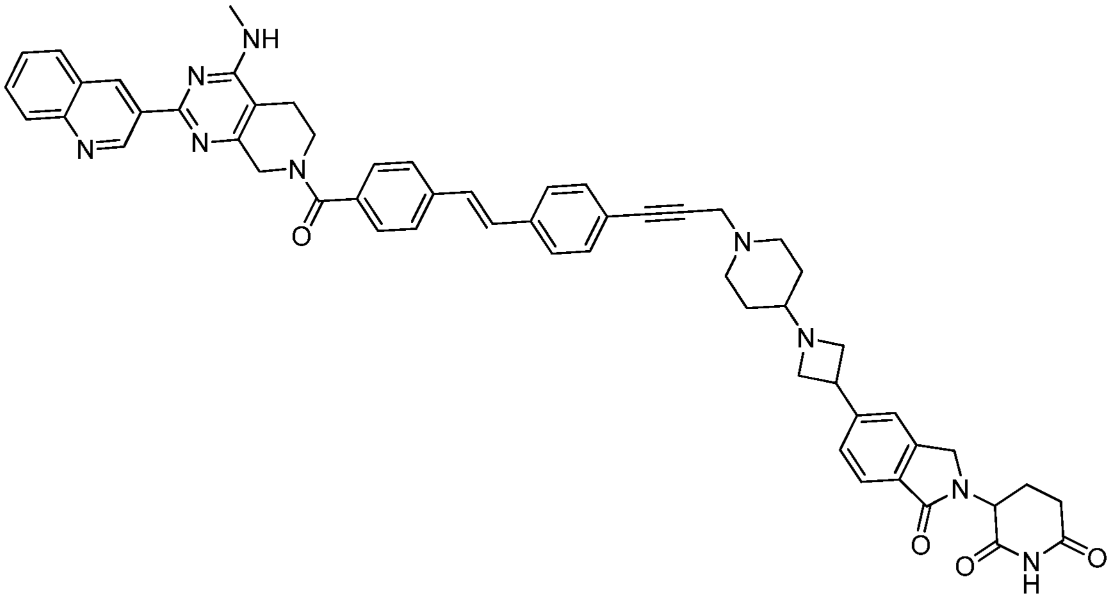

,

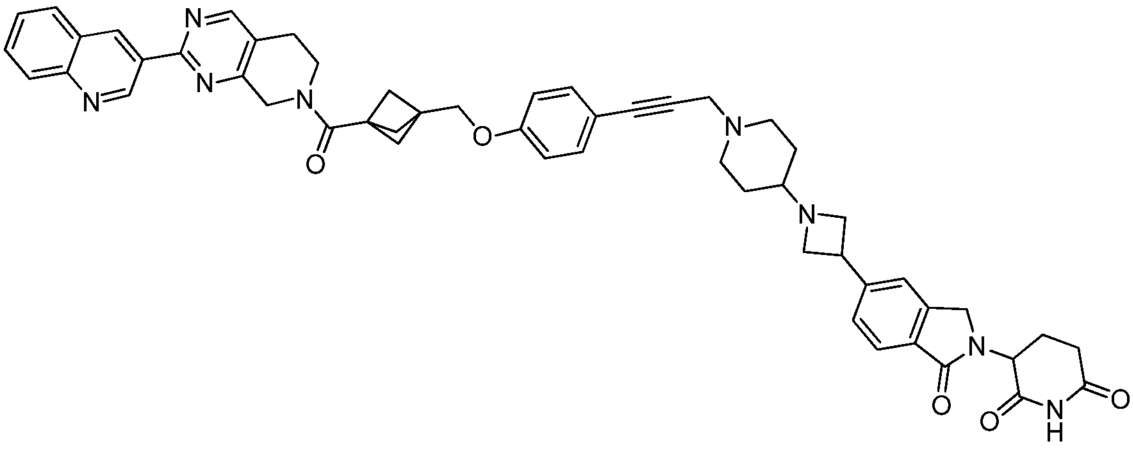

,

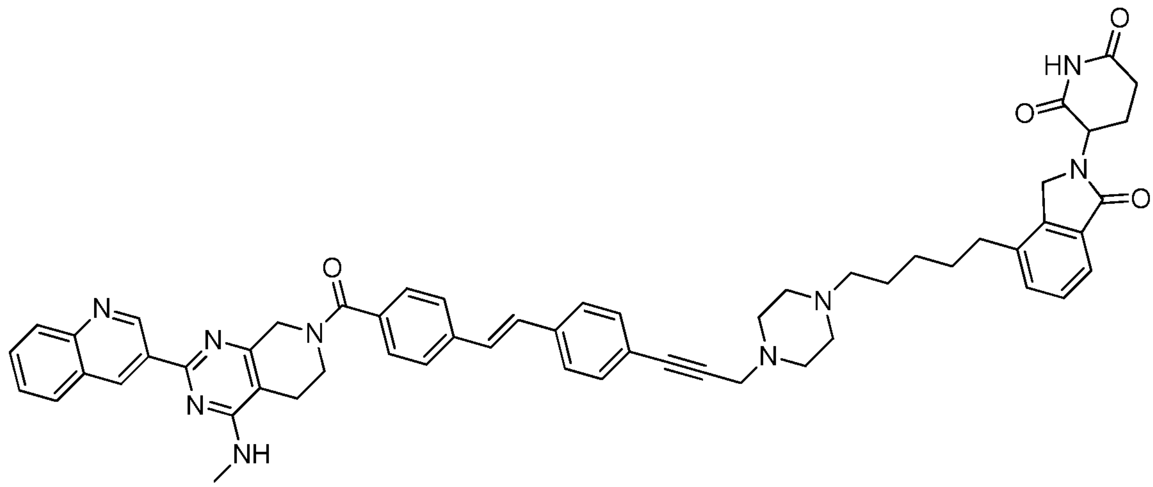

,

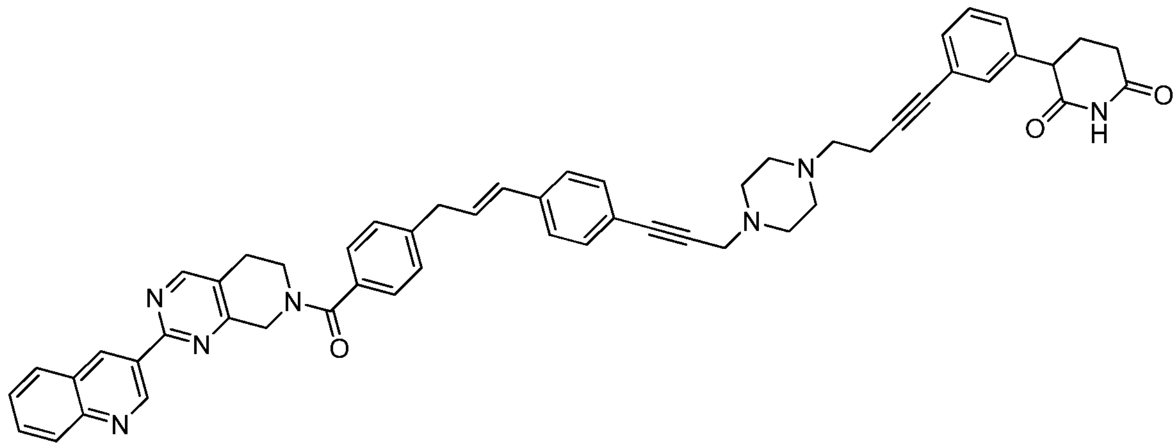

,

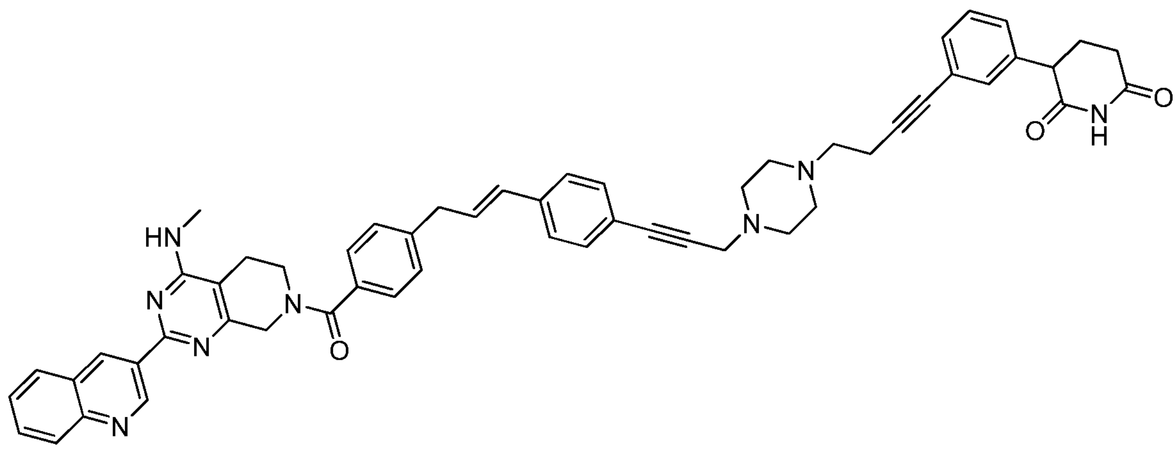

,

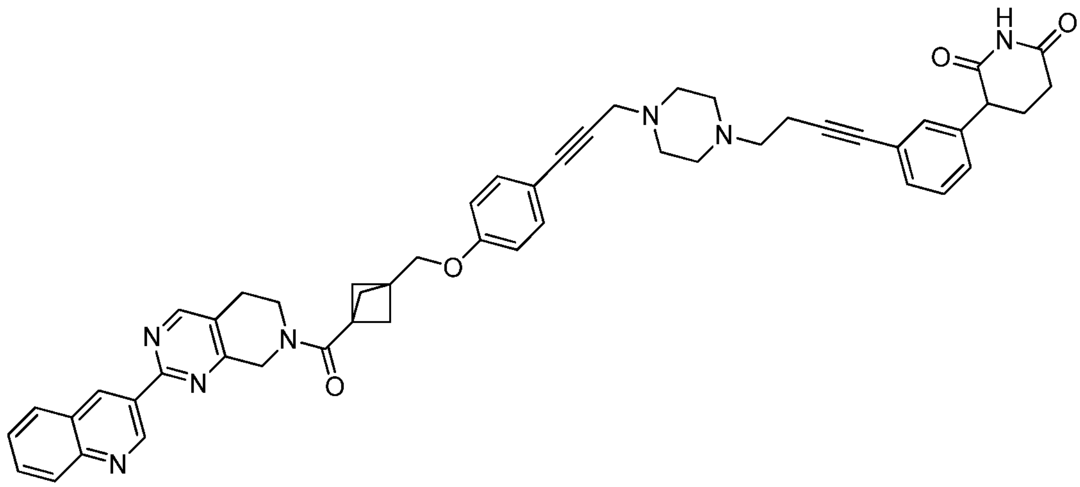

,

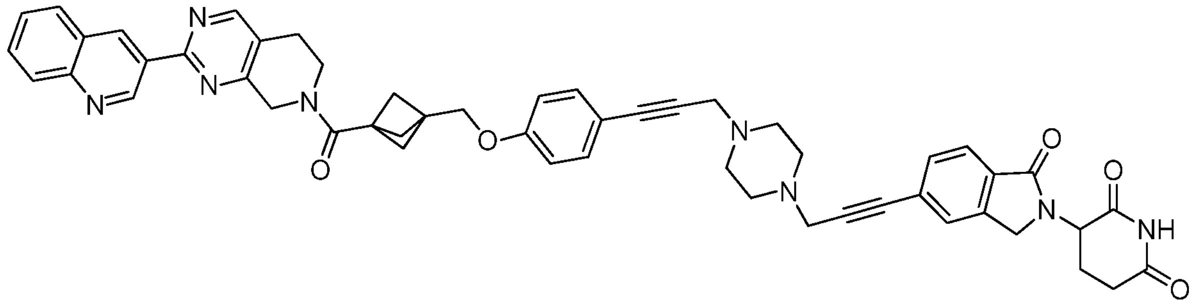

,

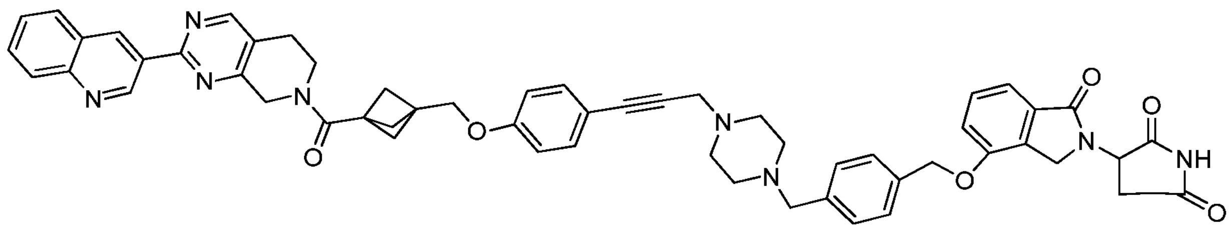

,

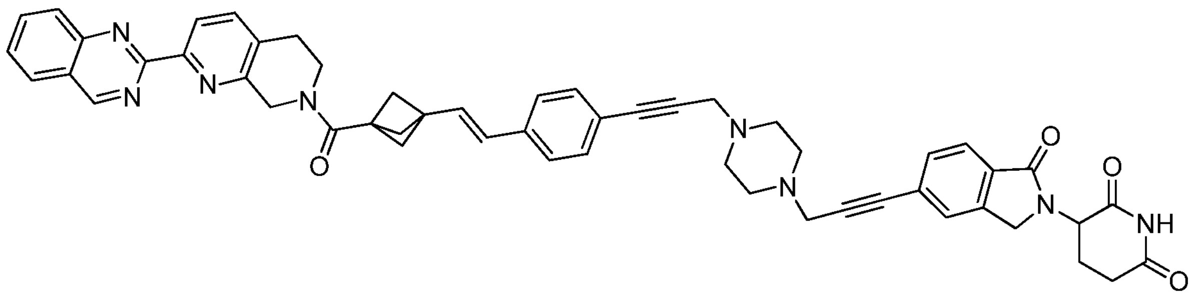

,

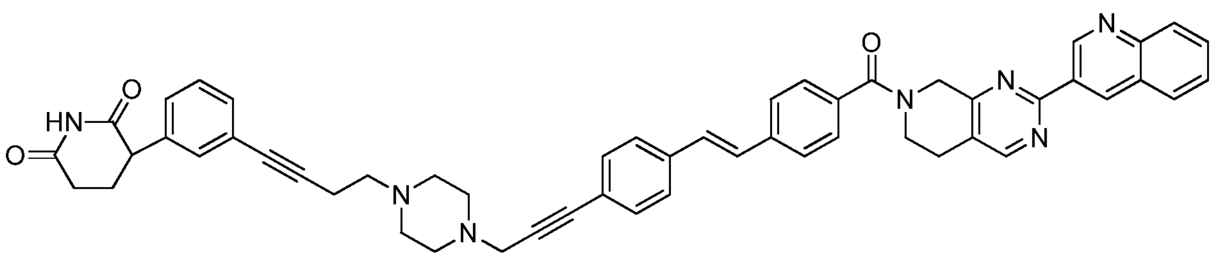

,

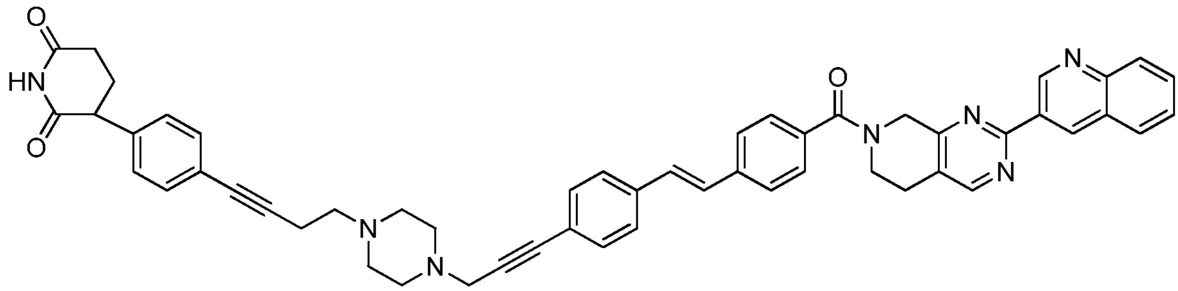

,

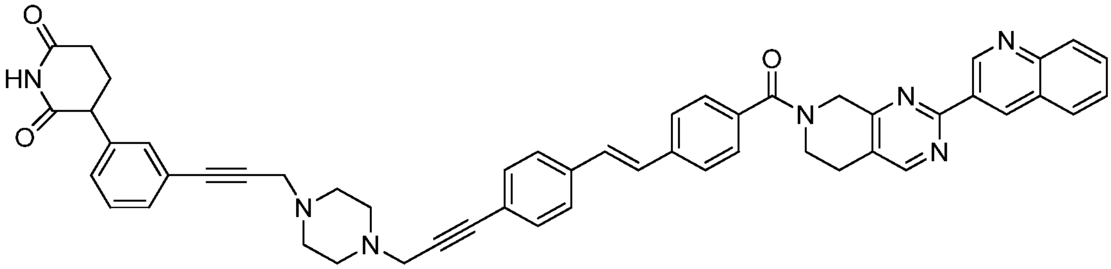

,

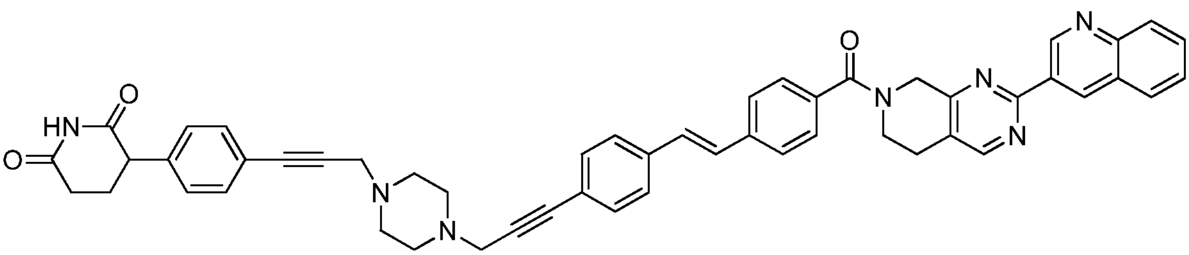

,

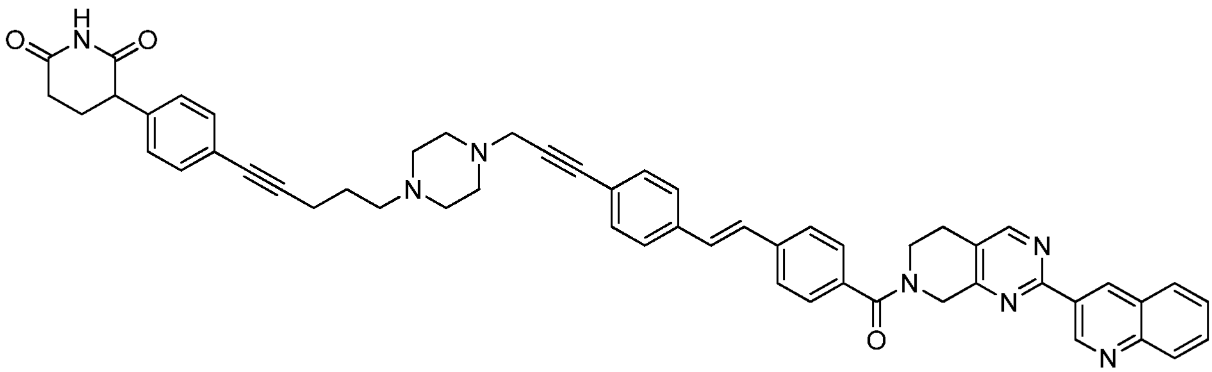

,

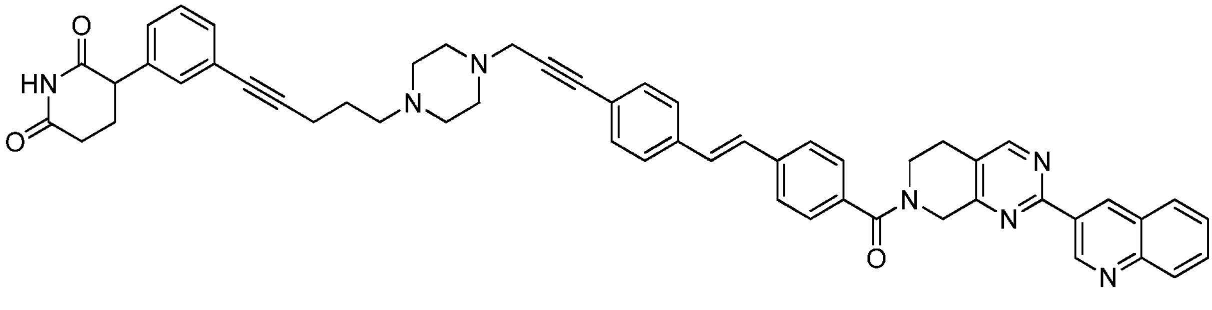

,

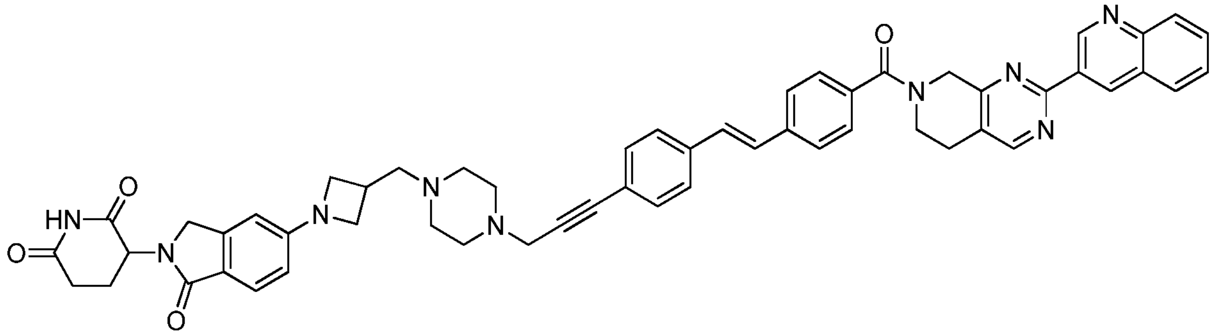

,

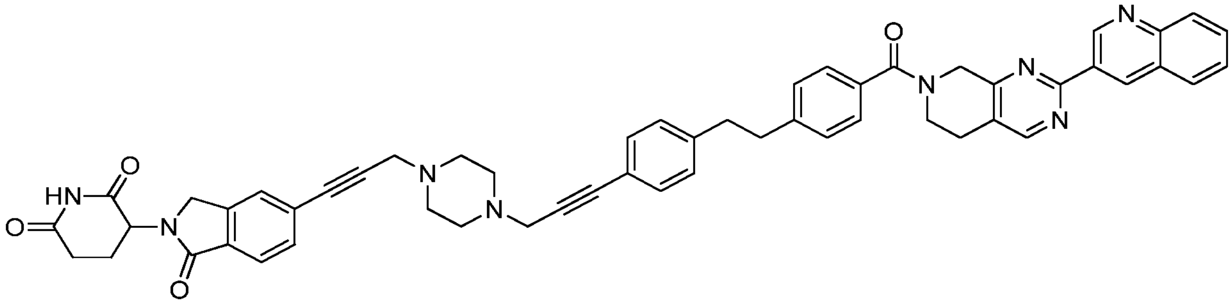

,

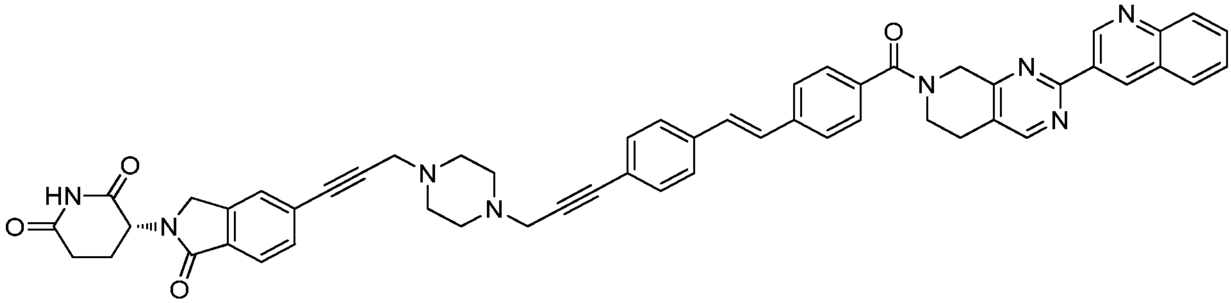

,

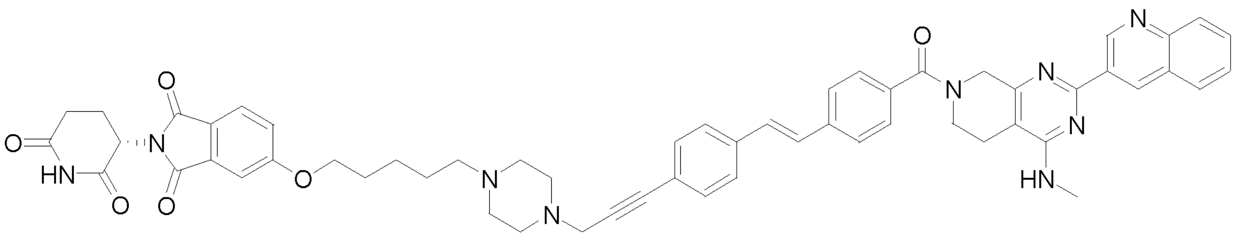

,

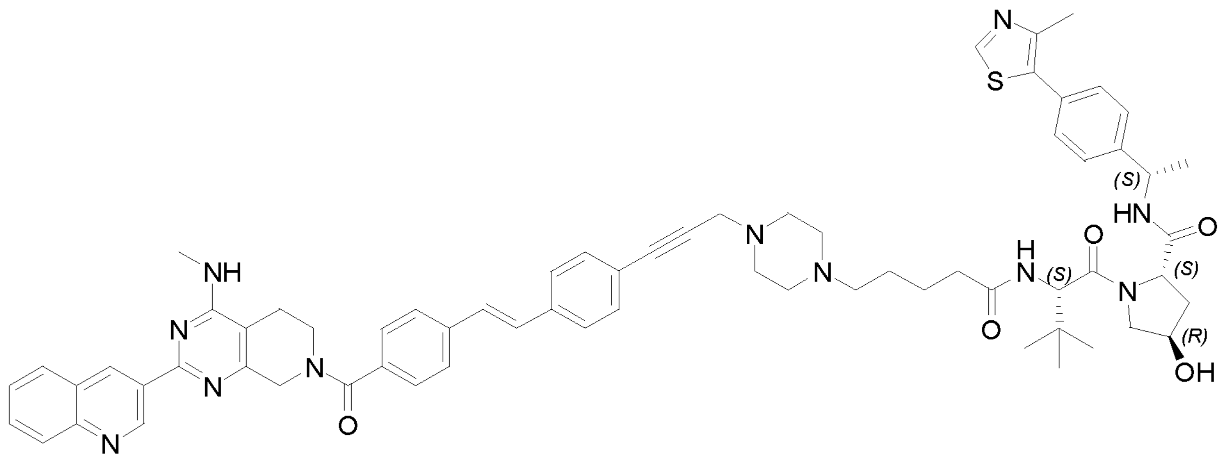

,

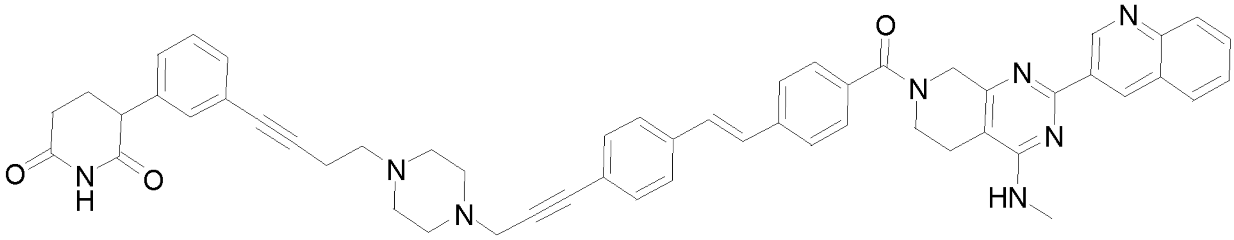

,

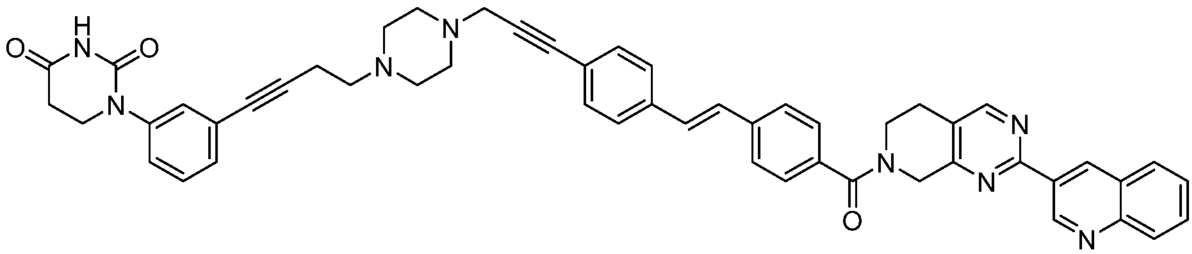

,

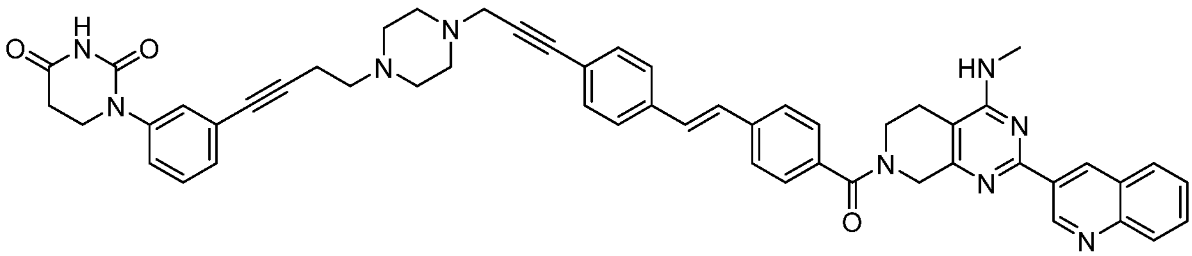

.

**21.** The compound according to any one of claims 1 to 20, wherein the compound is optionally and independently substituted by one or more (for example, 2, 3, 4, 5, 6, 7, 8, 9 or 10) $R^{YL'}$;

preferably, $R^{YL'}$ is independently selected from the group consisting of halogen, cyano, nitro, $C_{1\sim6}$ alkyl, halogen-substituted $C_{1\sim6}$ alkyl, -OR', and -N(R')$_2$; each R' is independently selected from the group consisting of hydrogen, halogen, -$C_{1\sim6}$ alkyl, halogen-substituted -$C_{1\sim6}$ alkyl, -$C_{0\sim2}$ alkylene-($C_{3\sim10}$ carbocyclic group), and -$C_{0\sim2}$ alkylene-(3- to 10-membered heterocycloalkyl);

preferably, $R^{YL'}$ is independently selected from the group consisting of halogen, cyano, nitro, $C_{1\sim6}$ alkyl, halogen-substituted $C_{1\sim6}$ alkyl, -OR', and -N(R')$_2$; each R' is independently selected from the group consisting of hydrogen, halogen, -$C_{1\sim6}$ alkyl, halogen-substituted -$C_{1\sim6}$ alkyl, -$C_{0\sim2}$ alkylene-($C_{3\sim8}$ carbocyclic group), and -$C_{0\sim2}$ alkylene-(3- to 8-membered heterocycloalkyl);

preferably, $R^{YL'}$ is independently selected from the group consisting of halogen, cyano, nitro, $C_{1\sim3}$ alkyl, halogen-substituted $C_{1\sim3}$ alkyl, -OR' and -N(R')$_2$; each R' is independently selected from the group consisting of hydrogen,

halogen, $-C_{1\sim3}$ alkyl, halogen-substituted $-C_{1\sim3}$ alkyl, $-C_{0\sim2}$ alkylene-($C_{3\sim6}$ carbocyclic group) and $-C_{0\sim2}$ alkylene-(3- to 6-membered heterocycloalkyl).

**22.** A pharmaceutical composition, comprising a preparation made from the compound, or deuterated compound thereof, or stereoisomer thereof, or tautomer thereof, or polymorph thereof, or solvate thereof, or N-oxide thereof, or isotopically labeled compound thereof, or metabolite thereof, or prodrug thereof, or pharmaceutically acceptable salt thereof according to any one of claims 1 to 21.

**23.** The pharmaceutical composition according to claim 22, further comprising a pharmaceutically acceptable carrier, excipient, vehicle.

**24.** Use of the compound, or deuterated compound thereof, or stereoisomer thereof, or tautomer thereof, or polymorph thereof, or solvate thereof, or N-oxide thereof, or isotopically labeled compound thereof, or metabolite thereof, or prodrug thereof, or pharmaceutically acceptable salt thereof according to any one of claims 1 to 21, or the pharmaceutical composition according to any one of claims 22 to 23 in the preparation of a medicament for preventing and/or treating a disease associated with BCL-XL activity.

**25.** The use according to claim 24, wherein the disease associated with BCL-XL activity is selected from the group consisting of autoimmune disease, bladder cancer, brain cancer, breast cancer, bone marrow cancer, cervical cancer, colorectal cancer, esophageal cancer, hepatocellular carcinoma, follicular lymphoma, lymphoid malignancy of T-cell or B-cell origin, melanoma, myeloma, oral cancer, ovarian cancer, non-small cell lung cancer, prostate cancer, leukemia, small cell lung cancer, or spleen cancer; the leukemia is preferably chronic lymphocytic leukemia, lymphoblastic leukemia or granulocytic leukemia.

**26.** Use of the compound, or deuterated compound thereof, or stereoisomer thereof, or tautomer thereof, or polymorph thereof, or solvate thereof, or N-oxide thereof, or isotopically labeled compound thereof, or metabolite thereof, or prodrug thereof, or pharmaceutically acceptable salt thereof according to any one of claims 1 to 21, or the pharmaceutical composition according to any one of claims 22 to 23 in the preparation of a drug for preventing and/or treating a cancer.

**27.** A method for preventing and/or treating a diseases associated with BCL-XL activity, comprising administering an effective amount of the compound, or deuterated compound thereof, or stereoisomer thereof, or tautomer thereof, or polymorph thereof, or solvate thereof, or N-oxide thereof, or isotopically labeled compound thereof, or metabolite thereof, or prodrug thereof, or pharmaceutically acceptable salt thereof according to any one of claims 1 to 21, or the pharmaceutical composition to a subject in need thereof.

**28.** The method according to claim 24, wherein the disease associated with BCL-XL activity is selected from the group consisting of autoimmune disease, bladder cancer, brain cancer, breast cancer, bone marrow cancer, cervical cancer, colorectal cancer, esophageal cancer, hepatocellular carcinoma, follicular lymphoma, lymphoid malignancy of T-cell or B-cell origin, melanoma, myeloma, oral cancer, ovarian cancer, non-small cell lung cancer, prostate cancer, small cell lung cancer, or spleen cancer; the leukemia is preferably chronic lymphocytic leukemia, lymphoblastic leukemia or granulocytic leukemia.

**INTERNATIONAL SEARCH REPORT**

International application No.
**PCT/CN2022/131968**

**A. CLASSIFICATION OF SUBJECT MATTER**

C07D 471/04(2006.01)i; A61K 31/45(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS; CNTXT; DWPI; SIPOABS; STNext; ISI Web of Science; CNKI: 刘川; 成都先导; 四川科伦博泰; 蛋白降解; 泛素连接酶; protein degradation; BCL-XL; ubiquitin; E3; PROTAC

**C. DOCUMENTS CONSIDERED TO BE RELEVANT**

| Category* | Citation of document, with indication, where appropriate, of the relevant passages | Relevant to claim No. |
|---|---|---|
| X | CN 112105360 A (BIOVENTURES, LLC.) 18 December 2020 (2020-12-18) claims 1-25 | 1, 14-15, 18-19, 21-28 |
| X | CN 109152933 A (BIOVENTURES, LLC.) 04 January 2019 (2019-01-04) claims 1-15 | 1, 14-15, 18-19, 21-28 |
| X | CN 112707900 A (SHANGHAITECH UNIVERSITY et al.) 27 April 2021 (2021-04-27) claims 1-62 | 1, 14-15, 18-19, 21-28 |
| X | CHUNG, Chun-wa et al. "Structural Insights into PROTAC-Mediated Degradation of Bcl-xL;" *ACS Chemical Biology;*, Vol. 15, 22 July 2020 (2020-07-22), pages 2316-2323; abstract, and figures 1-3 | 1, 14-15, 18-19, 21-28 |
| A | CN 113387932 A (CHENGDU HITGEN DEVELOPMENT CO., LTD.) 14 September 2021 (2021-09-14) entire document | 1-28 |
| A | 易享炎 等 (YI, Xiangyan et al.). "Bcl－XL蛋白拮抗剂分子设计的研究进展; (Advances in Molecular Design of Bcl-XL Protein Antagonists;)" *生物加工过程; (Chinese Journal of Bioprocess Engineering;),* Vol. 16, No. 6, 30 November 2018 (2018-11-30), pages 70-79; entire document | 1-28 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 January 2023** | **28 January 2023** |

Name and mailing address of the ISA/CN

**China National Intellectual Property Administration (ISA/CN)**
**No. 6, Xitucheng Road, Jimenqiao, Haidian District, Beijing 100088, China**

Facsimile No. **(86-10)62019451**

Authorized officer

Telephone No.

Form PCT/ISA/210 (second sheet) (January 2015)

**INTERNATIONAL SEARCH REPORT**

International application No.
**PCT/CN2022/131968**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **27-28**
because they relate to subject matter not required to be searched by this Authority, namely:

[1] Claims 27-28 relate to a method for preventing and/or treating a disease associated with the activity of BCL-XL, and do not comply with PCT Rule 39.1(iv), and a search was conducted on the basis of the technical solutions of the pharmaceutical uses of the corresponding claims.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/131968**

| Patent document cited in search report | | | Publication date (day/month/year) | Patent family member(s) | | | Publication date (day/month/year) |
|---|---|---|---|---|---|---|---|
| CN | 112105360 | A | 18 December 2020 | EP | 3743069 | A1 | 02 December 2020 |
| | | | | JP | 2021511342 | A | 06 May 2021 |
| | | | | WO | 2019144117 | A1 | 25 July 2019 |
| | | | | CA | 3088253 | A1 | 25 July 2019 |
| CN | 109152933 | A | 04 January 2019 | WO | 2017184995 | A1 | 26 October 2017 |
| | | | | WO | 2020081880 | A1 | 23 April 2020 |
| | | | | KR | 20180134908 | A | 19 December 2018 |
| | | | | JP | 2019514877 | A | 06 June 2019 |
| | | | | EP | 3445452 | A1 | 27 February 2019 |
| | | | | US | 2019135801 | A1 | 09 May 2019 |
| | | | | AU | 2017254687 | A1 | 18 October 2018 |
| | | | | US | 2020331905 | A1 | 22 October 2020 |
| | | | | CA | 3018991 | A1 | 26 October 2017 |
| CN | 112707900 | A | 27 April 2021 | | None | | |
| CN | 113387932 | A | 14 September 2021 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Energy Chemical, Chengdu Kelong Chemical. Accela ChemBio Co., Ltd